# EUROPEAN PATENT SPECIFICATION

(11) **EP 1 851 210 B1**
(45) Date of publication and mention of the grant of the patent: **19.10.2011**
(21) Application number: 05824122.5
(22) Date of filing: 29.12.2005
(51) Int. Cl.: C07D 295/145, C07D 295/096, C07D 207/50, C07D 207/335, C07D 213/74, C07D 231/12, C07D 237/14, A61K 31/495, A61K 31/496, A61P 25/24, C07D 271/12

(54) **NITRO-SUBSTITUTED PHENYL-PIPERAZINE COMPOUNDS, THEIR PREPARATION AND USE IN MEDICAMENTS**
NITROSUBSTITUIERTE PHENYLPIPERAZINVERBINDUNGEN, DEREN HERSTELLUNG UND DEREN VERWENDUNG IN MEDIKAMENTEN
COMPOSÉS DU PIPÉRAZINE NITRO SUBSTITUÉS, PROCÉDÉ POUR LEUR PRODUCTION ET LEUR APPLICATION COMME MÉDICAMENTS

(30) Priority: 30.12.2004 EP 04380288
(43) Date of publication of application: 07.11.2007
(73) Proprietor: LABORATORIOS DEL DR. ESTEVE, S.A., 08041 Barcelona (ES)
(72) Inventor: ASCHENBRENNER, Andrea, 81245 Munich (DE); KRAUS, Jürgen, 82319 Starnberg (DE); TASLER, Stefan, 82229 Seefeld-Hechendorf (DE); WUZIK, Andreas, 86836 Untermeitingen (DE); QUINTANA-RUIZ, Jordi-Ramon, E-08391 Tiana (ES)
(74) Representative: ABG Patentes, S.L.
(86) International application number: PCT/EP2005/014191
(87) International publication number: WO 2006/069808

(56) References cited:
- WO-A-95/14004
- WO-A-02/092585
- WO-A-02/100822
- WO-A-2004/080986
- DATABASE REGISTRY [Online] CHEMICAL ABSTRACTS SERVICE, COLUMBUS, OHIO, US; XP002334268 retrieved from STN
- DATABASE CAPLUS [Online] CHEMICAL ABSTRACTS SERVICE, COLUMBUS, OHIO, US; 10 May 1999 (1999-05-10), XP002334269 retrieved from STN Database accession no. 1999:284028 & J. B. SMAILL ET AL.: "DNA minor groove targeted alkylating agents based on bisbenzimidazole carriers: synthesis, cytotoxicity and sequence-specifity of DNA-alkylation" ANTI-CANCER DRUG DESIGN, vol. 13, no. 8, 1998, pages 857-880,
- B. YE ET AL.: "Synthesis and biological evaluation of piperazine-based derivatives as inhibitors of plasminogen acitvator inhibitor-1 (PAI-1)" BIOORG. MED. CHEM. LETT., vol. 14, 2004, pages 761-765, XP002334266
- J. S. YADAV ET AL.: "[Bmim]PF6 and BF4 ionic liquids as novel and recyclable reaction media for aromatic amination" TETRAHEDRON LETT., vol. 44, 2003, pages 2217-2220, XP002334267

## Description

The present invention relates to nitro-substituted phenyl-piperazine compounds of general formula 1, 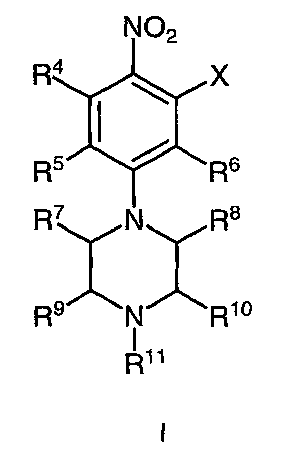 a process for their preparation, medicaments comprising said nitro-substituted phenyl-piperazine compounds as well as the use of said nitro-substituted phenyl-piperazine compounds for the preparation of medicaments, which are particularly suitable for the prophylaxis and/or treatment of disorders or diseases that are at least partially mediated via 5-HT₆ receptors.

The superfamily of serotonin receptors (5-HT) includes 7 classes (5-HT₁-5-HT₇) encompassing 14 human subclasses [D. Hoyer, et al., Neuropharmacology, 1997, 36, 419]. The 5-HT₆ receptor is the latest serotonin receptor identified by molecular cloning both in rats [F.J. Monsma et al., Mol. Pharmacol., 1993, 43, 320; M. Ruat et al., Biochem. Biophys. Res. Commun., 1993, 193, 268] and in humans [R. Kohen, et al., J. Neurochem., 1996, 66, 47].

Compounds with 5-HT₆ receptor affinity are useful for the treatment of various disorders of the Central Nervous System and of the gastrointestinal tract, such as irritable intestine syndrome. Compounds with 5-HT₆ receptor affinity are also useful in the treatment of anxiety, depression and cognitive memory disorders [M. Yoshioka et al., Ann. NY Acad. Sci., 1998, 861, 244; A. Bourson et al., Br. J. Pharmacol. , 1998, 125,1562; D.C. Rogers et al., Br. J. Pharmacol. Suppl., 1999,127, 22P; A. Bourson et al., J. Pharmacol. Exp. Ther., 1995, 274, 173; A.J. Sleight, et al., Behav. Brain Res. , 1996, 73, 245; T.A. Branchek et al., Annu. Rev. Pharmacol. Toxicol., 2000, 40, 319; C. Routledge et al., Br. J. Pharmacol. , 2000, 130, 1606]. It has been shown that typical and atypical antipsychotic drugs for treating schizophrenia have a high affinity for 5-HT₆ receptors [B.L. Roth et al., J. Pharmacol. Exp. Ther., 1994, 268, 1403; C.E. Glatt et al., Mol. Med., 1995, 1, 398; F.J. Mosma,et al., Mol. Pharmacol. , 1993, 43, 320; T. Shinkai et al., Am. J. Med. Genet., 1999, 88,120]. Compounds with 5-HT₆ receptor affinity are useful for treating infant hyperkinesia (ADHD, attention deficit / hyperactivity disorder) [W.D. Hirst et al., Br. J. Pharmacol. , 2000, 130, 1597; C. Gérard et al., Brain Research , 1997, 746, 207; M.R. Pranzatelli, Drugs of Today, 1997, 33, 379J.

Recently, it has been shown that the 5-HT₆ receptor also plays a role in food ingestion [Neuropharmacology, 41, 2001, 210-219].

Food ingestion disorders, particularly obesity, are a serious, fast growing threat to the health of humans of all age groups, since they increase the risk of developing other serious, even life-threatening diseases such as diabetes or coronary diseases as well.

Therefore an object of the present invention was to provide compounds that are particularly suitable as active ingredients in medicaments, especially in medicaments for the prophylaxis and/or treatment of disorders or diseases related to 5-HT₆ receptors such as food intake related disorders.

Surprisingly, it has been found that the nitro-substituted phenyl-piperazine compounds of general formula I, la and lb given below show good to excellent affinity for 5-HT₆-receptors. These compounds are therefore particularly suitable as pharmacologically active agents in a medicament for the prophylaxis and/or treatment of disorders or diseases related to 5-HT₆-receptors such as food intake related disorders like obesity.

Thus, in one aspect the present invention relates to nitro-substituted phenyl piperazine compounds of general formula I, 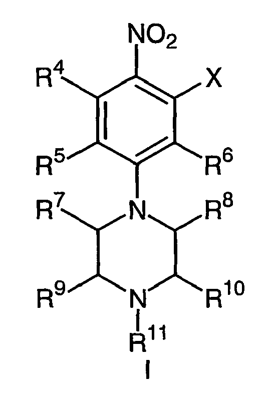 wherein
- X: represents a -NR¹R² moiety or a -OR3 moiety;
- R¹: represents a saturated or unsaturated 3- to 9-membered cycloaliphatic radical, which may be substituted with 1, 2 or 3 substituent(s) independently selected from the group consisting of oxo (=O), thioxo (=S), C₁₋₅-alkyl, -O-C₁₋₅-alkyl, -S- C₁₋₅-alkyl, -C(=O)-OH, -C(=O)-C₁₋₅-alkyl, -C(=O)-O-C₁₋₅-alkyl, -O-C(=O)-C₁-₅- alkyl, F, Cl, Br, I, -CN, -CF₃, -OCF₃, -SCF₃, -OH, -SH, -NH₂, -NH(C₁₋₅-alkyl), - N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may contain 1, 2 or 3 heteroatom(s) independently selected from the group consisting of nitrogen, oxygen and sulfur as ring member(s) and which is bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, - CN, -CF₃, -OCF₃, -SCF₃, -OH, -SH, -NH₂, -O-C₁₋₅-alkyl, -S-C₁₋₅-alkyl, -NH-(C₁₋₅-alkyl) and -N(C₁₋₅-alkyl)₂;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl which may be substituted with 1, 2, 3, 4 or 5 substituent(s) independently selected from the group consisting of C₁₋₅-alkyl, -O-C₁₋₅-alkyl, -O-C₁₋₅-alkyl, -C(=O)-OH, -C(=O)-C₁₋₅-alkyl, -C(=O)-O- C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂- phenyl, phenyl, phenoxy and benzyl;
an aryl or heteroaryl radical selected from the group consisting of phenyl, naphthyl, thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)-group and may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-C₁₋₅-alkyl, - C(=O)-O-C₁₋₅-alkyl, -O-C(=O)-C₁₋₅-alkyl, F, Br, I, -CN, -CF₃, -OCF₃, -SCF₃, - OH, -SH, -NH₂, -NH(C₁₋₅-alkyl), -N(C₁₋₅-alkl)₂, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁₋₅-alkyl, - S(=O)₂-phenyl, phenyl, phenoxy and benzyl;
an aryl or heteroaryl radical selected from the group consisting of naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₂-group and/or may be substituted with 1, 2, 3, 4 or 5 substituent(s) independently selected from the group consisting of C₁₋₅-alkyl, -O-C₁₋₅-alkyl, -S-C₁₋₅-alkyl), -C(=O)-OH, -C(=O)- C₁₋₅-alkyl, -C(=O)-O-C₁₋₅-alkyl, -O-C(=O)-C₁₋₅-alkyl, F, Cl, Br, I, -CN, -CF₃, - OCF₃, -SCF₃, -OH, -SH, -NH₂, -NH(C₁₋₅-alkyl), -N(C₁₋₅-alkyl)₂, -NO₂, -CHO, - CF₂H, -CFH₂, -C(=O)-NH₂, -C(=O)-NH(C₁₋₅-alkyl), -C(=O)-N(C₁-₅-alkyl)₂, - S(=O)₂-C₁₋₅-alkyl, -S(=O)₂-phenyl, phenyl, phenoxy and benzyl;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1- b]thiazolyl, whereby said aryl or heteroaryl radical is bonded via a -(CH₂)₃- group and/or may be substituted with 1, 2, 3, 4 or 5 substituent(s) independently selected from the group consisting of C₁₋₅-alkyl, -O-C₁₋₅-alkyl, - S-C₁₋₅-alkyl, -C(=O)-OH, -C(=O)-C₁₋₅-alkyl, -C(=O)-O-C₁₋₅-alkyl, -O-C(=O)-C₁₋₅- alkyl, F, Cl, Br, I, -CN, -CF₃, -OCF₃, -SCF₃, -OH, -SH, -NH₂, -NH(C₁₋₅-alkyl), - N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
or
a -C(=O)-R¹² moiety;
- R²: represents a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, - CN, -CF₃, -OCF₃, -SCF₃, -OH, -SH, -NH₂, -O-C₁₋₅-alkyl, -S-C₁₋₅-alkyl, -NH-(C₁₋₅-alkyl) and -N(C₁₋₅-alkyl)₂; or
- R¹ and R²: together with the bridging nitrogen a saturated or unsaturated, but not aromatic 3- to 6-membered heterocyclic ring which contains 1, 2 or 3 additional heteroatom(s) independently selected from the group consisting of nitrogen and sulfur as ring member(s) and which may be substituted with 1, 2 or 3 substituent(s) independently selected from the group consisting of C₁₋₅- alkyl, -O-C₁₋₅-alkyl, -S-C₁₋₅-alkyl, oxo (=O), thioxo (=S), -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, - CFH₂, -C(=O)-NH₂, -C(=O)-NH(C₁₋₅-alkyl), -C(=O)-N(C₁₋₅-alkyl)₂, -S(=O)₂-C₁₋₅- alkyl, -S(=O)₂-phenyl, phenyl, phenoxy and benzyl;
- R³: represents a 5- to 10-membered aryl or heteroaryl radical, which may be unsubstituted or may be substituted with 1, 2, 3, 4 or 5 substituent(s) independently selected from the group consisting of C₁₋₅-alkyl, -O-C₁₋₅-alkyl, - S-C₁₋₅-alkyl, -C(=O)-OH, -C(=O)-C₁₋₅-alkyl, -C(=O)-O-C₁₋₅-alkyl, -O-C(=O)-C₁₋₅- alkyl, F, Cl, Br, I, -CN, -CF₃, -OCF₃, -SCF₃, -OH, -SH, -NH₂, -NH(C₁₋₅-alkyl), - N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅- alkyl, -NH-(C₁₋₅-alkyl) and -N(C₁₋₅-alkyl)₂ and whereby the heteroaryl radical contains 1, 2 or 3 heteroatom(s) independently selected from the group consisting of nitrogen, oxygen and sulfur as ring member(s);
- R⁴, R⁵ and R⁶: each represent a hydrogen atom;
- R⁷ and R⁸,: identical or different, each represent a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, - OH, -SH, -NH₂, -O-C₁₋₅-alkyl, -S-C₁₋₅-alkyl, -NH-(C₁₋₅-alkyl) and -N(C₁₋₅-alkyl)₂;
- R⁹ and R¹⁰,: identical or different, each represent a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, - OH, -SH, -NH₂, -O-C₁₋₅-alkyl, -S-C₁₋₅-alkyl, -NH-(C₁₋₅-alkyl) and -N(C₁₋₅-alkyl)₂;
- R¹¹ and: represents a linear or branched, saturated or unsaturated, unsubstituted C₁₋₁₀ aliphatic radical or a 5- to 10-membered aryl or heteroaryl radical which may be unsubstituted or may be substituted with 1, 2, 3, 4 or 5 substituent(s) independently selected from the group consisting of C₁₋₅-alkyl, -O-C₁₋₅-alkyl, - S-C₁₋₅-alkyl, -C(=O)-OH, -C(=O)-C₁₋₅-alkyl, -C(=O)-O-C₁₋₅-alkyl, -O-C(=O)-C₁₋₅- alkyl, F, Cl, Br, I, -CN, -CF₃, -OCF₃, -SCF₃, -OH, -SH, -NH₂, -NH(C₁₋₅-alkyl), - N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and whereby the heteroaryl radical contains 1, 2 or 3 heteroatom(s) independently selected from the group consisting of nitrogen, oxygen and sulfur as ring member(s);
- R¹²: represents an aryl radical which may be substituted with 1, 2, 3, 4 or 5 substituent(s) independently selected from the group consisting of C₁₋₅-alkyl, - O-C₁₋₅-alkyl, -S-C₁₋₅-alkyl, -C(=O)-OH, -C(=O)-C₁₋₅-alkyl,-C(=O)-O-C₁₋₅-alkyl, - O-C(=O)-C₁₋₅-alkyl, F, Br, I, -CN, -CF₃, -OCF₃, -SCF₃, -SH, -NH₂, -NH(C₁₋₅- alkyl), -N(C₁₋₅-alkyl)₂, -CHO, -CF₂H, -CFH₂, -C(=O)-NH₂, -C(=O)-NH(C₁₋₅- alkyl), -C(=O)-N(C₁₋₅-alkyl)₂, -S(=O)₂-C₁₋₅-alkyl, -S(=O)₂-phenyl, phenyl, phenoxy and benzyl;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl which may be unsubstituted or may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, - CF₃, -OCF₃, -SCF₃, -OH, -SH, -NH₂, -NH(C₁₋₅-alkyl), -N(C₁₋₅-alkyl)₂, -NO₂, - CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be unsubstituted or may be substituted with 1, 2, 3, 4 or 5 substituent(s) independently selected from the group consisting of C₁₋₅-alkyl, -O-C₁₋₅-alkyl, - S-C₁₋₅-alkyl, -C(=O)-OH, -C(=O)-C₁₋₅-alkyl, -C(=O)-O-C₁₋₅-alkyl, -O-C(=O)-C₁₋₅- alkyl, F, Cl, Br, I, -CN, -CF₃, -OCF₃, -SCF₃, -OH, -SH, -NH₂, -NH(C₁₋₅-alkyl), - N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which is bonded via a -(CH₂)_{1, 2 or 3}-group;
optionally in form of one of their stereoisomers, preferably enantiomers or diasteromers, a racemate or in form of a mixture of at least two of their stereoisomers, preferably enantiomers and/or diastereomers, in any mixing ratio, or a physiologically acceptable salt thereof, or a corresponding solvate thereof.

Preferably the compound Benzyl-[5-(4-methyl-piperazin-1-yl)-2-nitro-phenyl]-amine may be excluded in any of the definitions given herein.

Preferred are compounds of general formula I given above, wherein R¹ represents
an (hetero)cycloaliphatic radical selected from the group consisting of cyclopropyl, cyclobutyl, cyclopentyl, cyclopentenyl, cyclohexyl, cyclohexenyl, cycloheptyl, cycloheptenyl, cyclooctyl, cyclooctenyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl whereby said (hetero)cylcoaliphatic radical can be bonded via a -(CH₂)_{1, 2 or 3}- group and/or may be substituted with 1, 2 or 3 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, - NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃,
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃,
an aryl or heteroaryl radical selected from the group consisting of phenyl, naphthyl, thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a - (CH₂)-group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)3, -C(=O)-CH₃, -C(=O)-C₂H₅, -C(=O)-CH₂-CH₂-CH₃, -C(=O)-CH(CH₃)₂, - C(=O)-C(CH₃)₃, F, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, - CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃,
an aryl or heteroaryl radical selected from the group consisting of naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₂-group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, - SH, -NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -NO₂, -CHO, -CF₂H, -CFH₂, -C(=O)-NH₂, -C(=O)-NH-CH₃, - C(=O)-NH-C₂H₅, -C(=O)-N(CH₃)₂, -C(=O)-N(C₂H₅)₂ and -S(=O)₂-CH₃,
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₃-group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, - SH, -NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -NO₂, -CHO, -CF₂H, -CFH₂, -C(=O)-NH₂, -C(=O)-NH-CH₃, - C(=O)-NH-C₂H₅, -C(=O)-N(CH₃)₂, -C(=O)-N(C₂H₅)₂ and -S(=O)₂-CH₃,
or
a -C(=O)-R¹² moiety;
preferably R¹ represents
an unsubstituted pyrrolyl radical;
an aryl or heteroaryl radical selected from the group consisting of phenyl and pyrrolyl, whereby said phenyl radical is bonded via a -(CH₂)-group and said pyrrolyl radical is bonded via a -(CH₂)₂-group and/or said phenyl or pyrrolyl radical may be substituted with 1, 2 or 3 substituent(s) independently selected from the group consisting of methyl, ethyl, n-propyl, isopropyl, n-butyl, tert-butyl, sec-butyl, isobutyl, n-penty), -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Br, I, -CN, -CF₃, -OCF₃, - SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ or
a -C(=O)-R¹² moiety;
more preferably R¹ represents an unsubstituted pyrrolyl radical;
an aryl or heteroaryl radical selected from the group consisting of phenyl and pyrrolyl, whereby said phenyl radical is bonded via a -(CH₂)-group and said pyrrolyl radical is bonded via a -(CH₂)₂-group and/or said phenyl or pyrrolyl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, F, Br and I or
a -C(=O)-R¹² moiety;
and X and R² to R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.
Furthermore such nitro-substituted phenyl-piperazine compounds of general formula are preferred, wherein
R² represents a hydrogen atom or a linear or branched, unsubstituted C₁₋₁₀ alkyl radical;
preferably R² represents a hydrogen atom or an alkyl radical selected from the group consisting of methyl, ethyl, n-propyl, isopropyl, n-butyl, sec-butyl, isobutyl and tert-butyl;
more preferably R² represents a hydrogen atom or a methyl radical;
and X, R¹ and R³ to R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such nitro-substituted phenyl-piperazine compounds of general formula I, wherein
R¹ and R² together with the bridging nitrogen atom form a moiety selected from the group consisting of 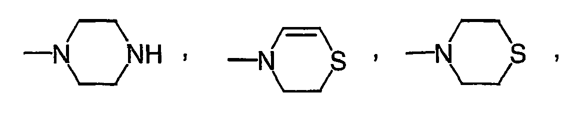 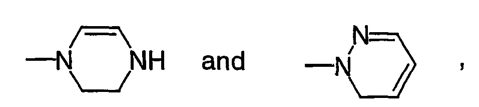 whereby each of these afore mentioned cyclic moieties may be substituted in any position including the -NH-groups with 1, 2 or 3 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, - SH, -NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -NO₂, -CHO, -CF₂H, -CFH₂, -C(=O)-NH₂, -C(=O)-NH-CH₃, - C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl;
preferably R¹ and R² together with the bridging nitrogen atom form a pyridazin-3-one moiety which may be substituted with 1, 2 or 3 substituent(s) independently selected from the group consisting of methyl, ethyl, n-propyl, isopropyl, n-butyl, tert-butyl, -O-CH₃, -O-C₂H₅, -C(=O)-OH, -C(=O)-O-CH₃, -C(=O)-O-C₂H₅, -C(=O)-CH₃, -C(=O)-C₂H₅, F, Cl, Br, I, -CN, -CF₃, -OCF₃, -SCF₃, -OH, -SH, -NH₂, -NH-CH₃, -NH-C₂H₅, - NO₂, -CHO, -CF₂H, -CFH₂, -C(=O)-NH₂, phenyl, phenoxy and benzyl;
more preferably R¹ and R² together with the bridging nitrogen atom form an unsubstituted pyridazin-3-one moiety;
and X and R³ to R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Moreover, such nitro-substituted phenyl-piperazine compounds of general formula I given above are preferred, wherein
R³ represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a -(CH₂)_{1, 2 or3}-group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
preferably R³ represents a phenyl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH3, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃;
more preferably R³ represents an unsubstituted phenyl radical;
and X, R¹, R² and R⁴ to R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are nitro-substituted phenyl-piperazine compounds of general formula I, wherein
R⁷ and R⁸, identical or different, each represent a hydrogen atom or a linear or branched, unsubstituted C₁₋₁₀ alkyl radical;
preferably R⁷ and R⁸, identical or different, each represent a hydrogen atom or an alkyl radical selected from the group consisting of methyl, ethyl, n-propyl, isopropyl, n-butyl, sec-butyl, isobutyl and tert-butyl;
more preferably R⁷ and R⁸ each represent a hydrogen atom;
and X, R¹ to R⁶ and R⁹ to R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are nitro-substituted phenyl-piperazine compounds of general formula I, wherein R⁹ and R¹⁰, identical or different, each represent a hydrogen atom or a linear or branched, unsubstituted C₁₋₁₀ alkyl radical;
preferably R⁹ and R¹⁰, identical or different, each represent a hydrogen atom or an alkyl radical selected from the group consisting of methyl, ethyl, n-propyl, isopropyl, n-butyl, sec-butyl, isobutyl and tert-butyl;
more preferably R⁹ and R¹⁰ each represent a hydrogen atom;
and X, R¹ to R⁸ and R¹¹ and R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are nitro-substituted phenyl-piperazine compounds of general formula I, wherein
R¹¹ represents an alkyl radical selected from the group consisting of methyl, ethyl, n-propyl, isopropyl, n-butyl, sec-butyl, isobutyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyridinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃;
preferably R¹¹ represents an alkyl radical selected from the group consisting of methyl, ethyl, n-propyl, isopropyl and n-butyl; or an aryl or heteroaryl radical selected from the group consisting of phenyl and pyridinyl whereby said aryl or heteroaryl may be substituted with 1, 2 or 3 substituent(s) independently selected from the group consisting of methyl, ethyl, n-propyl, isopropyl, n-butyl, tert-butyl, -O-CH₃, -O-C₂H₅, - C(=O)-OH, -C(=O)-O-CH₃, -C(=O)-O-C₂H₅, F, Cl, Br, I, -CN, -CF₃, -OCF₃, -SCF₃, - OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
more preferably R¹¹ represents an alkyl radical selected from the group consisting of methyl, ethyl, n-propyl and n-butyl; or an unsubstituted phenyl or pyridinyl radical;
and X, R¹ to R¹⁰ and R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are nitro-substituted phenyl-piperazine compounds of general formula I, wherein
R¹² represents an aryl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH3, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Br, I, -CN, -CF₃, -OCF₃, -SCF₃, -SH, - NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,7-b]thiazolyl whereby said heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH3, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃;
or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₁,_{2 or 3}- group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃;
preferably R¹² represents a phenyl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Br, I, -CN, -CF₃, -OCF₃, - SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, -C(=O)-NH₂, -C(=O)-NH-CH₃, - C(=O)-NH-C₂H₅, -C(-O)-N(CH₃)₂, -C(=O)-N(C₂H₅)₂ and -S(=O)₂-CH₃;
more preferably R¹² represents an unsubstituted phenyl radical;
and X and R¹ to R¹¹ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are nitro-substituted phenyl-piperazine compounds of general formula I, wherein
R¹ represents an unsubstituted pyrrolyl radical; an aryl or heteroaryl radical selected from the group consisting of phenyl and pyrrolyl, whereby said phenyl radical is bonded via a -(CH₂)-group and said pyrrolyl radical is bonded via a -(CH₂)₂-group and/or said phenyl or pyrrolyl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, - C(=O)-CH(CH₃)₂, -C(=O)-C(CH₃)₃, F, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ or a -C(=O)-R¹² moiety;
R² represents a hydrogen atom or an alkyl radical selected from the group consisting of methyl, ethyl, n-propyl, isopropyl, n-butyl, sec-butyl, isobutyl and tert-butyl;
or
R¹ and R² together with the bridging nitrogen atom form a pyridazin-3-one moiety which may be substituted with 1, 2 or 3 substituent(s) independently selected from the group consisting of methyl, ethyl, n-propyl, isopropyl, n-butyl, tert-butyl, -O-CH₃, - O-C₂H₅, -C(=O)-OH, -C(=O)-O-CH₃, -C(=O)-O-C₂H₅, -C(=O)-CH₃, -C(=O)-C₂H₅, F, Cl, Br, I, -CN, -CF₃, -OCF₃, -SCF₃, -OH, -SH, -NH₂, -NH-CH₃, -NH-C₂H₅, -NO₂, -CHO, -CF₂H, -CFH₂, -C(=O)-NH₂, phenyl, phenoxy and benzyl;
R³ represents a phenyl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH3, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(-O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH,-SH, -NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -NO₂, -CHO, -CF₂H, -CFH₂, -C(=O)-NH₂, -C(=O)-NH-CH₃, - C(=O)-NH-C₂H₅, -C(=O)-N(CH₃)₂, -C(=O)-N(C₂H₅)₂ and -S(=O)₂-CH₃;
R⁴, R⁵ and R⁶ each represent a hydrogen atom;
R⁷ and R⁸, identical or different, each represent a hydrogen atom or an alkyl radical selected from the group consisting of methyl, ethyl, n-propyl, isopropyl, n-butyl, sec-butyl, isobutyl and tert-butyl;
R⁹ and R¹⁰, identical or different, each represent a hydrogen atom or an alkyl radical selected from the group consisting of methyl, ethyl, n-propyl, isopropyl, n-butyl, sec-butyl, isobutyl and tert-butyl;
R¹¹ represents an alkyl radical selected from the group consisting of methyl, ethyl, n-propyl, isopropyl and n-butyl; or an aryl or heteroaryl radical selected from the group consisting of phenyl and pyridinyl whereby said aryl or heteroaryl may be substituted with 1, 2 or 3 substituent(s) independently selected from the group consisting of methyl, ethyl, n-propyl, isopropyl, n-butyl, tert-butyl, -O-CH₃, -O-C₂H₅, -C(=O)-OH, - C(=O)-O-CH₃, -C(=O)-O-C₂H₅, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
and
R¹² represents a phenyl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Br, I, -CN, -CF₃, -OCF₃, -SCF₃, -SH,-NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Particularly preferred are nitro-substituted phenyl-piperazine compounds of general formula I, wherein
R¹ represents an unsubstituted pyrrolyl radical; an aryl or heteroaryl radical selected from the group consisting of phenyl and pyrrolyl, whereby said phenyl radical is bonded via a -(CH₂)-group and said pyrrolyl radical is bonded via a -(CH₂)₂-group and/or said phenyl or pyrrolyl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, F, Br and l or a -C(=O)-R¹² moiety;
R² represents a hydrogen atom or a methyl radical;
or
R¹ and R² together with the bridging nitrogen atom form an unsubstituted pyridazin-3-one moiety;
R³ represents an unsubstituted phenyl radical;
R⁴, R⁵ and R⁶ each represent a hydrogen atom;
R⁷ and R⁸ each represent a hydrogen atom;
R⁹ and R¹⁰ each represent a hydrogen atom;
R¹¹ represents an alkyl radical selected from the group consisting of methyl, ethyl, n-propyl- and n-butyl; or an unsubstituted phenyl or pyridinyl radical
and
R¹² represents an unsubstituted phenyl radical;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Particularly preferred are nitro-substituted phenyl-piperazine compounds selected from the group consisting of
[1] [5-(4-Methyl-piperazin-1-yl)-2-nitro-phenyl]-(2-pyrrol-1-yl-ethyl)-amine,
[2] (4-Fluoro-benzyl)-[5-(4-methyl-piperazin-1-yl)-2-nitro-phenyl]-amine,
[3] N-[5-(4-Methyl-piperazin-1-yl)-2-nitro-phenyl]-benzamide,
[4] N-Methyl-N-[5-(4-methyl-piperazin-1-yl)-2-nitro-phenyl]-benzamide,
[5] [5-(4-Methyl-piperazin-1-yl)-2-nitro-phenyl]-pyrrol-1-yl-amine,
[6] 2-[5-(4-Methyl-piperazin-1-yl)-2-nitro-phenyl]-2H-pyridazin-3-one,
[7] 1-Methyl-4-(4-nitro-3-phenoxy-phenyl)-piperazine,
[8] Benzyl-[5-(4-butyl-piperazin-1-yl)-2-nitro-phenyl]-amine,
[9] Benzyl-[2-nitro-5-(4-pyridin-2-yl-piperazin-1-yl)-phenyl]-amine and
[10] Benzyl-[2-nitro-5-(4-phenyl-2-yl-piperazin-1-yl)-phenyl]-amine;
optionally in form of a physiologically acceptable salt thereof, or a corresponding solvate thereof.

In yet another aspect the present invention relates to nitro-substituted phenyl-piperazine compounds of general formula I, 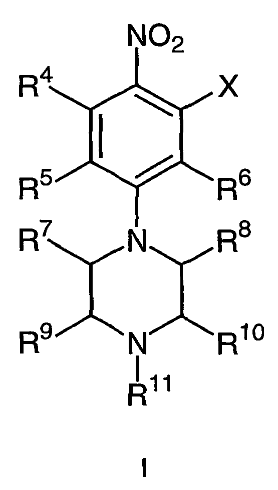 wherein
- X: represents a -NR¹R² moiety or a -OR³ moiety;
- R¹: represents a saturated or unsaturated, optionally at least mono-substituted 3- to 9-membered cycloaliphatic radical, which is bonded via a linear or branched, optionally at least mono-substituted C₁₋₆ alkylene, C₂₋₆ alkenylene or C₂₋₆ alkinylene group and/or which may contain 1, 2 or 3 heteroatom(s) independently selected from the group consisting of nitrogen, oxygen and sulphur as ring member(s);
an optionally at least mono-substituted heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1- b]thiazolyl;
an aryl or heteroaryl radical selected from the group consisting of phenyl, naphthyl, thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)-group and/or may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-C₁₋₅-alkyl, - C(=O)-O-C₁₋₅-alkyl, -O-C(=O)-C₁₋₅-alkyl, F, Br, I, -CN, -CF₃, -OCF₃, -SCF₃, - OH, -SH, -NH₂, -NH(C₁₋₅-alkyl), -N(C₁₋₅-alkyl)₂, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁₋₅-alkyl, - S(=O)₂-phenyl, phenyl, phenoxy and benzyl;
an aryl or heteroaryl radical selected from the group consisting of naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₂-group and/or may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)- C₁₋₅-alkyl, -C(=O)-O-C₁₋₅-alkyl, -O-C(=O)-C₁₋₅-alkyl, F, Cl, Br, I, -CN, -CF₃, - OCF₃, -SCF₃, -OH, -SH, -NH₂, -NH(C₁₋₅-alkyl), -N(C₁₋₅-alkyl)₂, -NO₂, -CHO, - CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₃-group and/or may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)- C₁₋₅-alkyl, -C(=O)-O-C₁₋₅-alkyl, -O-C(=O)-C₁-₅-alkyl, F, Cl, Br, I, -CN, -CF₃, - OCF₃, -SCF₃, -OH, -SH, -NH₂, -NH(C₁₋₅-alkyl), -N(C₁₋₅-alkyl)₂, -NO₂, -CHO, - CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl;
or
a -C(=O)-R¹² moiety;
- R²: represents a hydrogen atom or a linear or branched, saturated or unsaturated, optionally at least mono-substituted C₁₋₁₀ aliphatic radical;
- R¹ and R²: together with the bridging nitrogen form an optionally at least mono- substituted, saturated or unsaturated, but not aromatic 3- to 9-membered heterocyclic ring which may contain 1, 2 or 3 additional heteroatom(s) independently selected from the group consisting of nitrogen and sulphur as ring member(s);
- R³: represents an optionally at least mono-substituted 5- to 10-membered aryl or heteroaryl radical, which may be bonded via a linear or branched, optionally at least mono-substituted C₁₋₆ alkylene, C₂₋₆ alkenylene or C₂₋₆ alkinylene group and whereby the heteroaryl radical contains 1, 2 or 3 heteroatom(s) independently selected from the group consisting of nitrogen, oxygen and sulphur as ring member(s);
- R⁴, R⁵ and R⁶: each represent a hydrogen atom;
- R⁷ and R⁸,: identical or different, each represent a hydrogen atom or a linear or branched, saturated or unsaturated, optionally at least mono-substituted C₁₋₁₀ aliphatic radical;
- R⁹ and: R¹⁰, identical or different, each represent a hydrogen atom or a linear or branched, saturated or unsaturated, optionally at least mono-substituted C₁₋₁₀ aliphatic radical;
R¹¹ represents a linear or branched, saturated or unsaturated, unsubstituted C₁₋₁₀ aliphatic radical or an optionally at least mono-substituted 5- to 10-membered aryl or heteroaryl radical whereby the heteroaryl radical contains 1, 2 or 3 heteroatom(s) independently selected from the group consisting of nitrogen, oxygen and sulphur as ring member(s); and
- R¹²: represents an aryl radical which may be substituted with 1, 2, 3, 4 or 5 substituent(s) independently selected from the group consisting of C₁₋₅-alkyl, - O-C₁₋₅-alkyl, -S-C₁₋₅-alkyl, -C(=O)-OH, -C(=O)-C₁₋₅-alkyl,-C(=O)-O-C₁₋₅-alkyl, - O-C(=O)-C₁₋₅-alkyl, F, Br, I, -CN, -CF₃, -OCF₃, -SCF₃, -SH, -NH₂, -NH(C₁₋₅- alkyl), -N(C₁₋₅-alkyl)₂, -CHO, -CF₂H, -CFH₂, -C(=O)-NH₂, -C(=O)-NH(C₁₋₅- alkyl), -C(=O)-N(C₁₋₅-alkyl)₂, -S(=O)₂-C₁₋₅-alkyl, -S(=O)₂-phenyl, phenyl, phenoxy and benzyl;
an optionally at least mono-substituted heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1- b]thiazolyl;
or
an optionally at least mono-substituted aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)_{1,2 or 3}- group;
with the proviso that the following compound
(1) Benzyl-[5-(4-methyl-piperazin-1-yl)-2-nitro-phenyl]-amine may preferably be excluded;
   optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.
   If any of the substituents represents or comprises a (hetero)cycloaliphatic radical [(hetero)cycloaliphatic group], said (hetero)cycloaliphatic radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of oxo (=O), thioxo (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; whereby in each occurence C₁₋₅-alkyl may be linear or branched and whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, - SCF₃, -OH, -SH, -NH₂ and NO₂.

More preferably said substituents may be selected independently from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃ -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂,-NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl; whereby in each occurence said cyclic substituents may be unsubstituted or substituted by 1, 2 or 3 substituent(s) independently selected from the group consisting of methyl, ethyl, n-propyl, isopropyl, methoxy, ethoxy, F, Cl, Br, - CN, -CF₃, -OCF₃, -SCF₃, -OH, -SH, -NH₂ and NO₂.

If any of the substituents represents or comprises a cycloaliphatic radical, which contains one or more, preferably 1, 2 or 3 heteroatom(s) as ring member(s), unless defined otherwise, each of these heteroatom(s) may preferably be selected independently from the group consisting of N, O and S.

Suitable (hetero)cycloaliphatic radicals, which may be unsubstituted or at least mono-substituted, include cyclopropyl, cyclobutyl, cyclopentyl, cyclopentenyl, cyclohexyl, cyclohexenyl, cycloheptyl, cycloheptenyl, cyclooctyl, cyclooctenyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl.

If any of the substituents represents or comprises an aryl radical (aryl group), including a phenyl or naphthyl group, said aryl radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -SH, -NH₂, -NH(C₁₋₅-alkyl), -N(C₁₋₅-alkyl)₂, -NO₂,-CHO, -CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl; whereby in each occurrence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and NO₂.

More preferably said substituents may be selected independently from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂Hₛ)₂ and -S(=O)₂-CH₃.

Preferred aryl radicals, which may optionally be at least mono-substituted, are phenyl and naphthyl.

If any of the substituents represents or comprises a heteroaryl radical (heteroaryl group), said heteroaryl radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Br, I, -CN, -CF₃, -OCF₃, -SCF₃, -SH, -NH₂, -NH(C₁₋₅-alkyl), -N(C₁₋₅-alkyl)₂, -CHO, -CF₂H, - CFH₂, -C(=O)-NH₂, -C(=O)-NH(C₁₋₅-alkyl), -C(=O)-N(C₁₋₅-alkyl)₂, -S(=O)2-C₁₋₅-alkyl, - S(=O)₂-phenyl, phenyl, phenoxy and benzyl; whereby in each occurrence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, - CN, -CF₃, -OCF₃, -SCF₃, -OH, -SH, -NH₂ and NO₂.

More preferably said substituents may be selected independently from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.

The heteroatom(s), which are present as ring member(s) in the heteroaryl radical, may, unless defined otherwise, independently be selected from the group consisting of nitrogen, oxygen and sulphur. Preferably the heteroaryl radical comprises 1, 2 or 3 heteroatom(s).

Suitable heteroaryl radicals, which may optionally be at least mono-substituted, may preferably be selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl.

If at least two of the substituents together with the bridging nitrogen atom form a saturated or unsaturated heterocyclic ring, said heterocyclic ring may preferably be selected from the group consisting of

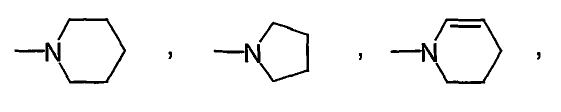

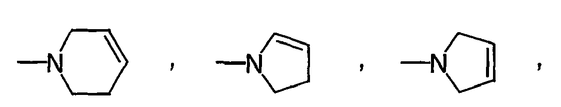

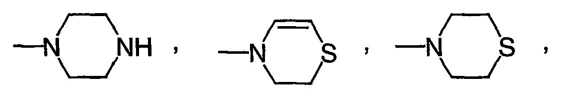

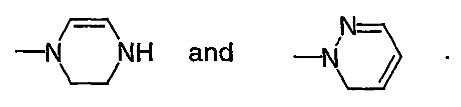

Those skilled in the art understand that the afore mentioned cyclic moieties, which contain an NH group may also be substituted in this position, i.e. the hydrogen atom may be exchanged for another substituent.

Said heterocyclic rings may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅-alkyl, oxo (=O), thioxo (=S), -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; whereby in each occurrence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and NO₂.

More preferably said substituents may be selected independently from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂,-NH-CH₃, -NH-C₂H₅, -NH-CH₂-CH₂-CH₃, -NH-CH(CH₃)₂, -NH-C(CH₃)₃, -N(CH₃)₂, - N(C₂H,₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl; whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and NO₂.

If any of the substituents represents a saturated or unsaturated aliphatic radical (aliphatic group), i.e. an alkyl radical, an alkenyl radical or an alkinyl radical, said aliphatic radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of -O-C₁₋₅-alkyl, -S-C₁₋₅-alkyl, F, Cl, Br, I, -CN, -CF₃, -OCF₃, -SCF₃, - OH, -SH, -NH₂, -NH(C₁₋₅-alkyl) and -N(C₁₋₅-alkyl)₂, whereby in each occurence C₁₋₅-alkyl may be linear or branched. More preferably said substituent(s) may preferably be selected independently from the group consisting of -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, F, Cl, Br, I, -CN, -CF₃, -OCF₃, -SCF₃, -OH, -SH, -NH₂, NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂.

An alkenyl radical comprises at least one carbon-carbon double bond, an alkinyl radical comprises at least one carbon-carbon triple bond.

Suitable alkyl radicals, which may be substituted by one or more substituents, may preferably be selected from the group consisting of methyl, ethyl, n-propyl, isopropyl, n-butyl, isobutyl, sec-butyl, tert-butyl, n-pentyl, n-hexyl, n-heptyl, n-octyl, n-nonyl and n-decyl.

Suitable alkenyl radicals, which may be substituted by one or more substituents, may preferably be selected from the group consisting of vinyl, 1-propenyl, 2-propenyl, 1-butenyl, 2-butenyl and 3-butenyl.

Suitable alkinyl radicals, which may be substituted by one or more substituents, may preferably be selected from the group consisting of ethinyl, 1-propinyl, 2-propinyl, 1-butinyl, 2-butinyl and 3-butinyl.

Preferred are compounds of general formula I given above, wherein R¹ represents an (hetero)cycloaliphatic radical selected from the group consisting of cyclopropyl, cyclobutyl, cyclopentyl, cyclopentenyl, cyclohexyl, cyclohexenyl, cycloheptyl, cycloheptenyl, cyclooctyl, cyclooctenyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl whereby said (hetero)cylcoaliphatic radical can be bonded via a linear or branched, optionally at least mono-substituted C₁₋₆ alkylene, C₂₋₆ alkenylene or C₂₋₆ alkinylene group and/or may be substituted with 1, 2 or 3 substituent(s) independently selected from the group consisting of oxo (=O), thioxo (=S), C₁₋₅-alkyl, -O-C₁₋₅-alkyl, -S-C₁₋₅-alkyl, - C(=O)-OH, -C(=O)-C₁₋₅-alkyl, -C(=O)-O-C₁₋₅-alkyl, -O-C(=O)-C₁₋₅-alkyl, F, Cl, Br, I, - CN, -CF₃, -OCF₃, -SCF₃, -OH, -SH, -NH₂, -NH(C₁₋₅-alkyl), -N(C₁₋₅-alkyl)₂, -NO₂, - CHO, -CF₂H, -CFH₂, -C(=O)-NH₂, -C(=O)-NH(C₁₋₅-alkyl), -C(=O)-N(C₁₋₅-alkyl)2, - S(=O)₂-C₁₋₅-alkyl, -S(=O)₂-phenyl, phenyl, phenoxy and benzyl;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said heteroaryl radical may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl),-N(C₁₋₅-alkyl)₂, -NO₂, -CHO, -CF₂H, -CFH₂, -C(=O)-NH₂, -C(=O)-NH(C₁₋₅-alkyl), - C(=O)-N(C₁₋₅-alkyl)₂, -S(=O)₂-C₁₋₅-alkyl, -S(=O)₂-phenyl, phenyl, phenoxy and benzyl;
an aryl or heteroaryl radical selected from the group consisting of phenyl, naphthyl, thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a - (CH₂)-group and/or may be substituted with 1, 2 or 3 substituent(s) independently selected from the group consisting of C₁₋₅-alkyl, -O-C₁₋₅-alkyl, -S-C₁₋₅-alkyl, -C(=O)-C₁₋₅-alkyl, -C(=O)-O-C₁₋₅-alkyl, -O-C(=O)-C₁₋₅-alkyl, F, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
an aryl or heteroaryl radical selected from the group consisting of naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₂-group and/or may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), C(=O)-N(C₁₋₅-alkyl)₂, - S(=O)₂-C₁₋₅-alkyl, -S(=O)₂-phenyl, phenyl, phenoxy and benzyl;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₃-group and/or may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl;
or
a -C(=O)-R¹² moiety;
preferably R¹ represents an (hetero)cycloaliphatic radical selected from the group consisting of cyclopropyl, cyclobutyl, cyclopentyl, cyclopentenyl, cyclohexyl, cyclohexenyl, cycloheptyl, cycloheptenyl, cyclooctyl, cyclooctenyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl whereby said (hetero)cylcoaliphatic radical can be bonded via a -(CH₂)_{1, 2 or 3}- group and/or may be substituted with 1, 2 or 3 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, CI, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃,
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H_{5,} -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃,
an aryl or heteroaryl radical selected from the group consisting of phenyl, naphthyl, thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a - (CH₂)-group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, - CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃,
an aryl or heteroaryl radical selected from the group consisting of naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₂-group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH₃, -S-C₂H₅, -S-CH₂-CH₂-CH₃, -S-CH(CH₃)₂, -S- , C(CH₃)₃, -C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, - SH, -NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -NO₂, -CHO, -CF₂H, -CFH₂, -C(=O)-NH₂, -C(=O)-NH-CH₃, - C(=O)-NH-C₂H₅, -C(=O)-N(CH₃)₂, -C(=O)-N(C₂H₅)₂ and -S(=O)₂-CH₃,
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₃-group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃,-C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, - SH, -NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -NO₂, -CHO, -CF₂H, -CFH₂, -C(=O)-NH₂, -C(=O)-NH-CH₃, - C(=O)-NH-C₂H₅, -C(=O)-N(CH₃)₂, -C(=O)-N(C₂H₅)₂ and -S(=O)₂-CH₃,
or
a -C(=O)-R¹² moiety;
more preferably R¹ represents an unsubstituted pyrrolyl radical; an aryl or heteroaryl radical selected from the group consisting of phenyl and pyrrolyl, whereby said phenyl is bonded via a -(CH₂)-group and said pyrrolyl radical is bonded via a -(CH₂)_{1 or 2}- group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, - CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ or a -C(=O)-R¹² moiety
and X and R² to R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Furthermore such nitro-substituted phenyl-piperazine compounds of general formula I are preferred, wherein R² represents a hydrogen atom or a linear or branched, unsubstituted C₁₋₁₀ alkyl radical;
preferably R² represents a hydrogen atom or an alkyl radical selected from the group consisting of methyl, ethyl, n-propyl, isopropyl, n-butyl, sec-butyl, isobutyl and tert-butyl;
more preferably R² represents a hydrogen atom or a methyl radical;
and X, R¹ and R³ to R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such nitro-substituted phenyl-piperazine compounds of general formula I, wherein R¹ and R² together with the bridging nitrogen atom form a moiety selected from the group consisting of 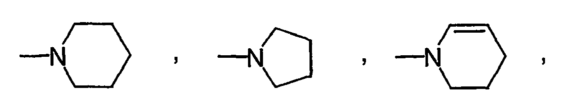 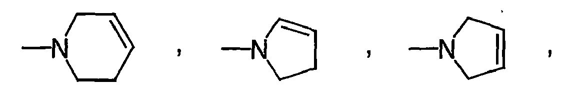 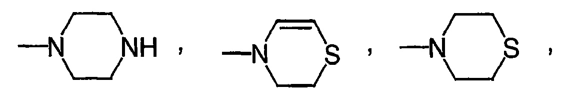 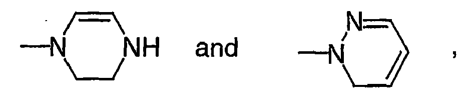 whereby each of these afore mentioned cyclic moieties may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH, -C(=O)-C₁₋₅-alkyl, -C(=O)-O-C₁₋₅-alkyl, -O-C(=O)-C₁₋₅-alkyl, F, CI, Br, I, -CN, -CF₃, -OCF₃, -SCF₃, -OH, -SH, - NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
preferably R¹ and R² together with the bridging nitrogen atom form a moiety selected from the group consisting of 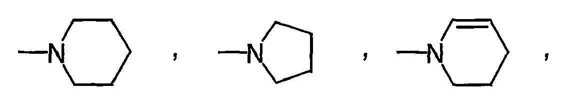 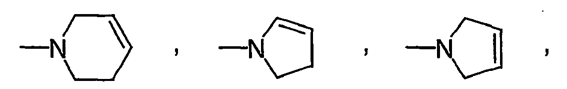 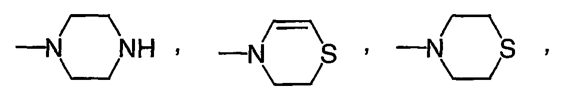 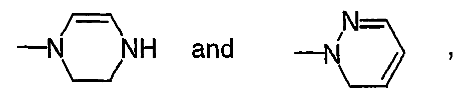 whereby each of these afore mentioned cyclic moieties may be substituted with 1, 2 or 3 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl;
more preferably R¹ and R² together with the bridging nitrogen atom form a pyridazin-3-one moiety;
and X and R³ to R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Moreover, such nitro-substituted phenyl-piperazine compounds of general formula I given above are preferred, wherein R³ represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a - (CH₂)_{1, 2 or 3}-group and/or may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, CI, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl;
preferably R³ represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a -(CH₂)_{1,2 or 3}-group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, - CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃,
more preferably R³ represents a phenyl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH3, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, -CF₃,-OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃;
and X, R¹, R² and R⁴ to R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are nitro-substituted phenyl-piperazine compounds of general formula I, wherein R⁷ and R⁸, identical or different, each represent a hydrogen atom or a linear or branched, unsubstituted C₁₋₁₀ alkyl radical;
preferably R⁷ and R⁸, identical or different, each represent a hydrogen atom or an alkyl radical selected from the group consisting of methyl, ethyl, n-propyl, isopropyl, n-butyl, sec-butyl, isobutyl and tert-butyl;
more preferably R⁷ and R⁸ each represent a hydrogen atom;
and X, R¹ to R⁶ and R⁹ to R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are nitro-substituted phenyl-piperazine compounds of general formula I, wherein R⁹ and R¹⁰, identical or different, each represent a hydrogen atom or a linear or branched, unsubstituted C₁₋₁₀ alkyl radical;
preferably R⁹ and R¹⁰, identical or different, each represent a hydrogen atom or an alkyl radical selected from the group consisting of methyl, ethyl, n-propyl, isopropyl, n-butyl, sec-butyl, isobutyl and tert-butyl;
more preferably R⁹ and R¹⁰ each represent a hydrogen atom;
and X, R¹ to R⁸ and R¹¹ and R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are nitro-substituted phenyl-piperazine compounds of general formula I, wherein R¹¹ represents a linear or branched, unsubstituted C₁₋₁₀ alkyl radical; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl whereby said aryl or heteroaryl may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, - C(=O)-O-C₁₋₅-alkyl, -O-C(=O)-C₁₋₅-alkyl, F, CI, Br, I, -CN, -CF₃, -OCF₃, -SCF₃, -OH, - SH, -NH₂, -NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
preferably R¹¹ represents an alkyl radical selected from the group consisting of methyl, ethyl, n-propyl, isopropyl, n-butyl, sec-butyl, isobutyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, CI, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
more preferably R¹¹ represents an alkyl radical selected from the group consisting of methyl, ethyl, n-propyl, isopropyl and n-butyl; or an unsubstituted phenyl or pyridinyl radical;
and X, R¹ to R¹⁰ and R¹² have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are nitro-substituted phenyl-piperazine compounds of general formula I, wherein R¹² represents an aryl radical which may be substituted with 1, 2 or 3 substituent(s) independently selected from the group consisting of whereby said aryl radical may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Br, I, -CN, -CF₃, -OCF₃, -SCF₃, - SH, -NH₂, -NH(C₁₋₅-alkyl), -N(C₁₋₅-alkyl)₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
an optionally at least mono-substituted heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl whereby said heteroaryl radical may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, CI, Br, I, -CN, - CF₃, -OCF₃, -SCF₃, -OH, -SH, -NH₂, -NH(C₁₋₅-alkyl), -N(C₁₋₅-alkyl)₂, -NO₂, -CHO, - CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)_{1,2} or ₃- group and/or may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, CI, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
preferably R¹² represents an aryl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH3, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Br, I, -CN, -CF₃, -OCF₃, - SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, -C(=O)-NH₂, -C(=O)-NH-CH₃, - C(=O)-NH-C₂H₅, -C(=O)-N(CH₃)₂, -C(=O)-N(C₂H₅)₂ and -S(=O)₂-CH₃;
an optionally at least mono-substituted heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl whereby said heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH3, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, CI, Br, I, -CN, -CF₃, -OCF₃, -SCF₃, -OH, -SH, -NH₂, - NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)_{1,2 or 3}- group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, CI, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃;
more preferably R¹² represents a phenyl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Br, I, -CN, -CF₃, -OCF₃, - SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, -C(=O)-NH₂, -C(=O)-NH-CH₃, - C(=O)-NH-C₂H₅, -C(=O)-N(CH₃)₂, -C(=O)-N(C₂H₅)₂ and -S(=O)₂-CH₃;
and X and R¹ to R¹¹ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are nitro-substituted phenyl-piperazine compounds of general formula I, wherein
- X: represents a -NR¹R² moiety or a -OR³ moiety;
- R¹: represents an unsubstituted pyrrolyl radical; an aryl or heteroaryl radical selected from the group consisting of phenyl and pyrrolyl, whereby said pheny radical is bonded via a (CH₂)-group and said pyrrolyl radical is bonded via a - (CH₂)_{1 or 2}- group and may be substituted with 1 fluorine atom; or a -C(=O)-R¹² moiety;
- R²: represents a hydrogen atom or a methyl radical; or

- R¹ and R²: together with the bridging nitrogen atom form a pyridazin-3-one moiety;
- R³: represents an unsubstituted phenyl radical;
- R⁴, R⁵ and R⁶: each represent a hydrogen atom;
- R⁷ and R⁸: each represent a hydrogen atom;
- R⁹ and R¹⁰: each represent a hydrogen atom;
- R¹¹: represents an alkyl radical selected from the group consisting of methyl and n-butyl; or an unsubstituted phenyl or pyridinyl radical and
- R¹²: represents an unsubstituted phenyl radical;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Particularly preferred are nitro-substituted phenyl-piperazine compounds selected from the group consisting of
[1] [5-(4-Methyl-piperazin-1-yl)-2-nitro-phenyl]-(2-pyrrol-1-yl-ethyl)-amine,
[2] (4-Fluoro-benzyl)-[5-(4-methyl-piperazin-1-yl)-2-nitro-phenyl]-amine,
[3] N-[5-(4-Methyl-piperazin-1-yl)-2-nitro-phenyl]-benzamide,
[4] N-Methyl-N-[5-(4-methyl-piperazin-1-yl)-2-nitro-phenyl]-benzamide,
[5] [5-(4-Methyl-piperazin-1-yl)-2-nitro-phenyl]-pyrrol-1-yl-amine,
[6] 2-[5-(4-Methyl-piperazin-1-yl)-2-nitro-phenyl]-2H-pyridazin-3-one,
[7] 1-Methyl-4-(4-nitro-3-phenoxy-phenyl)-piperazine,
[8] Benzyl-[5-(4-butyl-piperazin-1-yl)-2-nitro-phenyl]-amine,
[9] Benzyl-[2-nitro-5-(4-pyridin-2-yl-piperazin-1-yl)-phenyl]-amine and
[10] Benzyl-[2-nitro-5-(4-phenyl-2-yl-piperazin-1-yl)-phenyl]-amine;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Another aspect of the present invention relates to a process for the preparation of a nitro-substituted phenyl-piperazine compound of general formula I wherein X represents -NR¹R², wherein at least one nitrobenzene compound of general formula II, 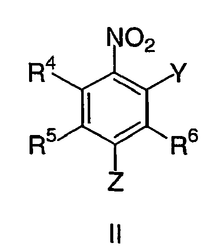 wherein R⁴ to R⁶ have any of the above given meanings, Y represents a chlorine atom, and Z represents a bromine or iodine atom; is reacted with at least one compound of general formula III, 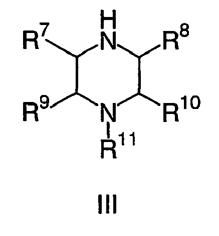 wherein R⁷ to R¹¹ have any of the above given meaningsin a suitable reaction medium, preferably in at least an organic solvent, more preferably in at least an organic solvent selected from the group consisting of tetrahydrofuran, toluene and dioxane, preferably in the presence of at least one catalyst, more preferably in the presence of at least a palladium source, even more preferably in the presence of PdCI₂(dppf) wherein dppf is 1,1-bis(diphenylphosphino)-ferrocene, and/or at least one auxiliary agent, preferably 1,1-bis(diphenylphosphino)-ferrocene, and/or at least one base, preferably sodium tert-pentoxide, to yield a compound of general formula IV, 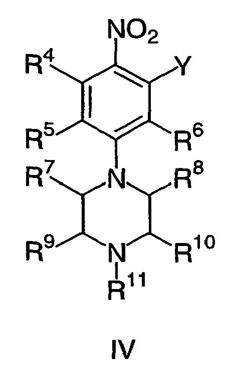 wherein R⁴ to R¹¹ have any of the above given meanings and Y represents a chlorine atom; which is optionally purified and/or isolated, and the compound of general formula IV is reacted with at least one compound of general formula V, 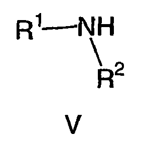 wherein R¹ and R² have any of the above given meanings, in a suitable reaction medium, preferably in at least an organic solvent, more preferably in at least an organic solvent selected from the group consisting of toluene or dimethoxyethane, preferably in the presence of at least one catalyst, more preferably in the presence of at least a palladium source, even more preferably in the presence of at least a palladium source selected from the group consisting of Pd(OAc)₂, wherein OAc is acetate, and Pd₂dba₃, wherein dba is dibenzylidene acetone, and/or at least one auxiliary agent, preferably (biph)P(*t*Bu)₂, wherein biph is biphenyl and *t*Bu is *tert-*butyl, and/or at least one base, preferably at least one base selected from the group consisting of K₃PO₄ and sodium *tert-*pentoxide to yield a compound of general formula VI, 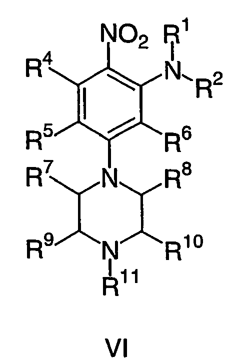 wherein R¹, R² and R⁴ to R¹¹ have any of the above given meanings which is optionally purified and/or isolated.

In another aspect the invention relates to a process for the preparation of a nitro-substituted phenyl-piperazine compound of general formula I wherein X represents -NR¹R², wherein at least one nitrobenzene compound of general formula VII, 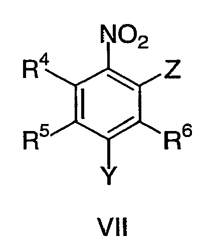 wherein R⁴ to R⁶ have any of the above given meanings, Z represents a bromine or iodine atom, and Y represents a chlorine atom, is reacted with at least one compound of general formula V, 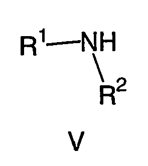 wherein R¹ and R² have any of the above given meaningsin a suitable reaction medium, preferably in at least an organic solvent, more preferably in at least an organic solvent selected from the group consisting of toluene and dioxane, preferably in the presence of at least one catalyst, more preferably in the presence of at least a palladium and/or copper source, even more preferably in the presence of at least a palladium and/or copper source selected from the group consisting of Pd(OAc)₂, wherein OAc is acetate, Pd₂dba₃, wherein dba is dibenzylidene acetone, and copper(I)iodide, and/or at least one auxiliary agent, preferably at least one auxiliary agent selected from the group consisting of 4,5-bis(diphenylphosphino)-9,9-dimethylxanthene (Xantphos), 1,1-bis(diphenylphosphino-ferrocene and P(*t*Bu)₃, wherein *t*Bu is tert-Butyl, and/or at least one base, preferably at least one base selected from the group consisting of K₃PO₄, CS₂CO₃ and trans-1,2-diaminomethylcyclohexane, to yield a compound of general formula VIII, 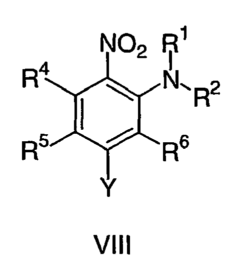 wherein R¹, R² and R⁴ to R⁶ have any of the above given meanings and Y represents a chlorine atom; which is optionally purified and/or isolated, and the compound of general formula VIII is reacted with at least one compound of general formula III, 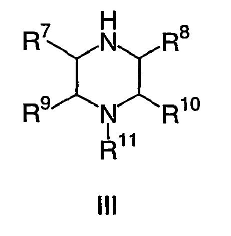 wherein R⁷ to R¹¹ have any of the above given meanings, in a suitable reaction medium, preferably in at least an organic solvent, more preferably in at least an organic solvent selected from the group consisting of toluene, tetrahydrofuran and dimethoxyethane, preferably in the presence of at least one catalyst, more preferably in the presence of at least a palladium source, even more preferably in the presence of at least a palladium source selected from the group consisting of Pd(OAc)₂, wherein OAc is acetate, and Pd₂dba₃, wherein dba is dibenzylidene acetone, and/or at least one auxiliary agent, preferably (biph)P(*t*Bu)₂, wherein biph is biphenyl and tBu is tert-butyl, and/or at least one base, preferably at least one base selected from the group consisting of K3PO₄ or sodium *tert*-pentoxide, to yield a compound of general formula VI, 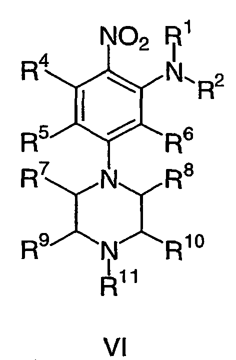 wherein R¹, R² and R⁴ to R¹¹ have any of the above given meanings, which is optionally purified and/or isolated.

Another aspect of the present invention relates to a process for the preparation of a nitro-substituted phenyl-piperazine compound of general formula I wherein X represents -OR³, wherein at least one nitrobenzene compound of general formula II, 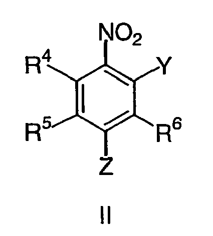 wherein R⁴ to R⁶ have any of the above given meanings, Y represents a chlorine atom, and Z represents a bromine or iodine atom; is reacted with at least one compound of general formula III, 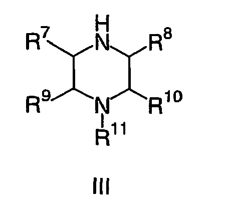 wherein R⁷ to R¹¹ have any of the above given meanings, in a suitable reaction medium, preferably in at least an organic solvent, more preferably in at least an organic solvent selected from the group consisting of tetrahydrofuran or dioxane, preferably in the presence of at least one catalyst, more preferably in the presence of at least a palladium source, even more preferably in the presence of PdCl₂(dppf), wherein dppf is 1,1-bis(diphenylphosphino)-ferrocene, and/or at least one auxiliary agent, preferably 1,1-bis(diphenylphosphino)-ferrocene, and/or at least one base, preferably sodium *tert*-pentoxide, to yield a compound of general formula IV, 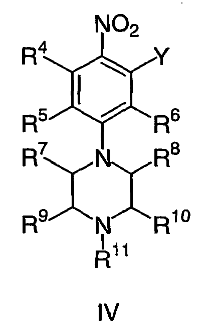 wherein R⁴ to R¹¹ have any of the above given meanings and Y represents a chlorine atom; which is optionally purified and/or isolated, and the compound of general formula IV is reacted with at least one compound of general formula IX, 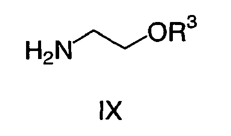 wherein R³ has any of the above given meanings, in a suitable reaction medium, preferably in at least an organic solvent, more preferably in at least an organic solvent selected from the group consisting of toluene or dimethoxyethane, preferably in the presence of at least one catalyst, more preferably in the presence of at least a palladium source, even more preferably in the presence of at least a palladium source selected from the group consisting of Pd(OAc)₂, wherein OAc is acetate, and Pd₂dba₃, wherein dba is dibenzylidene acetone, and/or at least one auxiliary agent, preferably (biph)P(*t*Bu)₂, wherein biph is biphenyl and *t*Bu is *tert*-butyl, and/or at least one base, preferably at least one base selected from the group consisting of K₃PO₄ and sodium *tert*-pentoxideto yield a compound of general formula X, 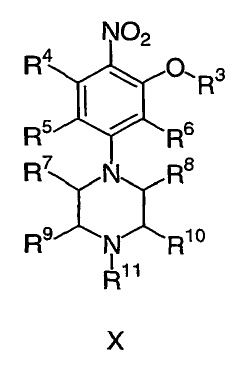 wherein R³ to R¹¹ have any of the above given meanings, which is optionally purified and/or isolated.

Suitable reaction media include organic solvents, such as dialkyl ether, preferably diethyl ether and dimethoxyethane, or a cyclic ether, preferably tetrahydrofuran or dioxane; or a halogenated hydrocarbon, preferably dichloromethane or chloroform; an alcohol, preferably methanol or ethanol; an aprotic solvent, preferably acetonitrile, toluene, pyridine or dimethylformamide, or any other suitable reaction medium. Of course, mixtures of at least two classes of solvents or of at least two solvents of one class may also be used.

All of above mentioned reactions are preferably carried out in an oven-dried vial. The catalyst, the auxiliary agent, the base and the compound of general formula II, IV, VII or VIII are added in each case and the vial is subsequently evacuated and purged with argon. The organic solvent and the compound of general formula III, V or IX are added and the reaction is carried out in a sealed vial at a temperature between 100 °C and 110 °C, preferably at 100 °C in case of tetrahydrofurane or toluene as the organic solvent and at 110 °C in case of dimethoxyethane and dioxane as the organic solvent.

Suitable reaction conditions for carrying out the reaction between compounds of general formula II, IV, VII or VIII and compounds of general formula III, V or IX are described in the references of J.F. Hartwig et al., J. Am. Chem. Soc. 1996, 118, 7217-7218; S.L. Buchwald et al., J. Am. Chem. Soc. 2002, 124, 6043-6048; S. L. Buchwald et al. J. Am. Chem. Soc. 2002, 124, 7241-7424 and S.L. Buchwald et al., J. Am. Chem. Soc. 2002, 124, 11684-11688. The respective part of the description is hereby incorporated by reference and forms part of the present disclosure.

The compounds of general formulas IV, VI, VIII and X given above may be purified and/or isolated according to methods well known to those skilled in the art.

The compounds of general formulas IV, VI, VIII and X may be isolated by evaporating the reaction medium, addition of water and adjusting the pH value to obtain the compound in form of a solid that can be isolated by filtration, or by extraction with a solvent that is not miscible with water such as chloroform and purification by chromatography or recrystallisation from a suitable solvent.

Preferably, the compounds of general formula IV, VI, VIII and X may be obtained by filtration of the reaction mixture and subsequent separation of the reaction mixture on a TLC plate. Alternatively, the compounds of general formula I may be isolated by addition of water and methanol to the reaction mixture, evaporating the reaction mixture and purifying the residue by preparative HPLC.

The compounds of general formula II and VII are commercially available or may be prepared according to methods well known in the art, for example, analogous to the methods described in the bibliography of A. McKillop et al., Tetrahedron 1987, 43, 1753. The respective part of the literature description cited above is hereby incorporated by reference and forms part of the disclosure.

The compounds of general formula III, V and IX are commercially available or may be prepared according to methods well known in the art.

During some synthetic reactions described above or while preparing the compounds of general formulas III, V, VI, IX or X the protection of sensitive or reactive groups may be necessary and/or desirable. This can be performed by using conventional protective groups like those described in Protective groups in Organic Chemistry, ed. J. F. W. McOmie, Plenum Press, 1973; T.W. Greene & P.G.M. Wuts and Protective Groups in Organic Chemistry, John Wiley & sons, 1991. The respective parts of the description is hereby incorporated by reference and forms part of the disclosure. The protective groups may be eliminated when convenient by means well-known to those skilled in the art.

If the nitro-substituted phenyl-piperazine compounds of general formula I are obtained in form of a mixture of stereoisomers, particularly enantiomers or diastereomers, said mixtures may be separated by standard procedures known to those skilled in the art, e.g. chromatographic methods or crystallization with chiral reagents.

The nitro-substituted phenyl-piperazine compounds of general formula I and in each case stereoisomers thereof may be obtained in form of a corresponding salt according to methods well known to those skilled in the art, e.g. by reacting said compound with at least one inorganic and/or organic acid, preferably in a suitable reaction medium. Suitable reaction media include, for example, any of the ones given above.

The term "salt" is to be understood as meaning any form of the nitro substituted phenyl-piperazine compounds of general formula I in which they assume an ionic form or are charged and are coupled with a counter-ion (a cation or anion) or are in solution. By this are also to be understood complexes of the active compound with other molecules and ions, in particular complexes which are complexed via ionic interactions.

The term "physiologically acceptable salt" is understood in particular, in the context of this invention, as salt (as defined above) formed either with a physiologically tolerated acid, that is to say salts of the particular active compound with inorganic or organic acids which are physiologically tolerated - especially if used on humans and/or mammals - or with at least one, preferably inorganic, cation which are physiologically tolerated - especially if used on humans and/or mammals. Examples of physiologically tolerated salts of particular acids are salts of: hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid picric acid and/or aspartic acid. Examples of physiologically tolerated salts of particular bases are salts of alkali metals and alkaline earth metals and with NH₄.

Solvates, preferably hydrates, of the nitro subsituted phenyl-piperazine compounds of general formula I or in each case of corresponding stereoisomers may also be obtained by standard procedures known to those skilled in the art.

The term "solvate" according to this invention is to be understood as meaning any form of the nitro substituted phenyl-piperazine compounds of general formula I in which they have attached to it via non-covalent binding another molecule (most likely a polar solvent) especially including hydrates and alcoholates, e.g. methanolate.

A further aspect of the present invention relates to a medicament comprising at least one nitro-substituted phenyl-piperazine compound of general formula Ia, 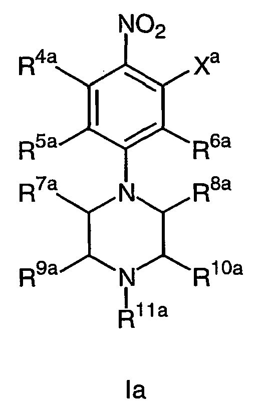 wherein
- X^{a}: represents a -NR^{1a}R^{2a} moiety or a -OR^{3a} moiety;
- R^{1a}: represents a linear or branched, saturated or unsaturated C₁₋₁₀ aliphatic radical which is substituted with 1, 2 or 3 substituent(s) independently selected from the group consisting of F, Cl, Br, I, -CN, -CF₃, -OCF₃, -SCF₃, -OH, -SH, -NH₂, - O-C₁₋₅-alkyl, -S-C₁₋₅-alkyl, -NH-(C₁₋₅-alkyl) and -N(C₁₋₅-alkyl)₂;
a saturated or unsaturated 3- to 9-membered cycloaliphatic radical, which may be substituted with 1, 2 or 3 substituent(s) independently selected from the group consisting of oxo (=O), thioxo (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which is bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, - CN, -CF₃, -OCF₃, -SCF₃, -OH, -SH, -NH₂, -O-C₁₋₅-alkyl, -S-C₁₋₅-alkyl, phenyl, - NH-(C₁₋₅-alkyl) and -N(C₁₋₅-alkyl)₂ and/or which may contain 1, 2 or 3 heteroatom(s) independently selected from the group consisting of nitrogen, oxygen and sulfur as ring member(s);
a 5- to 10-membered aryl or heteroaryl radical which may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, - NH₂, -NH(C₁₋₅-alkyl), -N(C₁-₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and whereby said heteroaryl radical contains 1, 2 or 3 heteroatom(s) independently selected from the group consisting of nitrogen, oxygen and sulfur as ring member(s);
a 5- to 10-membered aryl radical whereby said aryl radical may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-O-C₁₋₅-alkyl, -O- C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)2-C₁₋₅-alkyl, -S(=O)₂-phenyl, phenyl, phenoxy and benzyl and said aryl radical is bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, - OH, -SH, -NH₂, -O-C₁₋₅-alkyl, -S-C₁₋₅-alkyl, phenyl, -NH-(C₁₋₅-alkyl) and -N(C₁₋₅-alkyl)₂ and which may contain 1, 2 or 3 heteroatom(s) independently selected from the group consisting of nitrogen, oxygen and sulfur as chain member(s);
a 5- to 10-membered heteroaryl radical, which may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which is bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O- C₁₋₅-alkyl, -S-C₁₋₅-alkyl, phenyl, -NH-(C₁₋₅-alkyl) and -N(C₁₋₅-alkyl)₂ and which may contain 1, 2 or 3 heteroatom(s) independently selected from the group consisting of nitrogen, oxygen and sulfur as chain member(s) and whereby the heteroaryl radical contains 1, 2 or 3 heteroatom(s) independently selected from the group consisting of nitrogen, oxygen and sulfur as ring member(s);
or a -C(=O)-R^{12a} moiety;
- R^{2a} or: represents a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, - CN, -CF₃, -OCF₃, -SCF₃, -OH, -SH, -NH₂, -O-C₁₋₅-alkyl, -S-C₁₋₅-alkyl, -NH-(C₁₋₅-alkyl) and -N(C₁₋₅-alkyl)₂;
- R^{1a} and R^{2a}: together with the bridging nitrogen form a saturated, unsaturated or aromatic 3- to 6-membered heterocyclic ring which may be substituted with 1, 2 or 3 substituent(s) independently selected from the group consisting of C₁₋₅- alkyl, -O-C₁₋₅-alkyl, -S-C₁₋₅-alkyl, oxo (=O), thioxo (=S), -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alky), -N(C₁₋₅-alkyl)₂, -NO₂, -CHO, -CF₂H, - CFH₂, -C(=O)-NH₂, -C(=O)-NH(C₁₋₅-alkyl), -C(=O)-N(C₁₋₅-alkyl)₂, -S(=O)₂-C₁₋₅- alkyl, -S(=O)₂-phenyl, phenyl, phenoxy and benzyl and which may contain 1, 2 or 3 additional heteroatom(s) independently selected from the group consisting of nitrogen, oxygen and sulfur as ring member(s) and/or which may be condensed with a mono- or bicyclic ring system which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH, -C(=O)- C₁₋₅-alkyl, -C(=O)-O-C₁₋₅-alkyl, -O-C(=O)-C₁₋₅-alkyl, F, Cl, Br, I, -CN, -CF₃, - OCF₃, -SCF₃, -OH, -SH, -NH₂, -NH(C₁₋₅-alkyl), -N(C₁₋₅-alkyl)₂, -NO₂, -CHO, - CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl whereby said cyclic substituent(s) phenyl, phenoxy and benzyl may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and NO₂;
whereby the rings of the ring system are 5- 6- or 7-membered and may contain 1, 2 or 3 heteroatom(s) independently selected from the group consisting of nitrogen, oxygen and sulfur;
- R^{3a}: represents a 5- to 10-membered aryl or heteroaryl radical which may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅- alkyl, -C(=O)-O-C₁₋₅-alkyl, -O-C(=O)-C₁₋₅-alkyl, F, Cl, Br, I, -CN, -CF₃, -OCF₃, - SCF₃, -OH, -SH, -NH₂, -NH(C₁₋₅-alkyl), -N(C₁₋₅-alkyl)₂, -NO₂, -CHO, -CF₂H, - CFH₂, -C(=O)-NH₂, -C(=O)-NH(C₁₋₅-alkyl), -C(=O)-N(C₁₋₅-alkyl)₂, -S(=O)₂-C₁₋₅- alkyl, -S(=O)₂-phenyl, phenyl, phenoxy and benzyl and which may be bonded via a linear or branched C₆ alkylene, C₂₋₆ alkenylene or C₂₋₆ alkinylene group and which may be unsubstituted or substituted with 1, 2 or 3 substituent(s) independently selected from the group consisting of F, Cl, Br, I, -CN, -CF₃, - OCF₃, -SCF₃, -OH, -SH, -NH₂, -O-C₁₋₅-alkyl, -S-C₁₋₅-alkyl, -NH-(C₁₋₅-alkyl) and -N(C₁₋₅-alkyl)₂ and whereby the heteroaryl radical contains 1, 2 or 3 heteroatom(s) independently selected from the group consisting of nitrogen, oxygen and sulfur as ring member(s);
- R^{4a}, R^{5a} and R^{6a},: identical or different, each represent a hydrogen atom or a halogen atom;
or
- R^{4a} and R^{5a}: together with the bridging carbon atoms form an unsubstituted 5- or 6- membered heterocyclic ring which contains 1, 2 or 3 heteroatom(s) independently selected from the group consisting of nitrogen, oxygen and sulfur as ring member(s) and which together with the phenyl ring which it is fused with forms a 9- or 10-membered bicyclic aromatic ring system;
- R^{7a} and R^{8a},: identical or different, each represent a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, - OH, -SH, -NH₂, -O-C₁₋₅-alkyl, -S-C₁₋₅-alkyl, -NH-(C₁₋₅-alkyl) and -N(C₁₋₅-alkyl)₂;
- R^{9a} and R^{10a},: identical or different, each represent a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, - OH, -SH, -NH₂, -O-C₁₋₅-alkyl, -S-C₁₋₅-alkyl, -NH-(C₁₋₅-alkyl) and -N(C₁-₅-alkyl)₂;
- R^{11a}: represents a hydrogen atom; a linear or branched, saturated or unsaturated C₁₋₁₀ aliphatic radical which may be unsubstituted or substituted with 1, 2 or 3 substituent(s) independently selected from the group consisting of F, Cl, Br, I, - CN, -CF₃, -OCF₃, -SCF₃, -OH, -SH, -NH₂, -O-C₁₋₅-alkyl, -S-C₁₋₅-alkyl, -NH-(C₁₋₅-alkyl) and -N(C₁₋₅-akyl)₂ and which may contain 1, 2 or 3 heteroatom(s) independently selected from the group consisting of nitrogen, oxygen and sulfur as chain member(s);
a 5- to 10-membered aryl or heteroaryl radical which may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, - NH₂, -NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may be bonded via a linear or branched, C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, - OH, -SH, -NH₂, -O-C₁₋₅-alkyl, -S-C₁₋₅-alkyl, -NH-(C₁₋₅-alkyl) and -N(C₁₋₅-alkyl)₂ and whereby the heteroaryl radical contains 1, 2 or 3 heteroatom(s) independently selected from the group consisting of nitrogen, oxygen and sulfur as ring member(s); a -C(=O)-R^{13a} moiety or a -S(=O)₂-R^{14a} moiety;
- R^{12a}: represents a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, - OCF₃, -SCF₃, -OH, -SH, -NH₂, -O-C₁₋₅-alkyl, -S-C₁₋₅-alkyl, -NH-(C₁₋₅-alkyl) and -N(C₁₋₅-alkyl)₂;
or a 5- to 10-membered aryl or heteroaryl radical, which may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)- O-C₁₋₅-alkyl, -O-C(=O)-C₁₋₅-alkyl, F, Cl, Br, I, -CN, -CF₃, -OCF₃, -SCF₃, -OH, - SH, -NH₂, -NH(C₁₋₅-alkyl), -N(C₁₋₅-alkyl)₂, -NO₂, -CHO, -CF₂H, -CFH₂, -C(=O)- NH₂, -C(=O)-NH(C₁₋₅-alkyl), -C(=O)-N(C₁₋₅-alkyl)₂, -S(=O)₂-C₁₋₅-alkyl, -S(=O)₂- phenyl, phenyl, phenoxy and benzyl and which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, - OH, -SH, -NH₂, -O-C₁₋₅-alkyl, -S-C₁₋₅-alkyl, -NH-(C₁₋₅-alkyl) and -N(C₁₋₅-alkyl)₂ and whereby the heteroaryl radical contains 1, 2 or 3 heteroatom(s) independently selected from the group consisting of nitrogen, oxygen and sulfur as ring member(s);
and
- R^{13a} and R^{14a},: identical or different, each represent a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅- alkyl, -NH-(C₁₋₅-alkyl) and -N(C₁₋₅-alkyl)₂;
or a 5- to 10-membered aryl or heteroaryl radical, which may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)- O-C₁₋₅-alkyl, -O-C(=O)-C₁₋₅-alkyl, F, Cl, Br, I, -CN, -CF₃, -OCF₃, -SCF₃, -OH, - SH, -NH₂, -NH(C₁₋₅-alkyl), -N(C₁₋₅-alkyl)₂, -NO₂, -CHO, -CF₂H, -CFH₂, -C(=O)- NH₂, -C(=O)-NH(C₁₋₅-alkyl), -C(=O)-N(C₁₋₅-alkyl)₂, -S(=O)₂-C₁₋₅-alkyl, -S(=O)₂- phenyl, phenyl, phenoxy and benzyl and/or which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, - OH, -SH, -NH₂, -O-C₁₋₅-alkyl, -S-C₁₋₅-alkyl, -NH-(C₁₋₅-alkyl) and -N(C₁₋₅-alkyl)₂ and whereby the heteroaryl radical contains 1, 2 or 3 heteroatom(s) independently selected from the group consisting of nitrogen, oxygen and sulfur as ring member(s).
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof;
and optionally at least one physiologically compatible auxiliary agent.

Also preferred are medicaments comprising at least one nitro-substituted phenyl piperazine compound of general formula la, wherein
R^{1a} represents an alkyl radical selected from the group consisting of methyl, ethyl, n-propyl, isopropyl, n-butyl, sec-butyl, isobutyl and tert-butyl which is substituted with 1, 2 or 3 substituent(s) independently selected from the group consisting of F, Cl, Br, I, - CN, -CF₃, -OCF₃, -SCF₃, -OH, -SH and -NH₂;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl;
an aryl radical selected from the group consisting of phenyl and naphthyl which may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and said aryl radical is bonded via a linear or branched C₁₋₆ alkylene group which may contain 1, 2 or 3 oxygen atom(s) as chain member(s) and which may be substituted with 1 phenyl radical;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said heteroaryl radical may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, - NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and said heteroaryl radical is bonded via a linear or branched C₁₋₆ alkylene group which may contain 1, 2 or 3 oxygen atom(s) as chain member(s);
or a -C(=O)-R^{12a} moiety;
preferably R^{1a} represents an alkyl radical selected from the group consisting of methyl, ethyl, n-propyl and isopropyl, whereby said alkyl radical is substituted with 1, 2 or 3 substituent(s) independently selected from the group consisting of -OH, F, Cl, Br and -CN;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said aryl or heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -SH, - NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
an aryl radical selected from the group consisting of phenyl and naphthyl whereby said aryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, - CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, -C(=O)-NH₂, - C(=O)-NH-CH₃, -C(=O)-NH-C₂H₅, -C(=O)-N(CH₃)₂, -C(=O)-N(C₂H₅)₂ and -S(=O)₂-CH₃ and/or is bonded via an alkylene group selected form the group consisting of - CH₂-, -CH(CH₃)-, -CH(Phenyl)-, -CH₂-CH₂-, -CH₂-CH₂-CH₂- and -CH₂-CH₂-O-;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, - C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or is bonded via an alkylene group selected form the group consisting of -CH₂-, -CH(CH₃)-, -CH₂-CH₂-, -CH₂-CH₂-CH₂- and -CH₂-CH₂-O-;
or a -C(=O)-R^{12a} moiety;
more preferably R^{1a} represents an alkyl radical selected from the group consisting of -CH₂-CH₂-OH and -CH₂-CH₂-CH₂-OH;
an unsubstituted phenyl or pyrrolyl radical;
a phenyl radical which may be substituted with 1, 2 or 3 substituent(s) independently selected from the group consisting of methyl, ethyl, tert-butyl, methoxy, F and Cl and said phenyl radical is bonded via an alkylene group selected form the group consisting of -CH₂-, -CH(CH₃)-, -CH(Phenyl)-, -CH₂-CH₂-, -CH₂-CH₂-CH₂- and -CH₂-CH₂-O-;
a heteroaryl radical selected from the group consisting of pyridinyl, furanyl and pyrrolyl, whereby said pyridinyl, furanyl or pyrrolyl radical may be substituted with 1, 2 or 3 substituent(s) independently selected from the group consisting of methyl, ethyl, tert-butyl, methoxy, F and Cl and said pyridinyl, furanyl or pyrrolyl radical is bonded via an alkylene group selected form the group consisting of -CH₂-, -CH(CH₃)-, -CH₂-CH₂-, -CH₂-CH₂-CH₂- and -CH₂-CH₂-O-;
or a -C(=O)-R^{12a} moiety;
and X^{a} and R^{2a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Furthermore, such medicaments comprising at least one nitro-substituted phenyl-piperazine compound of general formula la are preferred, wherein
R^{2a} represents a hydrogen atom or a linear or branched, unsubstituted C₁₋₁₀ alkyl radical;
preferably R^{2a} represents a hydrogen atom or an alkyl radical selected from the group consisting of methyl, ethyl, n-propyl, isopropyl, n-butyl, sec-butyl, isobutyl and tert-butyl;
more preferably R^{2a} represents a hydrogen atom or a methyl radical;
and X^{a}, R^{1a} and R^{3a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are medicaments comprising at least one nitro-substituted phenyl piperazine compound of general formula la, wherein
R^{1a} and R^{2a} together with the bridging nitrogen atom form a moiety selected from the group consisting of 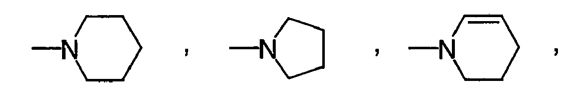 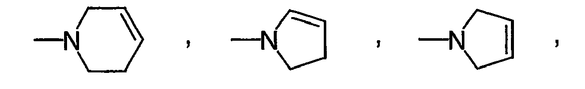 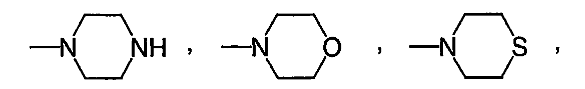 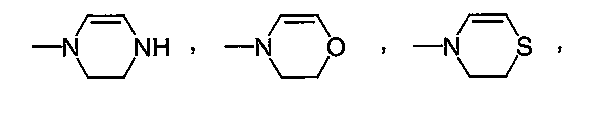 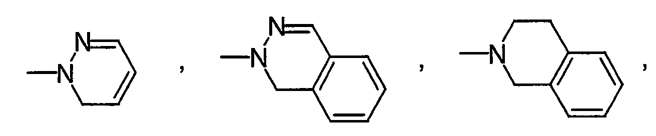 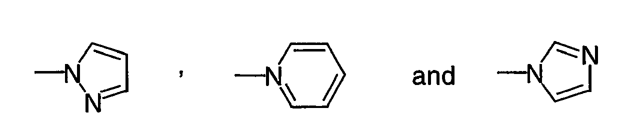 whereby each of these afore mentioned cyclic moieties may be substituted in any position including the -NH-groups with 1, 2 or 3 substituent(s) independently selected from the group consisting of C₁₋₅-alkyl, -O-C₁₋₅-alkyl, -S-C₁₋₅-alkyl, -C(=O)-OH, oxo (=O), thioxo (=S), -C(=O)-O-C₁₋₅-alkyl, -O-C(=O)-C₁₋₅-alkyl, F, Cl, Br, I, -CN, -CF₃, - OCF₃, -SCF₃, -OH, -SH, -NH₂, -NH(C₁-₅-alkyl), -N(C₁₋₅-alkyl)₂, -NO₂, -CHO, -CF₂H, - CFH₂, -C(=O)-NH₂, -C(=O)-NH(C₁₋₅-alkyl), -C(=O)-N(C₁₋₅-alk_{Y}I)₂, -S(=O)₂-C₁₋₅-alkyl, - S(=O)₂-phenyl, phenyl, phenoxy and benzyl; whereby said cyclic substituent(s) phenyl, phenoxy and benzyl may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH,-NH₂ and NO₂;
preferably R^{1a} and R^{2a} together with the bridging nitrogen atom form a moiety selected from the group consisting of 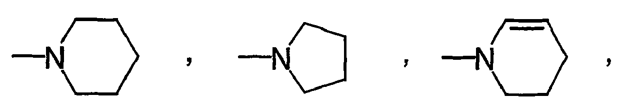 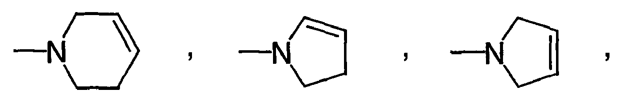 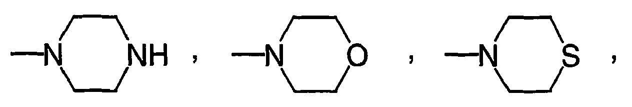 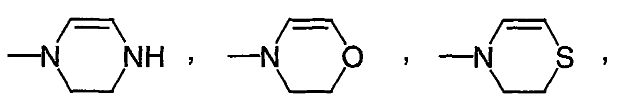 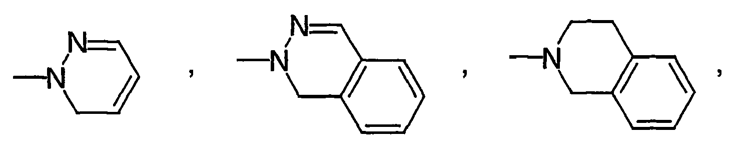 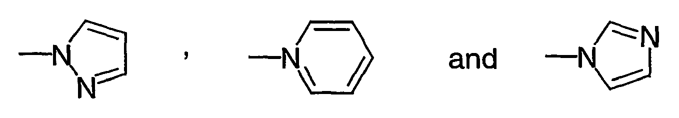 whereby each of these afore mentioned cyclic moieties may be substituted with 1, 2 or 3 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, - C(=O)-O-CH₃, -C(=O)-O-C₂H₅, -C(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, - NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=0)₂-CH₃, -S(=O)₂-phenyl, phenyl, phenoxy and benzyl; whereby said cyclic substituent(s) phenyl, phenoxy and benzyl may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and NO₂;
more preferably R^{1a} and R^{2a} together with the bridging nitrogen atom form a moiety selected from the group consisting of 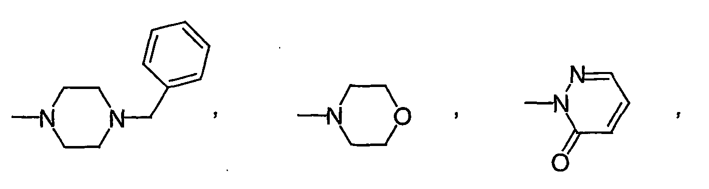 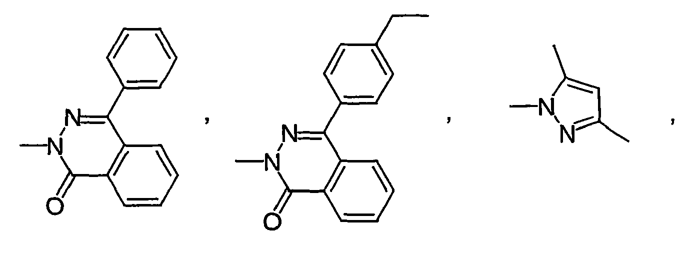 and 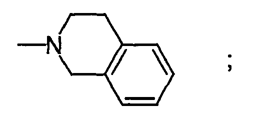 and X^{a} and R^{3a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are medicaments comprising at least one nitro-substituted phenyl-piperazine compound of general formula la, wherein
R^{3a} represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said aryl or heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -SH, -NH₂, -NH-CH₃, -NH-C₂H₅, -NH-CH₂-CH₂-CH₃, -NH-CH(CH₃)₂, - NH-C(CH₃)₃, -N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
preferably R^{3a} represents a phenyl radical which may be substituted with 1, 2 or 3 substituent(s) independently selected from the group consisting of methyl, ethyl, n-propyl, isopropyl, n-butyl, tert-butyl, -O-CH₃, -O-C₂H₅, -O-CH₂-CH₂-CH₃, - -C(=O)-OH, -C(=O)-O-CH₃, -C(=O)-O-C₂H₅, -C(=O)-CH₃, -C(=O)-C₂H₅, F, Cl, Br, I, -CN, -CF₃, - OCF₃, -SCF₃, -SH, -NH₂, -NH-CH₃, -NH-C₂H₅, -NH-CH₂-CH₂-CH₃, -NH-CH(CH₃)₂, - NH-C(CH₃)₃, -N(CH₃)₂, -N(C₂H₅)₂, -CHO, -CF₂H, -CFH₂ and -S(=O)₂-CH₃;
more preferably R^{3a} represents an unsubstituted phenyl radical;
and X^{a}, R^{1a}, R^{2a} and R^{4a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Moreover, such medicaments comprising at least one nitro-substituted phenyl-piperazine compound of general formula la are preferred, wherein
R^{4a}, R^{5a} and R^{6a}, identical or different, each represent a hydrogen atom or a halogen atom selected from the group consisting of F, Cl and Br;
preferably R^{4a}, R^{5a} and R^{6a}, identical or different, each represent a hydrogen atom or a fluorine atom;
and X^{a}, R^{1a} to R^{3a} and R^{7a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Furthermore, such medicaments comprising at least one nitro-substituted phenyl-piperazine compound of general formula la are preferred, whereinR^{4a} and R^{5a} together with the bridging carbon atoms form a moiety selected from the group consisting of 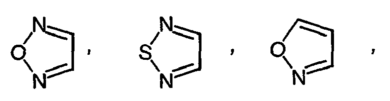 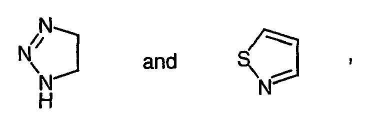 which together with the phenyl ring which it is fused with forms a substituted bicyclic aromatic ring system selected from the group consisting of 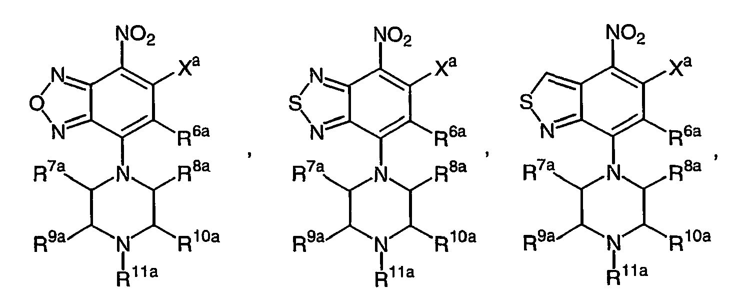 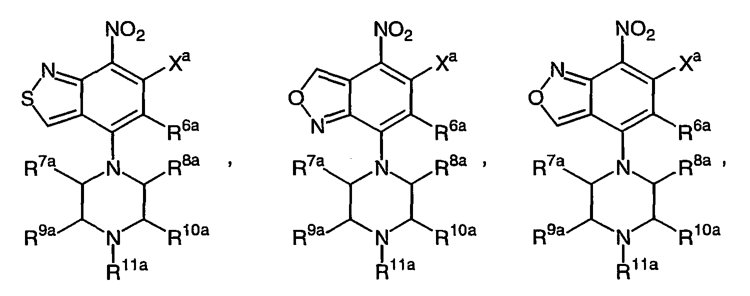 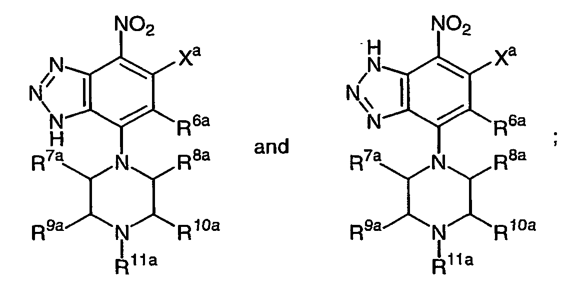 preferably R^{4a} and R^{5a} together with the bridging carbon atoms form the following moiety, 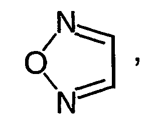 which together with the phenyl ring which it is fused with forms the following substituted bicyclic aromatic ring system 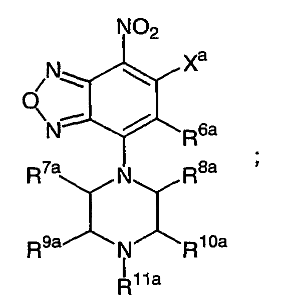 and X^{a}, R^{1a} to R^{3a} and R^{6a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl-piperazine compound of general formula la, wherein
R^{7a} and R^{8a}, identical or different, each represent a hydrogen atom or a linear or branched, unsubstituted C₁₋₁₀ alkyl radical;
preferably R^{7a} and R^{8a}, identical or different, each represent a hydrogen atom or an alkyl radical selected from the group consisting of methyl, ethyl, n-propyl, isopropyl, n-butyl, sec-butyl, isobutyl and tert-butyl;
more preferably R^{7a} and R^{8a} each represent a hydrogen atom;
and X^{a}, R^{1a} to R^{6a} and R^{9a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl-piperazine compound of general formula la, wherein
R^{9a} and R^{10a}, identical or different, each represent a hydrogen atom or a linear or branched, unsubstituted C₁₋₁₀ alkyl radical;
preferably R^{9a} and R^{10a}, identical or different, each represent a hydrogen atom or an alkyl radical selected from the group consisting of methyl, ethyl, n-propyl, isopropyl, n-butyl, sec-butyl, isobutyl and tert-butyl;
more preferably R^{9a} and R^{10a}, identical or different, each represent a hydrogen atom or a methyl radical;
and X^{a}, R^{1a} to R^{8a} and R^{11a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl piperazine compound of general formula la, wherein
R^{11a} represents a hydrogen atom;
an alkyl radical selected from the group consisting of methyl, ethyl, n-propyl, isopropyl, n-butyl, sec-butyl, isobutyl and tert-butyl whereby said alkyl radical may be substituted with 1, 2 or 3 substituent(s) independently selected from the group consisting of -OH, F, Cl, Br and -CN;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical
may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and/or may be bonded via a -(CH₂)_{1,2 or 3}- group;
a -C(=O)-R^{13a} moiety or a -S(=O)₂-R^{14a} moiety;
preferably R^{11a} represents a hydrogen atom;
an alkyl radical selected from the group consisting of methyl, ethyl, n-propyl, isopropyl, n-butyl, sec-butyl, isobutyl and tert-butyl whereby said alkyl radical may be substituted with 1, 2 or 3 substituent(s) independently selected from the group consisting of -OH, F, Cl, Br and -CN;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyridinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or may be bonded via a -(CH₂)₁, _{2 or 3}-group;
a -C(=O)-R^{13a} moiety or a -S(=O)₂-R^{14a} moiety;
more preferably R^{11a} represents a hydrogen atom;
an alkyl radical selected from the group consisting of methyl, n-butyl and -CH₂-CH₂-OH;
an unsubstituted phenyl or pyridinyl radical whereby said phenyl or pyridinyl radical may be bonded via a -(CH₂)- group;
a -C(=O)-R^{12a} moiety or a-S(=O)₂-R^{13a} moiety;
and X^{a}, R^{1a} to R^{10a} and R^{12a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl-piperazine compound of general formula la, wherein
R^{12a} represents an alkyl radical selected from the group consisting of methyl, ethyl, n-propyl, isopropyl, n-butyl, sec-butyl, isobutyl and tert-butyl;
or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and/or may be bonded via a -(CH₂)_{1,2 or 3}- group;
preferably R^{12a} represents an alkyl radical selected from the group consisting of methyl, ethyl, n-propyl and isopropyl,
or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said aryl or heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -SH, - NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or may be bonded via a -(CH₂)- group;
more preferably R^{12a} represents a phenyl or a thiophenyl radical whereby said phenyl or thiophenyl radical may be substituted with 1, 2 or 3 substituent(s) selected from the group consisting of methyl and chlorine;
and X^{a}, R^{1a} to R^{11a}, R^{13a} and R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl-piperazine compound of general formula la, wherein
R^{13a} represents an alkyl radical selected from the group consisting of methyl, ethyl, n-propyl, isopropyl, n-butyl, sec-butyl, isobutyl and tert-butyl whereby said alkyl radical may be substituted with 1, 2 or 3 substituent(s) independently selected from the group consisting of -OH, F, Cl, Br and -CN;
or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyridinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or may be bonded via a -(CH₂)_{1,2} or ₃-group;
preferably R^{13a} represents an alkyl radical selected from the group consisting of methyl, ethyl, n-propyl, isopropyl, n-butyl, sec-butyl, isobutyl and tert-butyl;
or an aryl or heteroaryl radical selected from the group consisting of phenyl, and thienyl (thiophenyl), whereby said aryl or heteroaryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃ and/or may be bonded via a -(CH₂)₁, _{2 or 3}-group;
more preferably R^{13a} represents a methyl radical or a phenyl or a thiophenyl radical whereby said phenyl or thiophenyl radical may be substituted with 1, 2 or 3 substituent(s) selected from the group consisting of methyl and chlorine;
and X^{a}, R^{1a} to R^{12a} and R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl-piperazine compound of general formula la, wherein
R^{14a} represents an alkyl radical selected from the group consisting of methyl, ethyl, n-propyl, isopropyl, n-butyl, sec-butyl, isobutyl and tert-butyl;
or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and/or may be bonded via a -(CH₂)_{1,2 or 3}- group;
preferably R^{14a} represents an alkyl radical selected from the group consisting of methyl, ethyl, n-propyl and isopropyl,
or a phenyl radical, which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(-O)-CH₂-CH₂-CH₃, -C(=O)-CH(CH₃)₂, -C(=O)-C(CH₃)₃, F, Cl, Br, I, -CN, -CF₃, -OCF₃, -SCF₃, -SH, - NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
more preferably R^{14a} represents a methyl radical or a phenyl radical which may be substituted with 1, 2 or 3 methyl radical(s).

Also preferred are medicaments comprising at least one nitro-substituted phenyl-piperazine compound of general formula la, wherein
X^{a} represents a -NR^{1a}R^{2a} moiety or a -OR^{3a} moiety;
R^{1a} represents an alkyl radical selected from the group consisting of -CH₂-CH₂-OH and -CH₂-CH₂-CH₂-OH;
an unsubstituted phenyl or pyrrolyl radical;
a phenyl radical which may be substituted with 1, 2 or 3 substituent(s) independently selected from the group consisting of methyl, ethyl, tert-butyl, methoxy, F and Cl and said phenyl radical is bonded via an alkylene group selected form the group consisting of -CH₂-, -CH(CH₃)-, -CH(Phenyl)-, -CH₂-CH₂-, -CH₂-CH₂-CH₂- and -CH₂-CH₂-O-;
a heteroaryl radical selected from the group consisting of pyridinyl, furanyl and pyrrolyl, whereby said pyridinyl, furanyl or pyrrolyl radical may be substituted with 1, 2 or 3 substituent(s) independently selected from the group consisting of methyl, ethyl, tert-butyl, methoxy, F and Cl and said pyridinyl, furanyl or pyrrolyl radical is bonded via an alkylene group selected form the group consisting of -CH₂-, -CH(CH₃)-, -CH₂-CH₂-, -CH₂-CH₂-CH₂- and -CH₂-CH₂-O-;
or a -C(=O)-R^{12a} moiety;
R^{2a} represents a hydrogen atom or a methyl radical;
or
R^{1a} and R^{2a} together with the bridging nitrogen atom form a moiety selected from the group consisting of 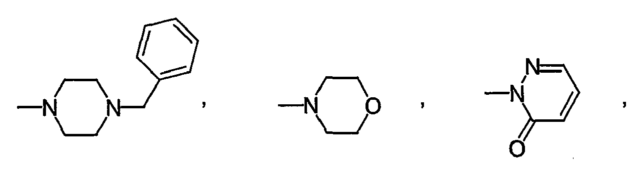 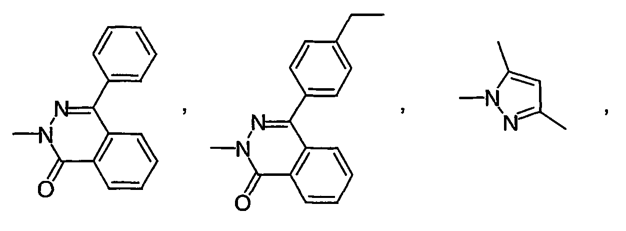 and 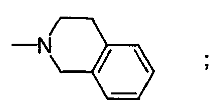 R^{3a} represents an unsubstituted phenyl radical;
R^{4a}, R^{5a} and R^{6a}, identical or different, each represent a hydrogen atom or a fluorine atom;
or
R^{4a} and R^{5a} together with the bridging carbon atoms form the following moiety, 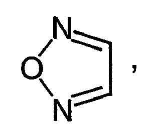 which together with the phenyl ring which it is fused with forms the following substituted bicyclic aromatic ring system 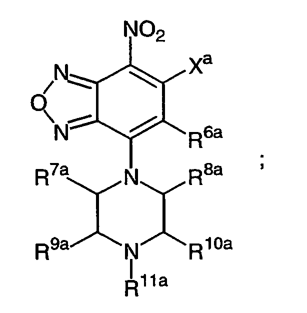 R^{7a} and R^{8a} each represent a hydrogen atom;
R^{9a} and R^{10a}, identical or different, each represent a hydrogen atom or a methyl radical;
R^{11a} represents a hydrogen atom;
an alkyl radical selected from the group consisting of methyl, n-butyl and -CH₂-CH₂-OH;
an unsubstituted phenyl or pyridinyl radical whereby said phenyl or pyridinyl radical may be bonded via a -(CH₂)- group;
a -C(=O)-R^{12a} moiety or a-S(=O)2-R^{13a} moiety;
R^{12a} represents a phenyl or a thiophenyl radical whereby said phenyl or thiophenyl radical may be substituted with 1, 2 or 3 substituent(s) selected from the group consisting of methyl and chlorine;
R^{13a} represents a methyl radical or a phenyl or a thiophenyl radical whereby said phenyl or thiophenyl radical may be substituted with 1, 2 or 3 substituent(s) selected from the group consisting of methyl and chlorine
and
R^{14a} represents a methyl radical or a phenyl radical which may be substituted with 1, 2 or 3 methyl radical(s);
optionally in form a physiologically acceptable salt thereof, or a corresponding solvate thereof.

Particularly preferred are medicaments comprising at least one nitro-substituted phenyl-piperazine compound of general formula la selected from the group consisting of
[1] [5-(4-Methyl-piperazin-1-yl)-2-nitro-phenyl]-(2-pyrrol-1-yl-ethyl)-amine,
[2] (4-Fluoro-benzyl)-[5-(4-methyl-piperazin-1-yl)-2-nitro-phenyl]-amine,
[3] N-[5-(4-Methyl-piperazin-1-yl)-2-nitro-phenyl]-benzamide,
[4] N-Methyl-N-[5-(4-methyl-piperazin-1-yl)-2-nitro-phenyl]-benzamide,
[5] [5-(4-Methyl)-piperazin-1-yl)-2-nitro-phenyl]-pyrrol-1-yl-amine,
[6] 2-[5-(4-Methyl-piperazin-1-yl)-2-nitro-phenyl]-2H-pyridazin-3-one,
[7] 1-Methyl-4-(4-nitro-3-phenoxy-phenyl)-piperazine,
[8] Benzyl-[5-(4-butyl-piperazin-1-yl)-2-nitro-phenyl]-amine,
[9] Benzyl-[2-nitro-5-(4-pyridin-2-yl-piperazin-1-yl)-phenyl]-amine and
[10] Benzyl-[2-nitro-5-(4-phenyl-2-yl-piperazin-1-yl)-phenyl]-amine;
[11] Furan-2-ylmethyl-[5-(4-methyl-piperazin-1-yl)-2-nitro-phenyl]-amine,
[12] 2-[4-(4-Nitro-3-phenethylamino-phenyl)-piperazin-1-yl]-ethanol,
[13] (2-Nitro-5-piperazin-1-yl-phenyl)-(2-o-tolyloxy-ethyl)-amine
[14] 2-[4-(3-Benzylamino-4-nitro-phenyl)-piperazin-1-yl]-ethanol,
[15] 4-[5-(4-Methyl-piperazin-1-yl)-2-nitro-phenyl]-morpholine,
[16] 2-[5-(4-Methyl-piperazin-1-yl)-2-nitro-phenyl]-4-phenyl-2H-phthalazin-1-one,
[17] [2-(4-Chloro-phenoxy)-ethyl]-[5-(3,5-dimethyl-piperazin-1-yl)-2-nitrophenyl]-amine,
[18] 2-[4-[3-(Benzhydryl-amino)-4-nitro-phenyl]-piperazin-1-yl]-ethanol,
[19] 4-[4-Fluoro-5-(4-methyl-piperazin-1-yl)-2-nitro-phenyl]-morpholine,
[20] 2-[2-Nitro-5-[4-(toluene-4-sulfonyl)-piperazin-1-yl]-phenyl]-1,2,3,4-tetrahydro-isoquinoline,
[21] 1-[3-(3,5-Dimethyl-pyrazol-1-yl)-4-nitro-phenyl]-4-methyl-piperazine,
[22] Benzyl-(2-nitro-5-piperazin-1-yl-phenyl)-amine,
[23] [5-(4-Methyl-piperazin-1-yl)-2-nitro-phenyl]-phenethyl-amine,
[24] [5-(4-Methyl-piperazin-1-yl)-2-nitro-phenyl]-pyridin-3-ylmethyl-amine,
[25] (3-Chloro-phenyl)-[4-[3-[(furanyl-2-ylmethyl)-amino]-4-nitro-phenyl]-piperazin-1-yl]-methanone,
[26] (2-Nitro-5-piperazin-1-yl-phenyl)-pyridin-3-ylmethyl-amine,
[27] 1-Benzyl-4-(2-nitro-5-piperazin-1-yl-phenyl)-piperazine,
[28] Furan-2-ylmethyl-[5-(4-methanesulfonyl-piperazin-1-yl)-2-nitro-phenyl]-amine,
[29] Benzhydryl-[5-(4-methyl-piperazin-1-yl)-2-nitro-phenyl]-amine,
[30] (2-Nitro-5-piperazin-1-yl-phenyl)-(2-phenoxy-ethyl)-amine,
[31] 2-[5-(4-Methyl-piperazin-1-yl)-2-nitro-phenyl]-4-phenyl-2H-phthalazin-1-one,
[32] 1-[3-(3,5-Dimethyl-pyrazol-1-yl)-4-nitro-phenyl]-piperazine,
[33] (2-Nitro-5-piperazin-1-yl-phenyl)-phenethyl-amine,
[34] [5-(4-Benzenesulfonyl-piperazin-1-yl)-2-nitro-phenyl]-furan-2-ylmethylamine,
[35] [2-(3,4-Dimethoxy-phenyl)-ethyl]-(2-nitro-5-piperazin-1-yl-phenyl)-amine,
[36] [4-[3-[(Furan-2-ylmethyl)-amino]-4-nitro-phenyl]-piperazin-1-yl]-m-tolyl-methanone,
[37] [4-[3-[(Furan-2-ylmethyl)-amino]-4-nitro-phenyl]-piperazin-1-yl]-phenyl-methanone,
[38] [4-[3-(3,5-Dimethyl-pyrazol-1-yl)-4-nitro-phenyl]-piperazin-1-yl]-thiophen-2-yl-methanone,
[39] 4-(4-Ethyl-phenyl)-2-[5-[4-methyl-piperazin-1-yl)-2-nitro-phenyl]-2H-phthalalazin-1-one,
[40] 3-[7-(4-Methyl-piperazin-1-yl)-4-nitro-benzo[1,2,5]oxadiazol-5-ylamino]-propan-1-ol,
[41] 3-[4-Nitro-7-(4-phenyl-piperazin-1-yl)-benzo[1,2,5]oxadiazol-5-ylamino]-ethan-1-ol,
[42] 3-[4-Nitro-7-(4-pyridin-piperazin-1-yl)-benzo[1,2,5]oxadiazol-5-ylamino]-propan-1-ol,
[43] [2-(3,4-Dimethoxy-phenyl)-ethyl]-(5-(4-methyl-piperazin-1-yl)-2-nitrophenyl]-amine,
[44] [2-(3,4-Dimethyl-phenyl)-ethyl]-[5-(4-methyl-piperazin-1-yl)-2-nitrophenyl]-amine,
[45] [2-(4-tert-Butyl-phenoxy)-ethyl]-[5-(4-methyl-piperazin-1-yl)-2-nitrophenyl]-amine,
[46] [2-(4-Methoxy-phenoxy)-ethyl]-[5-(4-methyl-piperazin-1-yl)-2-nitrophenyl]-amine,
[47] [5-(4-Methyl-piperazin-1-yl)-2-nitro-phenyl]-(2-m-tolyloxy-ethyl)-amine,
[48] [2-(4-Chloro-phenoxy)-ethyl]-[5-(4-methyl-piperazin-1-yl)-2-nitro-phenyl]-amine,
[49] (2-Nitro-5-piperazin-1-yl-phenyl)-(1-phenyl-ethyl)-amine,
[50] [2-(3-Methoxy-phenoxy)-ethyl]-[5-(4-methyl-piperazin-1-yl)-2-nitrophenyl]-amine,
[51] [2-(2-Methoxy-phenoxy)-ethyl]-[5-(4-methyl-piperazin-1-yl)-2-nitrophenyl]-amine,
[52] (4-Chloro-benzyl)-(2-nitro-5-piperazin-1-yl-phenyl)-amine,
[53] Benzyl-[5-(4-benzyl-piperazin-1-yl)-2-nitro-phenyl]-amine,
[54] Benzyl-[5-(4-methyl-piperazin-1-yl)-2-nitro-phenyl]-amine,
[55] [5-(4-Methyl-piperazin-1-yl)-2-nitro-phenyl]-(2-o-tolyloxy-ethyl)-amine,
[56] (4-Chloro-benzyl)-[5-(4-methyl-piperazin-1-yl)-2-nitro-phenyl]-amine,
[57] Furan-2-ylmethyl-(2-nitro-5-piperazin-1-yl-phenyl)-amine,
[58] [2-(4-Chloro-phenoxy)-ethyl]-(2-nitro-5-piperazin-1-yl-phenyl)-amine,
[59] [5-(4-Methyl-piperazin-1-yl)-2-nitro-phenyl]-(1-phenyl-ethyl)-amine and
[60] 1-[4-(3-Benzylamino-4-nitro-phenyl)-piperazin-1-yl]-ethanone,
optionally in form of a physiologically acceptable salt thereof, or a corresponding solvate thereof.

Yet a further aspect of the present invention relates to a medicament comprising at least one nitro-substituted phenyl-piperazine compound of general formula la, 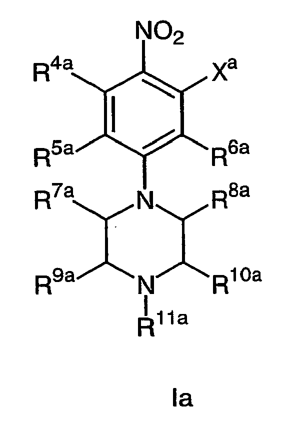 wherein
- X^{a}: represents a -NR^{1a}R^{2a} moiety or a -OR^{3a} moiety;
- R^{1a}: represents a linear or branched, saturated or unsaturated, at least mono- substituted aliphatic radical; a saturated or unsaturated, optionally at least mono-substituted, optionally at least one heteroatom as a ring member containing cycloaliphatic radical, which is bonded via a linear or branched, optionally at least mono-substituted alkylene, alkenylene or alkinylene group; an optionally at least mono-substituted aryl or heteroaryl radical; an aryl radical whereby said aryl radical may be substituted with 1, 2, 3, 4 or 5 substituent(s) independently selected from the group consisting of C₁₋₅-alkyl, -O-C₁₋₅-alkyl, - S-C₁₋₅-alkyl, -C(=O)-O-C₁₋₅-alkyl, -O-C(=O)-C₁₋₅-alkyl, F, Cl, Br, I, -CN, -CF₃, - OCF₃, -SCF₃, -OH, -SH, -NH₂, -NH(C₁₋₅-alkyl), -N(C₁₋₅-alkyl)₂, -NO₂, -CHO, - CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl and said aryl radical is bonded via a linear or branched, optionally at least mono-substituted, optionally at least one heteroatom as a chain member containing alkylene, alkenylene or alkinylene group; an optionally at least mono-substituted heteroaryl radical, which is bonded via a linear or branched, optionally at least mono-substituted, optionally at least one heteroatom as a chain member containing alkylene, alkenylene or alkinylene group; or a -C(=O)-R^{12a} moiety;
- R^{2a}: represents a hydrogen atom or a linear or branched, saturated or unsaturated, optionally at least mono-substituted aliphatic radical;
or
- R^{1a} and R^{2a}: together with the bridging nitrogen form an optionally at least mono- substituted; saturated, unsaturated or aromatic heterocyclic ring which may contain at least one further heteroatom as a ring member and/or which may be condensed with an optionally at least mono-substituted mono- or bicyclic ring system;
- R^{3a}: represents an optionally at least mono-substituted aryl or heteroaryl radical, which may be bonded via a linear or branched, optionally at least mono- substituted alkylene, alkenylene or alkinylene group;
- R^{4a}, R^{5a} and R^{6a},: identical or different, each represent a hydrogen atom or a halogen atom; or
- R^{4a} and R^{5a}: together with the bridging carbon atoms form an optionally at least mono- substituted, at least one heteroatom as a ring member containing heterocyclic ring which together with the phenyl ring which it is fused with forms a substituted bicyclic aromatic ring system;
- R^{7a} and R^{8a},: identical or different, each represent a hydrogen atom or a linear or branched, saturated or unsaturated, optionally at least mono-substituted aliphatic radical;
- R^{9a} and R^{10a},: identical or different, each represent a hydrogen atom or a linear or branched, saturated or unsaturated, optionally at least mono-substituted aliphatic radical;
- R^{11a}: represents a hydrogen atom; a linear or branched, saturated or unsaturated, optionally at least mono-substituted, optionally at least one heteroatom as a chain member containing aliphatic radical; an optionally at least mono- substituted aryl or heteroaryl radical, which may be bonded via a linear or branched, optionally at least mono-substituted alkylene, alkenylene or alkinylene group; a -C(=O)-R^{13a} moiety or a -S(=O)₂-R^{14a} moiety;
- R^{12a}: represents a linear or branched, saturated or unsaturated, optionally at least mono-substituted aliphatic radical; or an optionally at least mono-substituted aryl or heteroaryl radical, which may be bonded via a linear or branched, optionally at least mono-substituted alkylene, alkenylene or alkinylene group;
and
- R^{13a} and R^{14a},: identical or different, each represent a linear or branched, saturated or unsaturated, optionally at least mono-substituted aliphatic radical; or an optionally at least mono-substituted aryl or heteroaryl radical, which may be bonded via a linear or branched, optionally at least mono-substituted alkylene, alkenylene or alkinylene group;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof;
and optionally at least one physiologically compatible auxiliary agent.

A mono- or bicyclic ring system according to the present invention - if not defined otherwise - means a mono- or bicyclic hydrocarbon ring system that may be saturated, unsaturated or aromatic. Each of its different rings may show a different degree of saturation, i.e. it may be saturated, unsaturated or aromatic. Optionally each of the rings of the mono- or bicyclic ring system may contain one or more, preferably 1, 2 or 3, heteroatom(s) as ring member(s), which may be identical or different and which can preferably be selected from the group consisting of N, O and S. The rings of the mono- or bicyclic ring system are preferably 5-, 6- or 7-membered.

The term "condensed" according to the present invention means that a ring or ring system is attached to another ring or ring system, whereby the terms "annulated" or "annelated" are also used by those skilled in the art to designate this kind of attachment.

Such a mono- or bicyclic ring system may - if not defined otherwise - be unsubstituted or substituted by one or more substituents, preferably unsubstituted or substituted with 1, 2, 3, 4 or 5 substituent(s). Said substituents may preferably be selected independently from the group consisting of C₁₋₅-alkyl, -O-C₁₋₅-alkyl, -S-C₁₋₅-alkyl, -C(=O)-OH, oxo (=O), thioxo (=S), -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, - NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; whereby in each occurence C₁₋₅-alkyl may be linear or branched and whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and NO₂.

More preferably said substituents may be selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, - CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl; whereby in each occurence said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and NO₂.

If any of the substituents represents or comprises a (hetero)cycloaliphatic radical [(hetero)cycloaliphatic group], said (hetero)cycloaliphatic radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of oxo (=O), thioxo (=S), C₁₋₅-alkyl, -O-C₁₋₅alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; whereby in each occurence C₁₋₅-alkyl may be linear or branched and whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, - SCF₃, -OH, -SH, -NH₂ and NO₂.

More preferably said substituents may be selected independently from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, - NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl; whereby in each occurence said cyclic substituents may be unsubstituted or substituted by 1, 2 or 3 substituent(s) independently selected from the group consisting of methyl, ethyl, n-propyl, isopropyl, methoxy, ethoxy, F, Cl, Br, - CN, -CF₃, -OCF₃, -SCF₃, -OH, -SH, -NH₂ and NO₂.

If any of the substituents represents or comprises a cycloaliphatic radical, which contains one or more, preferably 1, 2 or 3 heteroatom(s) as ring member(s), unless defined otherwise, each of these heteroatom(s) may preferably be selected independently from the group consisting of N, O and S.

Suitable (hetero)cycloaliphatic radicals, which may be unsubstituted or at least mono-substituted, include cyclopropyl, cyclobutyl, cyclopentyl, cyclopentenyl, cyclohexyl, cyclohexenyl, cycloheptyl, cycloheptenyl, cyclooctyl, cyclooctenyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl.

If any of the substituents represents or comprises an aryl radical (aryl group), including a phenyl or naphthyl group, said aryl radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -SH, -NH₂, -NH(C₁₋₅-alkyl), -N(C₁₋₅-alkyl)₂, -NO₂, - CHO, -CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl; whereby in each occurrence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and-NO₂.

More preferably said substituents may be selected independently from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃,-S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.

Preferred aryl radicals, which may optionally be at least mono-substituted, are phenyl and naphthyl.

If any of the substituents represents or comprises a heteroaryl radical (heteroaryl group), said heteroaryl radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -SH, -NH₂, -NH(C₁₋₅-alkyl), -N(C₁₋₅-alkyl)₂, -NO₂, - CHO, -CF₂H, -CFH₂, -C(=O)-NH₂, -C(=O)-NH(C₁₋₅-alkyl), -C(=O)-N(C₁₋₅-alkyl)₂, - S(=O)2-C₁₋₅-alkyl, -S(=O)₂-phenyl, phenyl, phenoxy and benzyl; whereby in each occurrence C₁-₅-alkyl may be linear or branched and whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and NO₂.

More preferably said substituents may be selected independently from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.

The heteroatom(s), which are present as ring member(s) in the heteroaryl radical, may, unless defined otherwise, independently be selected from the group consisting of nitrogen, oxygen and sulphur. Preferably the heteroaryl radical comprises 1, 2 or 3 heteroatom(s).

Suitable heteroaryl radicals, which may optionally be at least mono-substituted, may preferably be selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl.

If at least two of the substituents together with the bridging nitrogen atom form a saturated or unsaturated heterocyclic ring which may be condensed with an optionally at least mono-substituted mono- or bicyclic ring system, said heterocyclic ring may preferably be selected from the group consisting of

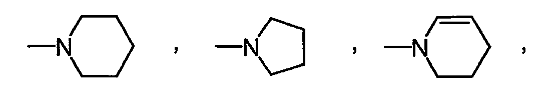

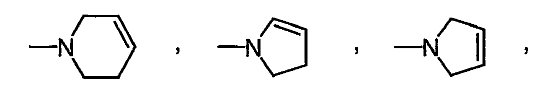

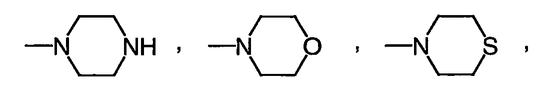

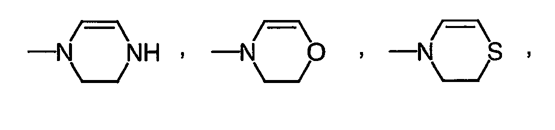

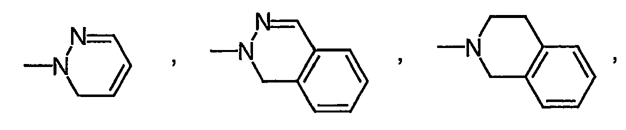

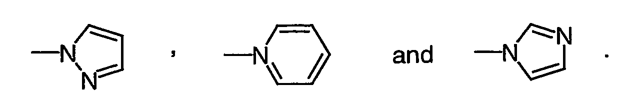

Those skilled in the art understand that the afore mentioned cyclic moieties, which contain an NH group may also be substituted in this position, i.e. the hydrogen atom may be exchanged for another substituent.

Said heterocyclic rings may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅-alkyl, oxo (=O), thioxo (=S), -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; whereby in each occurrence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and NO₂.

More preferably said substituents may be selected independently from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂,-NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl; whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and NO₂.

If any of the substituents represents a saturated or unsaturated aliphatic radical (aliphatic group), i.e. an alkyl radical, an alkenyl radical or an alkinyl radical, said aliphatic radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of -O-C₁₋₅-alkyl, -S-C₁-₅-alkyl, F, Cl, Br, I, -CN, -CF₃, -OCF₃, -SCF₃, - OH, -SH, -NH₂, -NH(C₁₋₅-alkyl) and -N(C₁₋₅-alkyl)₂, whereby in each occurence C₁₋₅-alkyl may be linear or branched. More preferably said substituent(s) may preferably be selected independently from the group consisting of -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, F, Cl, Br, I, -CN, -CF₃, -OCF₃, -SCF₃, -OH, -SH, -NH₂, NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂.

An alkenyl radical comprises at least one carbon-carbon double bond, an alkinyl radical comprises at least one carbon-carbon triple bond.

Suitable alkyl radicals, which may be substituted by one or more substituents, may preferably be selected from the group consisting of methyl, ethyl, n-propyl, isopropyl, n-butyl, isobutyl, sec-butyl, tert-butyl, n-pentyl, n-hexyl, n-heptyl, n-octyl, n-nonyl and n-decyl.

Suitable alkenyl radicals, which may be substituted by one or more substituents, may preferably be selected from the group consisting of vinyl, 1-propenyl, 2-propenyl, 1-butenyl, 2-butenyl and 3-butenyl.

Suitable alkinyl radicals, which may be substituted by one or more substituents, may preferably be selected from the group consisting of ethinyl, 1-propinyl, 2-propinyl, 1-butinyl, 2-butinyl and 3-butinyl.

If any of the substituents represents an alkylene group, an alkenylene group or an alkinylene group, which may be substituted, said alkylene group, alkenylene group or alkinylene group may - if not defined otherwise - be unsubstituted or substituted by one or more substituents, preferably unsubstituted or substituted with 1, 2 or 3 substituent(s). Said substituent(s) may preferably be selected independently from the group consisting of -O-C₁₋₅-alkyl, -S-C₁₋₅-alkyl, -F, Cl, Br, I, -CN, -CF₃, -OCF₃, -SCF₃, -OH, -SH, -NH₂, -NH(C₁₋₅-alkyl), -N(C₁₋₅-alkyl)₂ and phenyl, whereby in each occurence C₁₋₅-alkyl may be linear or branched and the phenyl radical is preferably unsubstituted. An alkenylene group comprises at least one carbon-carbon double bond, an alkinylene group comprises at least one carbon-carbon triple bond.

Suitable alkylene groups include -(CH₂)-, -CH(CH₃)-, -CH(phenyl), -(CH₂)₂-, -(CH₂)₃-, -(CH₂)₄-,-(CH₂)₅ and -(CH₂)₆-, suitable alkenylene groups include -CH=CH-, -CH₂-CH=CH- and -CH=CH-CH₂- and suitable alkinylene groups include -C≡C-, -CH₂-C≡C- and -C≡C-CH₂-.

If the alkylene, alkenylene or alkinylene group contains one or more, preferably 1, 2 or 3, more preferably 1, heteroatom(s) as chain member(s), unless defined otherwise, each of these heteroatom(s) may preferably be selected from the group consisting of N, O and S. Suitable alkylene groups which contain one or more heteroatom(s) include -CH₂-O-CH₂- and -CH₂-CH₂-O.

In another aspect, the invention relates to a medicament comprising at least one nitro-substituted phenyl-piperazine compound of general formula la,
wherein
- X^{a}: represents a -NR^{1a}R^{2a} moiety or a -OR^{3a} moiety;
- R^{1a}: represents a linear or branched, saturated or unsaturated, at least mono- substituted C₁₋₁₀ aliphatic radical; a saturated or unsaturated, optionally at least mono-substituted 3- to 9-membered cycloaliphatic radical, which is bonded via a linear or branched, optionally at least mono-substituted C₁₋₆ alkylene, C₂₋₆ alkenylene or C₂₋₆ alkinylene group and/or which may contain 1, 2 or 3 heteroatom(s) independently selected from the group consisting of nitrogen, oxygen and sulphur as ring member(s); an optionally at least mono-substituted 5- to 10-membered aryl or heteroaryl radical which contains 1, 2 or 3 heteroatom(s) independently selected from the group consisting of nitrogen, oxygen and sulphur as ring member(s); a 5- to 10-membered aryl radical whereby said aryl radical may be substituted with 1, 2, 3, 4 or 5 substituent(s) independently selected from the group consisting of C₁₋₅-alkyl, -O-C₁₋₅-alkyl, - S-C₁₋₅-alkyl, -C(=O)-O-C₁₋₅-alkyl, -O-C(=O)-C₁₋₅-alkyl, F, Cl, Br, I, -CN, -CF₃, - OCF₃, -SCF₃, -OH, -SH, -NH₂, -NH(C₁₋₅-alkyl), -N(C₁₋₅-alkyl)₂, -NO₂, -CHO, - CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl and said aryl radical is bonded via a linear or branched, optionally at least mono-substituted C₁₋₆ alkylene, C₂₋₆ alkenylene or C₂₋₆ alkinylene group which may contain 1, 2 or 3 heteroatom(s) independently selected from the group consisting of nitrogen, oxygen and sulphur as chain member(s); an optionally at least mono- substituted 5- to 10-membered heteroaryl radical, which is bonded via a linear or branched, optionally at least mono-substituted C₁₋₆ alkylene, C₂₋₆ alkenylene or C₂₋₆ alkinylene group which may contain 1, 2 or 3 heteroatom(s) independently selected from the group consisting of nitrogen, oxygen and sulphur as chain member(s) and whereby the heteroaryl radical contains 1, 2 or 3 heteroatom(s) independently selected from the group consisting of nitrogen, oxygen and sulphur as ring member(s); or a -C(=O)-R^{12a} moiety;
- R^{2a}: represents a hydrogen atom or a linear or branched, saturated or unsaturated, optionally at least mono-substituted C₁₋₁₀ aliphatic radical;
or
- R^{1a} and R^{2a}: together with the bridging nitrogen form an optionally at least mono- substituted; saturated, unsaturated or aromatic 3- to 9-membered heterocyclic ring which may contain 1, 2 or 3 additional heteroatom(s) independently selected from the group consisting of nitrogen, oxygen and sulphur as ring member(s) and/or which may be condensed with an optionally at least mono- substituted mono- or bicyclic ring system; whereby the rings of the ring system are 5- 6- or 7-membered and may contain 1, 2 or 3 heteroatom(s) independently selected from the group consisting of nitrogen, oxygen and sulphur;
- R^{3a}: represents an optionally at least mono-substituted 5- to 10-membered aryl or heteroaryl radical, which may be bonded via a linear or branched, optionally at least mono-substituted C₁₋₆ alkylene, C₂₋₆ alkenylene or C₂₋₆ alkinylene group and whereby the heteroaryl radical contains 1, 2 or 3 heteroatom(s) independently selected from the group consisting of nitrogen, oxygen and sulphur as ring member(s);
- R^{4a}, R^{5a} and R^{6a},: identical or different, each represent a hydrogen atom or a halogen atom;
or
- R^{4a} and R^{5a}: together with the bridging carbon atoms form an optionally at least mono- substituted 5- or 6- membered heterocyclic ring which contains 1, 2 or 3 heteroatom(s) independently selected from the group consisting of nitrogen, oxygen and sulphur as ring member(s) and which together with the phenyl ring which it is fused with forms a substituted 9- or 10-membered bicyclic aromatic ring system;
- R^{7a} and R^{8a},: identical or different, each represent a hydrogen atom or a linear or branched, saturated or unsaturated, optionally at least mono-substituted C₁₋₁₀ aliphatic radical;
- R^{9a} and R^{10a},: identical or different, each represent a hydrogen atom or a linear or branched, saturated or unsaturated, optionally at least mono-substituted C₁₋₁₀ aliphatic radical;
- R^{11a}: represents a hydrogen atom; a linear or branched, saturated or unsaturated, optionally at least mono-substituted C₁₋₁₀ aliphatic radical which may contain 1, 2 or 3 heteroatom(s) independently selected from the group consisting of nitrogen, oxygen and sulphur as chain member(s); an optionally at least mono- substituted 5- to 10-membered aryl or heteroaryl radical which may be bonded via a linear or branched, optionally at least mono-substituted C₁₋₆ alkylene, C₂₋₆ alkenylene or C₂₋₆ alkinylene group and whereby the heteroaryl radical contains 1, 2 or 3 heteroatom(s) independently selected from the group consisting of nitrogen, oxygen and sulphur as ring member(s); a -C(=O)-R^{13a} moiety or a -S(=O)₂-R^{14a} moiety;
- R^{12a}: represents a linear or branched, saturated or unsaturated, optionally at least mono-substituted C₁₋₁₀ aliphatic radical; or an optionally at least mono- substituted aryl or heteroaryl radical, which may be bonded via a linear or branched, optionally at least mono-substituted C₁₋₆ alkylene, C₂₋₆ alkenylene or C₂₋₆ alkinylene group and whereby the heteroaryl radical contains 1, 2 or 3 heteroatom(s) independently selected from the group consisting of nitrogen, oxygen and sulphur as ring member(s);
and
- R^{13a} and R^{14a},: identical or different, each represent a linear or branched, saturated or unsaturated, optionally at least mono-substituted C₁₋₁₀ aliphatic radical; or an optionally at least mono-substituted aryl or heteroaryl radical, which may be bonded via a linear or branched, optionally at least mono-substituted C₁₋₆ alkylene, C₂₋₆ alkenylene or C₂₋₆ alkinylene group and whereby the heteroaryl radical contains 1, 2 or 3 heteroatom(s) independently selected from the group consisting of nitrogen, oxygen and sulphur as ring member(s).

Also preferred are medicaments comprising at least one nitro-substituted phenyl-piperazine compound of general formula la, wherein R^{1a} represents a linear or branched, at least mono-substituted C₁₋₁₀ alkyl radical; an optionally at least mono-substituted aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl; an aryl radical selected from the group consisting of phenyl and naphthyl whereby said aryl radical may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and said aryl radical is bonded via an optionally at least mono-substituted C₁₋₆ alkylene group which may contain 1, 2 or 3 heteroatom(s) independently selected from the group consisting of nitrogen, oxygen and sulphur as chain member(s); an optionally at least mono-substituted heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said heteroaryl radical is bonded via an optionally at least mono-substituted C₁₋₆ alkylene group which may contain 1, 2 or 3 heteroatom(s) independently selected from the group consisting of nitrogen, oxygen and sulphur as chain member(s); or a -C(=O)-R^{12a} moiety;
preferably R^{1a} represents an alkyl radical selected from the group consisting of methyl, ethyl, n-propyl, isopropyl, n-butyl, sec-butyl, isobutyl and tert-butyl whereby said alkyl radical may be substituted with 1, 2 or 3 substituent(s) independently selected from the group consisting of -OH, F, Cl, Br and -CN; an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; an aryl radical selected from the group consisting of phenyl and naphthyl whereby said aryl radical may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and said aryl radical is bonded via a linear or branched C₁₋₆ alkylene group which may contain 1, 2 or 3 oxygen atom(s) as chain member(s) and which may be substituted with 1 phenyl radical; a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said heteroaryl radical may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -CF₃, -OCF₃, -SCF₃, - OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and said heteroaryl radical is bonded via a linear or branched C₁₋₆ alkylene group which may contain 1, 2 or 3 oxygen atom(s) as chain member(s); or a -C(=O)-R^{12a} moiety;
more preferably R^{1a} represents an alkyl radical selected from the group consisting of methyl, ethyl, n-propyl and isopropyl, whereby said alkyl radical may be substituted with 1, 2 or 3 substituent(s) independently selected from the group consisting of -OH, F, Cl, Br and -CN; an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said aryl or heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -SH, -NH₂, -NH-CH₃, -NH-C₂H₅, -NH-CH₂-CH₂-CH₃, -NH-CH(CH₃)₂, - NH-C(CH₃)₃, -N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
an aryl radical selected from the group consisting of phenyl and naphthyl whereby said aryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, - CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, -C(=O)-NH₂, - C(=O)-NH-CH₃, -C(=O)-NH-C₂H₅, -C(=O)-N(CH₃)₂, -C(=O)-N(C₂H₅)₂ and -S(=O)₂-CH₃ and/or is bonded via an alkylene group selected form the group consisting of - CH₂-, -CH(CH₃)-, -CH(Phenyl)-, -CH₂-CH₂-, -CH₂-CH₂-CH₂- and -CH₂-CH₂-O-; a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃,-C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂,-C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or is bonded via an alkylene group selected form the group consisting of -CH₂-, -CH(CH₃)-, -CH₂-CH₂-, -CH₂-CH₂-CH₂- and -CH₂-CH₂-O-; or a - C(=O)-R^{12a} moiety;
and X^{a} and R^{2a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Furthermore, such medicaments comprising at least one nitro-substituted phenyl-piperazine compound of general formula la are preferred, wherein R^{2a} represents a hydrogen atom or a linear or branched, unsubstituted C₁₋₁₀alkyl radical;
preferably R^{2a} represents a hydrogen atom or an alkyl radical selected from the group consisting of methyl, ethyl, n-propyl, isopropyl, n-butyl, sec-butyl, isobutyl and tert-butyl;
more preferably R^{2a} represents a hydrogen atom or a methyl radical;
and X^{a}, R^{1a} and R^{3a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are medicaments comprising at least one nitro-substituted phenyl-piperazine compound of general formula la, wherein R^{1a} and R^{2a} together with the bridging nitrogen atom form a moiety selected from the group consisting of 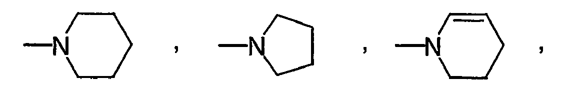 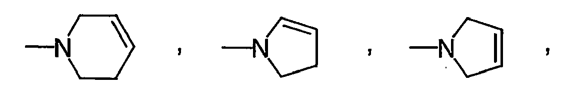 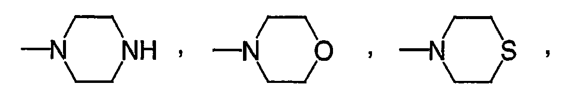 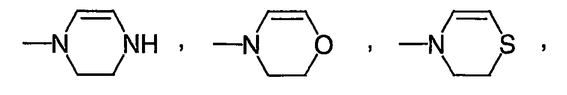 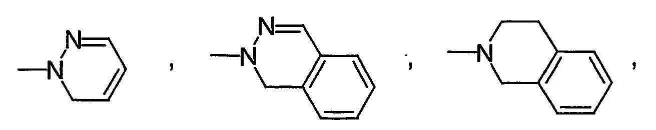 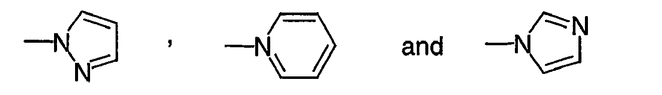 whereby each of these afore mentioned cyclic moieties may be substituted with 1, 2 or 3 substituent(s) independently selected from the group consisting of C₁₋₅-alkyl, -O-C₁₋₅-alkyl, -S-C₁₋₅-alkyl, -C(=O)-OH, oxo (=O), thioxo (=S), -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, - NH₂ and NO₂;
preferably R^{1a} and R^{2a} together with the bridging nitrogen atom form a moiety selected from the group consisting of 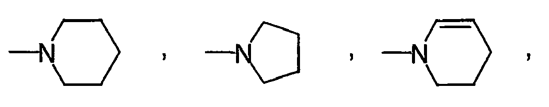 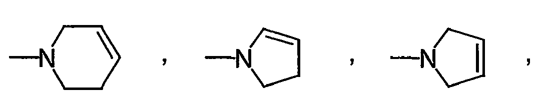 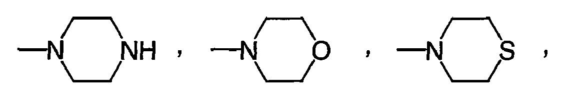 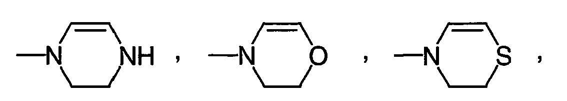 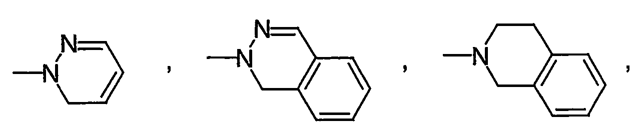 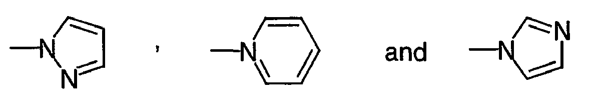 whereby each of these afore mentioned cyclic moieties may be substituted with 1, 2 or 3 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, - C(=O)-O-CH₃, -C(=O)-O-C₂H₅, -C(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, - NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl; whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and NO₂;
more preferably R^{1a} and R^{2a} together with the bridging nitrogen atom form a moiety selected from the group consisting of 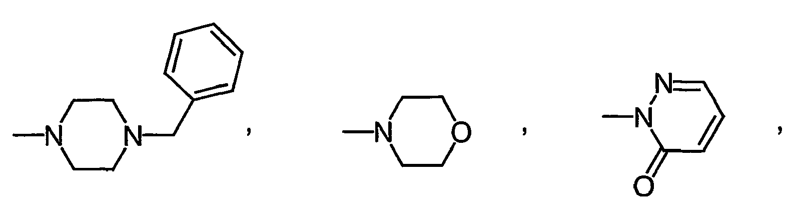 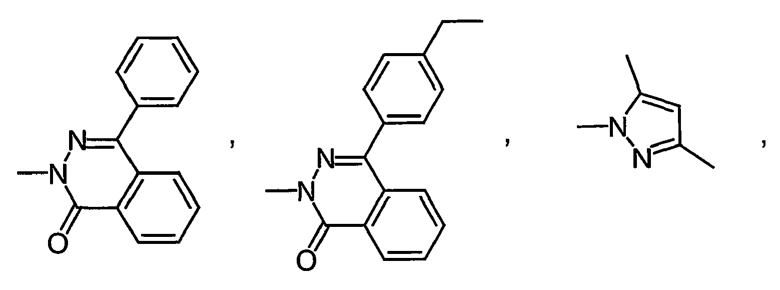 and 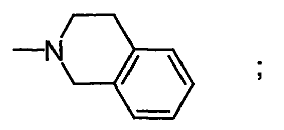 and X^{a} and R^{3a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are medicaments comprising at least one nitro-substituted phenyl-piperazine compound of general formula la, wherein R^{3a} represents an optionally at least mono-substituted aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via an optionally at least mono-substituted C₁₋₆ alkylene group;
preferably R^{3a} represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, -O-C(=O)-C₁₋₅-alkyl, F, Cl, Br, I, - CN, -CF₃, -OCF₃, -SCF₃, -OH, -SH, -NH₂, -NH(C₁₋₅-alkyl), -N(C₁₋₅-alkyl)₂, -NO₂, - CHO, -CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl and said aryl or heteroaryl radical may be bonded via a linear or branched C₁₋₆ alkylene group;
more preferably R^{3a} represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said aryl or heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -SH, -NH₂, -NH-CH₃, -NH-C₂H₅, -NH-CH₂-CH₂-CH₃, -NH-CH(CH₃)₂, - NH-C(CH₃)₃, -N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
and X^{a}, R^{1a}, R^{2a} and R^{4a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Moreover, such medicaments comprising at least one nitro-substituted phenyl-piperazine compound of general formula la are preferred, wherein R^{4a}, R^{5a} and R^{6a}, identical or different, each represent a hydrogen atom or a halogen atom selected from the group consisting of F, Cl and Br;
preferably R^{4a}, R^{5a} and R^{6a}, identical or different, each represent a hydrogen atom or a fluorine atom;
and X^{a}, R^{1a} to R^{3a} and R^{7a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Furthermore, such medicaments comprising at least one nitro-substituted phenyl-piperazine compound of general formula la are preferred, wherein R^{4a} and R^{5a} together with the bridging carbon atoms form a moiety selected from the group consisting of 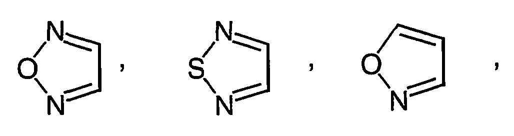 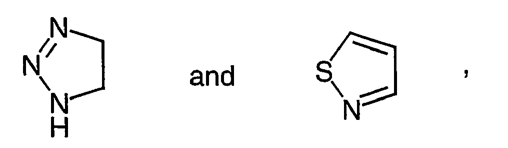 whereby each of these afore mentioned cyclic moieties may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂ and - NO₂; which together with the phenyl ring which it is fused with forms a substituted bicyclic aromatic ring system selected from the group consisting of 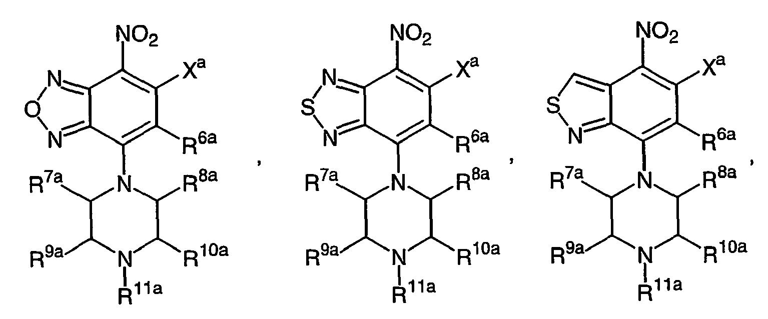 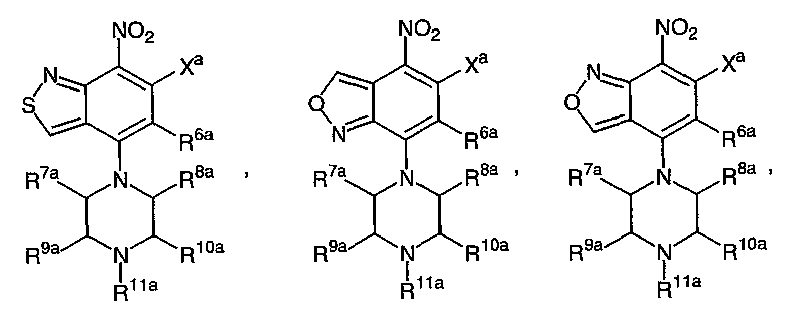 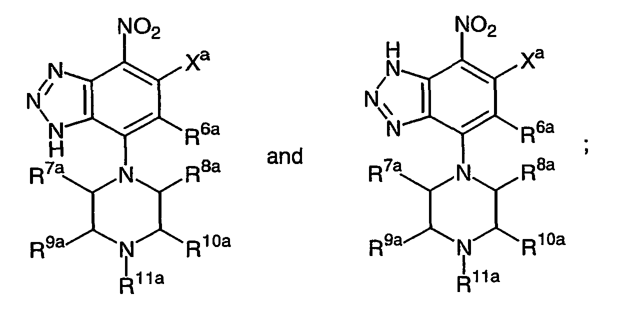 preferably R^{4a} and R^{5a} together with the bridging carbon atoms form the following moiety, 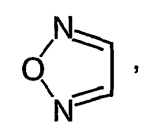 which together with the phenyl ring which it is fused with forms the following substituted bicyclic aromatic ring system 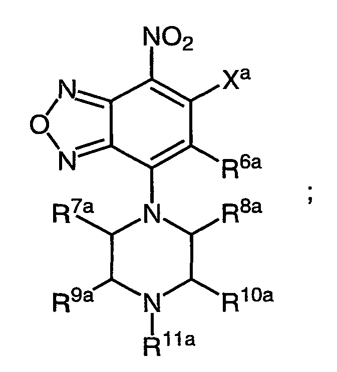 and X^{a}, R^{1a} to R^{3a} and R^{6a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl-piperazine compound of general formula la, wherein R^{7a} and R^{8a}, identical or different, each represent a hydrogen atom or a linear or branched, unsubstituted C₁₋₁₀ alkyl radical;
preferably R^{7a} and R^{8a}, identical or different, each represent a hydrogen atom or an alkyl radical selected from the group consisting of methyl, ethyl, n-propyl, isopropyl, n-butyl, sec-butyl, isobutyl and tert-butyl;
more preferably R^{7a} and R^{8a} each represent a hydrogen atom;
and X^{a}, R^{1a} to R^{6a} and R^{9a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl-piperazine compound of general formula la, wherein R^{9a} and R^{10a}, identical or different, each represent a hydrogen atom or a linear or branched, unsubstituted C₁₋₁₀ alkyl radical;
preferably R^{9a} and R^{10a}, identical or different, each represent a hydrogen atom or an alkyl radical selected from the group consisting of methyl, ethyl, n-propyl, isopropyl, n-butyl, sec-butyl, isobutyl and tert-butyl;
more preferably R^{9a} and R^{10a}, identical or different, each represent a hydrogen atom or a methyl radical;
and X^{a}, R^{1a} to R^{8a} and R^{11a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl piperazine compound of general formula la, wherein R^{11a} represents a hydrogen atom; a linear or branched, optionally at least mono-substituted C₁₋₁₀ alkyl radical; an aryl or heteroaryl radical selected from the group consisting of phenyl and pyridinyl whereby said aryl or heteroaryl radical is substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, -CN, - OCF₃, -SCF₃, -OH, -SH, -NH₂, -NH(C₁₋₅-alkyl), -N(C₁₋₅-alkyl)₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and/or may be bonded via a C₁₋₆-alkylene group; an aryl or heteroaryl radical selected from the group consisting of naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl whereby said aryl or heteroaryl may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, - C(=O)-O-C₁₋₅-alkyl, -O-C(=O)-C₁₋₅-alkyl, F, Cl, Br, I, -CN, -CF₃, -OCF₃, -SCF₃, -OH, - SH, -NH₂, -NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and/or may be bonded via a C₁₋₆-alkylene group; a - C(=O)-R^{13a} moiety or a -S(=O)₂-R^{14a} moiety;
preferably R^{11a} represents a hydrogen atom; an alkyl radical selected from the group consisting of methyl, ethyl, n-propyl, isopropyl, n-butyl, sec-butyl, isobutyl and tert-butyl whereby said alkyl radical may be substituted with 1, 2 or 3 substituent(s) independently selected from the group consisting of -OH, F, Cl, Br and -CN; an aryl or heteroaryl radical selected from the group consisting of phenyl and pyridinyl whereby said aryl or heteroaryl radical is substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, - O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, -CN, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -CHO, -CF₂H, -CFH₂, -C(=O)-NH₂, -C(=O)-NH-CH₃, -C(=O)-NH-C₂H₅, - C(=O)-N(CH₃)₂, -C(=O)-N(C₂H₅)₂ and -S(=O)₂-CH₃ and/or may be bonded via a - (CH₂)₁, _{2 or 3}-group; an aryl or heteroaryl radical selected from the group consisting of naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or may be bonded via a -(CH₂)_{1,2 or 3}-group; a - C(=O)-R^{13a} moiety or a -S(=O)₂-R^{14a} moiety;
and X^{a}, R^{1a} to R^{10a} and R^{12a} to R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl-piperazine compound of general formula la, wherein R^{12a} represents a linear or branched, optionally at least mono-substituted C₁₋₁₀ alkyl radical; an optionally at least mono-substituted aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl which may be bonded via a -(CH₂)_{1,2 or 3}- group;
preferably R^{12a} represents an alkyl radical selected from the group consisting of methyl, ethyl, n-propyl, isopropyl, n-butyl, sec-butyl, isobutyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and/or may be bonded via a-(CH₂)_{1,2 or 3}- group;
more preferably R^{12a} represents an alkyl radical selected from the group consisting of methyl, ethyl, n-propyl and isopropyl, or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said aryl or heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, - C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or may be bonded via a -(CH₂) - group;
and X^{a}, R^{1a} to R^{11a}, R^{13a} and R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl-piperazine compound of general formula la, wherein R^{13a} represents a linear or branched, optionally at least mono-substituted C₁₋₁₀ alkyl radical; an aryl or heteroaryl radical selected from the group consisting of phenyl and pyridinyl whereby said aryl or heteroaryl radical is substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, -CN, -OCF₃, -SCF₃, - OH, -SH, -NH₂, -NH(C₁₋₅-alkyl), -N(C₁₋₅-alkyl)₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and/or may be bonded via a C₁₋₆-alkylene group; an aryl or heteroaryl radical selected from the group consisting of naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl whereby said aryl or heteroaryl may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and/or may be bonded via a C₁₋₆-alkylene group; a -C(=O)-R^{13a} moiety or a -S(=O)₂-R^{14a} moiety;
preferably R^{13a} represents an alkyl radical selected from the group consisting of methyl, ethyl, n-propyl, isopropyl, n-butyl, sec-butyl, isobutyl and tert-butyl whereby said alkyl radical may be substituted with 1, 2 or 3 substituent(s) independently selected from the group consisting of -OH, F, Cl, Br and -CN; an aryl or heteroaryl radical selected from the group consisting of phenyl and pyridinyl whereby said aryl or heteroaryl radical is substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, - C(=O)-O-CH₃, -C(=O)-O-C₂H₅, -C(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, -CN, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or may be bonded via a -(CH₂)₁,_{2 or 3}-group; an aryl or heteroaryl radical selected from the group consisting of naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl, quinolinyl, isoquinolinyl, benzo[b]furanyl, benzo[b]thiophenyl, benzothiadiazolyl and imidazo[2,i-b]thiazolyl, whereby said aryl or heteroaryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃ and/or may be bonded via a -(CFH₂)₁,₂ or ₃-group; a -C(=O)-R^{13a} moiety or a -S(=O)2-R^{14a} moiety;
and X^{a}, R^{1a} to R^{12a} and R^{14a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such medicaments comprising at least one nitro-substituted phenyl-piperazine compound of general formula la, wherein R^{14a} represents a linear or branched, optionally at least mono-substituted C₁₋₁₀ alkyl radical; an optionally at least mono-substituted aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[byhiophenyl, benzothiadiazolyl and imidazo[2,1-b]thiazolyl which may be bonded via a -(CH2)1,2 or3- group;
preferably R^{14a} represents an alkyl radical selected from the group consisting of methyl, ethyl, n-propyl, isopropyl, n-butyl, sec-butyl, isobutyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁-₅-alkyl), -N(C₁₋₅-alkyl)₂, -NO₂, -CHO, -CF₂H, -CFH₂, -C(=O)-NH₂, -C(=O)-NH(C₁-₅-alkyl), -C(=O)-N(C₁₋₅-alkyl)₂, -S(=O)₂-C₁₋₅-alkyl, -S(=O)₂-phenyl, phenyl, phenoxy and benzyl and/or may be bonded via a -(CH₂)_{1,2 or 3}- group;
more preferably R^{14a} represents an alkyl radical selected from the group consisting of methyl, ethyl, n-propyl and isopropyl, or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said aryl or heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, - C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or may be bonded via a -(CH₂)- group;
and X^{a} and R^{1a} to R^{13a} have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are medicaments comprising at least one nitro-substituted phenyl-piperazine compound of general formula la, wherein
- X^{a}: represents a -NR^{1a} R^{2a} moiety or a -OR^{3a} moiety;
- R^{1a}: represents an alkyl radical selected from the group consisting of -CH₂-CH₂- OH and -CH₂-CH₂-CH₂-OH; an unsubstituted phenyl or pyrrolyl radical; a phenyl radical which may be substituted with 1, 2 or 3 substituent(s) independently selected from the group consisting of methyl, ethyl, tert-butyl, methoxy, F and Cl and said is bonded via an alkylene group selected form the group consisting of -CH₂-, -CH(CH₃)-, -CH(Phenyl)-, -CH₂-CH₂-, -CH₂-CH₂- CH₂- and -CH₂-CH₂-O-; a heteroaryl radical selected from the group consisting of pyridinyl, furanyl and pyrrolyl, whereby said pyridinyl, furanyl or pyrrolyl radical may be substituted with 1, 2 or 3 substituent(s) independently selected from the group consisting of methyl, ethyl, tert-butyl, methoxy, F and Cl and said pyridinyl, furanyl or pyrrolyl radical is bonded via an alkylene group selected form the group consisting of -CH₂-, -CH(CH₃)-, -CH₂-CH₂-, -CH₂-CH₂- CH₂- and -CH₂-CH₂-O-; or a -C(=O)-R^{12a} moiety;
- R^{2a}: represents a hydrogen atom or a methyl radical;
or 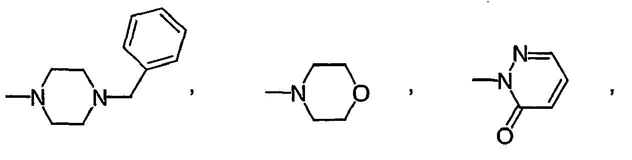 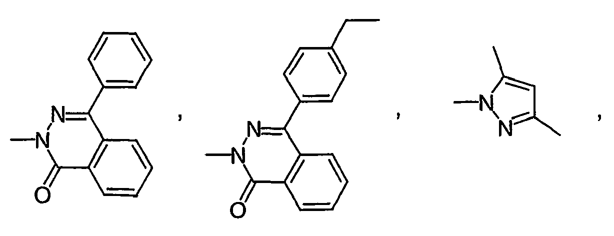 and 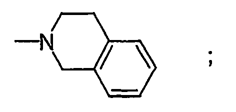 R^{1a} and R^{2a} together with the bridging nitrogen atom form a moiety selected from the group consisting of
R^{3a} represents an unsubstituted phenyl radical;
R^{4a}, R^{5a} and R^{6a}, identical or different, each represent a hydrogen atom or a fluorine atom;
or
R^{4a} and R^{5a} together with the bridging carbon atoms form the following moiety, 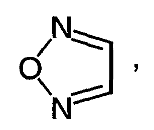 which together with the phenyl ring which it is fused with forms the following substituted bicyclic aromatic ring system 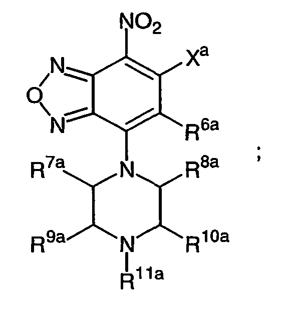 R^{7a} and R^{8a} each represent a hydrogen atom;
R^{9a} and R^{10a}, identical or different, each represent a hydrogen atom or a methyl radical;
R^{11a} represents a hydrogen atom; an alkyl radical selected from the group consisting of methyl, n-butyl and -CH₂-CH₂-OH; an unsubstituted phenyl or pyridinyl radical whereby said phenyl or pyridinyl radical may be bonded via a -(CH₂)- group; a -C(=O)-R^{12a} moiety or a -S(=O)₂-R^{13a} moiety;
R^{12a} represents a methyl radical or a phenyl or a thiophenyl radical whereby said phenyl or thiophenyl radical may be substituted with 1, 2 or 3 substituent(s) selected from the group consisting of methyl and chlorine;
R^{13a} represents a methyl radical or a phenyl or a thiophenyl radical whereby said phenyl or thiophenyl radical may be substituted with 1, 2 or 3 substituent(s) selected from the group consisting of methyl and chlorine;
and
R^{14a} represents a methyl radical or a phenyl radical which may be substituted with 1, 2 or 3 methyl radical(s);
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Particularly preferred are medicaments comprising at least one nitro-substituted phenyl-piperazine compound of general formula la selected from the group consisting of
[1] [5-(4-Methyl-piperazin-1-yl)-2-nitro-phenyl]-(2-pyrrol-1-yl-ethyl)-amine,
[2] (4-Fluoro-benzyl)-[5-(4-methyl-piperazin-1-yl)-2-nitro-phenyl]-amine,
[3] N-[5-(4-Methyl-piperazin-1-yl)-2-nitro-phenyl]-benzamide,
[4] N-Methyl-N-[5-(4-methyl-piperazin-1-yl)-2-nitro-phenyl]-benzamide,
[5] [5-(4-Methyl-piperazin-1-yl)-2-nitro-phenyl]-pyrrol-1-yl-amine,
[6] 2-[5-(4-Methyl-piperazin-1-yl)-2-nitro-phenyl]-2H-pyridazin-3-one,
[7] 1-Methyl-4-(4-nitro-3-phenoxy-phenyl)-piperazine,
[8] Benzyl-[5-(4-butyl-piperazin-1-yl)-2-nitro-phenyl]-amine,
[9] Benzyl-[2-nitro-5-(4-pyridin-2-yl-piperazin-1-yl)-phenyl]-amine and
[10] Benzyl-[2-nitro-5-(4-phenyl-2-yl-piperazin-1-yl)-phenyl]-amine;
[11] Furan-2-ylmethyl-[5-(4-methyl-piperazin-1-yl)-2-nitro-phenyl]-amine,
[12] 2-[4-(4-Nitro-3-phenethylamino-phenyl)-piperazin-1-yl]-ethanol,
[13] (2-Nitro-5-piperazin-1-yl-phenyl)-(2-o-tolyloxy-ethyl)-amine
[14] 2-[4-(3-Benzylamino-4-nitro-phenyl)-piperazin-1-yl)-ethanol,
[15] 4-[5-(4-Methyl-piperazin-1-yl)-2-nitro-phenyl]-morpholine,
[16] 2-[5-(4-Methyl-piperazin-1-yl)-2-nitro-phenyl]-4-phenyl-2H-phthalazin-1-one,
[17] [2-(4-Chloro-phenoxy)-ethyl]-[5-(3,5-dimethyl-piperazin-1-yl)-2-nitrophenyl]-amine,
[18] 2-[4-[3-(Benzhydryl-amino)-4-nitro-phenyl]-piperazin-1-yl]-ethanol,
[19] 4-[4-Fluoro-5-(4-methyl-piperazin-1-yl)-2-nitro-phenyl]-morpholine,
[20] 2-[2-Nitro-5-[4-(toluene-4-sulfonyl)-piperazin-1-yl]-phenyl]-1,2,3,4-tetrahydro-isoquinoline,
[21] 1-[3-(3,5-Dimethyl-pyrazol-1-yl)-4-nitro-phenyl]-4-methyl-piperazine,
[22] Benzyl-(2-nitro-5-piperazin-1-yl-phenyl)-amine,
[23] [5-(4-Methyl-piperazin-1-yl)-2-nitro-phenyl]-phenethyl-amine,
[24] [5-(4-Methyl-piperazin-1-yl)-2-nitro-phenyl]-pyridin-3-ylmethyl-amine,
[25] (3-Chloro-phenyl)-[4-[3-[(furanyl-2-ylmethyl)-amino]-4-nitro-phenyl]-piperazin-1-yl]-methanone,
[26] (2-Nitro-5-piperazin-1-yl-phenyl)-pyridin-3-ylmethyl-amine,
[27] 1-Benzyl-4-(2-nitro-5-piperazin-1-yl-phenyl)-piperazine,
[28] Furan-2-ylmethyl-[5-(4-methanesulfonyl-piperazin-1-yl)-2-nitro-phenyl]-amine,
[29] Benzhydryl-[5-(4-methyl-piperazin-1-yl)-2-nitro-phenyl]-amine,
[30] (2-Nitro-5-piperazin-1-yl-phenyl)-(2-phenoxy-ethyl)-amine,
[31] 2-[5-(4-Methyl-piperazin-1-yl)-2-nitro-phenyl]-4-phenyl-2H-phthalazin-1-one,
[32] 1-[3-(3,5-Dimethyl-pyrazol-1-yl)-4-nitro-phenyl]-piperazine,
[33] (2-Nitro-5-piperazin-1-yl-phenyl)-phenethyl-amine,
[34] [5-(4-Benzenesulfonyl-piperazin-1-yl)-2-nitro-phenyl]-furan-2-ylmethyl-amine,
[35] [2-(3,4-Dimethoxy-phenyl)-ethyl]-(2-nitro-5-piperazin-1-yl-phenyl)-amine,
[36] [4-[3-[(Furan-2-ylmethyl)-amino]-4-nitro-phenyl]-piperazin-1-yl]-m-tolyl-methanone,
[37] [4-[3-[(Furan-2-ylmethyl)-amino]-4-nitro-phenyl]-piperazin-1-yl]-phenyl-methanone,
[38] [4-[3-(3,5-Dimethyl-pyrazol-1-yl)-4-nitro-phenyl]-piperazin-1-yl]-thiophen-2-yl-methanone,
[39] 4-(4-Ethyl-phenyl)-2-[5-[4-methyl-piperazin-1-yl)-2-nitro-phenyl]-2H-phthalalazin-1-one,
[40] 3-[7-(4-Methyl-piperazin-1-yl)-4-nitro-benzo[1,2,5]oxadiazol-5-ylamino]-propan-1-ol,
[41] 3-[4-Nitro-7-(4-phenyl-piperazin-1-yl)-benzo[1,2,5]oxadiazol-5-ylamino]-ethan-1-ol,
[42] 3-[4-Nitro-7-(4-pyridin-piperazin-1-yl)-benzo[1,2,5]oxadiazol-5-ylamino]-propan-1-ol,
[43] [2-(3,4-Dimethoxy-phenyl)-ethyl]-[5-(4-methyl-piperazin-1-yl)-2-nitrophenyl]-amine,
[44] [2-(3,4-Dimethyl-phenyl)-ethyl]-[5-(4-methyl-piperazin-1-yl)-2-nitrophenyl]-amine,
[45] [2-(4-tert-Butyl-phenoxy)-ethyl]-[5-(4-methyl-piperazin-1-yl)-2-nitrophenyl]-amine,
[46] [2-(4-Methoxy-phenoxy)-ethyl]-[5-(4-methyl-piperazin-1-yl)-2-nitrophenyl]-amine,
[47] [5-(4-Methyl-piperazin-1-yl)-2-nitro-phenyl]-(2-m-tolyloxy-ethyl)-amine,
[48] [2-(4-Chloro-phenoxy)-ethyl]-[5-(4-methyl-piperazin-1-yl)-2-nitro-phenyl]-amine,
[49] (2-Nitro-5-piperazin-1-yl-phenyl)-(1-phenyl-ethyl)-amine,
[50] [2-(3-Methoxy-phenoxy)-ethyl]-[5-(4-methyl-piperazin-1-yl)-2-nitrophenyl]-amine,
[51] [2-(2-Methoxy-phenoxy)-ethyl]-[5-(4-methyl-piperazin-1-yl)-2-nitrophenyl]-amine,
[52] (4-Chloro-benzyl)-(2-nitro-5-piperazin-1-yl-phenyl)-amine,
[53] Benzyl-[5-(4-benzyl-piperazin-1-yl)-2-nitro-phenyl]-amine,
[54] Benzyl-[5-(4-methyl-piperazin-1-yl)-2-nitro-phenyl]-amine,
[55] [5-(4-Methyl-piperazin-1-yl)-2-nitro-phenyl]-(2-o-tolyloxy-ethyl)-amine,
[56] (4-Chloro-benzyl)-[5-(4-methyl-piperazin-1-yl)-2-nitro-phenyl]-amine,
[57] Furan-2-ylmethyl-(2-nitro-5-piperazin-1-yl-phenyl)-amine,
[58] [2-(4-Chloro-phenoxy)-ethyl]-(2-nitro-5-piperazin-1-yl-phenyl)-amine,
[59] [5-(4-Methyl-piperazin-1-yl)-2-nitro-phenyl]-(1-phenyl-ethyl)-amine
[60] 1-[4-(3-Benzylamino-4-nitro-phenyl)-piperazin-1-yl]-ethanone,
[61] 2-[4-[3-(4-Methylpiperazin-1-yl)-4-nitrophenyl]piperazin-1-yl]ethanol,
[62] 2-[4-[3-[2-(Naphthalen-2-yloxy)ethylamino]-4-nitrophenyl]piperazin-1-yl]ethanol,
[63] 2-[4-(3-{[1-(1-Adamantyl)ethyl]amino}-4-nitrophenyl)piperazin-1-yl]ethanol and
[64] 2-[4-[3-(3,4-Dimethoxyphenethylamino)-4-nitrophenyl]piperazin-1-yl]ethanol;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Said medicament is particularly suitable for 5-HT₆-receptor regulation and therefore for the prophylaxis and/or treatment of a disorder or a disease that is least partially mediated via 5-HT₆-receptors.

Preferably said medicament is suitable for the prophylaxis and/or treatment of a disorder or disease related to food intake, preferably for the regulation of appetite; for the maintenance, increase or reduction of body weight; for the prophylaxis and/or treatment of obesity, bulimia, anorexia, cachexia, type II diabetes (non insulin dependent diabetes mellitus), preferably type II diabetes that is caused by obesity; for the prophylaxis and/or treatment of stroke; migraine; head trauma; epilepsy; irritable colon syndrome; irritable bowl syndrome; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; chronic intermittent hypoxia; convulsions; hyperactivity disorder (ADHD, attention deficit/hyperactivity disorder); for improvement of cognition (cognitive enhancement) or cognitive memory (cognitive memory enhancement); for the prophylaxis and/or treatment of drug addiction and/or withdrawal; for the prophylaxis and/or treatment of alcohol addiction and/or withdrawal or for the prophylaxis and/or treatment of nicotine addiction and/or withdrawal.

More preferably said medicament is suitable for the prophylaxis and/or treatment of a disorder or disease related to food intake, preferably for the regulation of appetite; for the maintenance, increase or reduction of body weight; for the prophylaxis and/or treatment of obesity, bulimia, anorexia, cachexia or type II diabetes (non insulin dependent diabetes mellitus), preferably type II diabetes that is caused by obesity.

Furthermore, more preferably said medicament is suitable for improvement of cognition (cognitive enhancement) or cognitive memory (cognitive memory enhancement).

Furthermore, more preferably said medicament is suitable for the prophylaxis and/or treatment of drug addiction and/or withdrawal; for the prophylaxis and/or treatment of alcohol addiction and/or withdrawal or for the prophylaxis and/or treatment of nicotine addiction and/or withdrawal.

Most preferably, said medicament is suitable for the prophylaxis and/or treatment of obesity and/or disorders or diseases related thereto.

Any medicament according to the present invention may be in any form suitable for the application to humans and/or animals, preferably humans including infants, children and adults. The medicament can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modem Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The medicament of the present invention may, for example, be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or retarded release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments according to the present invention may also comprise an enteric coating, so that their dissolution is dependent on pH-value. Due to said coating the medicament can pass the stomach undissolved and the respective nitro-substituted phenyl-piperazine compound is liberated in the intestinal tract. Preferably the enteric coating is soluble at a pH value of 5 to 7.5. Suitable materials and methods for the preparation are known from the prior art.

Typically, the medicaments according to the present invention may contain 1-60 % by weight of one or more nitro-substituted phenyl-piperazine compounds as defined herein and 40-99 % by weight of one or more auxiliary substances (additives).

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from1 to 2000 mg, preferably 1 to 1500 mg, more preferably 1 to 1000 milligrams of active substance to be administered during one or several intakes per day.

The use of at least one nitro-substituted phenyl-piperazine compound of general formula I or la given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the prophylaxis and/or treatment of a disorder or disease related to food intake, preferably for the regulation of appetite; for the maintenance, increase or reduction of body weight; or for the prophylaxis and/or treatment of obesity, bulimia, anorexia, cachexia or type II diabetes (non insulin dependent diabetes mellitus), more preferably for the prophylaxis and/or treatment of obesity is preferred.

The use of at least one nitro-substituted phenyl-piperazine compound of general formula I or la given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the prophylaxis and/or treatment of stroke; migraine; head trauma; epilepsy; irritable colon syndrome; irritable bowl syndrome; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; chronic intermittent hypoxia; convulsions; or hyperactivity disorder (ADHD, attention deficit/hyperactivity disorder) is preferred.

Also preferred is the use of at least one nitro-substituted phenyl-piperazine compound of general formula I or la given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the improvement of cognition (cognitive enhancement) and/or for the improvement of cognitive memory (cognitive memory enhancement).

Also preferred is the use of at least one nitro-substituted phenyl-piperazine compound of general formula I or la given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the prophylaxis and/or treatment of drug addiction and/or withdrawal, preferably for the prophylaxis and/or treatment of addiction and/or withdrawal related to one or more of drugs selected from the group consisting of benzodiazepines, natural, semisynthetic or synthetic opioids like cocaine, ethanol and/or nicotine.

More preferred is the use of at least one nitro-substituted phenyl-piperazine compound of general formula I or la given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the prophylaxis and/or treatment of a disorder or disease related to food intake, preferably for the regulation of appetite; for the maintenance, increase or reduction of body weight; or for the prophylaxis and/or treatment of obesity, bulimia, anorexia, cachexia or type II diabetes (non insulin dependent diabetes mellitus), more preferably for the prophylaxis and/or treatment of obesity.

More preferred is the use of at least one nitro-substituted phenyl-piperazine compound of general formula I or la given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the improvement of cognition (cognitive enhancement) and/or for the improvement of cognitive memory (cognitive memory enhancement).

More preferred is the use of at least one nitro-substituted phenyl-piperazine compound of general formula I or la given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the prophylaxis and/or treatment of drug addiction and/or withdrawal, preferably for the prophylaxis and/or treatment of addiction and/or withdrawal related to one or more of drugs selected from the group consisting of benzodiazepines, natural, semisynthetic or synthetic opioids like cocaine, ethanol and/or nicotine.

Most preferred is the use of at least one nitro-substituted phenyl-piperazine compound of general formula I or la as defined above, optionally in form of one of its, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for the prophylaxis and/or treatment of obesity and/or disorders or diseases related thereto.

In the following methods for determining the pharmacological activity of the nitro-substituted phenyl-piperazine compounds are described.

### PHARMACOLOGICAL METHODS:

### I) BINDING TO SEROTONIN RECEPTOR 5-HT₆

Cell membranes of HEK-293 cells expressing the 5HT₆-human recombinant receptor were supplied by Receptor Biology. In said membranes the receptor concentration is 2.18 pmol/mg protein and the protein concentration is 9.17 mg/ml. The experimental protocol follows the method of B. L. Roth et al. [B. L. Roth, S. C. Craigo, M. S. Choudhary, A. Uluer, F. J. Monsma, Y. Shen, H. Y. Meltzer, D. R. Sibley: Binding of Typical and Atypical Antipsychotic Agents to 5-Hydroxytryptamine-6 and Hydroxytryptamine-7 Receptors. The Journal of Pharmacology and Experimental Therapeutics, 1994, 268, 1403] with the following slight changes. The respective part of the literature description is hereby incorporated by reference and forms part of the disclosure.

The commercial membrane is diluted (1:40 dilution) with the binding buffer: 50 mM Tris-HCl, 10 mM MgCl₂, 0.5 mM EDTA (pH 7.4). The radioligand used is [³H]-LSD at a concentration of 2.7 nM with a final volume of 200 µl. Incubation is initiated by adding 100 µl of membrane suspension, (= 22.9 µg membrane protein), and is prolonged for 60 minutes at a temperature of 37 °C. The incubation is ended by fast filtration in a Brandel Cell Harvester through fiber glass filters made by Schleicher & Schuell GF 3362 pretreated with a solution of polyethylenimine at 0.5 %. The filters are washed three times with three milliliters of buffer Tris-HCl 50 mM pH 7.4. The filters are transferred to flasks and 5 ml of Ecoscint H liquid scintillation cocktail are added to each flask. The flasks are allowed to reach equilibrium for several hours before counting with a Wallac Winspectral 1414 scintillation counter. Non-specific binding is determined in the presence of 100 µM of serotonin. Tests were made in triplicate. The inhibition constants (Kᵢ, nM) were calculated by non-linear regression analysis using the program EBDA/LIGAND described in Munson and Rodbard, Analytical Biochemistry, 1980, 107, 220, the respective part of which is hereby incorporated by reference and forms part of the disclosure.

### II.) FOOD INTAKE MEASUREMENT (BEHAVIOURAL MODEL):

Male W rats (200-270 g) obtained from Harlan, S.A. are used. The animals are acclimatized to the animal facility for at least 5 days before they are subjected to any treatment. During this period the animals are housed (in groups of five) in translucid cages and provided with food and water ad libitum. At least 24 hours before the treatment starts, the animals are adapted to single-housing conditions.

The acute effect of the substituted nitro-substituted phenyl-piperazine compounds according to the present invention in fasted rats is then determined as follows:
The rats were fasted for 23 hours in their single homecages. After this period, the rats are orally or intraperitoneally dosed with a composition comprising a nitro-substituted phenyl-piperazine compound or a corresponding composition (vehicle) without said nitro-substituted phenyl-piperazine compound. Immediately afterwards, the rat is left with preweighed food and cumulative food intake is measured after 1, 2, 4 and 6 hours.

Said method of measuring food intake is also described in the literature publications of Kask et al., European Journal of Pharmacology 414 (2001), 215-224 and Turnbull et al., Diabetes, Vol. 51, August 2002. The respective parts of the descriptions are hereby incorporated by reference and form part of the disclosure.

The present invention is illustrated below with the aid of examples. These illustrations are given solely by way of example and do not limit the general spirit of the present invention.

### Examples

### Abbreviations

- aq.: aqueous
- biph: biphenyl
- br: broad
- d: doublett
- dba: dibenzylidene acetone
- DCM: dichlormethane
- DME: 1,2-dimethoxyethan
- dppf: 1,1-bis(diphenylphosphino-ferrocene
- EA: ethyl acetate
- eq.: equivalent
- h: hours
- HPLC: high performance liquid chromatography
- m: multiplett
- MeOH: methanol
- OAc: acetate
- O*t*Pent: *tert*-pentoxide
- PE: petroleum ether
- prep.: preparative
- s: singulett
- t: triplett
- *t*Bu: tert-butyl
- THF: tetrahydrofuran
- TLC: thin layer chromatography
- xantphos: 4,5-bis(diphenylphosphino)-9,9-dimethylxanthene

### Preparation of example compounds 1, 2, 5, 7, 8, 9 and 10

### Step 1.

The starting material 4-bromo-2-chloro-1-nitro-benzene was obtained by oxidation from 4-bromo-2-chloro-aniline according to the method described in the reference by A. McKillop et al., Tetrahedron 1987, 43, 1753.

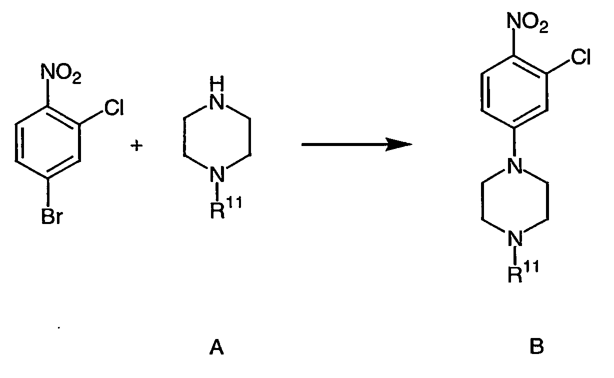

An oven-dried vial was charged subsequently with 0.05 eq. PdCl₂(dppf), 0.15 eq. dppf, 1.0 eq. 4-bromo-2-chloro-1-nitro-benzene and 1.25 eq. sodium *tert*-pentoxide. The vial was evacuated and purged with argon, and THF and piperazine of general formula A (1.1 eq.) were added. THF was added in a quantity to obtain a 0.5 M solution of 4-bromo-2-chloro-1-nitro-benzene. The vial was sealed and heated to 100°C for 3 h.

The reaction mixture was taken up in half-saturated brine and extracted three times with ethyl acetate. The volume of the combined organic phases was reduced in vacuo and extracted with 5% hydrochloric acid in water. The acidic aqueous phase was concentrated and the product was isolated by preparative HPLC. In order to remove any ammonium formate derivative formed during preparative HPLC on RP18, the resulting product was taken up in chloroform, extracted twice with saturated aqueous NaHCO₃, the combined aqueous phases were re-extracted with chloroform, and the combined organic phases were dried over MgSO₄ and concentrated in vacuo to obtain the desired product.

Said process was carried out according to the methode described in the reference of J. F. Hartwig et al., J. Am. Chem. Soc. 1996,118, 7217-7218. The respective part of the description is hereby incorporated by reference and forms part of the present disclosure.

### Step 2.

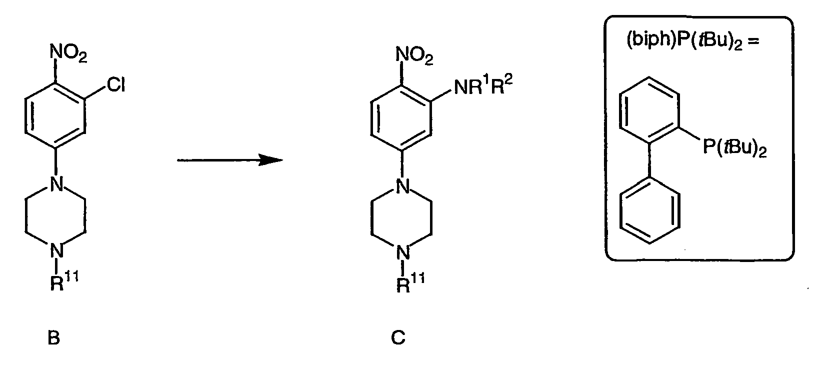

An oven-dried vial was subsequently charged with 0.1 eq. of a palladium source [Pd], 0.2 eq. (biph)P(*t*Bu)₂, 1.0 eq. arylhalide BI and 1.4 eq. base. The vial was evacuated and purged with argon, and the respective amine HNR¹R² (1.3 eq.) was added. The solvent was added to obtain a concentration of 1.0 M of the arylhalide B. The vial was sealed and heated to either 100°C in case of toluene as the solvent or 110°C in case of DME as the solvent in each case for 22 h. The mixture was worked up differently.

### Work-up 1:

The mixture was filtered through a pipette stuffed with cotton wool and then directly mounted on a preparative TLC plate (1 mm silica gel plate per 0.4 mmol starting aryl halide). The reaction solvent was removed in a vigorous stream of air, and separation was achieved using petroleum ether / ethyl acetate (PE/EA) mixtures.

### Work-up 2:

The reaction mixture was transferred into a flask using H₂O and MeOH and the total volume was reduced to less than 10 mL. Purification was achieved by prep. HPLC. In order to remove any ammonium formate derivative formed during preparative HPLC, the resulting product was taken up in chloroform, extracted twice with saturated aq. NaHCO₃, the combined aq. phases were re-extracted with chloroform, and the combined organic phases were dried over MgSO₄.

The different reaction conditions and work-up procedures are listed in table 1.
Said process was carried out according to the methode described in the reference of S.L. Buchwald et al. J. Am. Chem. Soc. 2002, 124, 6043-6048. The respective part of the description is hereby incorporated by reference and forms part of the present disclosure.

**Table 1.**

| **Example** | **[Pd]** | **base** | **solvent** | **R¹** | **R²** | **product** | **Work-up** |
|---|---|---|---|---|---|---|---|
| 2 | Pd(OAc)₂ | K₃PO₄ | DME | 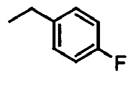 | H | 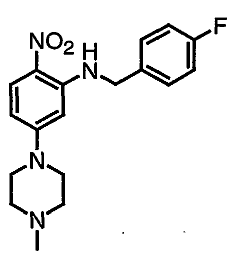 | 2 |
| 5 | Pd(OAc)₂ | K₃PO₄ | DME | 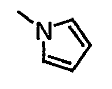 | H | 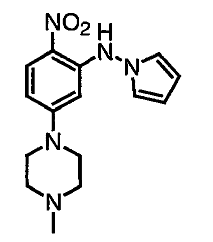 | 2 |
| 1 | Pd₂dba₃ | NaOtPent | toluene | 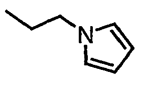 | H | 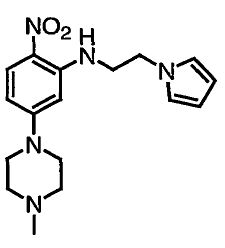 | 2 |
| 7 | Pd₂dba₃ | NaOtPent | toluene | 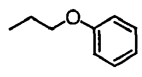 | H | 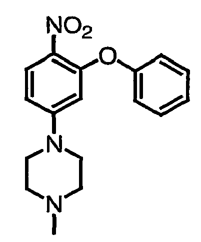 | 1 (CH₂Cl₂/ MeOH 12:1) +4 |
| 8 | Pd₂dba₃ | NaOtPent | toluene | 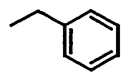 | H | 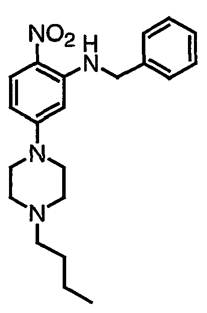 | 2 + prep. TLC (PE/CH₂C I₂/MeOH 16:4:1) |
| 10 | Pd₂dba₃ | NaOtPent | toluene | 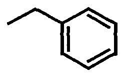 | H | 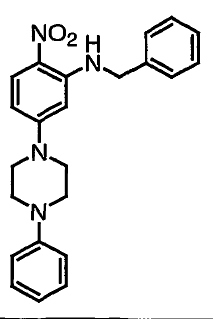 | 2 + prep. TLC (CH₂Cl₂/ MeOH 9:1) |
| 9 | Pd₂dba₃ | NaOtPent | toluene | 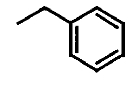 | H | 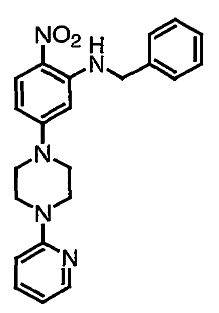 | 4 + prep. TLC (CH₂Cl₂/ MeOH 9:1) |

The ¹H-NMR for the example compounds 1, 2, 5, 7, 8, 9 and 10 are as follows:
Compound 1. [5-(4-Methyl-piperazin-1-yl)-2-nitro-phenyl]-(2-pyrrol-1-yl-ethyl)-amine
   ¹H (300 MHz, CDCl₃): δ 8.48 (*br* s, 1H), 8.06 (d, 1H), 6.71 (dd, 2H), 6.22 (dd, 1H), 6.17 (dd, 2H), 5.69 (d, 1H), 4.20 (dd, 2H), 3.62 (dd, 2H), 3.39 (dd, 4H), 2.54 (dd, 4H), 2.37 (s, 3H)
Compound 2. (4-Fluoro-benzyl)-[5-(4-methyl-piperazin-1-yl)-2-nitro-phenyl]-amine
   ¹H (300 MHz, CDCl₃): δ 8.72 (*br s,* 1H), 8.08 (d, 1H), 7.33 (t, 2H), 7.05 (t, 2H), 6.22 (dd, 1H), 5.82 (d, 1H), 4.85 (d, 2H), 3.36 (dd, 4H), 2.6 (*br* s*,* 4H), 2.41 (s, 3H)
Compound 5. [5-(4-Methyl-piperazin-1-yl)-2-nitro-phenyl]-pyrrol-1-yl-amine
   ¹H (300 MHz, CDCl₃CD₃OD): δ 8.17 (s, 1H), 8.10 (d, 1H), 6.76 (dd, 2H), 6.46 (dd, 1H), 6.25 (dd, 2H), 5.36 (d, 1H), 3.47 (dd, 4H), 3.10 (dd, 4H), 2.76 (s, 3H)
Compound 7. 1-Methyl-4-(4-nitro-3-phenoxy-phenyl)-piperazine
   ¹H (300 MHz, CDCl₃): δ 8.06 (d, 1H), 7.35 (t, 2H), 7.14 (t, 1H), 7.00 (*br* d, 2H), 6.60 (dd, 1H), 6.34 (d, 1H), 3.30 (t, 4H), 2.48 (t, 4H), 2.32 (s, 3H)
Compound 8. Benzyl-[5-(4-butyl-piperazin-1-yl)-2-nitro-phenyl]-amine
   ¹H (300 MHz, d₆-acetone): δ 8.78 (*br* s, 1H), 7.98 (d, 1H), 7.46 (d, 2H), 7.38 (m, 2H), 7.31 (m, 1H), 6.38 (dd, 1H), 6.08 (d, 1H), 4.65 (d, 2H), 3.36 (t, 4H), 2.45 (t, 4H), 2.32 (t, 2H), 1.49 - 1.33 (m, 4H), 0.91 (t, 3H)
Compound 9. Benzyl-[2-nitro-5-(4-pyridin-2-yl-piperazin-1-yl)-phenyl]-amine
   ¹H (300 MHz, CDCl₃): δ 8.21 (*br s,* 1H), 8.11 (d, 1H), 7.98 (d, 1H), 7.54 - 7.46 (m, 1H), 7.48 (d, 2H), 7.37 - 7.22 (m, 3H), 6.69 - 6.64 (m, 1H), 6.45 (dd, 1H), 6.24 (dd, 1H), 5.85 (d, 1H), 4.53 (d, 2H), 3.77 - 3.63 (m, 6H), 3.46 (m, 2H)
Compound 10. Benzyl-[2-nitro-5-(4-phenyl-2-yl-piperazin-1-yl)-phenyl]-amine
   ¹H (300 MHz, CDCl₃): δ 8.78 (*br s,* 1H), 8.14 (d, 1H), 7.47 - 7.27 (m, 10H), 6.24 (dd, ¹H),5.91 (d, 1H), 4.54 (d, 2H), 3.82 (m, 4H), 3.39 (m, 2H), 3.05 (m, 2H)

### Preparation of example compounds 3, 4, 6 and 21

The starting material 2-bromo-4-chloro-1-nitro-benzene was obtained by oxidation from 2-bromo-4-chloro-aniline according a method described in the reference by A. McKillop et al., Tetrahedron 1987, 43, 1753.

### Step 1.

### Preparation of intermediate compounds 3-1, 4-1 and 6-1

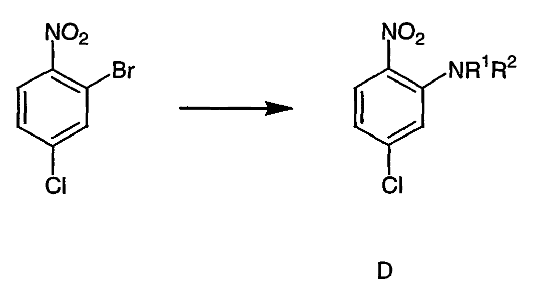

An oven-dried vial was subsequently charged with a palladium source [Pd], xantphos (1.5 eq. to Pd), 1.0 eq. 2-bromo-4-chloro-1-nitro-benzene, 1.1 eq. amide HNR¹R² and 1.4 eq. base. The tube was evacuated, purged with argon and dioxane was added to obtain a concentration of 1.0 M for 2-bromo-4-chloro-1-nitro-benzene. The vial was sealed and heated to 110°C for 21 h. The mixture was worked up differently.

### Work-up 1:

The mixture was filtered through a pipette stuffed with cotton wool and then directly mounted on a preparative TLC plate (1 mm silica gel plate per 0.4 mmol starting aryl halide). The reaction solvent was removed in a vigorous stream of air, and separation was achieved using petroleum ether / ethyl acetate (PE/EA) mixtures.

### Work-up 2:

The reaction mixture was taken up in half-saturated brine and extracted three times with ethyl acetate. The combined organic phases were dried over MgSO₄, concentrated and purified by preparative TLC (1 mm silica gel plate per 0.4 mmol starting aryl halide) using PE/EA mixtures.

### Work-up 3:

The reaction mixture was transferred into a flask using H₂O and MeOH. The total volume was reduced to less than 10 mL and purification was achieved by preparative HPLC. In order to remove any ammonium formate derivative formed during preparative HPLC on RP18, the resulting product was taken up in chloroform, extracted twice with saturated aq. NaHCO₃, the combined aq. phases were re-extracted with chloroform, and the combined organic phases were dried over MgSO₄.

Said process was carried out according to the methode described in the reference of S.L. Buchwald et al. J. Am. Chem. Soc. 2002, 124, 6043-6048. The respective part of the description is hereby incorporated by reference and forms part of the present disclosure.

The different reaction conditions and work-up procedures are listed in the following table 2.

**Table 2.**

| **Example** | **[Pd] (eq.)** | **base** | **amide** | **Work-up** |
|---|---|---|---|---|
| 4-I | Pd(OAc)₂ (0.03) | Cs₂CO₃ | 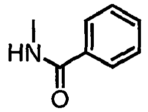 | 1 (PE/EA = 5:1) or 2 |
| 3-I | Pd(OAc)₂ (0.06) | Cs₂CO₃ | 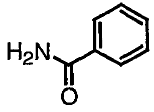 | 2 (PE/EA = 4:1) |
| 6-I | Pd₂(dba)₃ (0.02) | K₃PO₄ | 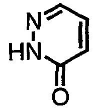 | 3 |

### Preparation of intermediate compound 21-I

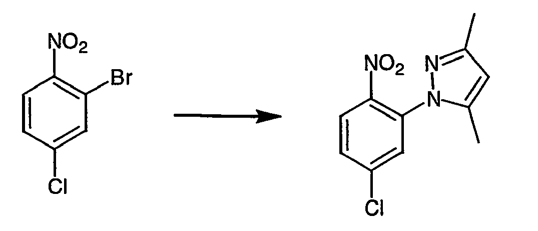

An oven-dried vial was subsequently charged with 0.1 eq. Cul, 1.0 eq. 2-bromo-4-chloro-1-nitro-benzene, 1.2 eq. 3,5-dimethylpyrazole and 2.1 eq. K₃PO₄. The vial was evacuated and purged with argon and 0.3 eq. *trans*-1,2-diaminomethyl-cyclohexane was added. Toluene was added to obtain a concentration of 1.0 M for 2-bromo-4-chloro-1-nitro-benzene . The vial was sealed and heated to 110°C for 21 h.

The mixture was filtered through a pipette stuffed with cotton wool and then directly mounted on a preparative TLC plate (1 mm silica gel plate per 0.4 mmol starting aryl halide). The reaction solvent was removed in a vigorous stream of air, and separation was achieved using petroleum ether / ethyl acetate (PE/EA 5:1 VolumeNolume) mixtures.

Said process was carried out according to the methode described in the reference of S.L. Buchwald et al. J. Am. Chem. Soc. 2002, 124, 11684-11688. The respective part of the description is hereby incorporated by reference and forms part of the present disclosure.

### Preparation of example compounds 4, 5, 6 and 21

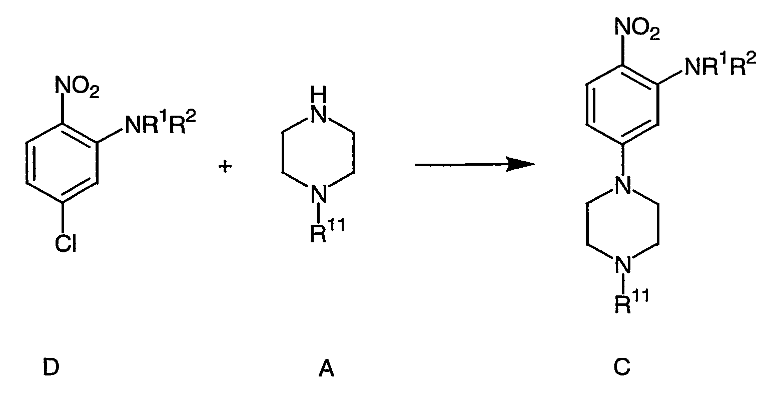

An oven-dried vial was subsequently charged with 0.1 eq. of a palladium source [Pd], 0.2 eq. (biph)P(*t*Bu)₂, 1.0 eq. arylhalide D and 1.4 eq. base. The vial was evacuated and purged with argon, and the respective piperazine A (1.3 eq.) was added. The solvent was added to obtain a concentration of 1.0 M of the arylhalide D. The vial was sealed and heated to either 100°C in case of toluene as the solvent or 110°C in case of DME as the solvent in each case for 22 h. The reaction mixture was transferred into a flask using H₂O and MeOH and the total volume was reduced to less than 10 mL. Purification was achieved by preparative HPLC on RP18. In order to remove any ammonium formate derivative formed during preparative HPLC, the resulting product was taken up in chloroform, extracted twice with saturated aq. NaHCO₃, the combined aq. phases were re-extracted with chloroform, and the combined organic phases were dried over MgSO₄.

Said process was carried out according to the methode described in the reference of S.L. Buchwald et al. J. Am. Chem. Soc. 2002, 124, 6043-6048. The respective part of the description is hereby incorporated by reference and forms part of the present disclosure.

The different reaction conditions are listed in the following table 3.

**Table 3.**

| **Example** | **[Pd]** | **base** | **solvent** | **amine** | **product** |
|---|---|---|---|---|---|
| 4 | Pd(OAc)₂ | K₃PO₄ | DME | 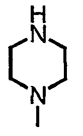 | 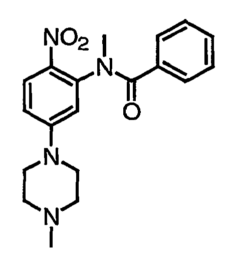 |
| 3 | Pd(OAc)₂ | K₃PO₄ | DME | 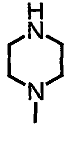 | 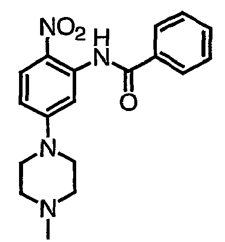 |
| 6 | Pd(OAc)₂ | K₃PO₄ | DME | 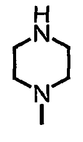 | 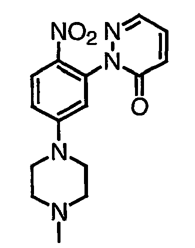 |
| 21 | Pd₂dba₃ | NaOtPent | toluene | 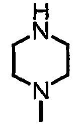 | 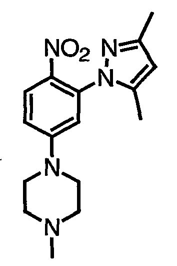 |

The ¹H-NMR for the example compounds 3, 4 and 6 are as follows:
Compound 3. N-[5-(4-Methyl-piperazin-1-yl)-2-nitro-phenyl]-benzamide
   ¹H (300 MHz, CDCl₃): δ 12.02 (s, 1H), 8.59 (d, 1H), 8.21 (d, 1H), 8.02 - 7.99 (m, 2H), 7.60 - 7.50 (m, 3H), 6.57 (dd, 1H), 3.57 (dd, 4H), 2.58 (dd, 4H), 2.38 (s, 3H)
Compound 4. N-Methyl-N-[5-(4-methyl-piperazin-1-yl)-2-nitro-phenyl]-benzamide
   ¹H (300 MHz, CDCl₃): δ 7.94 (*br s,* 1H), 7.28 - 7.18 (m, 5H), 6.63 *(br s,* 1H), 6.45 (*br* s, 1H), 3.38 (*brs,* 3H), 3.29 (*br s,* 4H), 2.48 *(br s,* 4H), 2.33 (s, 3H)
Compound 6. 2-[5-(4-Methyl-piperazin-1-yl)-2-nftro-phenyl]-2H-pyridazin-3-one
   ¹H (300 MHz, CDCl₃): δ 8.16 (d, 1H), 7.87 (dd, 1H), 7.28 (dd, 1H), 7.03 (dd, 1H), 6.88 (dd, 1H), 6.79 (d, 1H), 3.45 (t, 4H), 2.54 (t, 4H), 2.35 (s, 3H)

### Compound 21. 1-[3-(3,5-Dimethyl-pyrazol-1-yl)-4-nitro-phenyl]-4-methyl-piperazine

The compounds of the examples 11 to 20 and 22 to 64 have been prepared accordingly. Those skilled in the art will realize which starting materials were used to obtain the respective compounds.
[11] Furan-2-ylmethyl-[5-(4-methyl-piperazin-1-yl)-2-nitro-phenyl]-amine,
[12] 2-[4-(4-Nitro-3-phenethylamino-phenyl)-piperazin-1-yl]-ethanol,
[13] (2-Nitro-5-piperazin-1-yl-phenyl)-(2-o-tolyloxy-ethyl)-amine
[14] 2-[4-(3-Benzylamino-4-nitro-phenyl)-piperazin-1-yl]-ethanol,
[15] 4-[5-(4-Methyl-piperazin-1-yl)-2-nitro-phenyl]-morpholine,
[16] 2-[5-(4-Methyl-piperazin-1-yl)-2-nitro-phenyl]-4-phenyl-2H-phthalazin-1-one,
[17] [2-(4-Chloro-phenoxy)-ethyl]-[5-(3,5-dimethyl-piperazin-1-yl)-2-nitrophenyl]-amine,
[18] 2-[4-[3-(Benzhydryl-amino)-4-nitro-phenyl]-piperazin-1-yl]-ethanol,
[19] 4-[4-Fluoro-5-(4-methyl-piperazin-1-yl)-2-nitro-phenyl)-morpholine,
[20] 2-[2-Nitro-5-[4-(toluene-4-sulfonyl)-piperazin-1-yl]-phenyl]-1,2,3,4-tetrahydro-isoquinoline,
[22] Benzyl-(2-nitro-5-piperazin-1-yl-phenyl)-amine,
[23] [5-(4-Methyl-piperazin-1-yl)-2-nitro-phenyl]-phenethyl-amine,
[24] [5-(4-Methyl-piperazin-1-yl)-2-nitro-phenyl]-pyridin-3-ylmethyl-amine,
**[25] (3-Chloro-phenyl)-[4-[3-[(furanyl-2-ylmethyl)-amino]-4-nitro-phenyl]-piperazin-1-yl]-methanone,**
[26] (2-Nitro-5-piperazin-1-yl-phenyl)-pyridin-3-ylmethyl-amine,
[27] 1-Benzyl-4-(2-nitro-5-piperazin-1-yl-phenyl)-piperazine,
[28] Furan-2-ylmethyl-[5-(4-methanesulfonyl-piperazin-1-yl)-2-nitro-phenyl]-amine,
[29] Benzhydryl-[5-(4-methyl-piperazin-1-yl)-2-nitro-phenyl]-amine,
[30] (2-Nitro-5-piperazin-1-yl-phenyl)-(2-phenoxy-ethyl)-amine,
[31] 2-[5-(4-Methyl-piperazin-1-yl)-2-nitro-phenyl]-4-phenyl-2H-phthalazin-1-one,
[32] 1-[3-(3,5-Dimethyl-pyrazof-1-yl)-4-nitro-phenyl]-piperazine,
[33] (2-Nitro-5-piperazin-1-yl-phenyl)-phenethyl-amine,
[34] [5-(4-Benzenesulfonyl-piperazin-1-yl)-2-nitro-phenyl]-furan-2-ylmethyl-amine,
[35] [2-(3,4-Dimethoxy-phenyl)-ethyl]-(2-nitro-5-piperazin-1-yl-phenyl)-amine,
[36] [4-[3-[(Furan-2-ylmethyl)-amino]-4-nitro-phenyl]-piperazin-1-yl]-m-tolyl-methanone,
[37] [4-[3-[(Furan-2-ylmethyl)-amino]-4-nitro-phenyl]-piperazin-1-yl]-phenyl-methanone,
[38] [4-[3-(3,5-Dimethyl-pyrazol-1-yl)-4-nitro-phenyl]-piperazin-1-yl]-thiophen-2-yl-methanone,
[39] 4-(4-Ethyl-phenyl)-2-[5-[4-methyl-piperazin-1-yl)-2-nitro-phenyl]-2H-phthalalazin-1-one,
[40] 3-[7-(4-Methyl-piperazin-1-yl)-4-nitro-benzo[1,2,5]oxadiazol-5-ylamino]-propan-1-ol,
[41] 3-[4-Nitro-7-(4-pheny)-piperazin-1-yl)-benzo[1,2,5]oxadiazol-5-ylamino]-ethan-1-ol,
[42] 3-[4-Nitro-7-(4-pyddin-piperazin-1-yl)-benzo[1,2,5]oxadiazol-5-ylamino]-propan-1-ol,
[43] [2-(3,4-Dimethoxy-phenyl)-ethyl]-[5-(4-methyl-piperazin-1-yl)-2-nitrophenyl]-amine,
[44] [2-(3,4-Dimethyl-phenyl)-ethyl]-[5-(4-methyl-piperazin-1-yl)-2-nitrophenyl]-amine,
[45] [2-(4-tert-Butyl-phenoxy)-ethyl]-[5-(4-methyl-piperazin-1-yl)-2-nitrophenyl]-amine,
[46] [2-(4-Methoxy-phenoxy)-ethyl]-[5-(4-methyl-piperazin-1-yl)-2-nitrophenyl]-amine,
[47] [5-(4-Methyl-piperazin-1-yl)-2-nitro-phenyl]-(2-m-tolyloxy-ethyl)-amine,
[48] [2-(4-Chloro-phenoxy)-ethyl]-[5-(4-methyl-piperazin-1-yl)-2-nitro-phenyl]-amine,
[49] (2-Nitro-5-piperazin-1-yl-phenyl)-(1-phenyl-ethyl)-amine,
[50] [2-(3-Methoxy-phenoxy)-ethyl]-[5-(4-methyl-piperazin-1-yl)-2-nitrophenyl]-amine,
[51] [2-(2-Methoxy-phenoxy)-ethyl]-[5-(4-methyl-piperazin-1-yl)-2-nitrophenyl]-amine,
[52] (4-Chloro-benzyl)-(2-nitro-5-piperazin-1-yl-phenyl)-amine,
[53] Benzyl-[5-(4-benzyl-piperazin-1-yl)-2-nitro-phenyl]-amine,
[54] Benzyl-[5-(4-methyl-piperazin-1-yl)-2-nitro-phenyl]-amine,
[55] [5-(4-Methyl-piperazin-1-yl)-2-nitro-phenyl]-(2-o-tolyloxy-ethyl)-amine,
[56] (4-Chloro-benzyl)-[5-(4-methyl-piperazin-1-yl)-2-nitro-phenyl]-amine,
[57] Furan-2-ylmethyl-(2-nitro-5-piperazin-1-yl-phenyl)-amine,
[58] [2-(4-Chloro-phenoxy)-ethyl]-(2-nitro-5-piperazin-1-yl-phenyl)-amine,
[59] [5-(4-Methyl-piperazin-1-yl)-2-nitro-phenyl]-(1-phenyl-ethyl)-amine
[60] 1-[4-(3-Benzylamino-4-nitro-phenyl)-piperazin-1-yl]-ethanone
[61] 2-[4-[3-(4-Methylpiperazin-1-yl)-4-nitrophenyl]piperazin-1-yl]ethanol,
[62] 2-[4-[3-[2-(Naphthalen-2-yloxy)ethylamino]-4-nitrophenyl]piperazin-1-yl]ethanol,
[63] 2-[4-(3-{[1-(1-Adamantyl)ethyl]amino}-4-nitrophenyl)piperazin-1-yl]ethanol and
[64] 2-[4-[3-(3,4-Dimethoxyphenethylamino)-4-nitrophenyl]piperazin-1-yl]ethanol

### Pharmacological data:

The binding of the nitro-substituted phenyl-piperazine compounds to the 5-HT₆ receptor was determined as described above.

The binding results for some of these compounds are given in the following table :

| **Compound according to example:** | **Kᵢ (nM)** |
|---|---|
| 2 | 172.4 |
| 5 | 480.8 |
| 7 | 26.6 |
| 11 | 61.2 |
| 12 | 6332.2 |
| 13 | 162.5 |
| 15 | 2710.1 |
| 16 | 732.0 |
| 17 | 5002.7 |
| 21 | 249.5 |
| 22 | 27.5 |
| 23 | 118.5 |
| 24 | 1266.7 |
| 26 | 1511.5 |
| 27 | 3778.8 |
| 29 | 4489.0 |
| 30 | 114.1 |
| 31 | 1710.1 |
| 32 | 481.8 |
| 33 | 33.1 |
| 45 | 80.3 |
| 46 | 245.2 |
| 51 | 19.1 |
| 52 | 8.3 |
| 53 | 34.3 |
| 55 | 26.1 |
| 56 | 59.0 |
| 57 | 25.0 |
| 58 | 289.9 |
| 59 | 11.9 |

## Claims

1. A nitro-substituted phenyl-piperazine compound of general formula I, 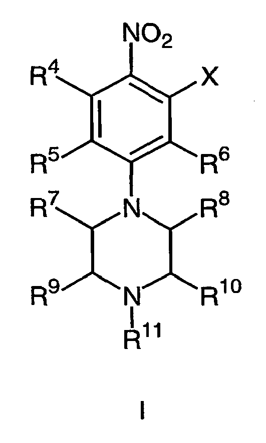 wherein
X represents a -NR¹R² moiety or a -OR³ moiety;
R¹ represents a saturated or unsaturated 3- to 9-membered cycloaliphatic radical, which may be substituted with 1, 2 or 3 substituent(s) independently selected from the group consisting of oxo (=O), thioxo (=S), C₁-₅-alkyl, -O-C₁₋₅-alkyl, -S-C₁₋₅-alkyl, -C(=O)-OH, -C(=O)-C₁₋₅- alkyl, -C(=O)-O-C₁₋₅-alkyl, -O-C(=O)-C₁₋₅-alkyl, F, Cl, Br, I, -CN, -CF₃, - OCF₃, -SCF₃, -OH, -SH, -NH₂, -NH(C₁₋₅-alkyl), -N(C₁₋₅-alkyl)₂, -NO₂, - CHO, -CF₂H, -CFH₂, -C(=O)-NH₂, -C(=O)-NH(C₁₋₅-alkyl), -C(=O)-N(C₁₋₅- alkyl)₂, -S(=O)₂-C₁₋₅-alkyl, -S(=O)₂-phenyl, phenyl, phenoxy and benzyl and which may contain 1, 2 or 3 heteroatom(s) independently selected from the group consisting of nitrogen, oxygen and sulfur as ring member(s) and which is bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH, - NH₂, -O-C₁₋₅-alkyl, -S-C₁₋₅-alkyl, -NH-(C₁₋₅-alkyl) and -N(C₁₋₅-alkyl)₂; with the proviso that R¹ does not represent a tetrahydrofuranyl radical;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1- b]thiazolyl which may be substituted with 1, 2, 3, 4 or 5 substituent(s) independently selected from the group consisting of C₁₋₅-alkyl, -O-C₁₋₅- alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, - NH₂, -NH(C₁₋₅-alkyl), -N(C₁₋₅-alkyl)₂, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁₋₅- alkyl, -S(=O)₂-phenyl, phenyl, phenoxy and benzyl;
an aryl or heteroaryl radical selected from the group consisting of phenyl, naphthyl, thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl, quinolinyl, isoquinolinyl, benzo[b]furanyl, benzo[b]thiophenyl, benzothiadiazolyl and imidazo[2,1- b]thiazolyl, whereby said aryl or heteroaryl radical is bonded via a - (CH₂)-group and may be substituted with 1, 2, 3, 4 or 5 substituent(s) independently selected from the group consisting of C₁₋₅-alkyl, -O-C₁₋₅- alkyl, -S-C₁₋₅-alkyl, -C(=O)-C₁₋₅-alkyl, -C(=O)-O-C₁₋₅-alkyl, -O-C(=O)-C₁- ₅-alkyl, F, Br, I, -CN, -CF₃, -OCF₃, -SCF₃, -OH, -SH, -NH₂, -NH(C₁₋₅- alkyl), -N(C₁₋₅-alkyl)₂, -NO₂, -CHO, -CF₂H, -CFH₂, -C(=O)-NH₂, -C(=O)- NH(C₁₋₅-alkyl), -C(=O)-N(C₁₋₅-alkyl)₂, -S(=O)₂-C₁₋₅-alkyl, -S(=O)₂-Phenyl, phenyl, phenoxy and benzyl;
an aryl or heteroaryl radical selected from the group consisting of naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₂-group and/or may be substituted with 1, 2, 3, 4 or 5 substituent(s) independently selected from the group consisting of C₁₋₅-alkyl, -O-C₁₋₅-alkyl, -S-C₁₋₅-alkyl, - C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), - N(C₁₋₅-alkyl)₂, -NO₂, -CHO, -CF₂H, -CFH₂, -C(=O)-NH₂, -C(=O)-NH(C₁₋₅- alkyl), -C(=O)-N(C₁₋₅-alkyl)₂, -S(=O)₂-C₁₋₅-alkyl, -S(=O)₂-phenyl, phenyl, phenoxy and benzyl;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₃-group and/or may be substituted with 1, 2, 3, 4 or 5 substituent(s) independently selected from the group consisting of C₁₋₅-alkyl, -O-C₁₋₅-alkyl, -S-C₁₋₅-alkyl, - C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁-₅-alkyl), - N(C₁₋₅-alkyl)₂, -NO₂, -CHO, -CF₂H, -CFH₂, -C(=O)-NH₂, -C(=O)-NH(C₁₋₅- alkyl), -C(=O)-N(C₁₋₅-alkyl)₂, -S(=O)₂-C₁₋₅-alkyl, -S(=O)₂-phenyl, phenyl, phenoxy and benzyl;
or
a -C(=O)-R¹² moiety;
R² represents a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, - NH₂, -O-C₁₋₅-alkyl, -S-C₁₋₅-alkyl, -NH-(C₁₋₅-alkyl) and -N(C₁₋₅-alkyl)₂; or
R¹ and R² together with the bridging nitrogen atom form a saturated or unsaturated, but not aromatic 3- to 6-membered heterocyclic ring which contains 1, 2 or 3 additional heteroatom(s) independently selected from the group consisting of nitrogen and sulfur as ring member(s) and which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, - NH₂, -NH(C₁₋₅-alkyl), -N(C₁₋₅-alkyl)₂, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁₋₅- alkyl, -S(=O)₂-phenyl, phenyl, phenoxy and benzyl;
R³ represents a 5- to 10-membered aryl or heteroaryl radical, which may be unsubstituted or may be substituted with 1, 2, 3, 4 or 5 substituent(s) independently selected from the group consisting of C₁₋₅-alkyl, -O-C₁₋₅- alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, - NH₂, -NH(C₁₋₅-alkyl), -N(C₁₋₅-alkyl)₂, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁₋₅- alkyl, -S(=O)₂-phenyl, phenyl, phenoxy and benzyl and which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH-(C₁-₅-alkyl) and -N(C₁₋₅-alkyl)₂ and whereby the heteroaryl radical contains 1, 2 or 3 heteroatom(s) independently selected from the group consisting of nitrogen, oxygen and sulfur as ring member(s);
R⁴, R⁵ and R⁶ each represent a hydrogen atom;
R⁷ and R⁸, identical or different, each represent a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, - SCF₃, -OH, -SH, -NH₂, -O-C₁₋₅-alkyl, -S-C₁₋₅-alkyl, -NH-(C₁₋₅-alkyl) and - N(C₁₋₅-alkyl)₂;
R⁹ and R¹⁰, identical or different, each represent a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, -CN, - CF₃, -OCF₃, -SCF₃, -OH, -SH, -NH₂, -O-C₁₋₅-alkyl, -S-C₁₋₅-alkyl, -NH- (C₁₋₅-alkyl) and -N(C₁₋₅-alkyl)₂;
R¹¹ represents a linear or branched, saturated or unsaturated, unsubstituted C₁₋₁₀ aliphatic radical or a 5- to 10-membered aryl or heteroaryl radical which may be unsubstituted or may be substituted with 1, 2, 3, 4 or 5 substituent(s) independently selected from the group consisting of C₁₋₅- alkyl, -O-C₁₋₅-alkyl, -S-C₁₋₅-alkyl, -C(=O)-OH, -C(=O)-C₁₋₅-alkyl, -C(=O)- O-C₁₋₅-alkyl, -O-C(=O)-C₁₋₅-alkyl, F, Cl, Br, I, -CN, -CF₃, -OCF₃, -SCF₃, - OH, -SH, -NH₂, -NH(C₁₋₅-alkyl), -N(C₁₋₅-alkyl)₂, -NO₂, -CHO, -CF₂H, - CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl and whereby the heteroaryl radical contains 1, 2 or 3 heteroatom(s) independently selected from the group consisting of nitrogen, oxygen and sulfur as ring member(s);
with the proviso that R¹¹ does not represent a thiazolyl radical;
and
R¹² represents a 5 to 10 membered aryl radical which may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)- C₁₋₅-alkyl,-C(=O)-O-C₁₋₅-alkyl, -O-C(=O)-C₁₋₅-alkyl, F, Br, I, -CN, -CF₃, - OCF₃, -SCF₃, -SH, -NH₂, -NH(C₁₋₅-alkyl), -N(C₁₋₅-alkyl)₂, -CHO, -CF₂H, - CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1- b]thiazolyl which may be unsubstituted or may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)- C₁₋₅-alkyl, -C(=O)-O-C₁₋₅-alkyl, -O-C(=O)-C₁₋₅-alkyl, F, Cl, Br, I, -CN, - CF₃, -OCF₃, -SCF₃, -OH, -SH, -NH₂, -NH(C₁₋₅-alkyl), -N(C₁₋₅-alkyl)₂, - NO₂, -CHO, -CF₂H, -CFH₂, -C(=O)-NH₂, -C(=O)-NH(C₁₋₅-alkyl), -C(=O)- N(C₁₋₅-alkyl)₂, -S(=O)₂-C₁₋₅-alkyl, -S(=O)₂-phenyl, phenyl, phenoxy and benzyl;
or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be unsubstituted or may be substituted with 1, 2, 3, 4 or 5 substituent(s) independently selected from the group consisting of C₁₋₅-alkyl, -O-C₁₋₅-alkyl, -S-C₁₋₅-alkyl, - C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), - N(C₁₋₅-alkyl)₂, -NO₂, -CHO, -CF₂H, -CFH₂, -C(=O)-NH₂, -C(=O)-NH(C₁₋₅- alkyl), -C(=O)-N(C₁₋₅-alkyl)₂, -S(=O)₂-C₁₋₅-alkyl, -S(=O)₂-phenyl, phenyl, phenoxy and benzyl and which is bonded via a -(CH₂)₁, _{2 or 3}-group;
with the proviso that the following compound
(1) Benzyl-[5-(4-methyl-piperazin-1-yl)-2-nitro-phenyl]-amine
is excluded;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

2. A compound according to claim 1, **characterized in that**
X represents a -NR¹ R² moiety or a -OR³ moiety;
R¹ represents an (hetero)cycloaliphatic radical selected from the group consisting of cyclopropyl, cyclobutyl, cyclopentyl, cyclopentenyl, cyclohexyl, cyclohexenyl, cycloheptyl, cycloheptenyl, cyclooctyl, cyclooctenyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrothiophenyl and azepanyl, which may be substituted with 1, 2 or 3 substituent(s) independently selected from the group consisting of oxo (=O), thioxo (=S), C₁₋₅-alkyl, -O-C₁₋₅₋alkyl, -S-C₁- ₅-alkyl, -C(=O)-OH, -C(=O)-C₁₋₅-alkyl, -C(=O)-O-C₁₋₅-alkyl, -O-C(=O)-C₁- ₅-alkyl, F, Cl, Br, I, -CN, -CF₃, -OCF₃, -SCF₃, -OH, -SH, -NH₂, -NH(C₁₋₅- alkyl), -N(C₁₋₅-alkyl)₂, -NO₂, -CHO, -CF₂H, -CFH₂, -C(=O)-NH₂, -C(=O)- NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which is bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, - CF₃, -OCF₃, -SCF₃, -OH, -SH, -NH₂, -O-C₁₋₅-alkyl, -S-C₁₋₅-alkyl, -NH- (C₁₅-alkyl) and -N(C₁₋₅-alkyl)₂;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1- b]thiazolyl which may be substituted with 1, 2, 3, 4 or 5 substituent(s) independently selected from the group consisting of C₁₋₅-alkyl, -O-C₁₋₅- alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, CI, Br, I, -CN, -CF₃, -OCF₃, -SCF₃, -OH, -SH, - NH₂, -NH(C₁₋₅-alkyl), -N(C₁₋₅-alkyl)₂, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-(₁₋₅- alkyl, -S(=O)₂-phenyl, phenyl, phenoxy and benzyl;
an aryl or heteroaryl radical selected from the group consisting of phenyl, naphthyl, thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl, quinolinyl, isoquinolinyl, benzo[b]furanyl, benzo[b]thiophenyl, benzothiadiazolyl and imidazo[2,1- b]thiazolyl, whereby said aryl or heteroaryl radical is bonded via a - (CH₂)-group and may be substituted with 1, 2, 3, 4 or 5 substituent(s) independently selected from the group consisting of C₁₋₅-alkyl, -O-C₁₋₅- alkyl, -S-C₁₋₅-alkyl, -C(=O)-C₁₋₅-alkyl, -C(=O)-O-C₁₋₅-alkyl, -O-C(=O)-C₁- ₅-alkyl, F, Br, I, -CN, -CF₃, -OCF₃, -SCF₃, -OH, -SH, -NH₂, -NH(C₁₋₅- alkyl), -N(C₁₋₅-alkyl)₂, -NO₂, -CHO, -CF₂H, -CFH₂, -C(=O)-NH₂, -C(=O)- NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
an aryl or heteroaryl radical selected from the group consisting of naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₂-group and/or may be substituted with 1, 2, 3, 4 or 5 substituent(s) independently selected from the group consisting of C₁₋₅-alkyl, -O-C₁₋₅-alkyl, -S-C₁₋₅-alkyl, - C(=O)-OH, -C(=O)-C₁₋₅-alkyl, -C(=O)-O-C₁₋₅-alkyl, -O-C(=O)-C₁₋₅-alkyl, F, CI, Br, I, -CN, -CF₃, -OCF₃, -SCF₃, -OH, -SH, -NH₂, -NH(C₁₋₅-alkyl), - N(C₁₋₅-alkyl)₂, -NO₂, -CHO, -CF₂H, -CFH₂, -C(=O)-NH₂, -C(=O)-NH(C₁₋₅- alkyl), -C(=O)-N(C₁₋₅-alkyl)₂, -S(=O)₂-C₁₋₅-alkyl, -S(=O)₂-phenyl, phenyl, phenoxy and benzyl;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₃-group and/or may be substituted with 1, 2, 3, 4 or 5 substituent(s) independently selected from the group consisting of C₁₋₅-alkyl, -O-C₁₋₅-alkyl, -S-C₁₋₅-alkyl, - C(=O)-OH, -C(=O)-C₁₋₅-alkyl, -C(=O)-O-C₁₋₅-alkyl, -O-C(=O)-C₁₋₅-alkyl, F, CI, Br, I, -CN, -CF₃, -OCF₃, -SCF₃, -OH, -SH, -NH₂, -NH(C₁₋₅-alkyl), - N(C₁₋₅-alkyl)₂, -NO₂, -CHO, -CF₂H, -CFH₂, -C(=O)-NH₂, -C(=O)-NH(C₁₋₅- alkyl), -C(=O)-N(C₁₋₅-alkyl)₂, -S(=O)₂-C₁₋₅-alkyl, -S(=O)₂-phenyl, phenyl, phenoxy and benzyl;
or
a -C(=O)-R¹² moiety;
R² represents a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, - NH₂, -O-C₁₋₅-alkyl, -S-C₁₋₅-alkyl, -NH-(C₁₋₅-alkyl) and -N(C₁₋₅-alkyl)₂; or
R¹ and R² together with the bridging nitrogen atom form a saturated or unsaturated, but not aromatic 3- to 6-membered heterocyclic ring which contains 1, 2 or 3 additional heteroatom(s) independently selected from the group consisting of nitrogen and sulfur as ring member(s) and which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH, -C(=O)-C₁₋₅-alkyl, -C(=O)-O-C₁₋₅-alkyl, - O-C(=O)-C₁₋₅-alkyl, F, CI, Br, I, -CN, -CF₃, -OCF₃, -SCF₃, -OH, -SH, - NH₂, -NH(C₁₋₅-alkyl), -N(C₁₋₅-alkyl)₂, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁₋₅- alkyl, -S(=O)₂-phenyl, phenyl, phenoxy and benzyl;
R³ represents a 5- to 10-membered aryl or heteroaryl radical, which may be unsubstituted or may be substituted with 1, 2, 3, 4 or 5 substituent(s) independently selected from the group consisting of C₁₋₅-alkyl, -O-C₁₋₅- alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, - NH₂, -NH(C₁₋₅-alkyl), -N(C₁₋₅-alkyl)₂, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁₋₅- alkyl, -S(=O)₂-phenyl, phenyl, phenoxy and benzyl and which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, CI, Br, I, -CN, -CF₃, -OCF₃, -SCF₃, -OH, -SH, -NH₂, -O-C₁₋₅-alkyl, -S-C₁- ₅-alkyl, -NH-(C₁₋₅-alkyl) and -N(C₁₋₅-alkyl)₂ and whereby the heteroaryl radical contains 1, 2 or 3 heteroatom(s) independently selected from the group consisting of nitrogen, oxygen and sulfur as ring member(s);
R⁴ R⁵ and R⁶ each represent a hydrogen atom;
R⁷ and R⁸, identical or different, each represent a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, - SCF₃, -OH, -SH, -NH₂, -O-C₁₋₅-alkyl, -S-C₁₋₅-alkyl, -NH-(C₁₋₅-alkyl) and - N(C₁₋₅-alkyl)₂;
R⁹ and R¹⁰, identical or different, each represent a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, -CN, - CF₃, -OCF₃, -SCF₃, -OH, -SH, -NH₂, -O-C₁₋₅-alkyl, -S-C₁₋₅-alkyl, -NH- (C₁₋₅-alkyl) and -N(C₁₋₅-alkyl)₂;
R¹¹ represents a linear or branched, saturated or unsaturated, unsubstituted C₁₋₁₀ aliphatic radical or or an aryl or heteroaryl radical selected from the group consisting of phenyl, naphthyl, furyl (furanyl), thienyl (thiophenyl), pyrrolyl, oxazolyl, isoxazolyl, isothiazolyl, imidazolyl, pyrazolyl, oxadiazolyl, thiadiazolyl, triazolyl, pyridazinyl, pyrimidinyl, pyridinyl, pyrazinyl, quinolinyl, isoquinolinyl, benzo[b]furanyl, benzo[b]thiophenyl, benzothiadiazolyl and imidazo[2,1-b]thiazolyl, which may be unsubstituted or may be substituted with 1, 2, 3, 4 or 5 substituent(s) independently selected from the group consisting of C₁₋₅-alkyl, -O-C₁₋₅- alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, - NH₂, -NH(C₁₋₅-alkyl), -N(C₁₋₅-alkyl)₂, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁₋₅- alkyl, -S(=O)₂-phenyl, phenyl, phenoxy and benzyl;
and
R¹² represents a 5 to 10 membered aryl radical which may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)- C₁₋₅-alkyl,-C(=O)-O-C₁₋₅-alkyl, -O-C(=O)-C₁₋₅-alkyl, F, Br, I, -CN, -CF₃, - OCF₃, -SCF₃, -SH, -NH₂, -NH(C₁₋₅-alkyl), -N(C₁₋₅-alkyl)₂, -CHO, -CF₂H, - CFH₂, -C(=O)-NH₂, -C(=O)-NH(C₁₋₅-alkyl), -C(=O)-N(C₁₋₅-alkyl)2, - S(=O)₂-C₁₋₅-alkyl, -S(=O)₂-phenyl, phenyl, phenoxy and benzyl;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1- b]thiazolyl which may be unsubstituted or may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)- C₁₋₅-alkyl, -C(=O)-O-C₁₋₅-alkyl, -O-C(=O)-C₁₋₅-alkyl, F, Cl, Br, I, -CN, - CF₃, -OCF₃, -SCF₃, -OH, -SH, -NH₂, -NH(C₁₋₅-alkyl), -N(C₁₋₅-alkyl)₂, - NO₂, -CHO, -CF₂H, -CFH₂, -C(=O)-NH₂, -C(=O)-NH(C₁₋₅-alkyl), -C(=O)- N(C₁₋₅-alkyl)₂, -S(=O)₂-C₁₋₅-alkyl, -S(=O)₂-phenyl, phenyl, phenoxy and benzyl;
or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be unsubstituted or may be substituted with 1, 2, 3, 4 or 5 substituent(s) independently selected from the group consisting of C₁₋₅-alkyl, -O-C₁₋₅-alkyl, -S-C₁₋₅-alkyl, - C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), - N(C₁₋₅-alkyl)₂, -NO₂, -CHO, -CF₂H, -CFH₂, -C(=O)-NH₂, -C(=O)-NH(C₁₋₅- alkyl), -C(=O)-N(C₁₋₅-alkyl)₂, -S(=O)₂-C₁₋₅-alkyl, -S(=O)₂-phenyl, phenyl, phenoxy and benzyl and which is bonded via a -(CH₂)₁, _{2 or3}- group;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

3. A compound according to claim 1 or 2, **characterized in that** R¹ represents
an (hetero)cycloaliphatic radical selected from the group consisting of cyclopropyl, cyclobutyl, cyclopentyl, cyclopentenyl, cyclohexyl, cyclohexenyl, cycloheptyl, cycloheptenyl, cyclooctyl, cyclooctenyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrothiophenyl and azepanyl whereby said (hetero)cylcoaliphatic radical can be bonded via a -(CH₂)_{1,2 or 3}- group and/or may be substituted with 1, 2 or 3 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, CI, Br, I, - CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)2, -NO₂, -CHO, -CF₂H, - CFH₂, -C(=O)-NH₂, -C(=O)-NH-CH₃, -C(=O)-NH-C₂H₅, -C(=O)-N(CH₃)₂, - C(=O)-N(C₂H₅)₂ and -S(=O)₂-CH₃,
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, CI, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, - C(=O)-NH-CH₃, -C(=O)-NH-C₂H₅, -C(=O)-N(CH₃)₂, -C(=O)-N(C₂H₅)₂ and - S(=O)₂-CH₃,
an aryl or heteroaryl radical selected from the group consisting of phenyl, naphthyl, thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)-group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, - C(=O)-O-CH₃, -C(=O)-O-C₂H₅, -C(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃,
an aryl or heteroaryl radical selected from the group consisting of naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₂-group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, CI, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, - C(=O)-NH-CH₃, -C(=O)-NH-C₂H₅, -C(=O)-N(CH₃)₂, -C(=O)-N(C₂H₅)₂ and - S(=O)₂-CH₃,
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyle, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)₃-group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, CI, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, - C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
or
a -C(=O)-R¹² moiety;
preferably R¹ represents
an unsubstituted pyrrolyl radical;
an aryl or heteroaryl radical selected from the group consisting of phenyl and pyrrolyl, whereby said phenyl radical is bonded via a -(CH₂)-group and said pyrrolyl radical is bonded via a -(CH₂)₂-group and/or said phenyl or pyrrolyl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Br, I, -CN, -CF₃, -OCF₃, -SCF₃, - OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, - C(=O)-NH-CH₃, -C(=O)-NH-C₂H₅, -C(=O)-N(CH₃)₂, -C(=O)-N(C₂H₅)₂ and - S(=O)₂-CH₃; or
a -C(=O)-R¹² moiety;
more preferably R¹ represents an unsubstituted pyrrolyl radical;
an aryl or heteroaryl radical selected from the group consisting of phenyl and pyrrolyl, whereby said phenyl radical is bonded via a -(CH₂)-group and said pyrrolyl radical is bonded via a -(CH₂)₂-group and/or said phenyl or pyrrolyl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, F, Br and I or
a -C(=O)-R¹² moiety.

4. A compound according to one or more of claims 1 to 3, **characterized in that**
R² represents a hydrogen atom or a linear or branched, unsubstituted C₁₋₁₀ alkyl radical;
preferably R² represents a hydrogen atom or an alkyl radical selected from the group consisting of methyl, ethyl, n-propyl, isopropyl, n-butyl, sec-butyl, isobutyl and tert-butyl;
more preferably R² represents a hydrogen atom or a methyl radical.

5. A compound according to one or more of claims 1 to 4, **characterized in that** R¹ and R² together with the bridging nitrogen atom form a moiety selected from the group consisting of 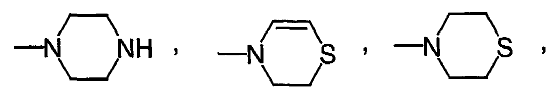 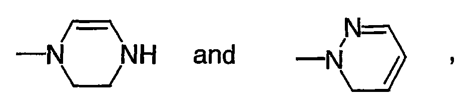 whereby each of these afore mentioned cyclic moieties may be substituted in any position including the -NH-groups with 1, 2 or 3 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, CI, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl;
preferably R¹ and R² together with the bridging nitrogen atom form a pyridazin-3-one moiety which may be substituted with 1, 2 or 3 substituent(s) independently selected from the group consisting of methyl, ethyl, n-propyl, isopropyl, n-butyl, tert-butyl, -O-CH₃, -O-C₂H₅, -C(=O)-OH, -C(=O)-O-CH₃, - C(=O)-O-C₂H₅, -C(=O)-CH₃, -C(=O)-C₂H₅, F, Cl, Br, I, -CN, -CF₃, -OCF₃, - SCF₃, -OH, -SH, -NH₂, -NH-CH₃, -NH-C₂H₅, -NO₂, -CHO, -CF₂H, -CFH₂, - C(=O)-NH₂, phenyl, phenoxy and benzyl;
more preferably R¹ and R² together with the bridging nitrogen atom form an unsubstituted pyridazin-3-one moiety.

6. A compound according to one or more of claims 1 to 5, **characterized in that**
R³ represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a - (CH₂)_{1,2or 3}-group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, - S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, - C(=O)-C₂H₅, -C(=O)-CH₂-CH₂-CH₃, -C(=O)-CH(CH₃)₂, -C(=O)-C(CH₃)₃, F, CI, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
preferably R³ represents a phenyl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH3, -S-C₂H₅, - S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
more preferably R³ represents an unsubstituted phenyl radical.

7. A compound according to one or more of claims 1 to 6, **characterized in that**
R⁷ and R⁸, identical or different, each represent a hydrogen atom or a linear or branched, unsubstituted C₁₋₁₀ alkyl radical;
preferably R⁷ and R⁸, identical or different, each represent a hydrogen atom or an alkyl radical selected from the group consisting of methyl, ethyl, n-propyl, isopropyl, n-butyl, sec-butyl, isobutyl and tert-butyl;
more preferably R⁷ and R⁸ each represent a hydrogen atom.

8. A compound according to one or more of claims 1 to 7, **characterized in that** R⁹ and R¹⁰, identical or different, each represent a hydrogen atom or a linear or branched, unsubstituted C₁₋₁₀ alkyl radical;
preferably R⁹ and R¹⁰, identical or different, each represent a hydrogen atom or an alkyl radical selected from the group consisting of methyl, ethyl, n-propyl, isopropyl, n-butyl, sec-butyl, isobutyl and tert-butyl;
more preferably R⁹ and R¹⁰ each represent a hydrogen atom.

9. A compound according to one or more of claims 1 to 8, **characterized in that**
R¹¹ represents an alkyl radical selected from the group consisting of methyl, ethyl, n-propyl, isopropyl, n-butyl, sec-butyl, isobutyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyridinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, - S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, - C(=O)-C₂H₅, -C(=O)-CH₂-CH₂-CH₃, -C(=O)-CH(CH₃)₂, -C(=O)-C(CH₃)₃, F, CI, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
preferably R¹¹ represents an alkyl radical selected from the group consisting of methyl, ethyl, n-propyl, isopropyl and n-butyl; or an aryl or heteroaryl radical selected from the group consisting of phenyl and pyridinyl whereby said aryl or heteroaryl may be substituted with 1, 2 or 3 substituent(s) independently selected from the group consisting of methyl, ethyl, n-propyl, isopropyl, n-butyl, tert-butyl, -O-CH₃, -O-C₂H₅, -C(=O)-OH, -C(=O)-O-CH₃, -C(=O)-O-C₂H₅, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
more preferably R¹¹ represents an alkyl radical selected from the group consisting of methyl, ethyl, n-propyl and n-butyl; or an unsubstituted phenyl or pyridinyl radical.

10. A compound according to one or more of claims 1 to 9, **characterized in that**
R¹² represents a 5 to 10 membered aryl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH3, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl whereby said heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH3, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, - C(=O)-NH-CH₃, -C(=O)-NH-C₂H₅, -C(=O)-N(CH₃)₂, -C(=O)-N(C₂H₅)₂ and - S(=O)₂-CH₃;
or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)_{1,2 or 3}- group and/or may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, l, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, - C(=O)-NH-CH₃, -C(=O)-NH-C₂H₅, -C(=O)-N(CH₃)₂, -C(=O)-N(C₂H₅)₂ and - S(=O)₂-CH₃;
preferably R**¹²** represents a phenyl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅,-S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
more preferably R¹² represents an unsubstituted phenyl radical.

11. A compound according to one or more of claims 1 to 10, **characterized in that**
X represents a -NR¹R² moiety or a -OR³ moiety;
R¹ represents an unsubstituted pyrrolyl radical; an aryl or heteroaryl radical selected from the group consisting of phenyl and pyrrolyl, whereby said phenyl radical is bonded via a -(CH₂)-group and said pyrrolyl radical is bonded via a - (CH₂)₂-group and/or said phenyl or pyrrolyl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, - S-CH₂-CH₂-CH₃, -S-CH(CH₃)₂, -S-C(CH₃)₃, -C(=O)-O-CH₃, -C(=O)-O-C₂H₅, - C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, - CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ or a -C(=O)-R¹² moiety;
R² represents a hydrogen atom or an alkyl radical selected from the group consisting of methyl, ethyl, n-propyl, isopropyl, n-butyl, sec-butyl, isobutyl and tert-butyl;
or
R¹ and R² together with the bridging nitrogen atom form a pyridazin-3-one moiety which may be substituted with 1, 2 or 3 substituent(s) independently selected from the group consisting of methyl, ethyl, n-propyl, isopropyl, n-butyl, tert-butyl, -O-CH₃, -O-C₂H₅, -C(=O)-OH, -C(=O)-O-CH₃, -C(=O)-O-C₂H₅, - C(=O)-CH₃, -C(=O)-C₂H₅, F, Cl, Br, l, -CN, -CF₃, -OCF₃, -SCF₃, -OH, -SH, - NH₂, -NH-CH₃, -NH-C₂H₅, -NO₂, -CHO, -CF₂H, -CFH₂, -C(=O)-NH₂, phenyl, phenoxy and benzyl;
R³ represents a phenyl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH3, -S-C₂H₅, - S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
R⁴, R⁵ and R⁶ each represent a hydrogen atom;
R⁷ and R⁸, identical or different, each represent a hydrogen atom or an alkyl radical selected from the group consisting of methyl, ethyl, n-propyl, isopropyl, n-butyl, sec-butyl, isobutyl and tert-butyl;
R⁹ and R¹⁰, identical or different, each represent a hydrogen atom or an alkyl radical selected from the group consisting of methyl, ethyl, n-propyl, isopropyl, n-butyl, sec-butyl, isobutyl and tert-butyl;
R¹¹ represents an alkyl radical selected from the group consisting of methyl, ethyl, n-propyl, isopropyl and n-butyl; or an aryl or heteroaryl radical selected from the group consisting of phenyl and pyridinyl whereby said aryl or heteroaryl may be substituted with 1, 2 or 3 substituent(s) independently selected from the group consisting of methyl, ethyl, n-propyl, isopropyl, n-butyl, tert-butyl, -O-CH₃, -O-C₂H₅, -C(=O)-OH, -C(=O)-O-CH₃, -C(=O)-O-C₂H₅, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
and
R¹² represents a phenyl radical which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅,-S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.

12. A compound according to one or more of claims 1 to 11, **characterized in that**
X represents a -NR¹R² moiety or a -OR³ moiety;
R¹ represents an unsubstituted pyrrolyl radical; an aryl or heteroaryl radical selected from the group consisting of phenyl and pyrrolyl, whereby said phenyl radical is bonded via a -(CH₂)-group and said pyrrolyl radical is bonded via a - (CH₂)₂-group and/or said phenyl or pyrrolyl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, F, Br and I or a -C(=O)-R¹² moiety;
R² represents a hydrogen atom or a methyl radical;
or
R¹ and R² together with the bridging nitrogen atom form an unsubstituted pyridazin-3-one moiety;
R³ represents an unsubstituted phenyl radical;
R⁴, R⁵ and R⁶ each represent a hydrogen atom;
R⁷ and R⁸ each represent a hydrogen atom;
R⁹ and R¹⁰ each represent a hydrogen atom;
R¹¹ represents an alkyl radical selected from the group consisting of methyl, ethyl, n-propyl and n-butyl; or an unsubstituted phenyl or pyridinyl radical
and
R¹² represents an unsubstituted phenyl radical.

13. A compound according to one or more of claims 1 to 12 selected from the group consisting of
[1] [5-(4-Methyl-piperazin-1-yl)-2-nitro-phenyl]-(2-pyrrol-1-yl-ethyl)-amine,
[2] (4-Fluoro-benzyl)-[5-(4-methyl-piperazin-1-yl)-2-nitro-phenyl]-amine,
[3] N-[5-(4-Methyl-piperazin-1-yl)-2-nitro-phenyl]-benzamide,
[4] N-Methyl-N-[5-(4-methyl-piperazin-1-yl)-2-nitro-phenyl]-benzamide,
[5] [5-(4-Methyl-piperazin-1-yl)-2-nitro-phenyl]-pyrrol-1-yl-amine,
[6] 2-[5-(4-Methyl-piperazin-1-yl)-2-nitro-phenyl]-2H-pyridazin-3-one,
[7] 1-Methyl-4-(4-nitro-3-phenoxy-phenyl)-piperazine,
[8] Benzyl-[5-(4-butyl-piperazin-1-yl)-2-nitro-phenyl]-amine,
[9] Benzyl-(2-nitro-5-(4-pyridin-2-yl-piperazin-1-yl)-phenyl]-amine and
[10] Benzyl-(2-nitro-5-(4-phenyl-2-yl-piperazin-1-yl)-phenyl]-amine;
optionally in form of a physiologically acceptable salt thereof, or a corresponding solvate thereof.

14. Process for the preparation of a compound according to one or more of claims 1 to 13, **characterized in that** at least one nitrobenzene compound of general formula II, 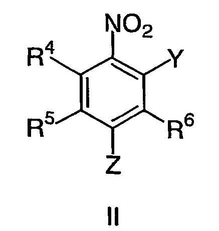 wherein R⁴ to R⁶ have the meaning according to one or more of claims 1 to 13, Y represents a chlorine atom, and Z represents a bromine or iodine atom; is reacted with at least one compound of general formula III, 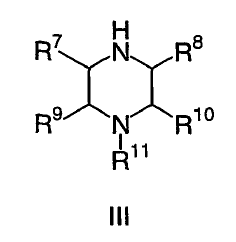 wherein R⁷ to R¹¹ have the meaning according to one or more of claims 1 to 13, in a suitable reaction medium, preferably in the presence of at least one catalyst and/or at least one auxiliary agent and/or at least one base to yield a compound of general formula IV, 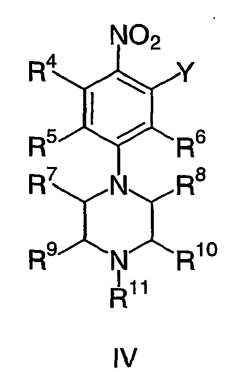 wherein R⁴ to R¹¹ have the meaning according to one or more of claims 1 to 13 and Y represents a chlorine atom; which is optionally purified and/or isolated, and the compound of general formula IV is reacted with at least one compound of general formula V, 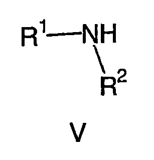 wherein R¹ and R² have the meaning according to one or more of claims 1 to 13, in a suitable reaction medium, preferably in the presence of at least one catalyst and/or at least one auxiliary agent and/or at least one base to yield a compound of general formula VI, 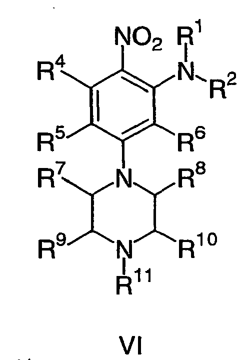 wherein R¹, R² and R⁴ to R¹¹have the meaning according to one or more of claims 1 to 13, which is optionally purified and/or isolated.

15. Process for the preparation of a compound according to one or more of claims 1 to 13, **characterized in that** at least one nitrobenzene compound of general formula VII, 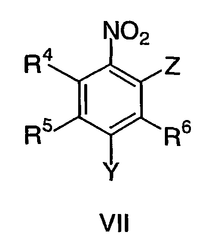 wherein R⁴ to R⁶ have the meaning according to one or more of claims 1 to 13, Z represents a bromine or iodine atom, and Y represents a chlorine atom, is reacted with at least one compound of general formula V, 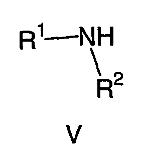 wherein R¹ and R² have the meaning according to one or more of claims 1 to 13, in a suitable reaction medium, preferably in the presence of at least one catalyst and/or at least one auxiliary agent and/or at least one base to yield a compound of general formula VIII, 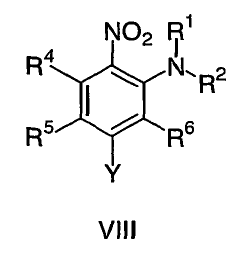 wherein R¹, R² and R⁴ to R⁶ have the meaning according to one or more of claims 1 to 13 and Y represents a chlorine atom; which is optionally purified and/or isolated, and the compound of general formula VIII is reacted with at least one compound of general formula III, 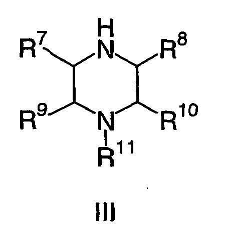 wherein R⁷ to R¹¹ have the meaning according to one or more of claims 1 to 13, in a suitable reaction medium, preferably in the presence of at least one catalyst and/or at least one auxiliary agent and/or at least one base to yield a compound of general formula VI, 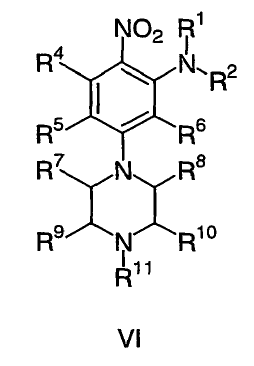 wherein R¹, R² and R⁴ to R¹¹ have the meaning according to one or more of claims 1 to 13, which is optionally purified and/or isolated.

16. Process for the preparation of a compound according to one or more of claims 1 to 13, **characterized in that** at least one nitrobenzene compound of general formula II, 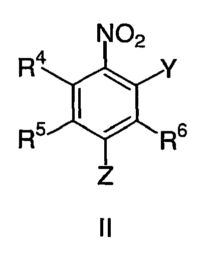 wherein R⁴ to R⁶ have the meaning according to one or more of claims 1 to 13, Y represents a chlorine atom, and Z represents a bromine or iodine atom; is reacted with at least one compound of general formula III, 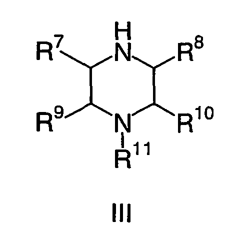 wherein R⁷ to R¹¹ have the meaning according to one or more of claims 1 to 13, in a suitable reaction medium, preferably in the presence of at least one catalyst and/or at least one auxiliary agent and/or at least one base to yield a compound of general formula IV, 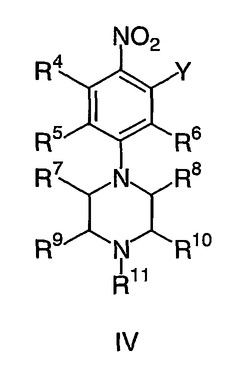 wherein R⁴ to R¹¹ have the meaning according to one or more of claims 1 to 13 and Y represents a chlorine atom; which is optionally purified and/or isolated, and the compound of general formula IV is reacted with at least one compound of general formula IX, 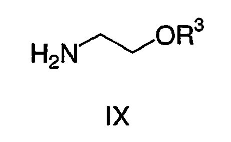 wherein R³ has the meaning according to one or more of claims 1 to 13, in a suitable reaction medium, preferably in the presence of at least one catalyst and/or at least one auxiliary agent and/or at least one base to yield a compound of general formula X, 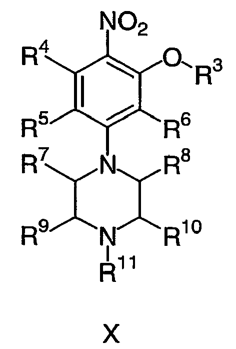 wherein R³ to R¹¹ have the meaning according to one or more of claims 1 to 13, which is optionally purified and/or isolated.

17. Medicament comprising at least one nitro-substituted phenyl-piperazine compound of general formula la, 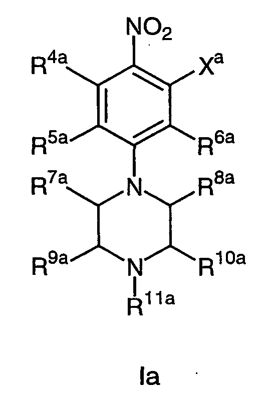 wherein
X^{a} represents a -NR^{1a}R^{2a} moiety or a -OR^{3a} moiety;
R^{1a} represents a linear or branched, saturated or unsaturated C₁₋₁₀ aliphatic radical which is substituted with 1, 2 or 3 substituent(s) independently selected from the group consisting of F, Cl, Br, I, -CN, -CF₃, -OCF₃, - SCF₃, -OH, -SH, -NH₂, -O-C₁₋₅-alkyl, S-C₁₋₅-alkyl, -NH-(C₁₋₅-alkyl) and - N(C₁₋₅-alkyl)₂;
a radical selected from the group consisting of adamantyl, bicyclo[2.2.1 ]heptyl and bicyclo[3.1.1]heptyl which may be substituted with 1, 2 or 3 substituent(s) independently selected from the group consisting of oxo (=O), thioxo (=S), C₁₋₅-alkyl, -O-C₁₋₅-alkyl, -S-C₁₋₅- alkyl, -C(=O)-OH, -C(=O)-C₁₋₅-alkyl, -C(=O)-O-C₁₋₅-alkyl, -O-C(=O)-C₁₋₅- alkyl, F, Cl, Br, I, -CN, -CF₃, -OCF₃, -SCF₃, -OH, -SH, -NH₂, -NH(C₁₋₅- alkyl), -N(C₁₋₅-alkyl)₂, -NO₂, -CHO, -CF₂H, -CFH₂, -C(=O)-NH₂, -C(=O)- NH(C₁₋₅-alkyl), -C(=O)-N(C₁₋₅-alkyl)₂ and -S(=O)₂-C₁₋₅-alkyl and/or which is bonded via a linear or branched C₁₋₆ alkylene group which may contain 1, 2 or 3 heteroatom(s) independently selected from the group consisting of nitrogen, oxygen and sulfur as chain member(s);
a saturated or unsaturated 3- to 9-membered cycloaliphatic radical, which may be substituted with 1, 2 or 3 substituent(s) independently selected from the group consisting of oxo (=O), thioxo (=S), C₁₋₅-alkyl, - O-C₁₋₅-alkyl, -S-C₁₋₅-alkyl, -C(=O)-OH, -C(=O)-C₁₋₅-alkyl, -C(=O)-O-C₁₋₅- alkyl, -O-C(=O)-C₁₋₅-alkyl, F, Cl, Br, I, -CN, -CF₃, -OCF₃, -SCF₃, -OH, - SH, -NH₂, -NH(C₁₋₅-alkyl), N(C₁₋₅-alkyl)₂, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁₋₅- alkyl, -S(=O)₂-phenyl, phenyl, phenoxy and benzyl and which is bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅- alkyl, phenyl, -NH-(C₁₋₅-alkyl) and -N(C₁₋₅-alkyl)₂ and/or which may contain 1, 2 or 3 heteroatom(s) independently selected from the group consisting of nitrogen, oxygen and sulfur as ring member(s);
a 5- to 10-membered aryl or heteroaryl radical which may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)- C₁₋₅-alkyl, -C(=O)-O-C₁₋₅-alkyl, -O-C(=O)-C₁₋₅-alkyl, F, Cl, Br, I, -CN, - CF₃, -OCF₃, -SCF₃, -OH, -SH, -NH₂, -NH(C₁₋₅-alkyl), -N(C₁₋₅-alkyl)₂, - NO₂, -CHO, -CF₂H, -CFH₂, -C(=O)-NH₂, -C(=O)-NH(C₁₋₅-alkyl), -C(=O)- N(C₁₋₅-alkyl)₂, -S(=O)₂-C₁₋₅-alkyl, -S(=O)₂-phenyl, phenyl, phenoxy and benzyl and whereby said heteroaryl radical contains 1, 2 or 3 heteroatom(s) independently selected from the group consisting of nitrogen, oxygen and sulfur as ring member(s);
a 5- to 10-membered aryl radical whereby said aryl radical may be substituted with 1, 2, 3, 4 or 5 substituent(s) independently selected from the group consisting of C₁₋₅-alkyl, -O-C₁₋₅-alkyl, -S-C₁₋₅-alkyl, - C(=O)-O-C₁₋₅-alkyl, -O-C(=O)-C₁₋₅-alkyl, F, Cl, Br, I, -CN, -CF₃, -OCF₃, - SCF₃, -OH, -SH, -NH₂, -NH(C₁₋₅-alkyl), -N(C₁₋₅-alkyl)₂, -NO₂, -CHO, - CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and said aryl radical is bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH, - NH₂, -O-C₁₋₅-alkyl, -S-C₁₋₅-alkyl, phenyl, -NH-(C₁₋₅-alkyl) and -N(C₁₋₅- alkyl)₂ and which may contain 1, 2 or 3 heteroatom(s) independently selected from the group consisting of nitrogen, oxygen and sulfur as chain member(s);
a 5- to 10-membered heteroaryl radical, which may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)- C₁₋₅-alkyl, -C(=O)-O-C₁₋₅-alkyl, -O-C(=O)-C₁₋₅-alkyl, F, Cl, Br, I, -CN, - CF₃, -OCF₃, -SCF₃, -OH, -SH, -NH₂, -NH(C₁₋₅-alkyl), -N(C₁₋₅-alkyl)₂, - NO₂, -CHO, -CF₂H, -CFH₂, -C(=O)-NH₂, -C(=O)-NH(C₁₋₅-alkyl), -C(=O)- N(C₁₋₅-alkyl)₂, -S(=O)₂-C₁₋₅-alkyl, -S(=O)₂-phenyl, phenyl, phenoxy and benzyl and which is bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH, - NH₂, -O-C₁₋₅-alkyl, -S-C₁₋₅-alkyl, phenyl, -NH-(C₁₋₅-alkyl) and -N(C₁₋₅- alkyl)₂ and which may contain 1, 2 or 3 heteroatom(s) independently selected from the group consisting of nitrogen, oxygen and sulfur as chain member(s) and whereby the heteroaryl radical contains 1, 2 or 3 heteroatom(s) independently selected from the group consisting of nitrogen, oxygen and sulfur as ring member(s);
or a -C(=O)-R^{12a} moiety;
R^{2a} represents a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, - NH₂, -O-C₁₋₅-alkyl, -S-C₁₋₅-alkyl, -NH-(C₁₋₅-alkyl) and -N(C₁₋₅-alkyl)₂;
or
R^{1a} and R^{2a} together with the bridging nitrogen form a saturated, unsaturated or aromatic 3- to 6-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH, -C(=O)-C₁₋₅-alkyl, -C(=O)-O-C₁₋₅-alkyl, -O- C(=O)-C₁₋₅-alkyl, F, Cl, Br, I -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C;(=O)-NH(C₁₋₅-alkyl), -C(=O)-N(C₁₋₅-alkyl)₂, -S(=O)₂-C₁₋₅-alkyl, -S(=O)₂- phenyl, phenyl, phenoxy and benzyl and which may contain 1, 2 or 3 additional heteroatom(s) independently selected from the group consisting of nitrogen, oxygen and sulfur as ring member(s) and/or which may be condensed with a mono- or bicyclic ring system which may be substituted with 1, 2 or 3 substituent(s) independently selected from the group consisting of C₁₋₅-alkyl, -O-C₁₋₅-alkyl, -S-C₁₋₅-alkyl, oxo =O), thioxo (=S), -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, - NH₂, -NH(C₁₋₅-alkyl), -N(C₁₋₅-alkyl)2, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁₋₅- alkyl, -S(=O)₂-phenyl, phenyl, phenoxy and benzyl whereby said cyclic substituent(s) phenyl, phenoxy and benzyl may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and NO₂;
whereby the rings of the ring system are 5- 6- or 7-membered and may contain 1, 2 or 3 heteroatom(s) independently selected from the group consisting of nitrogen, oxygen and sulfur;
R^{3a} represents a 5- to 10-membered aryl or heteroaryl radical which may be substituted with 1, 2 or 3 substituent(s) independently selected from the group consisting of C₁₋₅-alkyl, -O-C₁₋₅-alkyl, -S-C₁₋₅-alkyl, -C(=O)-OH, - C(=O)-C₁₋₅-alkyl, -C(=O)-O-C₁₋₅-alkyl, -O-C(=O)-C₁₋₅-alkyl, F, Cl, Br, I, - CN, -CF₃, -OCF₃, -SCF₃, -OH, -SH, -NH₂, -NH(C₁₋₅-alkyl), -N(C₁₋₅- alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may be bonded via a linear or branched C₁₋₆ alkylene, C₂₋₆ alkenylene or C₂₋₆ alkinylene group and which may be unsubstituted or substituted with 1, 2 or 3 substituent(s) independently selected from the group consisting of F, Cl, Br, I, -CN, -CF₃, -OCF₃, - SCF₃, -OH, -SH, -NH₂, -O-C₁₋₅-alkyl, -S-C₁₋₅-alkyl, -NH-(C₁₋₅-alkyl) and - N(C₁₋₅-alkyl)₂ and whereby the heteroaryl radical contains 1, 2 or 3 heteroatom(s) independently selected from the group consisting of nitrogen, oxygen and sulfur as ring member(s);
R^{4a}, R^{5a} and R^{6a}, identical or different, each represent a hydrogen atom or a halogen atom;
or
R^{4a} and R^{5a} together with the bridging carbon atoms form an unsubstituted 5- or 6- membered heterocyclic ring which contains 1, 2 or 3 heteroatom(s) independently selected from the group consisting of nitrogen, oxygen and sulfur as ring member(s) and which together with the phenyl ring which it is fused with forms a 9- or 10-membered bicyclic aromatic ring system;
R^{7a} and R^{8a}, identical or different, each represent a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, -CN, - CF₃, -OCF₃, -SCF₃, -OH, -SH, -NH₂, -O-C₁₋₅-alkyl, -S-C₁₋₅-alkyl, -NH- (C₁₋₅-alkyl) and N(C₁₋₅-alkyl)₂;
R^{9a} and R^{10a}, identical or different, each represent a hydrogen atom or a linear or branched, saturated or unsaturated C₁₋₁₀ aliphatic radical which may be unsubstituted or substituted with 1, 2 or 3 substituent(s) independently selected from the group consisting of F, Cl, Br, I, -CN, - CF₃, -OCF₃, -SCF₃, -OH, -SH, -NH₂, -O-C₁₋₅-alkyl, -S-C₁₋₅-alkyl, -NH- (C₁₋₅-alkyl) and -N(C₁₋₅-alkyl)2;
R^{11a} represents a hydrogen atom; a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, - NH2,-O-C₁₋₅-alkyl, -S-C₁₋₅-alkyl, -NH-(C₁₋₅-alkyl) and -N(C₁₋₅-alkyl)₂ and which may contain 1, 2 or 3 heteroatom(s) independently selected from the group consisting of nitrogen, oxygen and sulfur as chain member(s);
a 5- to 10-membered aryl or heteroaryl radical which may be substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)- C₁₋₅-alkyl, -C(=O)-O-C₁₋₅-alkyl, -O-C(=O)-C₁₋₅-alkyl, F, Cl, Br, I, -CN, - CF₃, -OCF₃, -SCF₃, -OH, -SH, NH₂, -NH(C₁₋₅-alkyl), -N(C₁₋₅-alkyl)₂, - NO₂, -CHO, -CF₂H, -CFH₂, -C(=O)-NH₂, -C(=O)-NH(C₁₋₅-alkyl), -C(=O)- N(C₁₋₅-alkyl)₂, -S(=O)2-C₁₋₅-alkyl, -S(=O)₂-phenyl, phenyl, phenoxy and benzyl and which may be bonded via a linear or branched, C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, - SCF₃, -OH, -SH, -NH₂, -O-C₁₋₅-alkyl, -S-C₁₋₅-alkyl, -NH-(C₁₋₅-alkyl) and - N(C₁₋₅-alkyl)₂ and whereby the heteroaryl radical contains 1, 2 or 3 heteroatom(s) independently selected from the group consisting of nitrogen, oxygen and sulfur as ring member(s); a -C(=O)-R^{13a} moiety or a-S(=O)₂-R^{14a}, moiety;
R^{12a} represents a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅- alkyl, -NH-(C₁₋₅-alkyl) and -N(C₁₋₅-alkyl)₂;
or a 5- to 10-membered aryl or heteroaryl radical, which may be substituted with 1, 2 or 3 substituent(s) independently selected from the group consisting of C₁₋₅-alkyl, -O-C₁₋₅-alkyl, -S-C₁₋₅-alkyl, -C(=O)-OH, - C(=O)-C₁₋₅-alkyl, -C(=O)-O-C₁₋₅-alkyl, -O-C(=O)-C₁₋₅-alkyl, F, Cl, Br, I, - CN, -CF₃, -OCF₃, -SCF₃, -OH, -SH, -NH₂, -NH(C₁₋₅-alkyl), -N(C₁₋₅- alkyl)₂, -NO₂, -CHO, -CF₂H, -CFH₂, -C(=O)-NH₂, -C(=O)-NH(c₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, - SCF₃, -OH, -SH, -NH₂, -O-C₁₋₅-alkyl, -S-C₁₋₅-alkyl, -NH-(C₁₋₅-alkyl) and - N(C₁₋₅-alkyl)₂ and whereby the heteroaryl radical contains 1, 2 or 3 heteroatom(s) independently selected from the group consisting of nitrogen, oxygen and sulfur as ring member(s);
and
R^{13a} and R^{14a}, identical or different, each represent a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, - SCF₃, -OH, -SH, -NH₂, -O-C₁₋₅-alkyl, -S-C₁₋₅-alkyl, -NH-(C₁₋₅-alkyl) and - N(C₁₋₅-alkyl)₂;
or a 5- to 10-membered aryl or heteroaryl radical, which may be substituted with 1, 2 or 3 substituent(s) independently selected from the group consisting of C₁₋₅-alkyl, -O-C₁₋₅-alkyl, -S-C₁₋₅-alkyl, -C(=O)-OH, - C(=O)-C₁₋₅-alkyl, -C(=O)-O-C₁₋₅-alkyl, -O-C(=O)-C₁₋₅-alkyl, F, Cl, Br, I, - CN, -CF₃, -OCF₃, -SCF₃, -OH, -SH, -NH₂, -NH(C₁₋₅-alkyl), -N(C₁₋₅- alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and/or which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, - CF₃, -OCF₃, -SCF₃, -OH, -SH, -NH₂, -O-C₁₋₅-alkyl, -S-C₁₋₅-alkyl, -NH- (C₁₋₅-alkyl) and -N(C₁₋₅-alkyl)₂ and whereby the heteroaryl radical contains 1, 2 or 3 heteroatom(s) independently selected from the group consisting of nitrogen, oxygen and sulfur as ring member(s).
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof;
and optionally at least one physiologically compatible auxiliary agent.

18. Medicament according to claim 17, **characterized in that**
R^{1a} represents an alkyl radical selected from the group consisting of methyl, ethyl, n-propyl, isopropyl, n-butyl, sec-butyl, isobutyl and tert-butyl which is substituted with 1, 2 or 3 substituent(s) independently selected from the group consisting of F, Cl, Br, I, -CN, -CF₃, -OCF₃, -SCF₃, -OH, -SH and -NH₂;
a radical selected from the group consisting of adamantyl, bicyclo[2.2.1]heptyl and bicyclo[3.1.1]heptyl which may be substituted with 1, 2 or 3 substituent(s) independently selected from the group consisting of C₁₋₅-alkyl, -O-C₁₋₅-alkyl, F, Cl, Br, I, -CN, -CF₃ and -OCF₃ and/or which is bonded via a linear or branched C₁₋₆ alkylene group which may contain 1, 2 or 3 oxygen atom(s) as chain member(s);
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, which may be substituted with 1, 2, 3, 4 or 5 substituent(s) independently selected from the group consisting of C₁₋₅-alkyl, -O-C₁₋₅-alkyl, - S-C₁₋₅-alkyl, -C(=O)-OH, -C(=O)-O-C₁₋₅-alkyl, -O-C(=O)-C₁₋₅-alkyl, F, Cl, Br, I, - CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
an aryl radical selected from the group consisting of phenyl and naphthyl which may be substituted with 1, 2, 3, 4 or 5 substituent(s) independently selected from the group consisting of C₁₋₅-alkyl, -O-C₁₋₅-alkyl, -S-C₁₋₅-alkyl, - C(=O)-O-C₁₋₅-alkyl, -O-C(=O)-C₁₋₅-alkyl, F, Cl, Br, I, -CN, -CF₃, -OCF₃, -SCF₃,-OH, -SH, -NH₂, -NH(C₁₋₅-alkyl), -N(C₁₋₅-alkyl)₂, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁-₅-alkyl, - S(=O)₂-phenyl, phenyl, phenoxy and benzyl and said aryl radical is bonded via a linear or branched C₁₋₆ alkylene group which may contain 1, 2 or 3 oxygen atom(s) as chain member(s) and which may be substituted with 1 phenyl radical;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said heteroaryl radical may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁₋₅-alkyl, - S(=O)₂-phenyl, phenyl, phenoxy and benzyl and said heteroaryl radical is bonded via a linear or branched C₁₋₆ alkylene group which may contain 1, 2 or 3 oxygen atom(s) as chain member(s);
or a -C(=O)-R^{12a} moiety;
preferably R^{1a} represents an alkyl radical selected from the group consisting of methyl, ethyl, n-propyl and isopropyl, whereby said alkyl radical is substituted with 1, 2 or 3 substituent(s) independently selected from the group consisting of -OH, F, Cl, Br and -CN;
a radical selected from the group consisting of adamantyl, bicyclo[2.2.1]heptyl and bicyclo[3.1.1]heptyl which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, F, Cl, Br, I, -CN, -CF₃ and -OCF₃ and/or which is bonded via an alkylene group selected form the group consisting of -CH₂-, -CH(CH₃)-, -CH₂-CH₂-, -CH₂-CH₂-CH₂- and -CH₂-CH₂-O-;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said aryl or heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Cl, Br, I, -CN, -CF₃, -OCF₃, -SCF₃, -SH, -NH₂, -NH-CH₃,-NH-C₂H₅, -NH-CH₂-CH₂-CH₃, -NH-CH(CH₃)₂, -NH-C(CH₃)₃, -N(CH₃)₂, - N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
an aryl radical selected from the group consisting of phenyl and naphthyl whereby said aryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, - S-CH(CH₃)₂, -S-C(CH₃)₃, -C(=O)-O-CH₃, -C(=O)-O-C₂H₅, -C(-O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN, - CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or is bonded via an alkylene group selected form the group consisting of -CH₂-, -CH(CH₃)-, -CH(Phenyl)-, -CH₂-CH₂-, -CH₂-CH₂-CH₂- and -CH₂-CH₂-O-;
a heteroaryl radical selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, - S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, - C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or is bonded via an alkylene group selected form the group consisting of -CH₂-, -CH(CH₃)-, -CH₂-CH₂-, -CH₂-CH₂-CH₂- and -CH₂-CH₂-O-;
or a -C(=O)-R^{12a} moiety;
more preferably R^{1a} represents an alkyl radical selected from the group consisting of -CH₂-CH₂-OH and -CH₂-CH₂-CH₂-OH;
an unsubstituted adamantyl radical;
an unsubstituted phenyl or pyrrolyl radical;
an unsubstituted napthyl radical which is bonded via an alkylene group selected form the group consisting of -CH₂-, -CH(CH₃)-, -CH₂-CH₂-, -CH₂-CH₂-CH₂- and -CH₂-CH₂-O-;
a phenyl radical which may be substituted with 1, 2 or 3 substituent(s) independently selected from the group consisting of methyl, ethyl, tert-butyl, methoxy, F and CI and said phenyl radical is bonded via an alkylene group selected form the group consisting of -CH₂-, -CH(CH₃)-, -CH(Phenyl)-, -CH₂-CH₂-, -CH₂-CH₂-CH₂- and -CH₂-CH₂-O-;
a heteroaryl radical selected from the group consisting of pyridinyl, furanyl and pyrrolyl, whereby said pyridinyl, furanyl or pyrrolyl radical may be substituted with 1, 2 or 3 substituent(s) independently selected from the group consisting of methyl, ethyl, tert-butyl, methoxy, F and Cl and said pyridinyl, furanyl or pyrrolyl radical is bonded via an alkylene group selected form the group consisting of -CH₂-, -CH(CH₃)-, -CH₂-CH₂-, -CH₂-CH₂-CH₂- and -CH₂-CH₂-O-;
or a -C(=O)-R^{12a} moiety.

19. Medicament according to claim 17 or 18, **characterized in that**
R^{2a} represents a hydrogen atom or a linear or branched, unsubstituted C₁₋₁₀ alkyl radical;
preferably R^{2a} represents a hydrogen atom or an alkyl radical selected from the group consisting of methyl, ethyl, n-propyl, isopropyl, n-butyl, sec-butyl, isobutyl and tert-butyl;
more preferably R^{2a} represents a hydrogen atom or a methyl radical.

20. Medicament according to one or more of claims 17 to 19, **characterized in that**
R^{1a} and R^{2a} together with the bridging nitrogen atom form a moiety selected from the group consisting of 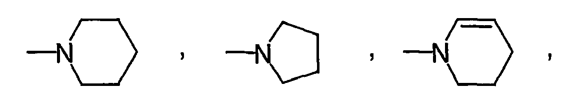 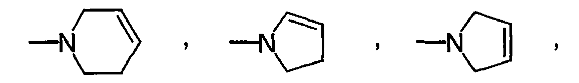 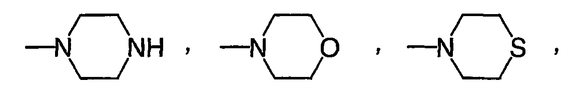 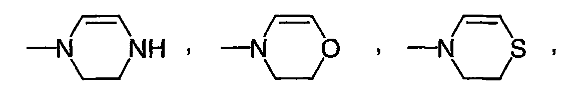 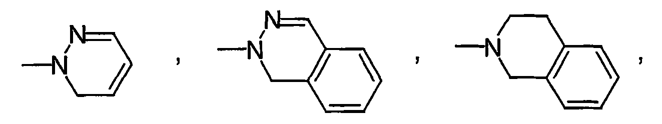 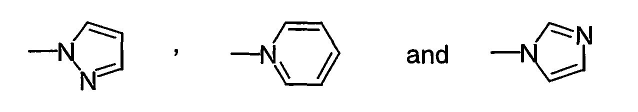 whereby each of these afore mentioned cyclic moieties may be substituted in any position including the -NH-groups with 1, 2 or 3 substituent(s) independently selected from the group consisting of C₁₋₅-alkyl, -O-C₁₋₅-alkyl, - S-C₁₋₅-alkyl, -C(=O)-OH, oxo (=O), thioxo (=S), -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; whereby said cyclic substituent(s) phenyl, phenoxy and benzyl may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, - NH₂ and NO₂;
preferably R^{1a} and R^{2a} together with the bridging nitrogen atom form a moiety selected from the group consisting of 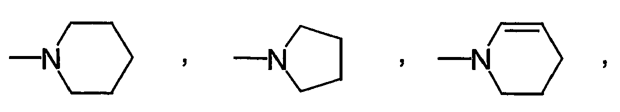 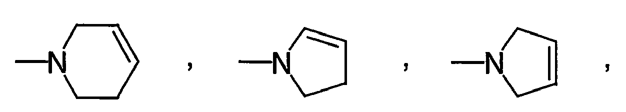 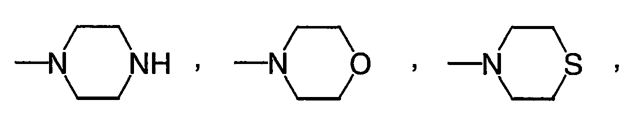 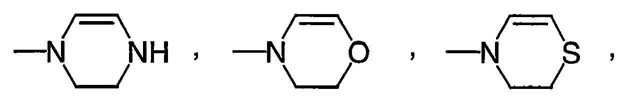 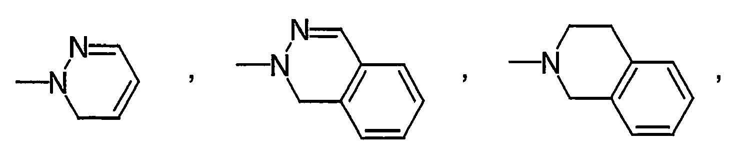 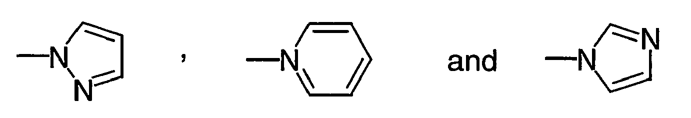 whereby each of these afore mentioned cyclic moieties may be substituted with 1, 2 or 3 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, - C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl; whereby said cyclic substituent(s) phenyl, phenoxy and benzyl may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, - OCF₃, -SCF₃, -OH, -SH, -NH₂ and NO₂;
more preferably R^{1a} and R^{2a} together with the bridging nitrogen atom form a moiety selected from the group consisting of

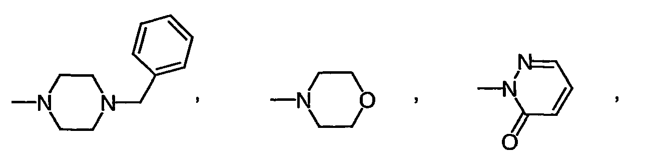

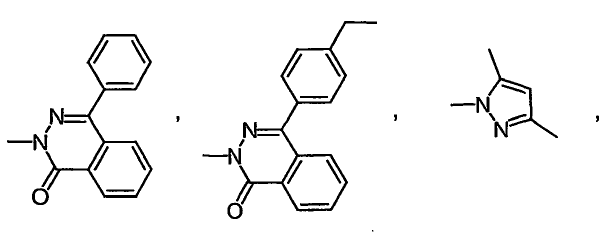

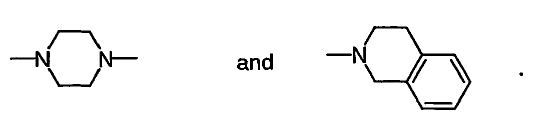

21. Medicament according to one or more of claims 17 to 20, **characterized in that**
R^{3a} represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said aryl or heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
preferably R^{3a} represents a phenyl radical which may be substituted with 1, 2 or 3 substituent(s) independently selected from the group consisting of methyl, ethyl, n-propyl, isopropyl, n-butyl, tert-butyl, -O-CH₃, -O-C₂H₅, -O-CH₂-CH₂-CH₃, - -C(=O)-OH, -C(=O)-O-CH₃, -C(=O)-O-C₂H₅, -C(=O)-CH₃, -C(=O)-C₂H₅, F, Cl, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, - CF₂H, -CFH₂ and -S(=O)₂-CH₃;
more preferably R^{3a} represents an unsubstituted phenyl radical.

22. Medicament according to one or more of claims 17 to 21, **characterized in that**
R^{4a}, R^{5a} and R^{6a}, identical or different, each represent a hydrogen atom or a halogen atom selected from the group consisting of F, Cl and Br;
preferably R^{4a}, R^{5a} and R^{6a}, identical or different, each represent a hydrogen atom or a fluorine atom.

23. Medicament according to one or more of claims 17 to 22, **characterized in that**
R^{4a} and R^{5a} together with the bridging carbon atoms form a moiety selected from the group consisting of 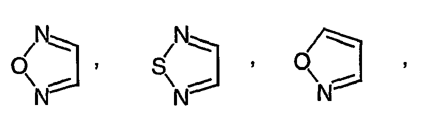 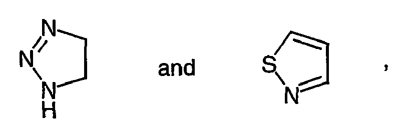 which together with the phenyl ring which it is fused with forms a substituted bicyclic aromatic ring system selected from the group consisting of 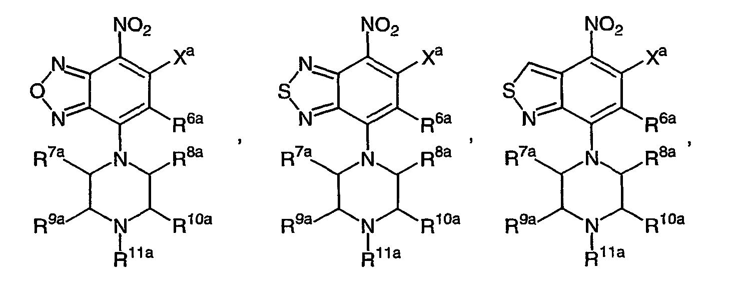 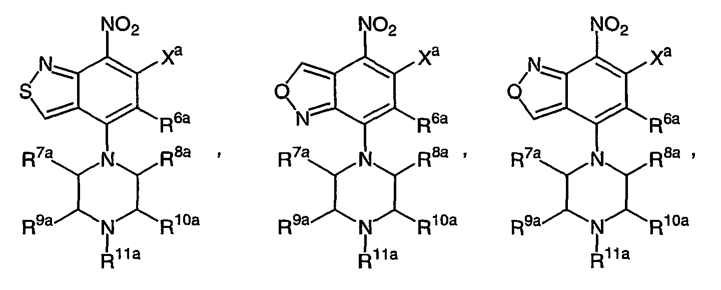 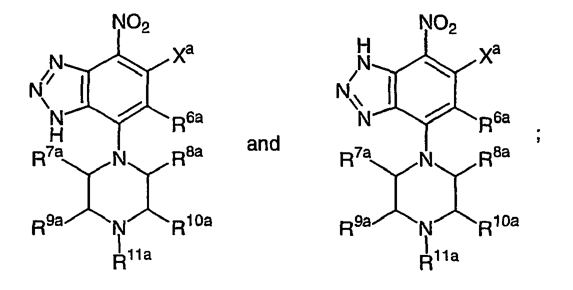 preferably R^{4a} and R^{5a} together with the bridging carbon atoms form the following moiety, 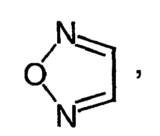 which together with the phenyl ring which it is fused with forms the following substituted bicyclic aromatic ring system

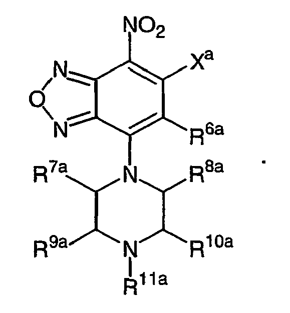

24. Medicament according to one or more of claims 17 to 23, **characterized in that**
R^{7a} and R^{8a}, identical or different, each represent a hydrogen atom or a linear or branched, unsubstituted C₁₋₁₀ alkyl radical;
preferably R^{7a} and R^{8a}, identical or different, each represent a hydrogen atom or an alkyl radical selected from the group consisting of methyl, ethyl, n-propyl, isopropyl, n-butyl, sec-butyl, isobutyl and tert-butyl;
more preferably R^{7a} and R^{8a} each represent a hydrogen atom.

25. Medicament according to one or more of claims 17 to 24, **characterized in that**
R^{9a} and R^{10a}, identical or different, each represent a hydrogen atom or a linear or branched, unsubstituted C₁₋₁₀ alkyl radical;
preferably R^{9a} and R^{10a}, identical or different, each represent a hydrogen atom or an alkyl radical selected from the group consisting of methyl, ethyl, n-propyl, isopropyl, n-butyl, sec-butyl, isobutyl and tert-butyl;
more preferably R^{9a} and R^{10a}, identical or different, each represent a hydrogen atom or a methyl radical.

26. Medicament according to one or more of claims 17 to 25, **characterized in that**
R^{11a} represents a hydrogen atom;
an alkyl radical selected from the group consisting of methyl, ethyl, n-propyl, isopropyl, n-butyl, sec-butyl, isobutyl and tert-butyl whereby said alkyl radical may be substituted with 1, 2 or 3 substituent(s) independently selected from the group consisting of -OH, F, Cl, Br and -CN;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-Cₗ₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and/or may be bonded via a -(CH₂)_{1,2 or 3}- group;
a -C(=O)-R^{13a} moiety or a -S(=O)₂-R^{14a} moiety;
preferably R^{11a} represents a hydrogen atom;
an alkyl radical selected from the group consisting of methyl, ethyl, n-propyl, isopropyl, n-butyl, sec-butyl, isobutyl and tert-butyl whereby said alkyl radical may be substituted with 1, 2 or 3 substituent(s) independently selected from the group consisting of -OH, F, Cl, Br and -CN;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyridinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or may be bonded via a -(CH₂)_{1,2 or 3}-group;
a -C(=O)-R^{13a} moiety or a -S(=O)₂-R^{14a} moiety;
more preferably R^{11a} represents a hydrogen atom;
an alkyl radical selected from the group consisting of methyl, n-butyl and - CH₂-CH₂-OH;
an unsubstituted phenyl or pyridinyl radical whereby said phenyl or pyridinyl radical may be bonded via a -(CH₂)- group;
a -C(=O)-R^{12a} moiety or a -S(=O)₂-R^{13a} moiety.

27. Medicament according to one or more of claims 17 to 26, **characterized in that**
R^{12a} represents an alkyl radical selected from the group consisting of methyl, ethyl, n-propyl, isopropyl, n-butyl, sec-butyl, isobutyl and tert-butyl;
or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), - N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and/or may be bonded via a -(CH₂)₁,_{2 or 3}- group;
preferably R^{12a} represents an alkyl radical selected from the group consisting of methyl, ethyl, n-propyl and isopropyl,
or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl and pyridinyl, whereby said aryl or heteroaryl radical may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Cl, Br, I, -CN, -CF₃, -OCF₃, -SCF₃, -SH, -NH₂, -NH-CH₃, - NH-C₂H₅, -NH-CH₂-CH₂-CH₃, -NH-CH(CH₃)₂, -NH-C(CH₃)₃, -N(CH₃)₂, - N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or may be bonded via a -(CH₂)- group;
more preferably R^{12a} represents a phenyl or a thiophenyl radical whereby said phenyl or thiophenyl radical may be substituted with 1, 2 or 3 substituent(s) selected from the group consisting of methyl and chlorine.

28. Medicament according to one or more of claims 17 to 27, **characterized in that**
R^{13a} represents an alkyl radical selected from the group consisting of methyl, ethyl, n-propyl, isopropyl, n-butyl, sec-butyl, isobutyl and tert-butyl whereby said alkyl radical may be substituted with 1, 2 or 3 substituent(s) independently selected from the group consisting of -OH, F, Cl, Br and -CN;
or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyridinyl, pyrazinyl, quinolinyl, isoquinolinyl, benzo[byuranyl, benzo[b]thiophenyl, benzothiadiazolyl and imidazo[2,1-b]thiazolyl, whereby said aryl or heteroaryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, - S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or may be bonded via a -(CH₂)_{1,2 or 3}-group;
preferably R^{13a} represents an alkyl radical selected from the group consisting of methyl, ethyl, n-propyl, isopropyl, n-butyl, sec-butyl, isobutyl and tert-butyl;
or an aryl or heteroaryl radical selected from the group consisting of phenyl, and thienyl (thiophenyl), whereby said aryl or heteroaryl may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, - C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃ and/or may be bonded via a -(CH₂)_{1, 2 or 3}-group;
more preferably R^{13a} represents a methyl radical or a phenyl or a thiophenyl radical whereby said phenyl or thiophenyl radical may be substituted with 1, 2 or 3 substituent(s) selected from the group consisting of methyl and chlorine.

29. Medicament according to one or more of claims 17 to 28, **characterized in that**
R^{14a} represents an alkyl radical selected from the group consisting of methyl, ethyl, n-propyl, isopropyl, n-butyl, sec-butyl, isobutyl and tert-butyl;
or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), - N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and/or may be bonded via a -(CH₂)₁,_{2 or 3}- group;
preferably R^{14a} represents an alkyl radical selected from the group consisting of methyl, ethyl, n-propyl and isopropyl,
or a phenyl radical, which may be substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, - S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, - C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃;
more preferably R^{14a} represents a methyl radical or a phenyl radical which may be substituted with 1, 2 or 3 methyl radical(s).

30. Medicament according to one or more of claims 17 to 29, **characterized in that**
X^{a} represents a -NR^{1a}R^{2a} moiety or a -OR3^{a} moiety;
R^{1a} represents an alkyl radical selected from the group consisting of -CH₂-CH₂-OH and -CH₂-CH₂-CH₂-OH;
an unsubstituted adamantyl radical;
an unsubstituted phenyl or pyrrolyl radical;
an unsubstituted napthyl radical which is bonded via an alkylene group selected form the group consisting of -CH₂-, -CH(CH₃)-, -CH₂-CH₂-, -CH₂-CH₂-CH₂- and -CH₂-CH₂-O-;
a phenyl radical which may be substituted with 1, 2 or 3 substituent(s) independently selected from the group consisting of methyl, ethyl, tert-butyl, methoxy, F and Cl and said phenyl radical is bonded via an alkylene group selected form the group consisting of -CH₂-, -CH(CH₃)-, -CH(Phenyl)-, -CH₂-CH₂-, -CH₂-CH₂-CH₂- and -CH₂-CH₂-O-;
a heteroaryl radical selected from the group consisting of pyridinyl, furanyl and pyrrolyl, whereby said pyridinyl, furanyl or pyrrolyl radical may be substituted with 1, 2 or 3 substituent(s) independently selected from the group consisting of methyl, ethyl, tert-butyl, methoxy, F and Cl and said pyridinyl, furanyl or pyrrolyl radical is bonded via an alkylene group selected form the group consisting of -CH₂-, -CH(CH₃)-, -CH₂-CH₂-, -CH₂-CH₂-CH₂- and -CH₂-CH₂-O-;
or a -C(=O)-R^{12a} moiety;
R^{2a} represents a hydrogen atom or a methyl radical;
or
R^{1a} and R^{2a} together with the bridging nitrogen atom form a moiety selected from the group consisting of 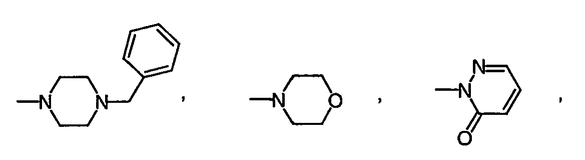 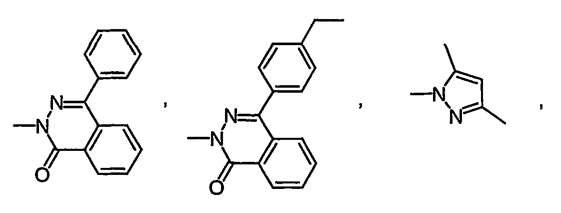 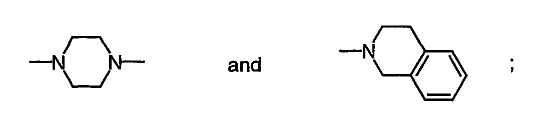 R^{3a} represents an unsubstituted phenyl radical;
R^{4a}, R^{5a} and R^{6a}, identical or different, each represent a hydrogen atom or a fluorine atom;
or
R^{4a} and R^{5a} together with the bridging carbon atoms form the following moiety, 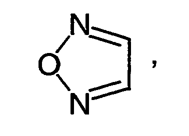 which together with the phenyl ring which it is fused with forms the following substituted bicyclic aromatic ring system 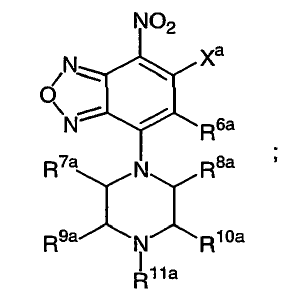 R^{7a} and R^{8a} each represent a hydrogen atom;
R^{9a} and R^{10a}, identical or different, each represent a hydrogen atom or a methyl radical;
R^{11a} represents a hydrogen atom;
an alkyl radical selected from the group consisting of methyl, n-butyl and - CH₂-CH₂-OH;
an unsubstituted phenyl or pyridinyl radical whereby said phenyl or pyridinyl radical may be bonded via a -(CH₂)- group;
a -C(=O)-R^{12a} moiety or a -S(=O)₂-R^{13a} moiety;
R^{12a} represents a phenyl or a thiophenyl radical whereby said phenyl or thiophenyl radical may be substituted with 1, 2 or 3 substituent(s) selected from the group consisting of methyl and chlorine;
R^{13a} represents a methyl radical or a phenyl or a thiophenyl radical whereby said phenyl or thiophenyl radical may be substituted with 1, 2 or 3 substituent(s) selected from the group consisting of methyl and chlorine
and
R^{14a} represents a methyl radical or a phenyl radical which may be substituted with 1, 2 or 3 methyl radical(s);
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

31. Medicament according to one or more of claims 17 to 30 comprising at least one compound selected from the group consisting of
[1] [5-(4-Methyl-piperazin-1-yl)-2-nitro-phenyl]-(2-pyrrol-1-yl-ethyl)-amine,
[2] (4-Fluoro-benzyl)-[5-(4-methyl-piperazin-1-yl)-2-nitro-phenyl]-amine,
[3] N-[5-(4-Methyl-piperazin-1-yl)-2-nitro-phenyl]-benzamide,
[4] N-Methyl-N-[5-(4-methyl-piperazin-1-yl)-2-nitro-phenyl]-benzamide,
[5] [5-(4-Methyl-piperazin-1-yl)-2-nitro-phenyl]-pyrrol-1-yl-amine,
[6] 2-[5-(4-Methyl-piperazin-1-yl)-2-nitro-phenyl]-2H-pyridazin-3-one,
[7] 1-Methyl-4-(4-nitro-3-phenoxy-phenyl)-piperazine,
[8] Benzyl-[5-(4-butyl-piperazin-1-yl)-2-nitro-phenyl]-amine,
[9] Benzyl-[2-nitro-5-(4-pyridin-2-yl-piperazin-1-yl)-phenyl]-amine and
[10] Benzyl-[2-nitro-5-(4-phenyl-2-yl-piperazin-1-yl)-phenyl]-amine;
[11] Furan-2-ylmethyl-[5-(4-methyl-piperazin-1-yl)-2-nitro-phenyl]-amine,
[12] 2-[4-(4-Nitro-3-phenethylamino-phenyl)-piperazin-1-yl]-ethanol,
[13] (2-Nitro-5-piperazin-1-yl-phenyl)-(2-o-tolyloxy-ethyl)-amine
[14] 2-[4-(3-Benzylamino-4-nitro-phenyl)-piperazin-1-yl]-ethanol,
[15] 4-[5-(4-Methyl-piperazin-1-yl)-2-nitro-phenyl]-morpholine,
[16] 2-[5-(4-Methyl-piperazin-1-yl)-2-nitro-phenyl]-4-phenyl-2H-phthalazin-1-one,
[17] [2-(4-Chloro-phenoxy)-ethyl]-[5-(3,5-dimethyl-piperazin-1-yl)-2-nitrophenyl]-amine,
[18] 2-[4-[3-(Benzhydryi-amino)-4-nitro-phenyl]-piperazin-1-yl]-ethanol,
[19] 4-[4-Fluoro-5-(4-methyl-piperazin-1-yl)-2-nitro-phenyl]-morpholine,
[20] 2-[2-Nitro-5-[4-(toluene-4-sulfonyl)-piperazin-1-yl]-phenyl]-1,2,3,4-tetrahydro-isoquinoline,
[21] 1-[3-(3,5-Dimethyl-pyrazol-1-yl)-4-nitro-phenyl]-4-methyl-piperazine,
[22] Benzyl-(2-nitro-5-piperazin-1-yl-phenyl)-amine,
[23] [5-(4-Methyl-piperazin-1-yl)-2-nitro-phenyl]-phenethyl-amine,
[24] [5-(4-Methyl-piperazin-1-yl)-2-nitro-phenyl]-pyridin-3-ylmethyl-amine,
[25] (3-Chloro-phenyl)-[4-[3-[(furanyl-2-ylmethyl)-amino]-4-nitro-phenyl]-piperazin-1-yl]-methanone,
[26] (2-Nitro-5-piperazin-1-yl-phenyl)-pyridin-3-ylmethyl-amine,
[27] 1-Benzyl-4-(2-nitro-5-piperazin-1-yl-phenyl)-piperazine,
[28] Furan-2-ylmethyl-[5-(4-methanesulfonyl-piperazin-1-yl)-2-nitro-phenyl]-amine,
[29] Benzhydryl-[5-(4-methyl-piperazin-1-yl)-2-nitro-phenyl]-amine,
[30] (2-Nitro-5-piperazin-1-yl-phenyl)-(2-phenoxy-ethyl)-amine,
[31] 2-[5-(4-Methyl-piperazin-1-yl)-2-nitro-phenyl]-4-phenyl-2H-phthalazin-1-one,
[32] 1-[3-(3,5-Dimethyl-pyrazol-1-yl)-4-nitro-phenyl]-piperazine,
[33] (2-Nitro-5-piperazin-1-yl-phenyl)-phenethyl-amine,
[34] [5-(4-Benzenesulfonyl-piperazin-1-yl)-2-nitro-phenyl]-furan-2-ylmethyl-amine,
[35] [2-(3,4-Dimethoxy-phenyl)-ethyl]-(2-nitro-5-piperazin-1-yl-phenyl)-amine,
[36] [4-[3-[(Furan-2-ylmethyl)-amino]-4-nitro-phenyl]-piperazin-1-yl]-m-tolyl-methanone,
[37] [4-[3-[(Furan-2-ylmethyl)-amino]-4-nitro-phenyl]-piperazin-1-yl]-phenyl-methanone,
[38] [4-[3-(3,5-Dimethyl-pyrazol-1-yl)-4-nitro-phenyl]-piperazin-1-yl]-thiophen-2-yl-methanone,
[39] 4-(4-Ethyl-phenyl)-2-[5-[4-methyl-piperazin-1-yl)-2-nitro-phenyl]-2H-phthalalazin-1-one,
[40] 3-[7-(4-Methyl-piperazin-1-yl)-4-nitro-benzo[1,2,5]oxadiazol-5-ylamino]-propan-1-ol,
[41] 3-[4-Nitro-7-(4-phenyl-piperazin-1-yl)-benzo[1,2,5]oxadiazol-5-ylamino]-ethan-1-ol,
[42] 3-[4-Nitro-7-(4-pyridin-piperazin-1-yl)-benzo[1,2,5]oxadiazol-5-ylamino]-propan-1-ol,
[43] [2-(3,4-Dimethoxy-phenyl)-ethyl]-[5-(4-methyl-piperazin-1-yl)-2-nitrophenyl]-amine,
[44] [2-(3,4-Dimethyl-phenyl)-ethyl]-[5-(4-methyl-piperazin-1-yl)-2-nitrophenyl]-amine,
[45] [2-(4-tert-Butyl-phenoxy)-ethyl]-[5-(4-methyl-piperazin-1-yl) -2-nitrophenyl]-amine,
[46] [2-(4-Methoxy-phenoxy)-ethyl]-[5-(4-methyl-piperazin-1-yl)-2-nitrophenyl]-amine,
[47] [5-(4-Methyl-piperazin-1-yl)-2-nitro-phenyl]-(2-m-tolyloxy-ethyl)-amine,
[48] [2-(4-Chloro-phenoxy)-ethyl]-[5-(4-methyl-piperazin-1-yl)-2-nitro-phenyl]-amine,
[49] (2-Nitro-5-piperazin-1-yl-phenyl)-(1-phenyl-ethyl)-amine,
[50] [2-(3-Methoxy-phenoxy)-ethyl]-[5-(4-methyl-piperazin-1-yl)-2-nitrophenyl]-amine,
[51] [2-(2-Methoxy-phenoxy)-ethyl]-[5-(4-methyl-piperazin-1-yl)-2-nitrophenyl]-amine,
[52] (4-Chloro-benzyl)-(2-nitro-5-piperazin-1-yl-phenyl)-amine,
[53] Benzyl-[5-(4-benzyl-piperazin-1-yl)-2-nitro-phenyl]-amine,
[54] Benzyl-[5-(4-methyl-piperazin-1-yl)-2-nitro-phenyl]-amine,
[55] [5-(4-Methyl-piperazin-1-yl)-2-nitro-phenyl]-(2-o-tolyloxy-ethyl)-amine,
[56] (4-Chloro-benzyl)-[5-(4-methyl-piperazin-1-yl)-2-nitro-phenyl]-amine,
[57] Furan-2-ylmethyl-(2-nitro-5-piperazin-1-yl-phenyl)-amine,
[58] [2-(4-Chloro-phenoxy)-ethyl]-(2-nitro-5-piperazin-1-yl-phenyl)-amine,
[59] [5-(4-Methyl-piperazin-1-yl)-2-nitro-phenyl]-(1-phenyl-ethyl)-amine
[60] 1-[4-(3-Benzylamino-4-nitro-phenyl)-piperazin-1-yl]-ethanone,
[61] 2-[4-[3-(4-Methylpiperazin-1-yl)-4-nitrophenyl]piperazin-1-yl]ethanol,
[62] 2-[4-[3-[2-(Naphthalen-2-yloxy)ethylamino]-4-nitrophenyl]piperazin-1-yl]ethanol,
[63] 2-[4-(3-{[1-(1-Adamantyl)ethyl]amino}-4-nitrophenyl)piperazin-1-yl]ethanol and
[64] 2-[4-[3-(3,4-Dimethoxyphenethylamino)-4-nitrophenyl]piperazin-1-yl]ethanol;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

32. Medicament according to one or more of claims 17 to 31 for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia, type II diabetes (non insulin dependent diabetes mellitus), preferably type II diabetes that is caused by obesity; for the prophylaxis and/or treatment of stroke; migraine; head trauma; epilepsy; irritable colon syndrome; irritable bowl syndrome; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; chronic intermittent hypoxia; convulsions; or hyperactivity disorder (ADHD, attention deficit/hyperactivity disorder); for improvement of cognition (cognitive enhancement) or cognitive memory (cognitive memory enhancement); for the prophylaxis and/or treatment of drug addiction and/or withdrawal; for the prophylaxis and/or treatment of alcohol addiction and/or withdrawal, for the prophylaxis and/or treatment of nicotine addiction and/or withdrawal.

33. Use of at least one compound of general formula la according to one or more of claims 17 to 31 for the manufacture of medicament for the prophylaxis and/or treatment of a disorder or disease related to food intake, preferably for the regulation of appetite; for the maintenance, increase or reduction of body weight; or for the prophylaxis and/or treatment of obesity, bulimia, anorexia, cachexia or type II diabetes (non insulin dependent diabetes mellitus), more preferably for the prophylaxis and/or treatment of obesity.

34. Use of at least one compound of general formula la according to one or more of claims 17 to 31 for the manufacture of a medicament for the prophylaxis and/or treatment of stroke; migraine; head trauma; epilepsy; irritable colon syndrome; irritable bowl syndrome; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; chronic intermittent hypoxia; convulsions; or hyperactivity disorder (ADHD, attention deficit/hyperactivity disorder).

35. Use of at least one compound of general formula la according to one or more of claims 17 to 31 for the manufacture of a medicament for the improvement of cognition (cognitive enhancement) and/or for the improvement of cognitive memory (cognitive memory enhancement).

36. Use of at least one compound of general formula la according to one or more of claims 17 to 31 for the manufacture of a medicament for the prophylaxis and/or treatment of drug addiction and/or withdrawal, preferably for the prophylaxis and/or treatment of addiction and/or withdrawal related to one or more of drugs selected from the group consisting of benzodiazepines, natural, semisynthetic or synthetic opioids like cocaine, ethanol and/or nicotine.

## Patentansprüche

1. Nitro-substituierte Phenyl-Piperazin Verbindung der allgemeinen Formel I, 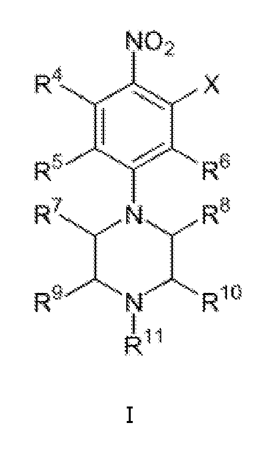 wobei
X eine -NR¹R² funktionelle Gruppe oder eine -OR³ funktionelle Gruppe darstellt;
R¹ stellt dar, ein gesättigtes oder ungesättigtes 3- bis 9- gliedriges cycloaliphatisches Radikal, welches mit 1, 2 oder 3 Substituent(en) substituiert sein kann, die unabhängig aus der Gruppe ausgewählt sind, die aus Oxo (=O), Thioxo (=S), C₁₋₅- Alkyl, -O-C-₁₋₅-Alkyl, -S-C₁₋₅-Alkyl, -C(=O)-OH, -C(=O)-C₁₋₅-Alkyl, - C(=O)-O-C₁₋₅-Alkyl, -O-C(=O)-C₁₋₅-Alkyl, F, CI, Br, I, -CN, -CF₃, - OCF₃, -SCF₃, -OH, -SH, -NH₂, -NH(C₁₋₅-Alkyl), -N(C₁₋₅-Alkyl)₂, -NO₂, -CHO, -CF₂H, -CFH₂, -C(=O)-NH₂, -C(=O)-NH(C₁₋₅-Alkyl), -C(=O)- N(C₁₋₅-Alkyl)₂, -S(=O)₂-C₁₋₅-Alkyl, -S(=O)₂-Phenyl, Phenyl, Phenoxy und Benzyl besteht, und welches 1, 2 oder 3 Heteroatom(e) enthalten kann, die unabhängig aus der Gruppe ausgewählt sind, die aus Stickstoff, Sauerstoff und Schwefel besteht, als Ringelement(e) und welches über eine lineare oder verzweigte C₁₋₆-Alkylen-, C₂₋₆-Alkenylen- oder C₂₋₆-Alkinylengruppe, welche unsubstituiert oder mit 1, 2 oder 3 Substituent(en) substituiert sein kann, die unabhängig aus der Gruppe ausgewählt sind, die aus F, Cl, Br, I, -CN, -CF₃, -OCF₃, -SCF₃, -OH, -SH, -NH₂, - O-C₁₋₅-Alkyl, -S-C₁₋₅-Alkyl, -NH-(C₁₋₅-Alkyl) und -N(C₁₋₅-Alkyl)₂ besteht, gebunden ist;
unter der Bedingung, dass R¹ nicht ein Tetrahydrofuranylradikal darstellt;
ein Heteroarylradikal, das aus der Gruppe ausgewählt ist, die aus Furyl (Furanyl), Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridinyl, Pyridazinyl, Pyrimidinyl, Pyrazinyl, Quinolinyl, Isoquinolinyl, Benzo[b]furanyl, Benzo[b]thiophenyl, Benzothiadiazolyl und Imidazo[2,1-b]thiazolyl besteht, welches mit 1, 2, 3, 4 oder 5 Substituent(en) substituiert sein kann, die unabhängig aus der Gruppe ausgewählt sind, die aus C₁₋₅-Alkyl, -O-C₁₋₅-Alkyl, -S-C₁₋₅-Alkyl, -C(=O)-OH, -C(=O)-C₁₋₅-Alkyl, -C(=O)-O-C₁₋₅-Alkyl, -O-C(=O)-C₁₋₅-Alkyl, F, Cl, Br, I, -CN, -CF₃, -OCF₃, -SCF₃, -OH, -SH, - NH₂, -NH(C₁₋₅-Alkyl), -N(C₁₋₅-Alkyl)₂, -NO₂, -CHO, -CF₂H, -CFH₂, - C(=O)-NH₂, -C(=O)-NH(C₁₋₅-Alkyl), -C(=O)-N(C₁₋₅-Alkyl)₂, -S(=O)₂-C₁₋₅-Alkyl, -S(=O)₂-Phenyl, Phenyl, Phenoxy und Benzyl besteht;
ein Aryl oder Heteroarylradikal, das aus der Gruppe ausgewählt ist, die aus Phenyl, Naphthyl, Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridazinyl, Pyrimidinyl, Pyrazinyl, Quinolinyl, Isoquinolinyl, Benzo[b]furanyl, Benzo[b]thiophenyl, Benzothiadiazolyl, und Imidazo[2,1-b]thiazolyl besteht, wobei das Aryl oder Heteroarylradikal über eine -(CH₂)- Gruppe gebunden ist und mit 1, 2, 3, 4 oder 5 Substituent(en) substituiert sein kann, die unabhängig aus der Gruppe ausgewählt sind, die aus C₁₋₅-Alkyl, -O-C₁₋₅-Alkyl, -S-C₁₋₅-Alkyl, -C(=O)-C₁₋₅-Alkyl, -C(=O)-O-C₁₋₅-Alkyl, -O-C(=O)-C₁₋₅-Alkyl, F, Br, I, -CN, -CF₃, -OCF₃, -SCF₃, -OH, - SH, -NH₂, -NH(C₁₋₅-Alkyl), -N(C₁₋₅-Alkyl)₂, -NO₂, -CHO, -CF₂H, -CFH₂, -C(=O)-NH₂, -C(=O)-NH(C₁₋₅-Alkyl), -C(=O)-N(C₁₋₅-Alkyl)₂, -S(=O)₂- C₁₋₅-Alkyl, -S(=O)₂-Phenyl, Phenyl, Phenoxy und Benzyl besteht;
ein Aryl oder Heteroarlyradikal, das aus der Gruppe ausgewählt ist, die aus Naphtyl, Furyl (Furanyl), Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridinyl, Pyridazinyl, Pyrimidinyl, Pyrazinyl, Quinlinyl, Isoquinolinyl, Benzo[b]furanyl, Benzo[b]thiophenyl, Benzothiadiazolyl und Imidazo[2,1-b]thiazolyl besteht, wobei das Aryl oder Heteroarylradikal über eine -(CFH₂)₂- Gruppe gebunden ist und/oder mit 1, 2, 3, 4 oder 5 Substituent(en) substituiert sein kann, die unabhängig aus der Gruppe ausgewählt sind, die aus C₁₋₅-Alkyl, -O-C₁₋₅-Alkyl, -S-C₁₋₅- Alkyl, -C(=O)-OH, -C(=O)-C₁₋₅-Alkyl, -C(=O)-O-C₁₋₅-Alkyl, -O-C(=O)- C₁₋₅-Alkyl, F, Cl, Br, I, -CN, -CF₃, -OCF₃, -SCF₃, -OH, -SH, -NH₂, -NH- (C₁₋₅-Alkyl), -N(C₁₋₅-Alkyl)₂, -NO₂, -CHO, -CF₂H, -CFH₂, -C(=O)-NH₂, - C(=O)-NH(C₁₋₅-Alkyl), -C(=O)-N(C₁₋₅-Alkyl)₂, -S(=O)₂-C₁₋₅-Alkyl, - S(=O)₂-Phenyl, Phenyl, Phenoxy und Benzyl besteht;
ein Aryl oder Heteroarylradikal, das aus der Gruppe ausgewählt ist, die aus Phenyl, Naphthyl, Furyl (Furanyl), Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridinyl, Pyridazinyl, Pyrimidinyl, Pyrazinyl, Quinolinyl, Isoquinolinyl, Benzo[b]furanyl, Benzo[b]thiophenyl, Benzothiadiazolyl, und Imidazo[2,1-b]thiazolyl besteht, wobei das Aryl oder Heteroarylradiakl über eine -(CFH₂)₃- Gruppe gebunden ist und/oder mit 1, 2, 3, 4 oder 5 Substituent(en) substituiert sein kann, die unabhängig aus der Gruppe ausgewählt sind, die aus C₁₋₅-Alkyl, -O-C₁₋₅-Alkyl, -S-C₁₋₅- Alkyl, -C(=O)-OH, -C(=O)-C₁₋₅-Alkyl, -C(=O)-O-C₁₋₅-Alkyl, -O- C(=O)-C₁₋₅-Alkyl, F, Cl, Br, I, -CN, -CF₃, -OCF₃, -SCF₃, -OH, -SH, - NH₂, -NH(C₁₋₅-Alkyl), -N(C₁₋₅-Alkyl)₂, -NO₂, -CHO, -CF₂H, -CFH, - C(=O)-NH₂, -C(=O)-NH(C₁₋₅-Alkyl), -C(=O)-N(C₁₋₅-Alkyl)₂, - S(=O)₂-C₁₋₅-Alkyl, -S(=O)₂-Phenyl, Phenyl, Phenoxy und Benzyl besteht;
oder
eine -C(=O)-R¹² funktionelle Gruppe;
R² ein Wasserstoffatom oder ein lineares oder verzweigtes, gesättigtes oder ungesättigtes C₁₋₁₀ aliphatisches Radikal darstellt, welches unsubstituiert oder mit 1, 2 oder 3 Substituent(en) substituiert sein kann, die unabhängig aus der Gruppe ausgewählt sind, die aus F, Cl, Br, I, -CN, -CF₃, -OCF₃, -SCF₃, -OH, -SH, -NH₂, -O-C₁₋₅-Alkyl, -S-C₁₋₅- Alkyl, -NH-(C₁₋₅-Alkyl) und -N(C₁₋₅-Alkyl)₂ besteht; oder
R¹und R² zusammen mit dem brückenbildenden Stickstoffatom einen gesättigten oder ungesättigten, aber nicht aromatischen 3- bis 6- gliedrigen heterozyklischen Ring bilden, welcher 1, 2 oder 3 zusätzliche Heteroatom(e) enthält, die unabhängig aus der Gruppe ausgewählt sind, die aus Stickstoff und Schwefel besteht, als Ringelement(e), und welcher mit 1, 2, oder 3 Substituent(en) substituiert sein kann, die unabhängig aus der Gruppe ausgewählt sind, die aus C₁₋₅-Alkyl, -O-C₁₋₅-Alkyl, -S-C₁₋₅-Alkyl, Oxo (=O), Thioxo (=S), -C(=O)-OH, -C(=O)-C₁₋₅-Alkyl, -C(=O)-O-C₁₋₅-Alkyl, -O-C(=O)- C₁₋₅-Alkyl, F, Cl, Br, I, -CN, -CF₃, -OCF₃, -SCF₃, -OH, -SH, -NH-, - NH(C₁₋₅-Alkyl), -N(C₁₋₅-Alkyl)₂, -NO₂, -CHO, -CF₂H, -CFH₂, -C(=O)- NH₂, -C(=O)-NH(C₁₋₅-Alkyl), -C(=O)-N(C₁₋₅-Alkyl)₂, -S(=O)₂-C₁₋₅- Alkyl, -S(=O)₂-Phenyl, Phenyl, Phenoxy und Benzyl besteht;
R³ ein 5- bis 10-gliedriges Aryl oder Heteroarylradikal darstellt, welches unsubstituiert sein kann oder mit 1, 2, 3, 4 oder 5 Substituent(en) substituiert sein kann, die unabhängig aus der Gruppe ausgewählt sind, die aus C₁₋₅-Alkyl, -O-C₁₋₅-Alkyl, -S-C₁₋₅-Alkyl, -C(=O)-OH, - C(=O)-C₁₋₅-Alkyl, -C(=O)-O-C₁₋₅-Alkyl, -O-C(=O)-C₁₋₅-Alkyl, F, Cl, Br, I, -CN, -CF₃, -OCF₃, -SCF₃, -OH, -SH, -NH₂, -NH(C₁₋₅-Alkyl), -N(C₁₋₅- Alkyl)₂, -NO₂, -CHO, -CF₂H, -CFH₂, -C(=O)-NH₂, -C(=O)-NH(C₁₋₅- Alkyl), -C(=O)-N(C₁₋₅-Alkyl)₂, -S(=O)₂-C₁₋₅-Alkyl, -S(=O)₂-Phenyl, Phenyl, Phenoxy und Benzyl besteht und welches über eine lineare oder verzweigte C₁₋₆ Alkylen, C₂₋₆ Alkenylen oder C₂₋₆ Alkinylengruppe, welche unsubstituiert oder mit 1, 2 oder 3 Substituent(en) substituiert sein kann, die unabhängig aus der Gruppe ausgewählt sind, die aus F, Cl, Br, I, -CN, -CF₃, -OCF₃, -SCF₃, -OH, SH, -NH₂, -O-C₁₋₅-Alkyl, -S-C₁₋₅-Alkyl, -NH-(C₁₋₅-Alkyl) und - N(C₁₋₅-Alkyl)₂ besteht, gebunden sein kann und wobei das Heteroarylradikal 1, 2 oder 3 Heteroatom(e) enthält, die unabhängig aus der Gruppe ausgewählt sind, die aus Stickstoff, Sauerstoff und Schwefel besteht, als Ringelement (e);
R⁴, R⁵ und R⁶ jeweils ein Wasserstoffatom darstellen;
R⁷ und R⁸, identisch oder verschieden, jeweils ein Wasserstoffatom oder ein lineares oder verzweigtes, gesättigtes oder ungesättigtes C₁₋₁₀ aliphatisches Radikal darstellen, welches unsubstituiert oder mit 1, 2 oder 3 Substituent(en) substituiert sein kann, die unabhängig aus der Gruppe ausgewählt sind, die aus F, Cl, Br, I, -CN, -CF₃, -OCF₃, -SCF₃, -OH, -SH, -NH₂, -O-C₁-₅Alkyl, -S-C₁₋₅-Alkyl, -NH-(C₁₋₅-Alkyl) und - N(C₁₋₅-Alkyl)₂ besteht;
R⁹ und R¹⁰ identisch oder verschieden, jeweils ein Wasserstoffatom oder ein lineares oder verzweigtes, gesättigtes oder ungesättigtes C₁₋₁₀ aliphatisches Radikal darstellen, welches unsubstituiert oder mit 1, 2 oder 3 Substituent(en) substituiert sein kann, die unabhängig aus der Gruppe ausgewählt sind, die aus F, Cl, Br, I, -CN, -CF₃, -OCF₃, -SCF₃, -OH, -SH, -NH₂, -O-C₁₋₅-Alkyl, -S-C₁₋₅-Alkyl, -NH-(C₁₋₅-Alkyl) und - N(C₁₋₅-Alkyl)₂ besteht;
R¹¹ ein lineares oder verzweigtes, gesättigtes oder ungesättigtes, unsubstituiertes C₁₋₁₀ aliphatisches Radikal oder ein 5- bis 10- gliedriges Aryl oder Heteroarylradikal darstellt, welches unsubstituiert sein kann oder mit 1, 2, 3, 4 oder 5 Substituent(en) substituiert sein kann, die unabhängig aus der Gruppe ausgewählt sind, die aus C₁₋₅- Alkyl, -O-C₁₋₅-Alkyl, -S-C₁₋₅-Alkyl, -C(=O)-OH, -C(=O)-C₁₋₅-Alkyl, - C(=O)-O-C₁₋₅-Alkyl, -O-C(=O)-C₁₋₅-Alkyl, F, Cl, Br, I, -CN, -CF₃, - OCF₃, -SCF₃, -OH, -SH, -NH₂, -NH(C₁₋₅-Alkyl), -N(C₁₋₅-Alkyl)₂, -NO₂, - CHO, -CF₂H, -CFH₂, -C(=O)-NH₂, -C(=O)-NH(C₁₋₅-Alkyl), -C(=O)- N(C₁₋₅-Alkyl)₂, -S(=O)₂-C₁₋₅-Alkyl, -S(=O)₂-Phenyl, Phenyl, Phenoxy und Benzyl besteht und wobei das Heteroarylradikal 1, 2 oder 3 Heteratom(e) enthält, die unabhängig aus der Gruppe ausgewählt sind, die aus Stickstoff, Sauerstoff und Schwefel besteht, als Ringelement(e);
unter der Bedingung, dass R¹¹ nicht ein Thiazolylradikal darstellt;
und
R¹² stellt dar, ein 5 bis 10 gliedriges Arylradikal, welches mit 1, 2, 3, 4 oder 5 Substituent(en) substituiert sein kann, die unabhängig aus der Gruppe ausgewählt sind, die aus C₁₋₅-Alkyl, -O-C₁₋₅-Alkyl, -S-C₁₋₅- Alkyl, -C(=O)-OH, -C(=O)-C₁₋₅-Alkyl, -C(=O)-O-C₁₋₅-Alkyl, -O-C(=O)- C₁₋₅-Alkyl, F, Br, I, -CN, -CF₃, -OCF₃, -SCF₃, -SH, -NH₂, -NH(C₁-₅- Alkyl), -N(C₁₋₅-Alkyl)₂, -CHO, -CF₂H, -CFH₂, -C(=O)-NHz, -C(=O)- NH(C₁₋₅-Alkyl), -C(=O)-N(C₁₋₅-Alkyl)₂, -S(=O)₂-C₁₋₅-Alkyl, -S(=O)₂- Phenyl, Phenyl, Phenoxy und Benzyl besteht;
ein Heteroarylradikal, das aus der Gruppe ausgewählt ist, die aus Furyl (Furanyl), Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridinyl, Pyridazinyl, Pyrimidinyl, Pyrazinyl, Quinolinyl, Isoquinolinyl, Benzo[b]furanyl, Benzo[b]thiophenyl, Benzothiadiazolyl und Imidazo[2,1-b]thiazolyl besteht, welches unsubstituiert sein kann oder mit 1, 2, 3, 4 oder 5 Substituent(en) substituiert sein kann, die unabhängig aus der Gruppe ausgewählt sind, die aus C₁₋₅-Alkyl, -O- C₁₋₅-Alkyl, -S-C₁₋₅-Alkyl, -C(=O)-OH, -C(=O)-C₁₋₅-Alkyl, -C(=O)-O-C₁₋₅-Alkyl, -O-C(=O)-C₁₋₅-Alkyl, F, Cl, Br, I, -CN, -CF₃, -OCF₃, -SCF₃, - OH, SH, -NH₂, -NH(C₁₋₅-Alkyl), -N(C₁₋₅-Alkyl)₂, -NO₂, -CHO, -CF₂H, - CFH₂, -C(=O)-NH₂, -C(=O)-NH(C₁₋₅-Alkyl), -C(=O)-N(C₁₋₅-Alkyl)₂, - S(=O)₂-C₁₋₅-Alkyl, -S(=O)₂-Phenyl, Phenyl, Phenoxy und Benzyl besteht;
oder
ein Aryl oder Heteroarylradikal, das aus der Gruppe ausgewählt ist, die aus Phenyl, Naphthyl, Furyl (Furanyl), Thienyl (Thiophenyl), Pyrrolyl, Oxazolyl, Isoxazolyl, Thiazolyl, Isothiazolyl, Pyrazolyl, Oxadiazolyl, Thiadiazolyl, Triazolyl, Pyridinyl, Pyridazinyl, Pyrimidinyl, Pyrazinyl, Quinlinyl, Isoquinolinyl, Benzo[b]furanyl, Benzo[b]thiophenyl, Benzothiadiazolyl und Imidazo[2,1-b]thiazolyl besteht, welches unsubstituiert sein kann oder mit 1, 2, 3, 4 oder 5 Substituent(en) substituiert sein kann, die unabhängig aus der Gruppe ausgewählt sind, die aus C₁₋₅-Alkyl, -O-C₁₋₅-Alkyl, -S-C₁₋₅- Alkyl, -C(=O)-OH, -C(=O)-C₁₋₅-Alkyl, -C(=O)-O-C₁₋₅-Alkyl, -O-C(=O)- C₁₋₅-Alkyl, F, Cl Br, I, -CN, -CF₃, -OCF₃, -SCF₃, -OH, -SH, -NH₂, - NH(C₁₋₅-Alkyl), -N(C₁₋₅-Alkyl)₂, -NO₂, -CHO, -CF₂H, -CFH₂, -C(=O)- NH, -C(=O)-NH(C₁-₅-Alkyl), -C(=O)-N(C₁₋₅-Alkyl)₂, -S(=O)₂-C₁₋₅- Alkyl, -S(=O)₂-Phenyl, Phenyl, Phenoxy und Benzyl besteht und welches über eine -(CH₂)₁,_{2 oder 3}-Gruppe gebunden ist;
unter der Bedingung, dass die folgende Verbindung
(1) Benzyl-[5-(4-methyl-piperazin-1-yl)-2-nitro-phenyl]-amin
ausgeschlossen ist;
optional in Form von einem seiner Stereoisomere, bevorzugt Enantiomere oder Diasteromere, eines Racemats oder in Form einer Mischung von zumindest zwei seiner Stereoisomeren, bevorzugt Enantiomeren und/oder Diasteromeren, in irgendeinem Mischungsverhältnis, oder eines physiologischen akzeptablen Salzes davon, oder eines korrespondierenden Solvats davon.

2. Verbindung nach Anspruch 1, **dadurch gekennzeichnet, dass**
X eine -NR¹R² funktionelle Gruppe oder eine -OR³ funktionelle Gruppe darstellt;
R¹ stellt dar, ein (hetero)cycloaliphatisches Radikal, das aus der Gruppe ausgewählt ist, die aus Cyclopropyl, Cyclobutyl, Cyclopentyl, Cyclopentenyl, Cyclohexyl, Cyclohexenyl, Cycloheptyl, Cycloheptenyl, Cyclooctyl, Cyclooctenyl, Cyclononyl, Imidazolidinyl, Aziridinyl, Azetidinyl, Pyrrolidinyl, Piperidinyl, Morpholinyl, Thiomorpholinyl, Piperazinyl, Pyrazolidinyl, Tetrahydrothiophenyl und Azepanyl besteht, welches mit 1, 2 oder 3 Substituent(en) substituiert sein kann, die unabhängig aus der Gruppe ausgewählt sind, die aus Oxo (=O), Thioxo (=S), C₁₋₅-Alkyl, -O-C-₁₋₅-Alkyl, -S-C₁₋₅-Alkyl, - C(=O)-OH, -C(=O)-C₁₋₅-Alkyl, -C(=O)-O-C₁₋₅-Alkyl, -O-C(=O)- C₁₋₅-Alkyl, F, Cl, Br, I, -CN, -CF₃, -OCF₃, -SCF₃, -OH, SH, -NH₂, -NH(C₁₋₅-Alkyl), -N(C₁₋₅-Alkyl)₂, -NO₂, -CHO, -CF₂H, -CFH₂, - C(=O)-NH₂, -C(=O)-NH(C₁₋₅-Alkyl), -C(=O)-N(C₁₋₅-Alkyl)₂, - S(=O)₂-C₁₋₅-Alkyl, -S(=O)₂-Phenyl, Phenyl, Phenoxy und Benzyl besteht und welches über eine lineare oder verzweigte C₁₋₆ Alkylen-, C₂₋₆ Alkenylen-, C₂₋₆ Alkinylengruppe gebunden ist, welche unsubstituiert oder mit 1, 2 oder 3 Substituent(en) substituiert sein kann, die unabhängig aus der Gruppe ausgewählt sind, die aus F, Cl, Br, I, -CN, -CF₃, -OCF₃, -SCF₃, - OH, -SH, -NH₂, -O-C₁₋₅-Alkyl, -S-C₁₋₅-Alkyl, -NH-(C₁₋₅-Alkyl) und -N(C₁₋₅-Alkyl)₂ besteht;
ein Heteroarylradikal, das aus der Gruppe ausgewählt ist, die aus Furyl (Furanyl), Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridinyl, Pyridazinyl, Pyrimidinyl, Pyrazinyl, Quinolinyl, Isoquinolinyl, Benzo[b]furanyl, Benzo[b]thiophenyl, Benzothiadiazolyl und Imidazo[2,1-b]thiazolyl besteht, welches mit 1, 2, 3, 4 oder 5 Substituent(en) substituiert sein kann, die unabhängig aus der Gruppe ausgewählt sind, die aus C₁₋₅-Alkyl, -O-C₁₋₅-Alkyl, -S- C₁₋₅-Alkyl, -C(=O)-OH, -C(=O)-C₁₋₅-Alkyl, -C(=O)-O-C₁₋₅-Alkyl, -O-C(=O)-C₁₋₅-Alkyl, F, Cl, Br, I, -CN, -CF₃, -OCF₃, -SCF₃, -OH, -SH, -NH₂, -NH(C₁₋₅-Alkyl), -N(C₁₋₅-Alkyl)₂, -NO₂, -CHO, -CF₂H, -CFH₂, -C(=O)-NH₂, -C(=O)-NH(C₁₋₅-Alkyl), -C(=O)-N(C₁₋₅- Alkyl)₂, -S(=O)₂-C₁₋₅-Alkyl, -S(=O)₂-Phenyl, Phenyl, Phenoxy und Benzyl besteht;
ein Aryl oder Heteroarylradikal, das aus der Gruppe ausgewählt ist, die aus Phenyl, Naphthyl, Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridazinyl, Pyrimidinyl, Pyrazinyl, Quinolinyl, Isoquinolinyl, Benzo[b]furanyl, Benzo[b]thiophenyl, Benzothiadiazolyl, und Imidazo[2,1-b]thiazolyl besteht, wobei das Aryl oder Heterolarylradikal über eine -(CH₂)-Gruppe gebunden ist und mit 1, 2, 3, 4 oder 5 Substituent(en) substituiert sein kann, die unabhängig aus der Gruppe ausgewählt sind, die aus C₁₋₅-Alkyl, -O-C₁₋₅-Alkyl, -S-C₁₋₅-Alkyl, -C(=O)-C₁₋₅-Alkyl, -C(=O)-O-C₁₋₅- Alkyl, -O-C(=O)-C₁₋₅-Alkyl, F, Br, I, -CN, -CF₃, -OCF₃, -SCF₃, - OH, -SH, -NH₂, -NH(C₁₋₅-Alkyl), -N(C₁₋₅-Alkyl)₂, -NO₂, -CHO, - CF₂H, -CFH₂, -C(=O)-NH2, -C(=O)-NH(C₁₋₅-Alkyl), -C(=O)-N(C₁₋₅-Alkyl)₂, -S(=O)₂-C₁₋₅-Alkyl, -S(=O)₂-Phenyl, Phenyl, Phenoxy und Benzyl besteht;
ein Aryl oder Heteroarylradikal, das aus der Gruppe ausgewählt ist, die aus Naphthyl, Furyl (Furanyl), Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridinyl, Pyridazinyl, Pyrimidinyl, Pyrazinyl, Quinolinyl, Isoquinolinyl, Benzo[b]furanyl, Benzo[b]thiophenyl, Benzothiadiazolyl und Imidazo[2,1-b]thiazolyl besteht, wobei das Aryl oder Heteroarylradikal über eine -(CH₂)₂-Gruppe gebunden ist und/oder mit 1, 2, 3, 4 oder 5 Substituent(en) substituiert sein kann, die unabhängig aus der Gruppe ausgewählt sind, die aus C₁₋₅-Alkyl, -O-C₁₋₅-Alkyl, -S-C₁₋₅-Alkyl, -C(=O)-OH, -C(=O)-C₁₋₅-Alkyl, -C(=O)-O-C₁₋₅-Alkyl, -O-C(=O)- C₁-₅-Alkyl, F, Cl, Br, I, -CN, -CF₃, -OCF₃, -SCF₃, -OH, -SH, -NH₂, -NH(C₁₋₅-Alkyl), -N(C₁₋₅-Alkyl)₂, -NO₂, -CHO, -CF₂H, -CFH₂, - C(=O)-NH₂, -C(=O)-NH(C₁₋₅-Alkyl), -C(=O)-N(C₁₋₅-Alkyl)₂, - S(=O)₂-C₁₋₅-Alkyl, -S(=O)₂-Phenyl, Phenyl, Phenoxy und Benzyl besteht;
ein Aryl oder Heteroarylradikal, das aus der Gruppe ausgewählt ist, die aus Phenyl, Naphthyl, Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridazinyl, Pyrimidinyl, Pyrazinyl, Quinolinyl, Isoquinolinyl, Benzo[b]furanyl, Benzo[b]thiophenyl, Benzothiadiazolyl, und Imidazo[2,1-b]thiazolyl besteht, wobei das Aryl oder Heteroarylradikal über eine -(CH₂)₃-Gruppe gebunden ist und/oder mit 1, 2, 3, 4 oder 5 Substituent(en) substituiert sein kann, die unabhängig aus der Gruppe ausgewählt sind, die aus C₁₋₅-Alkyl, -O-C₁₋₅-Alkyl, -S-C₁₋₅-Alkyl, -C(=O)-OH, -C(=O)-C₁₋₅- Alkyl, -C(=O)-O-C₁₋₅-Alkyl, -O-C(=O)-C₁₋₅-Alkyl, F, Cl, Br, I, - CN, -CF₃, -OCF₃, -SCF₃, -OH, -SH, -NH₂, -NH(C₁₋₅-Alkyl), -N(C₁₋₅-Alkyl)₂, -NO₂, -CHO, -CF₂H, -CFH₂, -C(=O)-NH₂, -C(=O)- NH(C₁₋₅-Alkyl), -C(=O)-N(C₁₋₅-Alkyl)₂, -S(=O)₂-C₁₋₅-Alkyl, - S(=O)₂-Phenyl, Phenyl, Phenoxy und Benzyl besteht;
oder
eine -C(=O)-R¹² funktionelle Gruppe;
R² ein Wasserstoffatom oder ein lineares oder verzweigtes, gesättigtes oder ungesättigtes C₁₋₁₀ aliphatisches Radikal darstellt, welches unsubstituiert oder substituiert mit 1, 2 oder 3 Substituent(en) sein kann, die unabhängig aus der Gruppe ausgewählt sind, die aus F, Cl, Br, I, -CN, -CF₃, -OCF₃, -SCF₃, - OH, -SH, -NH₂, -O-C₁₋₅-Alkyl, -S-C₁₋₅-Alkyl, -NH-(C₁₋₅-Alkyl) und -N(C₁₋₅-Alkyl)₂ besteht; oder
R¹ und R² bilden zusammen mit dem brückenbildenden Stickstoffatom einen gesättigten oder ungesättigten, aber nicht aromatischen 3- bis 6-gliedrigen heterozyklischen Ring, welcher 1, 2 oder 3 zusätzliche Heteroatom(en) enthält, die unabhängig aus der Gruppe ausgewählt sind, die aus Stickstoff und Schwefel besteht, als Ringelement(e) und welcher mit 1, 2, oder 3 Substituent(en) substituiert sein kann, die unabhängig aus der Gruppe ausgewählt sind, die aus C₁₋₅-Alkyl, -O-C₁₋₅-Alkyl, -S- C₁₋₅-Alkyl, Oxo (=O), Thioxo (=S), -C(=O)-OH, -C(=O)-C₁₋₅- Alkyl, -C(=O)-O-C₁₋₅-Alkyl, -O-C(=O)-C₁₋₅-Alkyl, F, Cl, Br, I, - CN, -CF₃, -OCF₃, -SCF₃, -OH, -SH, -NH₂, -NH(C₁₋₅-Alkyl), -N(C₁₋₅-Alkyl)₂, -NO₂, -CHO, -CF₂H, -CFH₂, -C(=O)-NH₂, -C(=O)- NH(C₁₋₅-Alkyl), -C(=O)-N(C₁₋₅-Alkyl)₂, -S(=O)₂-C₁₋₅-Alkyl, - S(=O)₂-Phenyl, Phenyl, Phenoxy und Benzyl besteht;
R³ ein 5- bis 10-gliedriges Aryl oder Heteroarylradikal darstellt, welches unsubstituiert sein kann oder mit 1, 2, 3, 4 oder 5 Substituent(en) substituiert sein kann, die unabhängig aus der Gruppe ausgewählt sind, die aus C₁₋₅-Alkyl, -O-C₁₋₅-Alkyl, -S- C₁₋₅-Alkyl, -C(=O)-OH, -C(=O)-C₁₋₅-Alkyl, -C(=O)-O-C₁₋₅-Alkyl, -O-C(=O)-C₁₋₅-Alkyl, F, Cl, Br, I, -CN, -CF₃, -OCF₃, -SCF₃, -OH, -SH, -NH₂, -NH(C₁₋₅-Alkyl), -N(C₁₋₅-Alkyl)₂, -NO₂, -CHO, -CF₂H, -CFH₂, -C(=O)-NH₂, -C(=O)-NH(C₁₋₅-Alkyl), -C(=O)-N(C₁₋₅- Alkyl)₂, -S(=O)₂-C₁₋₅-Alkyl, -S(=O)₂-Phenyl, Phenyl, Phenoxy und Benzyl besteht und welches über eine lineare oder verzweigte C₁₋₆ Alkylen-, C₂₋₆ Alkenylen- oder C₂₋₆ Alkinylengruppe, welche unsubstituiert oder mit 1, 2 oder 3 Substituent(en) substituiert sein kann, die unabhängig aus der Gruppe ausgewählt sind, die aus F, Cl, Br, I, -CN, -CF₃, -OCF₃, -SCF₃, -OH, -SH, -NH₂, -O-C₁₋₅-Alkyl, -S-C₁₋₅-Alkyl, -NH-(C₁₋₅- Alkyl) und -N(C₁₋₅-Alkyl)₂ besteht, gebunden sein kann und wobei das Heteroarylradikal 1, 2 oder 3 Heteroatom(e) enthält, die unabhängig aus der Gruppe ausgewählt sind, die aus Stickstoff, Sauerstoff und Schwefel besteht, als Ringelement(e);
R⁴, R⁵ und R⁶ jeweils ein Wasserstoffatom darstellen;
R⁷ und R⁸, identisch oder verschieden, jeweils ein Wasserstoffatom oder ein lineares oder verzweigtes, gesättigtes oder ungesättigtes C₁₋₁₀ aliphatisches Radikal darstellen, welches unsubstituiert oder mit 1, 2 oder 3 Substituent(en) substituiert sein kann, die unabhängig aus der Gruppe ausgewählt sind, die aus F, Cl, Br, I, -CN, -CF₃, -OCF₃, -SCF₃, -OH, -SH, -NH₂, -O-C₁₋₅-Alkyl, -S-C₁₋₅-Alkyl, -NH-(C₁₋₅-Alkyl) und -N(C₁₋₅-Alkyl)₂ besteht;
R⁹ und R¹⁰ identisch oder verschieden, jeweils ein Wasserstoffatom oder ein lineares oder verzweigtes, gesättigtes oder ungesättigtes C₁₋₁₀ aliphatisches Radikal darstellen, welches unsubstituiert oder mit 1, 2 oder 3 Substituent(en) substituiert sein kann, die unabhängig aus der Gruppe ausgewählt sind, die aus F, Cl, Br, I, -CN, -CF₃, -OCF₃, -SCF₃, -OH, -SH, -NH₂, -O-C₁₋₅-Alkyl, -S-C₁₋₅-Alkyl, -NH-(C₁₋₅-Alkyl) und -N(C₁₋₅-Alkyl)₂ besteht;
R¹¹ ein lineares oder verzweigtes, gesättigtes oder ungesättigtes, unsubstituiertes C₁₋₁₀ aliphatisches Radikal oder ein Aryl oder Heteroarylradikal darstellt, das aus der Gruppe ausgewählt ist, die aus Phenyl, Naphtyl, Furyl (Furanyl), Thienyl (Thiophenyl), Pyrrolyl, Oxazolyl, Isoxazolyl, Isothiazolyl, Imidazolyl, Pyrazolyl, Oxadiazolyl, Thiadiazolyl, Triazolyl, Pyridazinyl, Pyrimidinyl, Pyridinyl, Pyrazinyl, Quinolinyl, Isoquinolinyl, Benzo[b]furanyl, Benzo[b]thiophenyl, Benzothiadiazolyl und Imidazo[2,1-b]thiazolyl besteht, welches unsubstituiert sein kann oder mit 1, 2, 3, 4 oder 5 Substituent(en) substituiert sein kann, die unabhängig aus der Gruppe ausgewählt sind, die aus C₁₋₅-Alkyl, -O-C₁₋₅-Alkyl, -S-C₁₋₅-Alkyl, -C(=O)-OH, -C(=O)- C₁₋₅-Alkyl, -C(=O)-O-C₁₋₅-Alkyl, -O-C(=O)-C₁₋₅-Alkyl, F, Cl, Br, I, -CN, -CF₃, -OCF₃, -SCF₃, -OH, -SH, -NH₂, -NH(C₁₋₅-Alkyl), - N(C₁₋₅-Alkyl)₂, -NO₂, -CHO, -CF₂H, -CFH₂, -C(=O)-NH₂, -C(=O)- NH(C₁₋₅-Alkyl), -C(=O)-N(C₁₋₅-Alkyl)₂, -S(=O)₂-C₁₋₅-Alkyl, - S(=O)₂-Phenyl, Phenyl, Phenoxy und Benzyl besteht;
und
R¹² stellt dar, ein 5- bis 10-gliedriges Arylradikal, welches mit 1, 2, 3, 4 oder 5 Substituent(en) substituiert sein kann, die unabhängig aus der Gruppe ausgewählt sind, die aus C₁₋₅-Alkyl, -O-C₁₋₅-Alkyl, -S-C₁₋₅-Alkyl, -C(=O)-OH, -C(=O)-C₁₋₅-Alkyl, - C(=O)-O-C₁₋₅-Alkyl, -O-C(=O)-C₁₋₅-Alkyl, F, Br, I, -CN, -CF₃, - OCF₃, -SCF₃, -SH, -NH₂, -NH(C₁₋₅-Alkyl), -N(C₁₋₅-Alkyl)₂, -CHO, -CF₂H, -CFH₂, -C(=O)-NH₂, -C(=O)-NH(C₁₋₅-Alkyl), -C(=O)- N(C₁₋₅-Alkyl)₂, -S(=O)₂-C₁₋₅-Alkyl, -S(=O)₂-Phenyl, Phenyl, Phenoxy und Benzyl besteht;
ein Heteroarylradikal, das aus der Gruppe ausgewählt ist, die aus Furyl (Furanyl), Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridinyl, Pyridazinyl, Pyrimidinyl, Pyrazinyl, Quinolinyl, Isoquinolinyl, Benzo[b]furanyl, Benzo[b]thiophenyl, Benzothadiazolyl und Imidazo[2,1-b]thiazolyl besteht, welches unsubstituiert sein kann oder mit 1, 2, 3, 4 oder 5 Substituent(en) substituiert sein kann, die unabhängig aus der Gruppe ausgewählt sind, die aus C₁₋₅-Alkyl, -O-C₁₋₅-Alkyl, -S-C₁₋₅-Alkyl, -C(=O)-OH, -C(=O)- C₁₋₅-Alkyl, -C(=O)-O-C₁₋₅-Alkyl, -O-C(=O)-C₁₋₅-Alkyl, F, Cl, Br, I, -CN, -CF₃, -OCF₃, -SCF₃, -OH, -SH, -NH₂, -NH(C₁₋₅-Alkyl), - N(C₁₋₅-Alkyl)₂, -NO₂, -CHO, -CF₂H, -CFH₂, -C(=O)-NH₂, -C(=O)- NH(C₁₋₅-Alkyl), -C(=O)-N(C₁₋₅-Alkyl)₂, -S(=O)₂-C₁₋₅-Alkyl, - S(=O)₂-Phenyl, Phenyl, Phenoxy und Benzyl besteht;
oder
ein Aryl oder Heteroarylradikal, das aus der Gruppe ausgewählt ist, die aus Phenyl, Naphtyl, Furyl (Furanyl), Thienyl (Thiophenyl), Pyrrolyl, Oxazolyl, Isoxazolyl, Thiazolyl, Isothiazolyl, Pyrazolyl, Oxadiazolyl, Thiadiazolyl, Triazolyl, Pyridinyl, Pyridazinyl, Pyrimidinyl, Pyrazinyl, Quinolinyl, Isoquinolinyl, Benzo[b]furanyl, Benzo[b]thiophenyl, Benzothiadiazolyl und Imidazo[2,1-b]thiazolyl besteht, welche unsubstituiert sein kann oder mit 1, 2, 3, 4 oder 5 Substituent(en) substituiert sein kann, die unabhängig aus der Gruppe ausgewählt sind, die aus C₁₋₅-Alkyl, -O-C₁₋₅-Alkyl, -S- C₁₋₅-Alkyl, -C(=O)-OH, -C(=O)-C₁₋₅-Alkyl, -C(=O)-O-C₁₋₅-Alkyl, -O-C(=O)-C₁₋₅-Alkyl, F, Cl, Br, I, -CN, -CF₃, -OCF₃, -SCF₃, -OH, -SH, -NH₂, -NH(C₁₋₅-Alkyl), -N(C₁₋₅-Alkyl)₂, -NO₂, -CHO, -CF₂H, -CFH₂, -C(=O)-NH₂, -C(=O)-NH(C₁₋₅-Alkyl), -C(=O)-N(C₁₋₅- Alkyl)₂, -S(=O)₂-C₁₋₅-Alkyl, -S(=O)₂-Phenyl, Phenyl, Phenoxy und Benzyl besteht und welche über eine -(CH₂)_{1, 2 oder 3}-Gruppe gebunden ist;
optional in Form von einem seiner Stereoisomere, bevorzugt Enantiomere oder Diasteromere, eines Racemats oder in Form einer Mischung von zumindest zwei seiner Stereoisomeren, bevorzugt Enantiomeren und/oder Diasteromeren, in irgendeinem Mischungsverhältnis, oder eines physiologischen akzeptablen Salzes davon, oder eines korrespondierenden Solvats davon.

3. Verbindung nach Anspruch 1 oder 2, **dadurch gekennzeichnet, dass** R¹ stellt dar,
ein (hetero)cycloaliphatisches Radikal, das aus der Gruppe ausgewählt ist, die aus Cyclopropyl, Cyclobutyl, Cyclopentyl, Cyclopentenyl, Cyclohexyl, Cyclohexenyl, Cycloheptyl, Cycloheptenyl, Cyclooctyl, Cyclooctenyl, Cyclononyl, Imidazolidinyl, Aziridinyl, Azetidinyl, Pyrrolidinyl, Piperidinyl, Morpholinyl, Thiomorpholinyl, Piperazinyl, Pyrazolidinyl, Tetrahydrothiophenyl und Azepanyl besteht, wobei das (hetero)cycloaliphatische Radikal über eine -(CH₂)₁,_{2 oder 3}-Gruppe gebunden sein kann und/oder mit 1, 2 oder 3 Substituent(en) substituiert sein kann, die unabhängig aus der Gruppe ausgewählt sind, die aus Oxo (=O), Thioxo (=S), Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃,-OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, - C(=O)-NH₂, -C(=O)-NH-CH₃, -C(=O)-NH-C₂H₅, -C(=O)-N(CH₃)₂, -C(=O)-N(C₂H₅)₂ und -S(=O)₂-CH₃ besteht,
ein Heteroarylradikal, das aus der Gruppe ausgewählt ist, die aus Furyl (Furanyl), Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridinyl, Pyridazinyl, Pyrimidinyl, Pyrazinyl, Quinolinyl, Isoquinolinyl, Benzo[b]furanyl, Benzo[b]thiophenyl, Benzothiadiazolyl und Imidazo[2,1-b]thiazolyl besteht, wobei das Heteroarylradikal mit 1, 2 oder 3 Subtituent(en) substituiert sein kann, die unabhängig aus der Gruppe ausgewählt sind, die aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂-H₅, - C(=O)-CH₂-CH₂-CH₃, -C(=O)-CH(CH₃)₂, -C(=O)-C(CH₃)₃, F, Cl, Br, I, -CN,-CF₃, -OCF₃, -SCF₃, -OH, -SH, -NH₂, -NH-CH₃, -NH-C₂H₅, -NH-CH₂-CH₂-CH₃, - NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ und -S(=O)₂-CH₃ besteht,
ein Aryl oder Heteroarylradikal, das aus der Gruppe ausgewählt ist, die aus Phenyl, Naphthyl, Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridazinyl, Pyrimidinyl, Pyrazinyl, Quinolinyl, Isoquinolinyl, Benzo[b]furanyl, Benzo[b]thiophenyl, Benzothiadiazolyl und Imidazo[2,1-b]thiazolyl besteht, wobei das Aryl oder Heteroarylradikal über eine -(CH₂)-Gruppe gebunden ist und/oder mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - NO₂, -CHO, -CF₂H, -CFH₂, -C(=O)-NH₂, -C(=O)-NH-CH₃, -C(=O)-NH-C₂H₅, - C(=O)-N(CH₃)₂, -C(=O)-N(C₂H₅)₂ und -S(=O)₂-CH₃ besteht,
ein Aryl oder Heteroarylradikal, das aus der Gruppe ausgewählt ist, die aus Naphthyl, Furyl (Furanyl), Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridinyl, Pyridazinyl, Pyrimidinyl, Pyrazinyl, Quinolinyl, Isoquinolinyl, Benzo[b]furanyl, Benzo[b]thiophenyl, Benzothiadiazolyl und Imidazo[2,1-b]thiazolyl besteht, wobei das Aryl oder Heteroarylradikal über eine -(CH₂)₂-Gruppe gebunden ist und/oder mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, - S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN,-CF₃, -OCF₃, -SCF₃, -OH, -SH, -NH₂, -NH-CH₃, -NH-C₂H₅, -NH-CH₂-CH₂-CH₃, - NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ und -S(=O)₂-CH₃ besteht,
ein Aryl oder Heteroarylradikal, das aus der Gruppe ausgewählt ist, die aus Phenyl, Naphthyl, Furyl (Furanyl), Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridinyl, Pyridazinyl, Pyrimidinyl, Pyrazinyl, Quinolinyl, Isoquinolinyl, Benzo[b]furanyl, Benzo[b]thiophenyl, Benzothiadiazolyl und Imidazo[2,1-b]thiazolyl besteht, wobei das Aryl oder Heteroarylradikal über eine -(CH₂)₃-Gruppe gebunden ist und/oder mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, - S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN,-CF₃, -OCF₃, -SCF₃, -OH, -SH, -NH₂, -NH-CH₃, -NH-C₂H₅, -NH-CH₂-CH₂-CH₃, - NH-CH(CH₃)₂, -NH-C(CH₃)₃, -N(CH₃)₂, -N(C₂H₅)₂, -NO₂, -CHO, -CF₂H, -CFH₂, -C(=O)-NH2, -C(=O)-NH-CH₃, -C(=O)-NH-C₂H₅, -C(=O)-N(CH₃)₂, -C(=O)-N(C₂H₅)₂ und -S(=O)₂-CH₃ besteht;
oder
eine -C(=O)-R¹² funktionelle Gruppe;
bevorzugt stellt R¹ dar,
ein unsubstituiertes Pyrrolylradikal;
ein Aryl oder Heteroarylradikal, das aus der Gruppe ausgewählt ist, die aus Phenyl und Pyrrolyl besteht, wobei das Phenylradikal über eine -(CH₂)-Gruppe gebunden ist und das Pyrrolylradikal über eine -(CH₂)₂-Gruppe gebunden ist und/oder das Phenyl- oder Pyrrolylradikal mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Br, I, -CN, -CF₃, -OCF₃, - SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, - C(=O)-NH₂, -C(=O)-NH-CH₃, -C(=O)-NH-C₂H₅, -C(=O)-N(CH₃)₂, -C(=O)-N(C₂H₅)₂ und -S(=O)₂-CH₃ besteht; oder
eine -C(=O)-R¹² funktionelle Gruppe;
mehr bevorzugt stellt R¹ dar, ein unsubstituiertes Pyrrolylradikal;
ein Aryl oder Heteroarylradikal, das aus der Gruppe ausgewählt ist, die aus Phenyl und Pyrrolyl besteht, wobei das Phenylradikal über eine -(CH₂)-Gruppe gebunden ist, und das Pyrrolylradikal über eine -(CH₂)₂-Gruppe gebunden ist und/oder das Phenyl oder Pyrrolylradikal mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, sec-Butyl, Isobutyl, n-Pentyl, F, Br, und I besteht oder
eine -C(=O)-R¹² funktionelle Gruppe.

4. Verbindung nach einem oder mehreren der Ansprüche 1 bis 3, **dadurch gekennzeichnet, dass**
R² ein Wasserstoffatom oder ein lineares oder verzweigtes, unsubstituiertes C₁₋₁₀ Alkylradikal darstellt;
bevorzugt R² ein Wasserstoffatom oder ein Alkylradikal darstellt, das aus der Gruppe ausgewählt ist, die aus Methyl, Ethyl, n-Propyl, Isopropyl, n-Butyl, sec-Butyl, Isobutyl und tert-Butyl besteht;
mehr bevorzugt stellt R² ein Wasserstoffatom oder ein Methylradikal dar.

5. Verbindung nach einem oder mehreren der Ansprüche 1 bis 4, **dadurch gekennzeichnet, dass** R¹ und R² zusammen mit dem brückenbildenden Stickstoffatom eine funktionelle Gruppe bilden, die aus der Gruppe ausgewählt ist, die aus 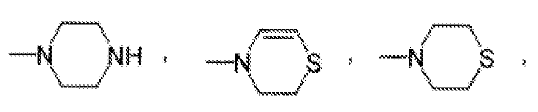 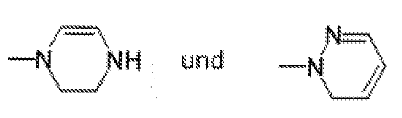 besteht, wobei jede von diesen vorher genannten zyklischen funktionellen Gruppen in irgendeiner Position einschließlich der -NH-Gruppen mit 1, 2 oder 3 Substituent(en) substituiert sein kann, die unabhängig aus der Gruppe ausgewählt sind, die aus Oxo (=O), Thioxo (=S), Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃,-NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-Phenyl, Phenyl, Phenoxy und Benzyl besteht; bevorzugt bilden R¹ und R² zusammen mit dem brückenbildenden Stickstoffatom eine Pyridazin-3-on funktionelle Gruppe, welche mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, -O-CH₃, -O-C₂H₅, -C(=O)-OH, -C(=O)-O-CH₃, -C(=O)-O-C₂H₅, - C(=O)-CH₃, -C(=O)-C₂H₅, F, Cl, Br, I, -CN, -CF₃, -OCF₃, -SCF₃, -OH, -SH,-NH₂, -NH-CH₃, -NH-C₂H₅, -NO₂, -CHO, -CF₂H, -CFH₂, -C(=O)-NH₂, Phenyl, Phenoxy und Benzyl besteht;
mehr bevorzugt bilden R¹ und R² zusammen mit dem brückenbildenden Stickstoffatom eine unsubstituiertes Pyridazin-3-on funktionelle Gruppe.

6. Verbindung nach einem oder mehreren der Ansprüche 1 bis 5, **dadurch gekennzeichnet, dass**
R³ ein Aryl oder Heteroarylradikal darstellt, das aus der Gruppe ausgewählt ist, die aus Phenyl, Naphthyl, Furyl (Furanyl), Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridinyl, Pyridazinyl, Pyrimidinyl, Pyrazinyl, Quinolinyl, Isoquinolinyl, Benzo[b]furanyl, Benzo[b]thiophenyl, Benzothiadiazolyl und Imidazo[2,1-b]thiazolyl besteht, wobei das Aryl oder Heteroarylradikal über eine -(CH₂)₁, _{2 oder 3}-Gruppe gebunden sein kann und/oder mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH,-NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ und -S(=O)₂-CH₃ besteht;
bevorzugt stellt R³ ein Phenylradikal dar, welches mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN,-CF₃, -OCF₃, -SCF₃, -OH, -SH, -NH₂, -NH-CH₃, -NH-C₂H₅, -NH-CH₂-CH₂-CH₃, - NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ und -S(=O)₂-CH₃ besteht;
mehr bevorzugt stellt R³ ein unsubstituiertes Phenylradikal dar.

7. Verbindung nach einer oder mehreren der Ansprüche 1 bis 6, **dadurch gekennzeichnet, dass**
R⁷ und R⁸, identisch oder verschieden, jeweils ein Wasserstoffatom oder ein lineares oder verzweigtes, unsubstituiertes C₁₋₁₀ Aklylradikal darstellen;
bevorzugt R⁷ und R⁸, identisch oder verschieden, jeweils ein Wasserstoffatom oder ein Alkylradikal darstellen, das aus der Gruppe ausgewählt ist, die aus Methyl, Ethyl, n-Propyl, Isopropyl, n-Butyl, sec-Butyl, Isobutyl und tert-Butyl besteht;
mehr bevorzugt stellt R⁷ und R⁸ jeweils ein Wasserstoffatom dar.

8. Verbindung nach einem oder mehreren der Ansprüche 1 bis 7, **dadurch gekennzeichnet, dass** R⁹ und R¹⁰, identisch oder verschieden, jeweils ein Wasserstoffatom oder ein lineares oder verzweigtes, unsubstituiertes C₁₋₁₀ Alkylradikal darstellen;
bevorzugt R⁹ und R¹⁰, identisch oder verschieden, jeweils ein Wasserstoffatom oder ein Alkylradikal darstellen, das aus der Gruppe ausgewählt ist, die aus Methyl, Ethyl, n-Propyl, Isopropyl, n-Butyl, sec-Butyl, Isobutyl und tert-Butyl besteht;
mehr bevorzugt stellt R⁹ und R¹⁰ jeweils ein Wasserstoffatom dar.

9. Verbindung nach einem oder mehreren der Ansprüche 1 bis 8, **dadurch gekennzeichnet, dass**
R¹¹ stellt dar, ein Alkylradikal, das aus der Gruppe ausgewählt ist, die aus Methyl, Ethyl, n-Propyl, Isopropyl, n-Butyl, sec-Butyl, Isobutyl und tert-Butyl besteht; oder ein Aryl oder Heteroarylradikal, das aus der Gruppe ausgewählt ist, die aus Phenyl Naphthyl, Furyl (Furanyl), Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridazinyl, Pyrimidinyl, Pyridinyl, Pyrazinyl, Quinolinyl, Isoquinolinyl, Benzo[b]furanyl, Benzo[b]thiophenyl, Benzothiadiazolyl und Imidazo[2,1-b]thiazolyl besteht, wobei das Aryl oder Heteroarylradikal mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃,-OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, - C(=O)-NH₂, -C(=O)-NH-CH₃, -C(=O)-NH-C₂H₅, -C(=O)-N(CH₃)₂, -C(=O)-N(C₂H₅)₂ und -S(=O)₂-CH₃ besteht;
bevorzugt R¹¹ stellt dar, ein Alkylradikal, das aus der Gruppe ausgewählt ist, die aus Methyl, Ethyl, n-Propyl, Isopropyl und n-Butyl besteht; oder ein Aryl oder Heteroarylradikal, das aus der Gruppe ausgewählt ist, die aus Phenyl und Pyridinyl besteht, wobei das Aryl oder Heteroarylradikal mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, -O-CH₃, -O-C₂H₅, -C(=O)-OH, -C(=O)-O-CH₃, -C(=O)-O-C₂H₅, F, Cl, Br, I, -CN, -CF₃, -OCF₃, -SCF₃, -OH, -SH, -NH₂, -NH-CH₃, -NH-C₂H₅, -NH-CH₂-CH₂CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ und -S(=O)₂-CH₃ besteht;
mehr bevorzugt stellt R¹¹ ein Alkylradikal dar, das aus der Gruppe ausgewählt ist, die aus Methyl, Ethyl, n-Propyl, und n-Butyl besteht; oder ein unsubstituiertes Phenyl oder Pyridinylradikal.

10. Verbindung nach einem oder mehreren der Ansprüche 1 bis 9, **dadurch gekennzeichnet, dass**
R¹² stellt dar, ein 5 bis 10 gliedriges Arylradikal, welches mit 1, 2 oder 3 Substituent(en) substituiert sein kann, das unabhängig aus der Gruppe ausgewählt ist, die aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ und -S(=O)₂-CH₃ besteht;
ein Heteroarylradikal, das aus der Gruppe ausgewählt ist, die aus Furyl (Furanyl), Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridinyl, Pyridazinyl, Pyrimidinyl, Pyrazinyl, Quinolinyl, Isoquinolinyl, Benzo[b]furanyl, Benzo[b]thiophenyl, Benzothiadiazolyl und Imidazo[2,1-b]thiazolyl besteht, wobei das Heteroarylradikal mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN,-CF₃, -OCF₃, -SCF₃, -OH, -SH, -NH₂, -NH-CH₃, -NH-C₂H₅, -NH-CH₂-CH₂-CH₃, - NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ und -S(=O)₂-CH₃ besteht;
oder
ein Aryl oder Heteroarylradikal, das aus der Gruppe ausgewählt ist, die aus Phenyl, Naphthyl, Furyl (Furanyl), Thienyl (Thiophenyl), Pyrrolyl, Oxazolyl, Isoxazolyl, Thiazolyl, Isothiazolyl, Pyrazolyl, Oxadiazolyl, Thiadiazolyl, Triazolyl, Pyridinyl, Pyridazinyl, Pyrimidinyl, Pyrazinyl, Quinolinyl, Isoquinolinyl, Benzo[b]furanyl, Benzo[b]thiophenyl, Benzothiadiazolyl und Imidazo[2,1-b]thiazolyl besteht, wobei das Aryl oder Heteroarylradikal über eine -(CH₂)_{1, 2 oder 3}-Gruppe gebunden ist und/oder mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN,-CF₃, -OCF₃, -SCF₃, -OH, -SH, -NH₂, -NH-CH₃, -NH-C₂H₅, -NH-CH₂-CH₂-CH₃, - NH-CH(CH₃)₂, -NH-C(CH₃)₃, -N(CH₃)₂, -N(C₂H₅)₂, -NO₂, -CHO, -CF₂H, -CFH₂, -C(=O)-NH2, -C(=O)-NH-CH₃, -C(=O)-NH-C₂H₅, -C(=O)-N(CH₃)₂, -C(=O)-N(C₂H₅)₂ und -S(=O)₂-CH₃ besteht;
bevorzugt stellt R¹² ein Phenylradikal dar, welches mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ und -S(=O)₂-CH₃ besteht;
mehr bevorzugt stellt R¹² ein unsubstituiertes Phenylradikal dar.

11. Verbindung nach einem oder mehreren der Ansprüche 1 bis 10, **dadurch gekennzeichnet, dass**
X eine -NR¹R² funktionelle Gruppe oder eine -OR³ funktionelle Gruppe darstellt;
R¹ stellt dar, ein unsubstituiertes Pyrrolylradikal; ein Aryl oder Heteroarylradikal, das aus der Gruppe ausgewählt ist, die aus Phenyl und Pyrrolyl besteht, wobei das Phenylradikal über eine -(CH₂) -Gruppe gebunden ist und das Pyrrolylradikal über eine -(CH₂)₂-Gruppe gebunden ist und/oder das Phenyl oder Pyrrolylradikal mit 1, 2 oder 3 Substituent(en) substituiert sein kann, die unabhängig von der Gruppe ausgewählt sind, die aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, - C(=O)-O-CH₃, -C(=O)-O-C₂-H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Br, I, -CN, -CF₃, -OCF₃, -SCF₃, - OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, - C(=O)-NH-CH₃, -C(=O)-NH-C₂H₅, -C(=O)-N(CH₃)₂, -C(=O)-N(C₂H₅)₂ und - S(=O)₂-CH₃ besteht oder eine -C(=O)-R¹² funktionelle Gruppe;
R² ein Wasserstoff oder ein Alklylradikal darstellt, das aus der Gruppe ausgewählt ist, die aus Methyl, Ethyl, n-Propyl, Isopropyl, n-Butyl, sec-Butyl, Isobutyl und tert-Butyl besteht;
oder
R¹ und R² bilden zusammen mit dem brückenbildenden Stickstoffatom eine Pyridazin-3-on funktionelle Gruppe, welche mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, -O-CH₃, -O-C₂H₅, -C(=O)-OH, -C(=O)-O-CH₃, -C(=O)-O-C₂H₅, C(=O)-CH₃, -C(=O)-C₂H₅, F, Cl, Br, I, -CN, -CF₃, -OCF₃, -SCF₃, -OH, -SH, -NH₂, -NH-CH₃, -NH-C₂H₅, -NO₂, - CHO, -CF₂H, -CFH₂, -C(=O)-NH₂, Phenyl, Phenoxy und Benzyl besteht;
R³ ein Phenylradikal darstellt, welches mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃,-C(=O)-OH, -C(=O)-O-CH₃, -C(=O)-O-C₂-H₅, -C(=O)-O-CH₂-CH₂-CH₃, - C(=O)-O-CH(CH₃)_{z}, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃,-OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, - C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃ und -S(=O)₂-CH₃ besteht;
R⁴, R⁵ und R⁶ jeweils ein Wasserstoffatom darstellen;
R⁷ und R⁸, identisch oder verschieden, jeweils ein Wasserstoffatom oder ein Alkylradikal darstellen, das aus der Gruppe ausgewählt ist, die aus Methyl, Ethyl, n-Propyl, Isopropyl, n-Butyl, sec-Butyl, Isobutyl und tert-Butyl besteht;
R⁹ und R¹⁰, identisch oder verschieden, jeweils ein Wasserstoffatom oder ein Alkylradikal darstellen, das aus der Gruppe ausgewählt ist, die aus Methyl, Ethyl, n-Propyl, Isopropyl, n-Butyl, sec-Butyl, Isobutyl und tert-Butyl besteht;
R¹¹ stellt dar, ein Alkylradikal, das aus der Gruppe ausgewählt ist, die aus Methyl, Ethyl, n-Propyl, Isopropyl und n-Butyl besteht; oder ein Aryl oder Heteroarylradikal, das aus der Gruppe ausgewählt ist, die aus Phenyl und Pyridinyl besteht, wobei das Aryl oder Heteroaryl mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, -O-CH₃, -O-C₂H₅, -C(=O)-OH, -C(=O)-O-CH₃, -C(=O)-O-C₂H₅, F, Cl, Br, I, -CN, -CF₃, -OCF₃, -SCF₃, -OH, -SH, -NH₂, -NH-CH₃, -NH-C₂H₅, -NH-CH₂-CH₂CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ und -S(=O)₂-CH₃ besteht;
und
R¹² ein Phenylradikal darstellt, das mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, - NH-C(CH₃)₃, -N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ und -S(=O)₂-CH₃ besteht;

12. Verbindung nach einem oder mehreren der Ansprüche 1 bis 11, **dadurch gekennzeichnet, dass**
X eine-NR¹R² funktionelle Gruppe oder eine -OR³ funktionelle Gruppe darstellt;
R¹ stellt dar, ein unsubstituiertes Pyrrolylradikal; ein Aryl oder Heteroarylradikal, das aus der Gruppe ausgewählt ist, die aus Phenyl und Pyrrolyl besteht, wobei das Phenylradikal über eine -(CH₂)-Gruppe gebunden ist und das Pyrrolylradikal über eine -(CH₂)₂-Gruppe gebunden ist und/oder das Phenyl oder Pyrrolylradikal mit 1, 2 oder 3 Subtituent(en) substituiert sein kann, die unabhängig aus der Gruppe ausgewählt sind, die aus Methyl, Ethyl, n-Propyl, Isopropyl, n-Butyl, tert-Butyl, sec-Butyl, Isobutyl, n-Pentyl, F, Br und I besteht oder eine -C(=O)-R¹² funktionelle Gruppe;
R² ein Wasserstoffatom oder ein Methylradikal darstellt;
oder
R¹ und R² bilden zusammen mit dem brückenbildenden Stickstoffatom eine unsubstituierte Pyridazin-3-on funktionelle Gruppe;
R³ ein unsubstituiertes Phenylradikal darstellt;
R⁴, R⁵ und R⁶ jeweils ein Wasserstoffatom darstellen;
R⁷ und R⁸ jeweils ein Wasserstoffatom darstellen;
R⁹ und R¹⁰ jeweils ein Wasserstoffatom darstellen;
R¹¹ stellt dar, ein Alkylradikal, das aus der Gruppe ausgewählt ist, die aus Methyl, Ethyl, n-Propyl und n-Butyl besteht; oder ein unsubstituiertes Phenyl oder Pyridinylradikal
und
R¹² ein unsubstituiertes Phenylradikal darstellt.

13. Verbindung nach einem oder mehreren der Ansprüche 1 bis 12, die aus der Gruppe ausgewählt ist, die aus
[1] [5-(4-Methyl-Piperazin-1-yl)-2-Nitro-Phenyl]-(2-Pyrrol-1-yl-Ethyl)-Amin,
[2] (4-Fluoro-Benzyl)-[5-(4-Methyl-Piperazin-1-yl)-2-Nitro-Phenyl]-Amin,
[3] N-[5-(4-Methyl-Piperazin-1-yl)-2-Nitro-Phenyl]-Benzamid,
[4] N-Methyl-N-[5-(4-Methyl-Piperazin-1-yl)-2-Nitro-Phenyl]-Benzamid,
[5] [5-(4-Methyl-Piperazin-1-yl)-2-Nitro-Phenyl]-Pyrrol-1-yl-Amin,
[6] 2-[5-(4-Methyl-Piperazin-1-yl)-2-Nitro-Phenyl]-2H-Pyridazin-3-On,
[7] 1-Methyl-4-(4-Nitro-3-Phenoxy-Phenyl)-Piperazin,
[8] Benzyl-[5-(4-Butyl-Piperazin-1-yl)-2-Nitro-Phenyl]-Amin,
[9] Benzyl-[2-Nitro-5-(4-Pyridin-2-yl-Piperazin-1-yl)-Phenyl]-Amin und
[10] Benzyl-[2-Nitro-5-(4-Phenyl-2-yl-Piperazin-1-yl)-Phenyl]-Amin;
optional in Form eines physiologisch akzeptablen Salzes davon oder eines korrespondierenden Solvats davon.

14. Verfahren zur Herstellung einer Verbindung nach einem oder mehreren der Ansprüche 1 bis 13, **dadurch gekennzeichnet, dass** zumindest eine Nitrobenzolverbindung der allgemeinen Formel II, 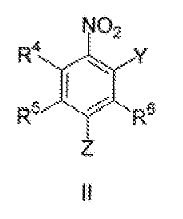 wobei R⁴ bis R⁶ die Bedeutung nach einem oder mehreren der Ansprüche 1 bis 13 haben, Y ein Chloratom darstellt, und Z ein Brom oder Jodatom darstellt; mit zumindest einer Verbindung der allgemeinen Formel III reagiert wird, 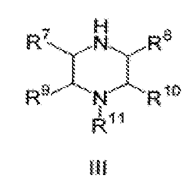 wobei R⁷ bis R¹¹ die Bedeutung nach einem oder mehreren der Ansprüche 1 bis 13 haben, in einem geeigneten Reaktionsmedium, bevorzugt in der Gegenwart von zumindest einem Katalysator und/oder zumindest einem Hilfsagens und/oder zumindest einer Base um eine Verbindung der allgemeinen Formel IV zu erhalten, 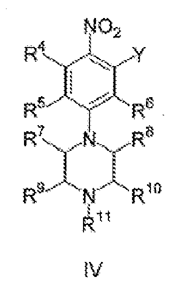 wobei R⁴ bis R¹¹ die Bedeutung nach einem oder mehreren der Ansprüche 1 bis 13 haben und Y ein Chloratom darstellt; welche optional gereinigt und/oder isoliert wird, und die Verbindung der allgemeinen Formel IV mit zumindest einer Verbindung der allgemeinen Formel V reagiert wird, 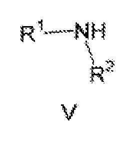 wobei R¹ und R² die Bedeutung nach einem oder mehreren der Ansprüche 1 bis 13 haben, in einem geeignetem Reaktionsmedium, bevorzugt in der Gegenwart von zumindest einem Katalysator und/oder zumindest einem Hilfsagents und/oder zumindest einer Base um eine Verbindung der allgemeinen Formel VI zu erhalten, 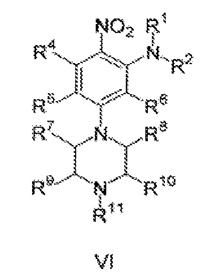 wobei R¹, R² und R⁴ bis R¹¹ die Bedeutung nach einem oder mehreren der Ansprüche 1 bis 13 haben, welche optional gereinigt und/oder isoliert wird.

15. Verfahren zur Herstellung einer Verbindung nach einem oder mehreren der Ansprüche 1 bis 13, **dadurch gekennzeichnet, dass** zumindest eine Nitrobenzolverbindung der allgemeinen Formel VII, 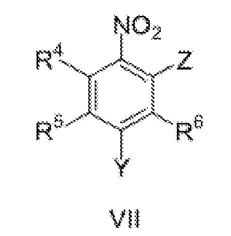 wobei R⁴ bis R⁶ die Bedeutung nach einem oder mehreren der Ansprüche 1 bis 13 haben, Z ein Brom- oder Jodatom darstellt und Y ein Chloratom darstellt, mit zumindest einer Verbindung der allgemeinen Formel V reagiert wird, 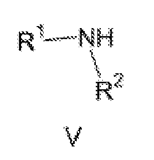 wobei R¹ und R² die Bedeutung nach einem oder mehreren der Ansprüche 1 bis 13 haben, in einem geeigneten Reaktionsmedium, bevorzugt in der Gegenwart von zumindest einem Katalysator und/oder zumindest einem Hilfsagens und/oder zumindest einer Base um eine Verbindung der allgemeinen Formel VIII zu erhalten, 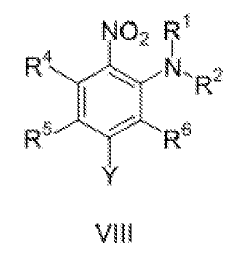 wobei R¹, R² und R⁴ bis R⁶ die Bedeutung nach einem oder mehreren der Ansprüche 1 bis 13 haben und Y ein Chloratom darstellt; welches optional gereinigt und/oder isoliert wird, und die Verbindung der allgemeinen Formel VIII mit zumindest einer Verbindung der allgemeinen Formel III reagiert wird, 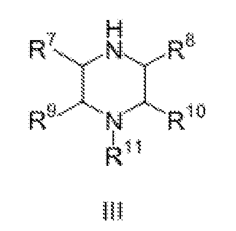 wobei R⁷ bis R¹¹ die Bedeutung nach einem oder mehreren der Ansprüche 1 bis 13 haben, in einem geeigneten Reaktionsmedium, bevorzugt in der Gegenwart von zumindest einem Katalysator und/oder zumindest einem Hilfsagens und/oder zumindest einer Base um eine Verbindung der allgemeinen Formel VI zu erhalten, 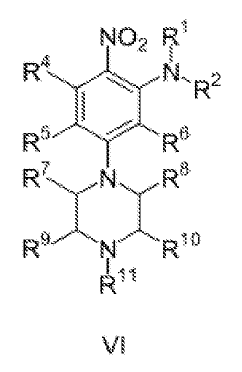 wobei R¹, R² und R⁴ bis R¹¹ die Bedeutung nach einem oder mehreren der Ansprüche 1 bis 13 haben, welche optional gereinigt und/oder isoliert wird.

16. Verfahren zur Herstellung einer Verbindung nach einem oder mehreren der Ansprüche 1 bis 13, **dadurch gekennzeichnet, dass** zumindest eine Nitrobenzolverbindung der allgemeinen Formel II, 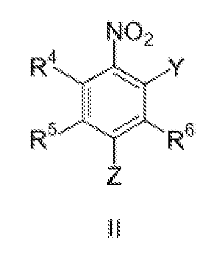 wobei R⁴ bis R⁶ die Bedeutung nach einem oder mehreren der Ansprüche 1 bis 13 haben, Y ein Chloratom darstellt und Z ein Brom- oder Jodatom darstellt; mit zumindest einer Verbindung der allgemeinen Formel III reagiert wird, 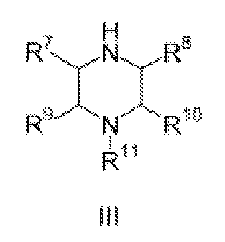 wobei R⁷ bis R¹¹ die Bedeutung nach einem oder mehreren der Ansprüche 1 bis 13 haben, in einem geeigneten Reaktionsmedium, bevorzugt in der Gegenwart von zumindest einem Katalysator und/oder zumindest einem Hilfsagens und/oder zumindest einer Base um eine Verbindung der allgemeinen Formel IV zu erhalten, 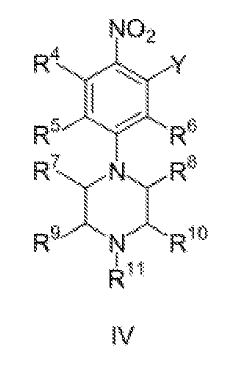 wobei R⁴ bis R¹¹ die Bedeutung nach einem oder mehreren der Ansprüche 1 bis 13 haben und Y ein Chloratom darstellt; welche optional gereinigt und/oder isoliert wird, und die Verbindung der allgemeinen Formel IV mit zumindest einer Verbindung der allgemeinen Formel IX reagiert wird, 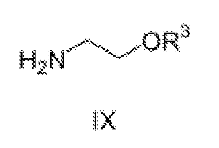 wobei R³ die Bedeutung nach einem oder mehreren der Ansprüche 1 bis 13 hat, in einem geeigneten Reaktionsmedium, bevorzugt in der Gegenwart von zumindest einem Katalysator und/oder zumindest einem Hilfsagens und/oder zumindest einer Base um eine Verbindung der allgemeinen Formel X zu erhalten, 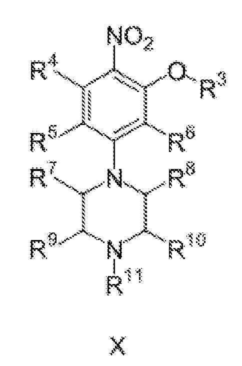 wobei R³ bis R¹¹ die Bedeutung nach einem oder mehreren der Ansprüche 1 bis 13 haben, welche optional gereinigt und/oder isoliert wird.

17. Medikament, das zumindest eine nitro-substituierte Phenyl-Piperazin Verbindung der allgemeinen Formel Ia umfasst, 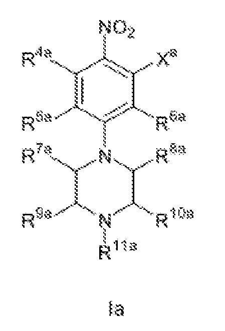 wobei
X^{a} eine -NR^{1a}R^{2a} funktionelle Gruppe oder eine -OR^{3a} funktionelle Gruppe darstellt;
R^{1a} stellt dar, ein lineares oder verzweigtes, gesättigtes oder ungesättigtes C₁₋₁₀ aliphatisches Radikal, welches mit 1, 2 oder 3 Substituent(en) substituiert ist, die unabhängig aus der Gruppe ausgewählt sind, die aus F, Cl, Br, I, -CN, -CF₃, -OCF₃, -SCF₃, -OH, - SH, -NH₂, -O-C₁₋₅-Alkyl, -S-C₁₋₅-Alkyl, -NH-(C₁₋₅-Alkyl) und -N(C₁₋₅- Alkyl)₂ besteht;
ein Radikal, das aus der Gruppe ausgewählt ist, die aus Adamantyl, Bicyclo[2.2.1]heptyl und Bicyclo[3.1.1]heptyl besteht, welches mit 1, 2 oder 3 Substituent(en) substituiert sein kann, die unabhängig aus der Gruppe ausgewählt sind, die aus Oxo (=O), Thioxo (=S), C₁₋₅- Alkyl, -O-C₁₋₅-Alkyl, -S-C₁₋₅-Alkyl, -C(=O)-OH, -C(=O)-C₁₋₅-Alkyl, - C(=O)-O-C₁₋₅-Alkyl, -O-C(=O)-C₁₋₅-Alkyl, F, Cl, Br, I, -CN, -CF₃, - OCF₃, -SCF₃, -OH, -SH, -NH₂, -NH(C₁₋₅-Alkyl), -N(C₁₋₅-Alkyl)₂, -NO₂, - CHO, -CF₂H, -CFH₂, -C(=O)-NH₂, -C(=O)-NH(C₁₋₅-Alkyl), -C(=O)- N(C₁₋₅-Alkyl)₂ und -S(=O)₂-C₁₋₅-Alkyl besteht und/oder welches über eine lineare oder verzweigte C₁₋₆ Alkylengruppe gebunden ist, welche 1, 2 oder 3 Heteroatom(e) enthalten kann, die unabhängig aus der Gruppe ausgewählt sind, die aus Stickstoff, Sauerstoff und Schwefel besteht, als Kettenelement(e);
ein gesättigtes oder ungesättigtes 3- bis 9-gliedriges cycloaliphatisches Radikal, welches mit 1, 2 oder 3 Substituent(en) substituiert sein kann, die unabhängig aus der Gruppe ausgewählt sind, die aus Oxo (=O), Thioxo (=S), C₁₋₅-Alkyl, -O-C₁₋₅-Alkyl, -S-C₁₋₅- Alkyl, -C(=O)-OH, -C(=O)-C₁₋₅-Alkyl, -C(=O)-O-C₁₋₅-Alkyl, -O-C(=O)- C₁₋₅-Alkyl, F, Cl, Br, I, -CN, -CF₃, -OCF₃, -SCF₃, -OH, -SH, -NH₂, - NH(C₁₋₅-Alkyl), -N(C₁₋₅-Alkyl)₂, -NO₂, -CHO, -CF₂H, -CFH₂, -C(=O)- NH₂, -C(=O)-NH(C₁₋₅-Alkyl), -C(=O)-N(C₁₋₅-Alkyl)₂, -S(=O)₂-C₁₋₅- Alkyl, -S(=O)₂-Phenyl, Phenyl, Phenoxy und Benzyl besteht und welches über eine lineare oder verzweigte C₁₋₆-Alkylen-, C₂₋₆- Alkenylen- oder C₂₋₆-Alkinylengruppe gebunden ist, welche unsubstituiert oder mit 1, 2 oder 3 Substituent(en) substituiert sein kann, die unabhängig aus der Gruppe ausgewählt sind, die aus F, Cl, Br, I, -CN, -CF₃, -OCF₃, -SCF₃, -OH, -SH, -NH₂, -O-C₁₋₅-Alkyl, -S-C₁₋₅- Alkyl, Phenyl, -NH-(C₁₋₅-Alkyl) und -N(C₁₋₅-Alkyl)₂ besteht und/oder welche 1, 2 oder 3 Heteroatom(e) enthalten kann, die unabhängig aus der Gruppe ausgewählt sind, die aus Stickstoff, Sauerstoff und Schwefel besteht, als Ringelement(e);
ein 5- bis 10-gliedriges Aryl oder Heteroarylradikal, welches mit 1, 2 oder 3 Substituent(en) substituiert sein kann, die unabhängig aus der Gruppe ausgewählt sind, die aus C₁₋₅-Alkyl, -O-C₁₋₅-Alkyl, -S-C₁₋₅- Alkyl, -C(=O)-OH, -C(=O)-C₁₋₅-Alkyl, -C(=O)-O-C₁₋₅-Alkyl, -O-C(=O)- C₁₋₅-Alkyl, F, Cl, Br, I, -CN, -CF₃, -OCF₃, -SCF₃, -OH, -SH, -NH₂, - NH(C₁₋₅-Alkyl), -N(C₁₋₅-Alkyl)₂, -NO₂, -CHO, -CF₂H, -CFH₂, -C(=O)- NH₂, -C(=O)-NH(C₁₋₅-Alkyl), -C(=O)-N(C₁₋₅-Alkyl)₂, -S(=O)₂-C₁₋₅- Alkyl, -S(=O)₂-Phenyl, Phenyl, Phenoxy und Benzyl besteht und wobei das Heteroarylradikal 1, 2 oder 3 Heteroatom(e) enthalten kann, die unabhängig aus der Gruppe ausgewählt sind, die aus Stickstoff, Sauerstoff und Schwefel besteht, als Ringelement(e);
ein 5- bis 10-gliedriges Arylradikal, wobei das Arylradikal mit 1, 2, 3, 4 oder 5 Substituent(en) substituiert sein kann, die unabhängig aus der Gruppe ausgewählt sind, die aus C₁₋₅-Alkyl, -O-C₁₋₅-Alkyl, -S-C₁₋₅- Alkyl, -C(=O)-O-C₁₋₅-Alkyl, -O-C(=O)-C₁₋₅-Alkyl, F, Cl, Br, I, -CN, - CF₃, -OCF₃, -SCF₃, -OH, -SH, -NH₂, -NH(C₁₋₅-Alkyl), -N(C₁₋₅-Alkyl)₂, - NO₂, -CHO, -CF₂H, -CFH₂, -C(=O)-NH₂, -C(=O)-NH(C₁₋₅-Alkyl), - C(=O)-N(C₁₋₅-Alkyl)₂, -S(=O)₂-C₁₋₅-Alkyl, -S(=O)₂-Phenyl, Phenyl, Phenoxy und Benzyl besteht und das Arylradikal über eine lineare oder verzweigte C₁₋₆-Alkylen-, C₂₋₆-Alkenylen- oder C₂₋₆- Alkinylengruppe gebunden ist, welche unsubstituiert oder mit 1, 2 oder 3 Substituent(en) substituiert sein kann, die unabhängig aus der Gruppe ausgewählt sind, die aus F, Cl, Br, I, -CN, -CF₃, -OCF₃, -SCF₃, -OH, -SH, -NH₂, -O-C₁₋₅-Alkyl, -S-C₁₋₅-Alkyl, Phenyl, -NH-(C₁₋₅-Alkyl) und -N(C₁₋₅-Alkyl)₂ besteht und welche 1, 2 oder 3 Heteroatom(e) enthalten kann, die unabhängig aus der Gruppe ausgewählt sind, die aus Stickstoff, Sauerstoff und Schwefel besteht, als Kettenelement(e);
ein 5- bis 10-gliedriges Heteroarylradikal, welches substituiert mit 1, 2 oder 3 Substituent(en) sein kann, die unabhängig aus der Gruppe ausgewählt sind, die aus C₁₋₅-Alkyl, -O-C₁₋₅-Alkyl, -S-C₁₋₅-Alkyl, - C(=O)-OH, -C(=O)-C₁₋₅-Alkyl, -C(=O)-O-C₁₋₅-Alkyl, -O-C(=O)-C₁₋₅- Alkyl, F, Cl, Br, I, -CN, -CF₃, -OCF₃, -SCF₃, -OH, -SH, -NH₂, -NH(C₁₋₅- Alkyl), -N(C₁₋₅-Alkyl)₂, -NO₂, -CHO, -CF₂H, -CFH₂, -C(=O)-NH₂, - C(=O)-NH(C₁₋₅-Alkyl), -C(=O)-N(C₁₋₅-Alkyl)₂, -S(=O)₂-C₁₋₅-Alkyl, - S(=O)₂-Phenyl, Phenyl, Phenoxy und Benzyl besteht und welches über eine lineare oder verzweigte C₁₋₅-Alkylen-, C₂₋₆-Alkenylen- oder C₂₋₆-Alkinylengruppe gebunden ist, welche unsubstituiert oder mit 1, 2 oder 3 Substituent(en) substituiert sein kann, die unabhängig aus der Gruppe ausgewählt sind, die aus F, Cl, Br, I, -CN, -CF₃, -OCF₃, - SCF₃, -OH, -SH, -NH₂, -O-C₁₋₅-Alkyl, -S-C₁₋₅-Alkyl, Phenyl, -NH-(C₁₋₅- Alkyl) und -N(C₁₋₅-Alkyl)₂ besteht und welche 1, 2 oder 3 Heteroatom(e) enthalten kann, die unabhängig aus der Gruppe ausgewählt sind, die aus Stickstoff, Sauerstoff und Schwefel besteht, als Kettenelement(e) und wobei das Heteroarylradikal 1, 2 oder 3 Heteroatom(e) enthält, die unabhängig aus der Gruppe ausgewählt sind, die aus Stickstoff, Sauerstoff und Schwefel besteht, als Ringelement(e);
oder eine -C( =0)-R^{12a} funktionelle Gruppe;
R^{2a} ein Wasserstoffatom oder ein lineares oder verzweigtes, gesättigtes oder ungesättigtes C₁₋₁₀ aliphatisches Radikal darstellt, welches unsubstituiert oder mit 1, 2 oder 3 Substituent(en) substituiert sein kann, die unabhängig aus der Gruppe ausgewählt sind, die aus F, Cl, Br, I, -CN, -CF₃, -OCF₃, -SCF₃, -OH, -SH, -NH₂, -O-C₁₋₅-Alkyl, -S-C₁₋₅- Alkyl, -NH-(C₁₋₅-Alkyl) und -N(C₁₋₅-Alkyl)₂ besteht;
oder
R^{1a} und R^{2a} bilden zusammen mit dem brückenbildenden Stickstoffatom einen gesättigten, ungesättigten oder aromatischen 3- bis 6- gliedrigen heterocyclischen Ring, welcher mit 1, 2 oder 3 Substituent(en) substituiert sein kann, die unabhängig aus der Gruppe ausgewählt sind, die aus C₁₋₅-Alkyl, -O-C₁₋₅-Alkyl, -S-C₁₋₅- Alkyl, Oxo (=O), Thioxo (=S), -C(=O)-OH, -C(=O)-C₁₋₅-Alkyl, - C(=O)-O-C₁₋₅-Alkyl, -O-C(=O)-C₁₋₅-Alkyl, F, Cl, Br, I, -CN, -CF₃, - OCF₃, -SCF₃, -OH, -SH, -NH₂, -NH(C₁₋₅-Alkyl), -N(C₁₋₅-Alkyl)₂, -NO₂, - CHO, -CF₂H, -CFH₂, -C(=O)-NH2, -C(=O)-NH(C₁₋₅-Alkyl), -C(=O)- N(C₁₋₅-Alkyl)₂, -S(=O)₂-C₁₋₅-Alkyl, -S(=O)₂-Phenyl, Phenyl, Phenoxy und Benzyl besteht und welcher 1, 2 oder 3 zusätzliche Heteroatom(e) enthalten kann, die unabhängig aus der Gruppe ausgewählt sind, die aus Stickstoff, Sauerstoff und Schwefel besteht, als Ringelement(e) und/oder welche mit einem mono- oder bicyclischen Ringsystem kondensiert sein kann, welches mit 1, 2 oder 3 Substituent(en) substituiert sein kann, die unabhängig aus der Gruppe ausgewählt sind, die aus C₁₋₅-Alkyl, -O-C₁₋₅-Alkyl, -S-C₁₋₅- Alkyl, Oxo (=O), Thioxo (=S), -C(=O)-OH, -C(=O)-C₁₋₅-Alkyl, - C(=O)-O-C₁₋₅-Alkyl, -O-C(=O)-C₁₋₅-Alkyl, F, Cl, Br, I, -CN, -CF₃, - OCF₃, -SCF₃, -OH, -SH, -NH₂, -NH(C₁₋₅-Alkyl), -N(C₁₋₅-Alkyl)₂, -NO₂, - CHO, -CF₂H, -CFH₂, -C(=O)-NH₂, -C(=O)-NH(C₁₋₅-Alkyl), -C(=O)- N(C₁₋₅-Alkyl)₂, -S(=O)₂-C₁₋₅-Alkyl, -S(=O)₂-Phenyl, Phenyl, Phenoxy und Benzyl besteht, wobei der cyclische Substituent(en) Phenyl, Phenoxy und Benzyl unsubstituiert oder substituiert durch 1, 2 oder 3 Substituent(en) sein kann, die unabhängig aus der Gruppe ausgewählt sind, die aus Methyl, Ethyl, n-Propyl, Isopropyl, Methoxy, Ethoxy, F, Cl, Br, -CN, -CF₃, -OCF₃, -SCF₃, -OH, -SH, -NH₂ und NO₂ besteht;
wobei die Ringe des Ringsystems 5-, 6- oder 7-Gliedrige sind und 1, 2 oder 3 Heteroatom(e) enthalten können, die unabhängig aus der Gruppe ausgewählt sind, die aus Stickstoff, Sauerstoff und Schwefel besteht;
R^{3a} ein 5- bis 10-gliedriges Aryl oder Heteroarylradikal darstellt, welches mit 1, 2 oder 3 Substituent(en) substituiert sein kann, die unabhängig aus der Gruppe ausgewählt sind, die aus C₁₋₅-Alkyl, -O- C₁₋₅-Alkyl, -S-C₁₋₅-Alkyl, -C(=O)-OH, -C(=O)-C₁₋₅-Alkyl, -C(=O)-O-C₁₋₅-Alkyl, -O-C(=O)-C₁₋₅-Alkyl, F, Cl, Br, I, -CN, -CF₃, -OCF₃, -SCF₃, - OH, -SH, -NH₂, -NH(C₁₋₅-Alkyl), -N(C₁₋₅-Alkyl)₂, -NO₂, -CHO, -CF₂H, - CFH₂, -C(=O)-NHz, -C(=O)-NH(C₁₋₅-Alkyl), -C(=O)-N(C₁₋₅-Alkyl)₂, - S(=O)₂-C₁₋₅-Alkyl, -S(=O)₂-Phenyl, Phenyl, Phenoxy und Benzyl besteht und welches über eine lineare oder verzweigte C₁₋₆-Alkylen-, C₂₋₆-Alkenylen- oder C₂₋₆-Alkinylengruppe gebunden sein kann und welche unsubstituiert oder mit 1, 2 oder 3 Substituent(en) substituiert sein kann, die unabhängig aus der Gruppe ausgewählt sind, die aus F, Cl, Br, I, -CN, -CF₃, -OCF₃, -SCF₃, -OH, -SH, -NH₂, -O- C₁₋₅-Alkyl, -S-C₁₋₅-Alkyl, -NH-(C₁₋₅-Alkyl) und -N(C₁₋₅-Alkyl)₂ besteht und wobei das Heteroarylradikal 1, 2 oder 3 Heteroatom(e) enthält, die unabhängig aus der Gruppe ausgewählt sind, die aus Stickstoff, Sauerstoff und Schwefel besteht, als Ringelement(e);
R^{4a}, R^{5a} und R^{6a}, identisch oder verschieden, jeweils ein Wasserstoffatom oder ein Halogenatom darstellen;
oder
R^{4a} und R^{5a} zusammen mit den brückenbildenden Kohlenstoffatomen einen unsubstituierten 5- oder 6-gliedrigen heterozyklischen Ring bilden, welcher 1, 2 oder 3 Heteroatom(e) enthält, die unabhängig aus der Gruppe ausgewählt sind, die aus Stickstoff, Sauerstoff und Schwefel besteht, als Ringelement(e) und welche zusammen mit dem Phenylring, mit welchem er fusioniert ist, ein 9- oder 10-gliedriges bicyclisches aromatisches Ringsystem bildet;
R^{7a} und R^{8a}, identisch oder verschieden, jeweils ein Wasserstoffatom oder ein lineares oder verzweigtes, gesättigtes oder ungesättigtes C₁₋₁₀ aliphatisches Radikal darstellen, welches unsubstituiert oder substituiert mit 1, 2 oder 3 Substituent(en) substituiert sein kann, die unabhängig aus der Gruppe ausgewählt sind, die aus F, Cl, Br, I, -CN, -CF₃, -OCF₃, -SCF₃, -OH, -SH, -NH₂, -O-C₁₋₅-Alkyl, -S-C₁₋₅-Alkyl, -NH- (C₁₋₅-Alkyl) und -N(C₁₋₅-Alkyl)₂ besteht;
R^{9a} und R^{10a}, identisch oder verschieden, jeweils ein Wasserstoffatom oder ein linear oder verzweigtes, gesättigtes oder ungesättigtes C₁₋₁₀ aliphatisches Radikal darstellen, welches unsubstituiert oder mit 1, 2 oder 3 Substituent(en) substituiert sein kann, die unabhängig aus der Gruppe ausgewählt sind, die aus F, Cl, Br, I, -CN, -CF₃, -OCF₃, -SCF₃, -OH, -SH, -NH₂, -O-C₁-₅-Alkyl, -S-C₁₋₅-Alkyl, -NH-(C₁₋₅-Alkyl) und - N(C₁₋₅-Alkyl)₂ besteht;
R^{11a} stellt dar, ein Wasserstoffatom; ein linear oder verzweigtes, gesättigtes oder ungesättigtes C₁₋₁₀ aliphatisches Radikal, welches unsubstituiert oder mit 1, 2 oder 3 Substituent(en) substituiert sein kann, die unabhängig aus der Gruppe ausgewählt sind, die aus F, Cl, Br, I, -CN, -CF₃, -OCF₃, -SCF₃, -OH, -SH, -NH₂, -O-C₁₋₅-Alkyl, -S-C₁₋₅- Alkyl, -NH-(C₁₋₅-Alkyl) und -N(C₁₋₅-Alkyl)₂ besteht und welches 1, 2 oder 3 Heteroatom(e) enthalten kann, die unabhängig aus der Gruppe ausgewählt sind, die aus Stickstoff, Sauerstoff und Schwefel besteht, als Kettenelement(e);
ein 5- bis 10-gliedriges Aryl oder Heteroarylradikal, welches mit 1, 2 oder 3 Substituent(en) substituiert sein kann, die unabhängig aus der Gruppe ausgewählt sind, die aus C₁₋₅-Alkyl, -O-C₁₋₅-Alkyl, -S-C₁₋₅- Alkyl, -C(=O)-OH, -C(=O)-C₁₋₅-Alkyl, -C(=O)-O-C₁₋₅-Alkyl, -O-C(=O)- C₁₋₅-Alkyl, F, Cl, Br, I, -CN, -CF₃, -OCF₃, -SCF₃, -OH, -SH, -NH₂, - NH(C₁₋₅-Alkyl), -N(C₁₋₅-Alkyl)₂, -NO₂, -CHO, -CF₂H, -CFH₂, -C(=O)- NH₂, -C(=O)-NH(C₁₋₅-Alkyl), -C(=O)-N(C₁₋₅-Alkyl)₂, -S(=O)₂-C₁₋₅- Alkyl, -S(=O)₂-Phenyl, Phenyl, Phenoxy und Benzyl besteht und welches über eine lineare oder verzweigte, C₁₋₆-Alkylen-, C₂₋₆- Alkenylen- oder C₂₋₆-Alkinylengruppe gebunden sein kann, welche unsubstituiert oder mit 1, 2 oder 3 Substituent(en) substituiert sein kann, die unabhängig aus der Gruppe ausgewählt sind, die aus F, Cl, Br, I, -CN, -CF₃, -OCF₃, -SCF₃, -OH, -SH, -NH₂, -O-C₁₋₅-Alkyl, -S-C₁₋₅- Alkyl, -NH-(C₁₋₅-Alkyl) und -N(C₁₋₅-Alkyl)₂ besteht und wobei das Heteroarylradikal 1, 2 oder 3 Heteroatom(e) enthält, die unabhängig aus der Gruppe ausgewählt sind, die aus Stickstoff, Sauerstoff und Schwefel besteht, als Ringelement(e); eine -C(=O)-R^{13a} funktionelle Gruppe oder eine -S(=O)₂-R^{14a} funktionelle Gruppe;
R^{12a} stellt dar, ein lineares oder verzweigtes, gesättigtes oder ungesättigtes C₁₋₁₀ aliphatisches Radikal, welches unsubstituiert oder mit 1, 2 oder 3 Substituent(en) substituiert sein kann, die unabhängig aus der Gruppe ausgewählt sind, die aus F, Cl, Br, I, -CN, -CF₃, -OCF₃, -SCF₃, -OH, -SH, -NH₂, -O-C₁₋₅-Alkyl, -S-C₁₋₅-Alkyl, -NH- (C₁₋₅-Alkyl) und -N(C₁₋₅-Alkyl)₂ besteht;
oder ein 5- bis 10-gliedriges Aryl oder Heteroarylradikal, welches mit 1, 2 oder 3 Substituent(en) substituiert sein kann, die unabhängig aus der Gruppe ausgewählt sind, die aus C₁₋₅-Alkyl, -O-C₁₋₅-Alkyl, -S- C₁₋₅-Alkyl, -C(=O)-OH, -C(=O)-C₁₋₅-Alkyl, -C(=O)-O-C₁₋₅-Alkyl, -O- C(=O)-C₁₋₅-Alkyl, F, Cl, Br, I, -CN, -CF₃, -OCF₃, -SCF₃, -OH, -SH, - NH₂, -NH(C₁₋₅-Alkyl), -N(C₁₋₅-Alkyl)₂, -NO₂, -CHO, -CF₂H, -CFH₂, - C(=O)-NH₂, -C(=O)-NH(C₁₋₅-Alkyl), -C(=O)-N(C₁₋₅-Alkyl)₂, -S(=O)₂- C₁₋₅-Alkyl, -S(=O)₂-Phenyl, Phenyl, Phenoxy und Benzyl besteht und welches über eine lineare oder verzweigte C₁₋₆ Alkylen-, C₂₋₆ Alkenylen- oder C₂₋₆ Alkinylengruppe gebunden sein kann, welche unsubstituiert oder mit 1, 2 oder 3 Substituent(en) substituiert sein kann, die unabhängig aus der Gruppe ausgewählt sind, die aus F, Cl, Br, I, -CN, -CF₃, -OCF₃, -SCF₃, -OH, -SH, -NH₂, -O-C₁₋₅-Alkyl, -S-C₁₋₅- Alkyl, -NH-(C₁₋₅-Alkyl) und -N(C₁₋₅-Alkyl)₂ besteht und wobei das Heteroarylradikal 1, 2 oder 3 Heteroatom(e) enthält, die unabhängig aus der Gruppe ausgewählt sind, die aus Stickstoff, Sauerstoff und Schwefel besteht, als Ringelement(e);
und
R^{13a} und R^{14a}, identisch oder verschieden, stellt jeweils dar, ein lineares oder verzweigtes, gesättigtes oder ungesättigtes C₁₋₁₀ aliphatisches Radikal, welches unsubstituiert oder mit 1, 2 oder 3 Substituent(en) substituiert sein kann, die unabhängig aus der Gruppe ausgewählt sind, die aus F, Cl, Br, I, -CN, -CF₃, -OCF₃, -SCF₃, -OH, -SH, -NH₂, -O- C₁₋₅-Alkyl, -S-C₁₋₅-Alkyl, -NH-(C₁₋₅-Alkyl) und -N(C₁₋₅-Alkyl)₂ besteht;
oder ein 5- bis 10-gliedriges Aryl oder Heteroarylradikal, welches mit 1, 2 oder 3 Substituent(en) substituiert sein kann, die unabhängig aus der Gruppe ausgewählt sind, die aus C₁₋₅-Alkyl, -O-C₁₋₅-Alkyl, -S- C₁₋₅-Alkyl, -C(=O)-OH, -C(=O)-C₁₋₅-Alkyl, -C(=O)-O-C₁₋₅-Alkyl, -O- C(=O)-C₁₋₅-Alkyl, F, Cl, Br, I, -CN, -CF₃, -OCF₃, -SCF₃, -OH, -SH, - NH₂, -NH(C₁₋₅-Alkyl), -N(C₁₋₅-Alkyl)₂, -NO₂, -CHO, -CF₂H, -CFH₂, - C(=O)-NH₂, -C(=O)-NH(C₁₋₅-Alkyl),-C(=O)-N(C₁₋₅-Alkyl)₂, -S(=O)₂-C₁₋₅-Alkyl, -S(=O)₂-Phenyl, Phenyl, Phenoxy und Benzyl besteht und/oder welches über eine lineare oder verzweigte C₁₋₆ Alkylen-, C₂₋₆ Alkenylen- oder C₂₋₆Alkinylengruppe gebunden sein kann, welche unsubstituiert oder mit 1, 2 oder 3 Substituent(en) substituiert sein kann, die unabhängig aus der Gruppe ausgewählt sind, die aus F, Cl, Br, I, -CN, -CF₃, -OCF₃, -SCF₃, -OH, -SH, -NH₂, -O-C₁₋₅-Alkyl, -S-C₁₋₅- Alkyl, -NH-(C₁₋₅-Alkyl) und -N(C₁₋₅-Alkyl)₂ besteht und wobei das Heteroarylradikal 1, 2 oder 3 Heteroatom(e) enthält, die unabhängig aus der Gruppe ausgewählt sind, die aus Stickstoff, Sauerstoff und Schwefel besteht, als Ringelement(e).
optional in Form von einem seiner Stereoisomere, bevorzugt Enantiomere oder Diasteromere, eines Racemats oder in Form von einer Mischung aus zumindest zwei seiner Stereoisomeren, bevorzugt Enantiomeren und/oder Diasteromeren, in irgendeinem Mischungsverhältnis, oder eines physiologisch akzeptablen Salzes davon, oder eines korrespondierenden Solvats davon;
und optional zumindest ein physiologisch kompatibles Hilfsagens.

18. Medikament nach Anspruch 17, **dadurch gekennzeichnet, dass**
R^{1a} stellt dar, ein Alkyradikal, das aus der Gruppe ausgewählt ist, die aus Methyl, Ethyl, n-Propyl, Isopropyl, n-Butyl, sec-Butyl, Isobutyl und tert-Butyl besteht, welches mit 1, 2 oder 3 Substituent(en) substituiert ist, die unabhängig aus der Gruppe ausgewählt sind, die aus F, CI, Br, I, -CN, -CF₃, -OCF₃, -SCF₃, -OH, -SH und -NH₂ besteht;
ein Radikal, das aus der Gruppe ausgewählt ist, die aus Adamantyl, Bicyclo[2.2.1]heptyl und Bicyclo[3.1.1]heptyl besteht, welches mit 1, 2 oder 3 Substituent(en) substituiert sein kann, die unabhängig aus der Gruppe ausgewählt sind, die aus C₁₋₅-Alkyl, -O-C₁₋₅-Alkyl, F, CI, Br, I, -CN, -CF₃ und -OCF₃ besteht und/oder welches über eine lineare oder verzweigte C₁₋₆ Alkylengruppe gebunden ist, welche 1, 2 oder 3 Sauerstoffatom(e) als Kettenelement(e) enthalten kann;
ein Aryl oder Heteroarylradikal, das aus der Gruppe ausgewählt ist, die aus Phenyl, Naphthyl, Furyl (Furanyl), Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridinyl, Pyridazinyl, Pyrimidinyl, Pyrazinyl, Quinolinyl, Isoquinolinyl, Benzo[b]furanyl, Benzo[b]thiophenyl, Benzothiadiazylol und Imidazo[2,1-b]thiazolyl besteht, welches mit 1, 2, 3, 4 oder 5 Substituent(en) substituiert sein kann, die unabhängig aus der Gruppe ausgewählt sind, die aus C₁₋₅-Alkyl, -O-C₁₋₅-Alkyl, -S-C₁₋₅-Alkyl, - C(=O)-OH, -C(=O)-O-C₁₋₅-Alkyl, -O-C(=O)-C₁₋₅-Alkyl, F, CI, Br, I, -CN, -CF₃, -OCF₃, -SCF₃, -OH, -SH, -NH₂, NH(C₁₋₅-Alkyl), -N(C₁₋₅-Alkyl)₂, -NO₂, -CHO, - CF₂H, -CFH₂, -C(=O)-NH₂, -C(=O)-NH(C₁₋₅-Alkyl), -C(=O)-N(C₁₋₅-Alkyl)₂, - S(=O)₂-C₁₋₅-Alkyl, -S(=O)₂-Phenyl, Phenyl, Phenoxy und Benzyl besteht;
ein Arylradikal, das aus der Gruppe ausgewählt ist, die aus Phenyl und Naphthyl besteht, welches mit 1, 2, 3, 4 oder 5 Substituent(en) substituiert sein kann, die unabhängig aus der Gruppe ausgewählt sind, die aus C₁₋₅-Alkyl, -O-C₁₋₅-Alkyl, -S-C₁₋₅-Alkyl, -C(=O)-O-C₁₋₅-Alkyl, -O-C(=O)-C₁₋₅-Alkyl, F, Cl, Br, I, -CN, -CF₃, -OCF₃, -SCF₃, -OH, -SH, NH₂, -NH(C₁₋₅-Alkyl), -N(C₁₋₅-Alkyl)₂, -NO₂, -CHO, -CF₂H, -CFH₂, -C(=O)-NH₂, -C(=O)-NH(C₁₋₅-Alkyl), - C(=O)-N(C₁₋₅-Alkyl)₂, -S(=O)₂-C₁₋₅-Alkyl, -S(=O)₂-Phenyl, Phenyl, Phenoxy und Benzyl besteht und das Arylradikal ist über eine lineare oder verzweigte C₁₋₆ Alkylengruppe gebunden, welche 1, 2 oder 3 Sauerstoffatom(e) als Kettenelement(e) enthalten kann und welche mit 1 Phenylradikal substituiert sein kann;
ein Heteroarylradikal, das aus der Gruppe ausgewählt ist, die aus Furyl (Furanyl), Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridinyl, Pyridazinyl, Pyrimidinyl, Pyrazinyl, Quinolinyl, Isoquinolinyl, Benzo[b]furanyl, Benzo[b]thiophenyl, Benzothiadiazolyl und Imidazo[2,1-b]thiazolyl besteht, wobei das Heteroarylradikal mit 1, 2, 3, 4 oder 5 Substituent(en) substituiert sein kann, die unabhängig aus der Gruppe ausgewählt sind, die aus C₁₋₅-Alkyl, -O-C₁₋₅-Alkyl, -S-C₁₋₅-Alkyl, -C(=O)-OH, -C(=O)-O-C₁₋₅-Alkyl, -O-C(=O)-C₁₋₅-Alkyl, F, Cl, Br, I, -CN, -CF₃, -OCF₃,-SCF₃, -OH, -SH, -NH₂, NH(C₁₋₅-Alkyl), -N(C₁₋₅-Alkyl)₂, -NO₂, -CHO, -CF₂H, - CFH₂, -C(=O)-NH₂, -C(=O)-NH(C₁₋₅-Alkyl), -C(=O)-N(C₁₋₅-Alkyl)₂, -S(=O)₂-C₁₋₅-Alkyl, -S(=O)₂-Phenyl, Phenyl, Phenoxy und Benzyl besteht und das Heteroarylradikal ist über eine lineare oder verzweigte C₁₋₆ Alkylengruppe gebunden, welche 1, 2 oder 3 Sauerstoffatom(e) enthalten kann, als Kettenelement(e);
oder eine -C(=O)-R^{12a} funktionelle Gruppe;
bevorzugt stellt R^{1a} dar, ein Alkylradikal, das aus der Gruppe ausgewählt ist, die aus Methyl, Ethyl, n-Propyl und Isopropyl besteht, wobei das Alkylradikal mit 1, 2 oder 3 Substituent(en) substituiert ist, die unabhängig aus der Gruppe ausgewählt sind, die aus -OH, F, Cl, Br und -CN besteht;
ein Radikal, das aus der Gruppe ausgewählt ist, die aus Adamantyl, Bicyclo[2.2.1]heptyl und Bicyclo[3.1.1]heptyl besteht, welches mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, F, CI, Br, I, -CN, -CF₃ und -OCF₃ besteht und/oder welches über eine Alkylengruppe gebunden ist, die aus der Gruppe ausgewählt ist, die aus -CH₂-, -CH(CH₃)-, -CH₂-CH₂-, -CH₂-CH₂-CH₂- und - CH₂-CH₂-O- besteht;
ein Aryl oder Heteroarylradikal, das aus der Gruppe ausgewählt ist, die aus Phenyl, Naphthyl, Furyl (Furanyl), Thienyl (Thiophenyl), Pyrrolyl, Oxazolyl, Isoxazolyl, Thiazolyl, Isothiazolyl, Imidiazolyl, Pyrazolyl, Oxadiazolyl, Thiadiazolyl, Triazolyl und Pyridinyl besteht, wobei das Aryl oder Heteroarylradikal mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -CHO, -CF₂H, -CFH₂, -C(=O)-NH₂, -C(=O)-NH-CH₃, - C(=O)-NH-C₂H₅, -C(=O)-N(CH₃)₂, -C(=O)-N(C₂H₅)₂ und -S(=O)₂-CH₃ besteht;
ein Arylradikal, das aus der Gruppe ausgewählt ist, die aus Phenyl und Naphthyl besteht, wobei das Aryl mit 1, 2 oder 3 Substituent(en) substituiert sein kann, die unabhängig aus der Gruppe ausgewählt sind, die Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -CHO, -CF₂H, -CFH₂, -C(=O)-NH₂, -C(=O)-NH-CH₃, - C(=O)-NH-C₂H₅, -C(=O)-N(CH₃)₂, -C(=O)-N(C₂H₅)₂ und -S(=O)₂-CH₃ besteht und/oder über eine Alkylengruppe gebunden ist, die aus der Gruppe ausgewählt ist, die aus -CH₂-, -CH(CH₃)-, -CH(Phenyl)-, -CH₂-CH₂-, -CH₂-CH₂-CH₂- und -CH₂-CH₂-O- besteht;
ein Heteroarylradikal, das aus der Gruppe ausgewählt ist, die aus Furyl (Furanyl), Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl und Pyridinyl besteht, wobei das Heteroarylradikal mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, F, Cl, Br, I, -CN,-CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ und -S(=O)₂-CH₃ besteht und/oder über eine Alkylengruppe gebunden ist, die aus der Gruppe ausgewählt ist, die aus -CH₂-, -CH(CH₃)-, -CH₂-CH₂-, - CH₂-CH₂-CH₂- und -CH₂-CH₂-O- besteht;
oder eine -C(=O)-R^{12a} funktionelle Gruppe;
mehr bevorzugt stellt R^{1a} dar, ein Alkylradikal, das aus der Gruppe ausgewählt ist, die aus -CH₂-CH₂-OH und -CH₂-CH₂-CH₂-OH;
ein unsubstituiertes Adamantylradikal;
ein unsubstituiertes Phenyl oder Pyrrolylradikal;
ein unsubstituiertes Naphthylradikal, welches über eine Alkylengruppe gebunden ist, das aus der Gruppe ausgewählt ist, die aus -CH₂-, -CH(CH₃)-, -CH₂-CH₂-, -CH₂-CH₂-CH₂- und -CH₂-CH₂-O- besteht;
ein Phenylradikal, welches mit 1, 2 oder 3 Substituent(en) substituiert sein kann, die unabhängig aus der Gruppe ausgewählt sind, die aus Methyl, Ethyl, tert-Butyl, Methoxy, F und Cl besteht und das Phenylradikal ist über eine Alkylengruppe gebunden, die aus der Gruppe ausgewählt ist, die aus - CH₂-, -CH(CH₃)-, -CH(Phenyl)-, -CH₂-CH₂-, -CH₂-CH₂-CH₂- und -CH₂-CH₂-O-besteht;
ein Heteroarylradikal, das aus der Gruppe ausgewählt ist, die aus Pyridinyl, Furanyl und Pyrrolyl besteht, wobei das Pyridinyl, Furanyl oder Pyrrolylradikal mit 1, 2 oder 3 Substituent(en) substituiert sein kann, die unabhängig aus der Gruppe ausgewählt sind, die aus Mehtyl, Ethyl, tert-Butyl, Methoxy, F und Cl besteht und das Pyridinyl, Furanyl oder Pyrrolylradikal ist über eine Alkylengruppe gebunden, die aus der Gruppe ausgewählt ist, die aus -CH₂-, -CH(CH₃)-, -CH₂-CH₂-, -CH₂-CH₂-CH₂- und - CH₂-CH₂-O- besteht;
oder eine -C(=O)-R^{12a} funktionelle Gruppe.

19. Medikament nach Anspruch 17 oder 18, **dadurch gekennzeichnet, dass**
R^{2a} ein Wasserstoffatom oder ein lineares oder verzweigtes, unsubstituiertes C₁₋₁₀ Alkylradikal darstellt;
bevorzugt stellt R^{2a} ein Wasserstoffatom oder ein Alkylradikal dar, das aus der Gruppe ausgewählt ist, die aus Methyl, Ethyl, n-Propyl, Isopropyl, n-Butyl, sec-Butyl, Isobutyl und tert-Butyl besteht;
mehr bevorzugt stellt R^{2a} ein Wasserstoffatom oder ein Methylradikal dar.

20. Medikament nach einem oder mehreren der Ansprüche 17 bis 19, **dadurch gekennzeichnet, dass**
R^{1a} und R^{2a} zusammen mit dem brückenbildenden Stickstoffatom eine funktionelle Gruppe bilden, die aus der Gruppe ausgewählt ist, die aus 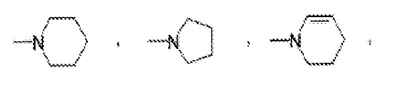 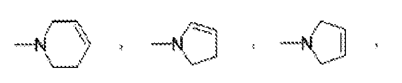 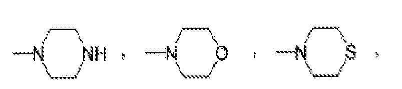 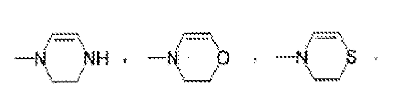 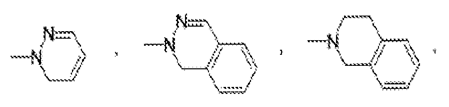 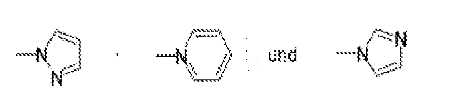 besteht,
wobei jede von diesen zuvor genannten zyklischen funktionellen Gruppen an irgendeiner Position einschließlich der -NH-Gruppen mit 1, 2 oder 3 Substituent(en) substituiert sein kann, die unabhängig aus der Gruppe ausgewählt sind, die aus C₁₋₅-Alkyl, -O-C₁₋₅-Alkyl, -S-C₁₋₅-Alkyl, -C(=O)-OH, Oxo (=O), Thioxo (=S), -C(=O)-O-C₁₋₅-Alkyl, -O-C(=O)-C₁₋₅-Alkyl, F, Cl, Br, I, -CN, -CF₃, -OCF₃, -SCF₃, -OH, -SH, -NH₂, -NH(C₁₋₅-Alkyl), -N(C₁₋₅-Alkyl)₂, -NO₂, -CHO, -CF₂H, -CFH₂, -C(=O)-NH₂, -C(=O)-NH(C₁₋₅-Alkyl), -C(=O)-N(C₁₋₅-Alkyl)₂, -S(=O)₂-C₁₋₅-Alkyl, -S(=O)₂-Phenyl, Phenyl, Phenoxy und Benzyl besteht; wobei der zyklische Substituent(en) Phenyl, Phenoxy und Benzyl unsubstituiert oder durch 1, 2 oder 3 Substituent(en) substituiert sein kann, die unabhängig aus der Gruppe ausgewählt sind, die aus Methyl, Ethyl, n-Propyl, Isopropyl, Methoxy, Ethoxy, F, Cl, Br, -CN, -CF₃, -OCF₃,-SCF₃, -OH, -SH, -NH₂ und NO₂ besteht;
bevorzugt bilden R^{1a} und R^{2a} zusammen mit dem brückenbildenden Stickstoffatom eine funktionelle Gruppe, die aus der Gruppe ausgewählt ist, die aus 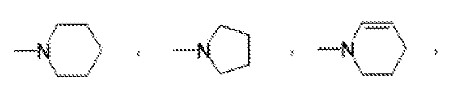 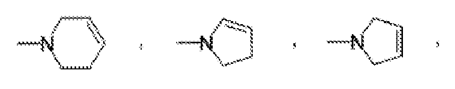 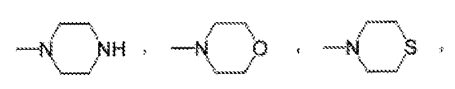 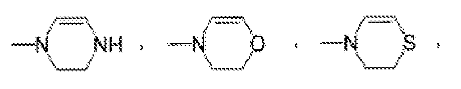 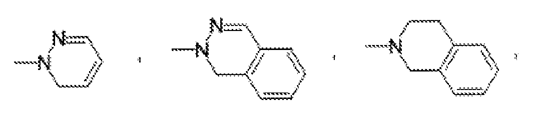 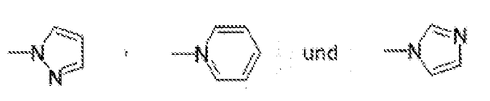 besteht,
wobei jede von diesen zuvor genannten zyklischen funktionellen Gruppen mit 1, 2 oder 3 Substituent(en) substituiert sein kann, die unabhängig aus der Gruppe ausgewählt sind, die aus Oxo (=O), Thioxo (=S), Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, - S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH,-NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -NO₂, -CHO, -CF₂H, CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-Phenyl, Phenyl, Phenoxy und Benzyl besteht; wobei der zyklische Substituent(en) Phenyl, Phenoxy und Benzyl unsubstituiert oder durch 1, 2 oder 3 Substituent(en) substituiert sein kann, die unabhängig aus der Gruppe ausgewählt sind, die aus Methyl, Ethyl, n-Propyl, Isopropyl, Methoxy, Ethoxy, F, Cl, Br, -CN, -CF₃, -OCF₃, -SCF₃, -OH, -SH, -NH₂ und NO₂ besteht;
mehr bevorzugt bilden R^{1a} und R^{2a} zusammen mit dem brückenbildenden Stickstoffatom eine funktionelle Gruppe, die aus der Gruppe ausgewählt ist, die aus 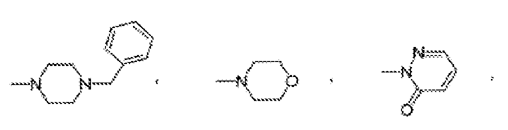 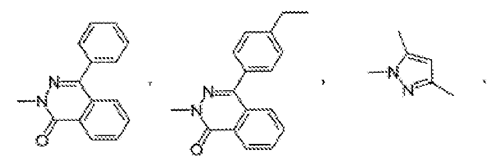 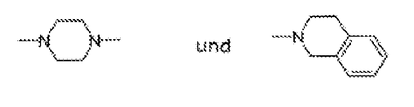 besteht.

21. Medikament nach einem oder mehreren der Ansprüche 17 bis 20, **dadurch gekennzeichnet, dass**
R^{3a} ein Aryl oder Heteroarylradikal darstellt, das aus der Gruppe ausgewählt ist, die aus Phenyl, Naphthyl, Furyl (Furanyl), Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl und Pyridinyl besteht, wobei das Aryl oder Heteroarylradikal mit 1, 2 oder 3 Substituent(en) substituiert sein kann, die unabhängig aus der Gruppe ausgewählt sind, die aus Mehtyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -CHO, -CF₂H, CFH₂, -C(=O)-NH₂, -C(=O)-NH-CH₃, - C(=O)-NH-C₂H₅, -C(=O)-N(CH₃)₂, -C(=O)-N(C₂H₅)₂ und -S(=O)₂-CH₃ besteht;
bevorzugt stellt R^{3a} ein Phenylradikal dar, welches mit 1, 2 oder 3 Substituent(en) substituiert sein kann, die unabhängig aus der Gruppe ausgewählt sind, die aus Mehtyl, Ethyl, n-Propyl, Isopropyl, n-Butyl, tert-Butyl, -O-CH₃, -O-C₂H₅, -O-CH₂-CH₂-CH₃, -C(=O)-OH, -C(=O)-O-CH₃, - C(=O)-O-C₂H₅, -C(=O)-CH₃, -C(=O)-C₂H₅, F, Cl, Br, I, -CN, -CF₃, -OCF₃,-SCF₃, -SH, -NH₂, -NH-CH₃, -NH-C₂H₅, -NH-CH₂-CH₂-CH₃, -NH-CH(CH₃)₂, - NH-C(CH₃)₃, -N(CH₃)₂, -N(C₂H₅)₂, -CHO, -CF₂H, -CFH₂ und -S(=O)₂-CH₃ besteht;
mehr bevorzugt stellt R^{3a} ein unsubstituiertes Phenylradikal dar.

22. Medikament nach einem oder mehreren der Ansprüche 17 bis 21, **dadurch gekennzeichnet, dass**
R^{4a}, R^{5a} und R^{6a}, identisch oder verschieden, jeweils ein Wasserstoffatom oder ein Halogenatom darstellen, das aus der Gruppe ausgewählt ist, die aus F, Cl und Br besteht;
bevorzugt stellen R^{4a}, R^{5a} und R^{6a}, identisch oder verschieden, jeweils ein Wasserstoffatom oder Fluoratom dar.

23. Medikament nach einem oder mehreren der Ansprüche 17 bis 22, **dadurch gekennzeichnet, dass**
R^{4a} und R^{5a} zusammen mit dem brückenbildenden Kohlenstoffatomen eine funktionelle Gruppe bilden, die aus der Gruppe ausgewählt ist, die aus 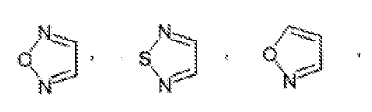 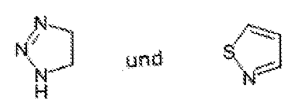 besteht, welche zusammen mit dem Phenylring, mit welchem Sie fusioniert ist, ein substituiertes bizyklisches aromatisches Ringsystem bildet, das aus der Gruppe ausgewählt ist, die aus 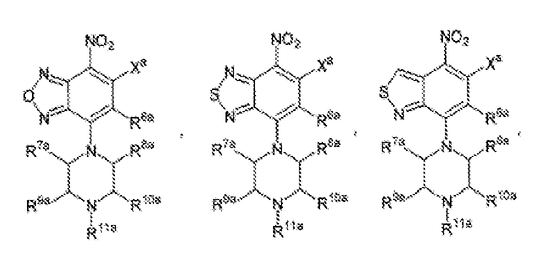 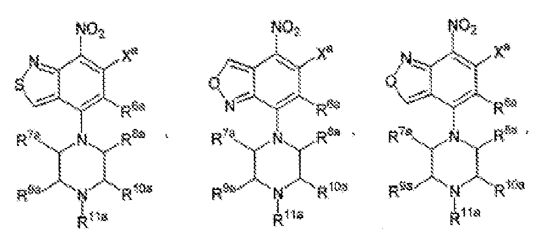 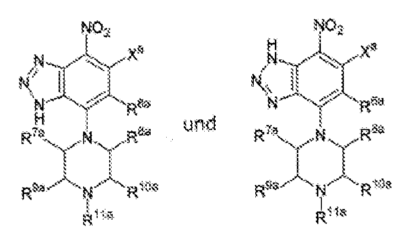 besteht;
bevorzugt bilden R^{4a} und R^{5a} zusammen mit den brückenbildenden Kohlenstoffatomen die folgende funktionelle Gruppe, 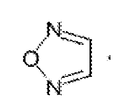 welche zusammen mit dem Phenylring, mit welchem Sie fusioniert ist, das folgende substituierte bizyklische aromatische Ringsystem bildet

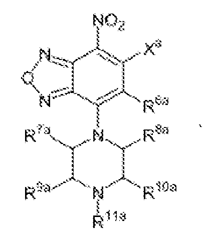

24. Medikament nach einem oder mehreren der Ansprüche 17 bis 23, **dadurch gekennzeichnet, dass**
R^{7a} und R^{8a}, identisch oder verschieden, jeweils ein Wasserstoffatom oder ein lineares oder verzweigtes, unsubstituiertes (₁₋₁₀ Alkylradikal darstellen;
bevorzugt R^{7a} und R^{8a}, identisch oder verschieden, jeweils ein Wasserstoffatom oder ein Alkylradikal darstellenn, das aus der Gruppe ausgewählt ist, die aus Methyl, Ethyl, n-Propyl, Isopropyl, n-Butyl, sec-Butyl, Isobutyl und tert-Butyl besteht;
mehr bevorzugt stellt R^{7a} und R^{8a} jeweils ein Wasserstoffatom dar.

25. Medikament nach einem oder mehreren der Ansprüche 17 bis 24, **dadurch gekennzeichnet, dass**
R^{9a} und R^{10a}, identisch oder verschieden, jeweils ein Wasserstoffatom oder ein lineares oder verzweigtes, unsubstituiertes C₁₋₁₀ Alkylradikal darstellen;
bevorzugt R^{9a} und R^{10a}, identisch oder verschieden, jeweils ein Wasserstoffatom oder ein Alkylradikal darstellen, das aus der Gruppe ausgewählt ist, die aus Methyl, Ethyl, n-Propyl, Isopropyl, n-Butyl, sec-Butyl, Isobutyl und tert-Butyl besteht;
mehr bevorzugt stellt R^{9a} und R^{10a}, identisch oder verschieden, jeweils ein Wasserstoffatom oder ein Methylradikal dar.

26. Medikament nach einem oder mehreren der Ansprüche 17 bis 25, **dadurch gekennzeichnet, dass**
R^{11a} stellt dar, ein Wasserstoffatom;
ein Alkylradikal, das aus der Gruppe ausgewählt ist, die aus Methyl, Ethyl, n-Propyl, Isopropyl, n-Butyl, sec-Butyl, Isobutyl und tert-Butyl besteht, wobei das Alkylradikal mit 1, 2 oder 3 Substituent(en) substituiert sein kann, die unabhängig aus der Gruppe ausgewählt sind, die aus -OH, F, Cl, Br und -CN besteht;
ein Aryl oder Heteroarylradikal, das aus der Gruppe ausgewählt ist, die aus Phenyl, Naphthyl, Furyl (Furanyl), Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridinyl, Pyridazinyl, Pyrimidinyl, Pyrazinyl, Quinolinyl, Isoquinolinyl, Benzo[b]furanyl, Benzo[b]thiophenyl, Benzothiadiazolyl und Imidazo[2,1-b]thiazolyl besteht, wobei das Aryl oder Heteroarylradikal mit 1, 2, 3, 4 oder 5 Substituent(en) substituiert sein kann, die unabhängig aus der Gruppe ausgewählt sind, die aus C₁₋₅-Alkyl, - O-C₁₋₅-Alkyl, -S-C₁₋₅-Alkyl, -C(=O)-OH, -C(=O)-O-C₁₋₅-Alkyl, -O-C(=O)-C₁₋₅-Alkyl, F, CI, Br, I, -CN, -CF₃, -OCF₃, -SCF₃, -OH, -SH, -NH₂, NH(C₁₋₅-Alkyl), - N(C₁₋₅-Alkyl)₂, -NO₂, -CHO, -CF₂H, -CFH₂, -C(=O)-NH₂, -C(=O)-NH(C₁₋₅-Alkyl), -C(=O)-N(C₁₋₅-Alkyl)₂, -S(=O)₂-C₁₋₅-Alkyl, -S(=O)₂-Phenyl, Phenyl, Phenoxy und Benzyl besteht und/oder über eine -(CH₂)_{1,2} oder ₃-Gruppe gebunden sein kann;
eine -C(=O)-R^{13a} funktionelle Gruppe oder eine -SC=O)₂-R^{14a} funktionelle Gruppe;
bevorzugt R^{11a} stellt dar, ein Wasserstoffatom;
ein Alkylradikal, das aus der Gruppe ausgewählt ist, die aus Methyl, Ethyl n-Propyl, Isopropyl, n-Butyl, sec-Butyl, Isobutyl und tert-Butyl besteht, wobei das Alkylradikal mit 1, 2 oder 3 Substituent(en) substituiert sein kann, die unabhängig aus der Gruppe ausgewählt sind, die aus -OH, F, Cl, Br und-CN besteht;
ein Aryl oder Heteroarylradikal, das aus der Gruppe ausgewählt ist, die aus Phenyl, Naphthyl, Furyl (Furanyl), Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridazinyl, Pyrimidinyl, Pyridinyl, Pyrazinyl, Quinolinyl, Isoquinolinyl, Benzo[b]furanyl, Benzo[b]thiophenyl, Benzothiadiazolyl und Imidazo[2,1-b]thiazolyl besteht, wobei das Aryl oder Heteroaryl mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ und -S(=O)₂-CH₃ besteht und/oder über eine -(CH₂)_{1,2} oder ₃-Gruppe gebunden sein kann;
eine -C(=O)-R^{13a} funktionelle Gruppe oder eine -SC(=O)₂-R^{14a} funktionelle Gruppe;
mehr bevorzugt R^{11a} stellt dar, ein Wasserstoffatom;
ein Alkylradikal, das aus der Gruppe ausgewählt ist, die aus Methyl, n-Butyl und -CH₂-CH₂-OH besteht;
ein unsubstituiertes Phenyl oder Pyridinylradikal, wobei das Phenyl oder Pyridinylradikal über eine -(CH₂)-Gruppe gebunden sein kann;
eine -C(=O)-R^{12a} funktionelle Gruppe oder eine -SC=O)₂R^{13a} funktionelle Gruppe.

27. Medikament nach einem oder mehreren der Ansprüche 17 bis 26, **dadurch gekennzeichnet, dass**
R^{12a} darstellt, ein Alkylradikal, das aus der Gruppe ausgewählt ist, die aus Methyl, Ethyl, n-Propyl, Isopropyl, n-Butyl, sec-Butyl, Isobutyl und tert-Butyl besteht;
oder ein Aryl oder Heteroarylradikal, das aus der Gruppe ausgewählt ist, die aus Phenyl, Naphthyl, Furyl (Furanyl), Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridinyl, Pyridazinyl, Pyrimidinyl, Pyrazinyl, Quinolinyl, Isoquinolinyl, Benzo[b]furanyl, Benzo[b]thiophenyl, Benzothiadiazolyl und Imidazo[2,1-b]thiazolyl besteht wobei das Aryl oder Heteroaryl mit 1, 2, 3, 4 oder 5 Substituent(en) substituiert sein kann, die unabhängig aus der Gruppe ausgewählt sind, die aus C₁₋₅-Alkyl, -O-C₁₋₅-Alkyl, -S-C₁₋₅-Alkyl, -C(=O)-OH, -C(=O)-O-C₁₋₅-Alkyl, -O-C(=O)-C₁₋₅-Alkyl, F, CI, Br, I, -CN, -CF₃, -OCF₃, -SCF₃, -OH, -SH, -NH₂, NH(C₁₋₅-Alkyl), -N(C₁₋₅-Alkyl)₂, -NO₂, -CHO, -CF₂H, -CFH₂, -C(=O)-NH₂, -C(=O)-NH(C₁₋₅-Alkyl), - C(=O)-N(C₁₋₅-Alkyl)₂, -S(=O)₂-C₁₋₅-Alkyl, -S(=O)₂-Phenyl, Phenyl, Phenoxy und Benzyl besteht und/oder über eine -(CH₂)_{1,2 oder 3}-Gruppe gebunden sein kann;
bevorzugt R^{12a} stellt dar, ein Alkylradikal, das aus der Gruppe ausgewählt ist, die aus Methyl, Ethyl, n-Propyl und Isopropyl besteht,
oder ein Aryl oder Heteroarylradikal, das aus der Gruppe ausgewählt ist, die aus Phenyl, Naphthyl, Furyl (Furanyl), Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl und Pyridinyl besteht, wobei das Aryl oder Heteroarylradikal mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -CHO, -CF₂H, -CFH₂, -C(=O)-NH₂, -C(=O)-NH-CH₃, - C(=O)-NH-C₂H₅, -C(=O)-N(CH₃)₂, -C(=O)-N(C₂H₅)₂ und -S(=O)₂-CH₃ besteht und/oder über eine -(CH₂)-Gruppe gebunden sein kann;
mehr bevorzugt stellt R^{12a} ein Phenyl oder ein Thiophenylradikal dar, wobei das Phenyl oder Thiophenylradikal mit 1, 2 oder 3 Substituent(en) substituiert sein kann, die aus der Gruppe ausgewählt sind, die aus Methyl und Chlor besteht.

28. Medikament nach einem oder mehreren der Ansprüche 17 bis 27, **dadurch gekennzeichnet, dass**
R^{13a} stellt dar, ein Alkylradikal, das aus der Gruppe ausgewählt ist, die aus Methyl, Ethyl, n-Propyl, Isopropyl, n-Butyl, sec-Butyl, Isobutyl und tert-Butyl besteht, wobei das Alkylradikal mit 1, 2 oder 3 Substituent(en) substituiert sein kann, die unabhängig aus der Gruppe ausgewählt sind, die aus -OH, F, Cl, Br und -CN besteht;
oder ein Aryl oder Heteroarylradikal, das aus der Gruppe ausgewählt ist, die aus Phenyl, Naphthyl, Furyl (Furanyl), Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridazinyl, Pyrimidinyl, Pyridinyl, Pyrazinyl, Quinolinyl, Isoquinolinyl, Benzo[b]furanyl, Benzo[b]thiophenyl, Benzothiadiazolyl und Imidazo[2,1-b]thiazolyl besteht, wobei das Aryl oder Heteroaryl mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH,-NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ und -S(=O)₂-CH₃ besteht und/oder über eine -(CH₂)_{1,2} oder ₃-Gruppe gebunden sein kann;
bevorzugt stellt R^{13a} dar, ein Alkylradikal, das aus der Gruppe ausgewählt ist, die aus Methyl, Ethyl, n-Propyl, Isopropyl, n-Butyl, sec-Butyl, Isobutyl und tert-Butyl besteht;
oder ein Aryl oder Heteroarylradikal, das aus der Gruppe ausgewählt ist, die aus Phenyl und Thienyl (Thiophenyl) besteht, wobei das Aryl oder Heteroaryl mit 1 oder 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)--CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH,-NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ und -S(=O)₂-CH₃ besteht und/oder über eine -(CH₂)_{1,2} oder ₃-Gruppe gebunden sein kann;
mehr bevorzugt stellt R^{13a} ein Methylradikal oder ein Phenyl oder ein Thiophenylradikal dar, wobei das Phenyl oder Thiophenylradikal mit 1, 2 oder 3 Substituent(en) substituiert sein kann, die aus der Gruppe ausgewählt sind, die aus Methyl und Chlor besteht.

29. Medikament nach einem oder mehreren der Ansprüche 17 bis 28, **dadurch gekennzeichnet, dass**
R^{14a} stellt dar, ein Alkylradikal, das aus der Gruppe ausgewählt ist, die aus Methyl, Ethyl, n-Propyl, Isopropyl, n-Butyl, sec-Butyl, Isobutyl und tert-Butyl besteht;
oder ein Aryl oder Heteroarylradikal, das aus der Gruppe ausgewählt ist, die aus Phenyl, Naphthyl, Furyl (Furanyl), Thienyl (Thiophenyl), Pyrrolyl, Oxazolyl, Isoxazolyl, Thiazolyl, Isothiazolyl, Imidazolyl, Pyrazolyl, Oxadiazolyl, Thiadiazolyl, Triazolyl, Pyridazinyl, Pyrimidinyl, Pyridinyl, Pyrazinyl, Quinolinyl, Isoquinolinyl, Benzo[b]furanyl, Benzo[b]thiophenyl, Benzothiadiazolyl und Imidazo[2,1-b]thiazolyl besteht, wobei das Aryl oder Heteroaryl mit 1, 2, 3, 4 oder 5 Substituent(en) substituiert sein kann, die unabhängig aus der Gruppe ausgewählt sind, die aus C₁₋₅-Alkyl, -O-C₁₋₅-Alkyl, -S-C₁₋₅-Alkyl, -C(=O)-OH, -C(=O)-O-C₁₋₅-Alkyl, -O-C(=O)-C₁₋₅-Alkyl, F, Cl, Br, I, -CN, -CF₃, -OCF₃, -SCF₃, -OH, -SH, -NH₂, -NH(C₁₋₅-Alkyl), ₅-Alkyl)₂, -NO₂, -CHO, -CF₂H, -CFH₂, -C(=O)-NH₂, -C(=O)-NH(C₁₋₅-Alkyl), - C(=O)-N(C₁₋₅-Alkyl)₂, -S(=O)₂-C₁₋₅-Alkyl, -S(=O)₂-Phenyl, Phenyl, Phenoxy und Benzyl besteht und/oder über eine -(CH₂)_{1,2 oder 3}-Gruppe gebunden sein kann;
bevorzugt stellt R^{14a} dar, ein Alkylradikal, das aus der Gruppe ausgewählt ist, die aus Methyl, Ethyl, n-Propyl und Isopropyl besteht,
oder ein Phenylradikal, welches mit 1, 2 oder 3 Substituent(en) substituiert sein kann, die unabhängig aus der Gruppe ausgewählt sind, die aus Methyl, Ethyl, n-Propyl, Isopropyl, n-Butyl, tert-Butyl, sec-Butyl, Isobutyl, n-Pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)--CH₂-CH₂-CH₃, -C(=O)-CH(CH₃)₂, -C(=O)-C(CH₃)₃, F, Cl, Br, I, -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -CHO, -CF₂H, -CFH₂, -C(=O)-NH₂, -C(=O)-NH-CH₃, - C(=O)-NH-C₂H₅, -C(=O)-N(CH₃)₂, -C(=O)-N(C₂H₅)₂ und -S(=O)₂-CH₃ besteht;
mehr bevorzugt stellt R^{14a} ein Methylradikal oder ein Phenylradikal dar, welches mit 1, 2 oder 3 Methylradikal(en) substituiert sein kann.

30. Medikament nach einem oder mehreren der Ansprüche 17 bis 29, **dadurch gekennzeichnet, dass**
X^{a} eine -NR^{1a}R^{2a} funktionelle Gruppe oder eine -OR^{3a} funktionelle Gruppe darstellt;
R^{1a} stellt dar, ein Alkylradikal, das aus der Gruppe ausgewählt ist, die aus - CH₂-CH₂-OH und -CH₂-CH₂-CH₂-OH besteht;
ein unsubstituiertes Adamantylradikal;
ein unsubstituiertes Phenyl oder Pyrrolylradikal;
ein unsubstituiertes Naphtylradikal, welches über eine Alkylengruppe gebunden ist, die aus der Gruppe ausgewählt ist, die aus -CH₂-, -CH(CH₃)-, -CH₂-CH₂-, -CH₂-CH₂-CH₂ und -CH₂-CH₂-O- besteht;
ein Phenylradikal, welches mit 1, 2 oder 3 Substituent(en) substituiert sein kann, die unabhängig aus der Gruppe ausgewählt sind, die aus Methyl, Ethyl, tert-Butyl, Methoxy, F und Cl besteht und das Phenylradikal über eine Alkylengruppe gebunden ist, die aus der Gruppe ausgewählt ist, die aus - CH₂-, -CH(CH₃)-, -CH(Phenyl)-, -CH₂-CH₂, -CH₂-CH₂-CH₂- und -CH₂-CH₂-O-besteht;
ein Heteroarylradikal, das aus der Gruppe ausgewählt ist, die aus Pyridinyl, Furanyl und Pyrrolyl besteht, wobei das Pyridinyl, oder Pyrrolylradikal mit 1, 2 oder 3 Substituent(en) substituiert sein kann, die unabhängig aus der Gruppe ausgewählt sind, die aus Methyl, Ethyl, tert-Butyl, Methoxy, F und Cl besteht und das Pyridinyl, Furanyl oder Pyrrolylradikal über einer Alkylengruppe gebunden ist, die aus der Gruppe ausgewählt ist, die aus - CH₂-, -CH(CH₃)-, -CH₂-CH₂-, -CH₂-CH₂-CH₂- und -CH₂-CH₂-O- besteht;
oder eine -C(=O)=R^{12a} funktionelle Gruppe;
R^{2a} stellt dar, ein Wasserstoffatom oder ein Methylradikal;
oder
R^{1a} und R^{2a} bilden zusammen mit dem brückenbildenden Stickstoffatom eine funktionelle Gruppe, die aus der Gruppe ausgewählt ist, die aus 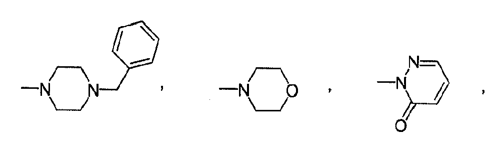 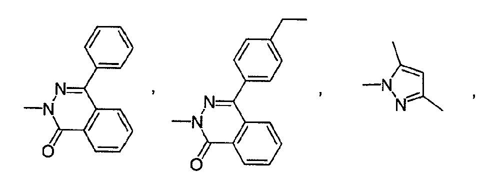 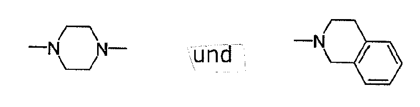 besteht;
R^{3a} stellt ein unsubstituiertes Phenylradikal dar;
R^{4a}, R^{5a} und R^{6a} stellen, identisch oder verschieden, jeweils ein Wasserstoffatom oder ein Fluoratom dar;
oder
R^{4a} und R^{5a} bilden zusammen mit den brückenbildenden Kohlenstoffatomen die folgende funktionelle Gruppe, 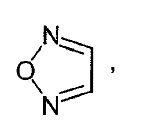 welche zusammen mit dem Phenylring, mit welchem Sie fusioniert ist, das folgende substituierte bizyklische aromatische Ringsystem bildet 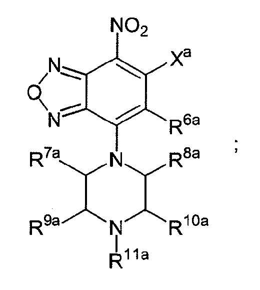 R^{7a} und R^{8a} jeweils ein Wasserstoffatom darstellen;
R^{9a} und R^{10a}, identisch oder verschieden, jeweils ein Wasserstoffatom oder ein Methylradikal darstellen;
R^{11a} stellt dar, ein Wasserstoffatom;
ein Alkylradikal, das aus der Gruppe ausgewählt ist, die aus Methyl, n-Butyl und -CH₂-CH₂-OH besteht;
ein unsubstituiertes Phenyl oder Pyridinylradikal, wobei das Phenyl oder Pyridinylradikal über eine -(CH₂)-Gruppe gebunden sein kann;
eine -C(=O)-R^{12a} funktionelle Gruppe oder eine -S(=O)₂-R^{13a} funktionelle Gruppe;
R^{12a} stellt ein Phenyl oder ein Thiophenylradikal dar, wobei das Phenyl oder Thiophenylradikal mit 1, 2 oder 3 Substituent(en) substituiert sein kann, die aus der Gruppe ausgewählt sind, die aus Methyl und Chlor besteht;
R^{13a} stellt ein Methylradikal oder ein Phenyl oder ein Thiophenylradikal dar, wobei das Phenyl oder Thiophenylradikal mit 1, 2 oder 3 Substituent(en) substituiert sein kann, die aus der Gruppe ausgewählt sind, die aus Methyl und Chlor besteht,
und
R^{14a} stellt ein Methylradikal oder ein Phenylradikal dar, welches mit 1, 2 oder 3 Methylradikal(en) substituiert sein kann;
optional in der Form eines seiner Steroisomere, bevorzugt Enatiomere oder Diasteromere, eines Racemats oder in Form von einer Mischung von zumindest zwei seiner Steroisomeren, bevorzugt Enatimoeren und/oder Diasteromeren, in irgendeinem Mischungsverhältnis, oder eines physiologisch akzeptablen Salzes davon, oder eines korrespondierenden Solvats davon.

31. Medikament nach einem oder mehreren der Ansprüche 17 bis 30, das zumindest eine Verbindung umfasst, die aus der Gruppe ausgewählt ist, die aus
[1] [5-(4-Methyl-piperazin-1-yl)-2-nitro-phenyl]-(2-pyrrol-1-yl-ethyl)-amin,
[2] (4-Fluoro-benzyl)-[5-(4-methyl-piperazin-1-yl)-2-nitro-phenyl]-amin,
[3] N-[5-(4-Methyl-piperazin-1-yl)-2-nitro-phenyl]-benzamid,
[4] N-Methyl-N-[5-(4-methyl-piperazin-1-yl)-2-nitro-phenyl]-benzamid,
[5] [5-(4-Methyl-piperazin-1-yl)-2-nitro-phenyl]-pyrrol-1-yl-amin,
[6] 2-[5-(4-Methyl-piperazin-1-yl)-2-nitro-phenyl]-2H-pyridazin-3-on,
[7] 1-Methyl-4-(4-nitro-3-phenoxy-phenyl)-piperazin,
[8] Benzyl-[5-(4-butyl-piperazin-1-yl)-2-nitro-phenyl]-amin,
[9] Benzyl-[2-nitro-5-(4-pyridin-2-yl-piperazin-1-yl)-phenyl]-amin und
[10] Benzyl-[2-nitro-5-(4-phenyl-2-yl-piperazin-1-yl)-phenyl]-amin,
[11] Furan-2-ylmethyl-[5-(4-methyl-piperazin-1-yl)-2-nitro-phenyl]-amin,
[12] 2-[4-(4-Nitro-3-phenethylamino-phenyl)-piperazin-1-yl]-ethanol,
[13] (2-Nitro-5-piperazin-1-yl-phenyl)-(2-o-tolyloxy-ethyl)-amin,
[14] 2-[4-(3-Benzylamino-4-nitro-phenyl)-piperazin-1-yl]-ethanol,
[15] 4-[5-(4-Methyl-piperazin-1-yl)-2-nitro-phenyl]-morpholin,
[16] 2-[5-(4-Methyl-piperazin-1-yl)-2-nitro-phenyl]-4-phenyl-2H-phthalazin-1-on,
[17] [2-(4-Chloro-phenoxy)-ethyl]-[5-(3,5-dimethyl-piperazin-1-yl)-2-nitro-phenyl]-amin,
[18] 2-[4-[3-(Benzhydryl-amino)-4-nitro-phenyl]-piperazin-1-yl]-ethanol,
[19] 4-[4-Fluoro-5-(4-methyl-piperazin-1-yl)-2-nitro-phenyl]-morpholin,
[20] 2-[2-Nitro-5-[4-(toluen-4-sulfonyl)-piperazin-1-yl]-phenyl]-1,2,3,4-tetrahydro-isoquinolin,
[21] 1-[3-(3,5-Dimethyl-pyrazol-1-yl)-4-nitro-phenyl]-4-methyl-piperazin,
[22] Benzyl-(2-nitro-5-piperazin-1-yl-phenyl)-amin,
[23] [5-(4-Methyl-piperazin-1-yl)-2-nitro-phenyl]-phenethyl-amin,
[24] [5-(4-Methyl-piperazin-1-yl)-2-nitro-phenyl]-pyridin-3-ylmethylamin,
[25] (3-Chloro-phenyl)-[4-[3-[(furanyl-2-ylmethyl) -amino]-4-nitrophenyl]-piperazin-1-yl]-methanon,
[26] (2-Nitro-5-piperazin-1-yl-phenyl)-pyridin-3-ylmethyl-amin,
[27] 1-Benzyl-4-(2-nitro-5-piperazin-1-yl-phenyl)-piperazin,
[28] Furan-2-ylmethyl-[5-(4-methansulfonyl-piperazin-1-yl)-2-nitrophenyl]-amin,
[29] Benzhydryl-[5-(4-methyl-piperazin-1-yl)-2-nitro-phenyl]-amin,
[30] (2-Nitro-5-piperazin-1-yl-phenyl)-(2-phenoxy-ethyl)-amin,
[31] 2-[5-(4-Methyl-piperazin-1-yl)-2-nitro-phenyl]-4-phenyl-2H-phthalazin-1-on,
[32] 1-[3-(3,5-Dimethyl-pyrazol-1-yl)-4-nitro-phenyl]-piperazin,
[33] (2-Nitro-5-piperazin-1-yl-phenyl)-phenethyl-amin,
[34] [5-(4-Benzensulfonyl-piperazin-1-yl)-2-nitro-phenyl]-furan-2-ylmethyl-amin,
[35] [2-(3,4-Dimethoxy-phenyl)-ethyl]-(2-nitro-5-piperazin-1-yl-phenyl)-amin,
[36] [4-[3-[(Furan-2-ylmethyl)-amino]-4-nitro-phenyl]-piperazin-1-yl]-m-tolyl-methanon,
[37] [4-[3-[(Furan-2-ylmethyl)-amino]-4-nitro-phenyl]-piperazin-1-yl]-phenyl-methanon,
[38] [4-[3-(3,5-Dimethyl-pyrazol-1-yl)-4-nitro-phenyl]-piperazin-1-yl]-thiophen-2-yl-methanon,
[39] 4-(4-Ethyl-phenyl)-2-[5-[4-methyl-piperazin-1-yl]-2-nitro-phenyl]-2H-phthalalazin-1-on,
[40] 3-[7-(4-Methyl-piperazin-1-yl)-4-nitro-benzo[1,2,5]oxadiazol-5-ylamino]-propan-1-ol,
[41] 3-[4-Nitro-7-(4-phenyl-piperazin-1-yl)-benzo[1,2,5]oxadiazol-5-ylamino]-ethan-1-ol,
[42] 3-[4-Nitro-7-(4-pyridin-piperazin-1-yl)-benzo[1,2,5]oxadiazol-5-ylamino]-propan-1-ol,
[43] [2-(3,4-Dimethoxy-phenyl)-ethyl]-[5-(4-methyl-piperazin-1-yl)-2-nitro-phenyl]-amin,
[44] [2-(3,4-Dimethyl-phenyl)-ethyl]-[5-(4-methyl-piperazin-1-yl)-2-nitro-phenyl]-amin,
[45] [2-(4-tert-butyl-phenoxy)-ethyl]-[5-(4-methyl-piperazin-1-yl)-2-nitro-phenyl]-amin,
[46] [2-(4-Methoxy-phenoxy)-ethyl]-[5-(4-methyl-piperazin-1-yl)-2-nitrophenyl]-amin,
[47] [5-(4-Methyl-piperazin-1-yl)-2-nitro-phenyl]-(2-m-tolyloxy-ethyl)-amin,
[48] [2-(4-Chloro-phenoxy)-ethyl]-[5-(4-methyl-piperazin-1-yl)-2-nitrophenyl]-amin,
[49] (2-Nitro-5-piperazin-1-yl-phenyl)-(1-phenyl-ethyl)-amin,
[50] [2-(3-Methoxy-phenoxy)-ethyl]-[5-(4-methyl-piperazin-1-yl)-2-nitrophenyl]-amin,
[51] [2-(2-Methoxy-phenoxy)-ethyl]-[5-(4-methyl-piperazin-1-yl)-2-nitrophenyl]-amin,
[52] (4-Chloro-benzyl)-(2-nitro-5-piperazin-1-yl-phenyl)-amin,
[53] Benzyl-[5-(4-benzyl-piperazin-1-yl)-2-nitro-phenyl]-amin,
[54] Benzyl-[5-(4-methyl-piperazin-1-yl)-2-nitro-phenyl]-amin,
[55] [5-(4-Methyl-piperazin-1-yl)-2-nitro-phenyl]-(2-o-tolyloxy-ethyl)-amin,
[56] (4-Chloro-benzyl)-[5-(4-methyl-piperazin-1-yl)-2-nitro-phenyl]-amin,
[57] Furan-2-ylmethyl-(2-nitro-5-piperazin-1-yl-phenyl)-amin,
[58] [2-(4-Chloro-phenoxy)-ethyl]-(2-nitro-5-piperazin-1-yl-phenyl)-amin,
[59] [5-(4-Methyl-piperazin-1-yl)-2-nitro-phenyl]-(1-phenyl-ethyl)-amin,
[60] 1-[4-(3-Benzylamino-4-nitro-phenyl)-piperazin-1-yl]-ethanon,
[61] 2-[4-[3-(4-Methylpiperazin-1-yl)-4-nitrophenyl]piperazin-1-yl]ethanol,
[62] 2-[4-[3-[2-(Naphthalen-2-yloxy)ethylamino]-4-nitrophenyl]piperazin-1-yl]ethanol,
[63] 2-[4-(3-{[1-(1-Adamantyl)ethyl]amino}-4-nitrophenyl)piperazin-1-yl]ethanol,
[64] 2-[4-[3-(3,4-Dimethoxyphenethylamino)-4-nitrophenyl]piperazin-1-yl]ethanol;
optional in Form von einem seiner Stereoisomere, bevorzugt Enantiomere oder Diasteromere, eines Racemats oder in Form von einer Mischung von zumindest zwei seiner Stereoisomeren, bevorzugt Enantiomeren und/oder Diastereomeren, in irgendeinem Mischungsverhältnis, oder eines physiologisch akzeptablen Salzes davon, oder eines korrespondierenden Solvats davon.

32. Medikament nach einem oder mehreren der Ansprüche 17 bis 31 für die Prophylaxe und/oder Behandlung von einer Störung oder Krankheit verbunden mit Nahrungsaufnahme, bevorzugt für die Regulierung des Appetits, für die Aufrechterhaltung, Zunahme oder Reduktion des Körpergewichts, für die Prophylaxe und/oder Behandlung von Fettleibigkeit, Bulimie, Anorexie, Kachexie, Typ II Diabetes (nicht Insulin-abhängige Diabetes Mellitus), bevorzugt Typ II Diabetes, die durch Fettleibigkeit verursacht ist; für die Prophylaxe und/oder Behandlung von Schlaganfall; Migräne; Schädeltrauma; Epilepsie; gereiztem Dickdarmsyndrom; Reizkolon; Störung des zentralen Nervensystems; Angst; Panikattacken; Depressionen; bipolare Störungen; Zwangsneurosen; kognitiven Störungen; kognitiven Dysfunktionen in Zusammenhang mit psychiatrischen Krankheiten; Gedächtnisstörungen; senile Demenz; Stimmungsstörungen; Schlafstörungen, Psychosen; neurodegenerativen Störungen, bevorzugt ausgewählt aus der Gruppe, die aus Morbus Alzheimer, Morbus Parkinson, Morbus Huntington und Multipler Sklerose besteht; Schizophrenie; chronische diskontinuierliche Hypoxie; Krämpfe; oder hyperaktive Störung (ADHD, Aufmerksamkeitsdefizit/hyperaktive Störung); zur Verbesserung von Kognition (kognitive Steigerung) oder kognitive Erinnerung (kognitive Erinnerungssteigerung); für die Prophylaxe und/oder Behandlung von Drogenabhängigkeit und/oder -entzug; für die Prophylaxe und/oder Behandlung von Alkoholabhängigkeit und/oder -entzug, für die Prophylaxe und/oder Behandlung von Nikotinabhängigkeit und/oder -entzug.

33. Verwendung zumindest einer Verbindung der allgemeinen Formel Ia nach einem oder mehreren der Ansprüche 17 bis 31 für die Herstellung eines Medikaments für die Prophylaxe und/oder Behandlung von einer Störung oder Krankheit verbunden mit Nahrungsaufnahme, bevorzugt für die Regulierung des Appetits; für die Aufrechterhaltung, Zunahme oder Reduktion des Körpergewichts; oder für die Prophylaxe und/oder Behandlung von Fettleibigkeit, Bulimie, Anorexie, Kachexie oder Typ II Diabetes (nicht Insulin-abhängige Diabetes Mellitus), mehr bevorzugt für die Prophylaxe und/oder Behandlung von Fettleibigkeit.

34. Verwendung zumindest einer Verbindung der allgemeinen Formel Ia nach einem oder mehreren der Ansprüche 17 bis 31 für die Herstellung eines Medikaments für die Prophylaxe und/oder Behandlung von Schlaganfall; Migräne; Schädeltrauma; Epilepsie; gereiztem Dickdarmsyndrom; Reizkolon; Störungen des zentralen Nervensystems; Angst; Panikattacken; Depression; bipolare Störungen; Zwangsneurosen; kognitiven Störungen; kognitiven Dysfunktionen in Zusammenhang mit psychiatrischen Störungen; Gedächtnisstörungen; seniler Demenz; Gefühlsstörungen; Schlafstörungen; Psychosen; neurodegenerativen Störungen, bevorzugt ausgewählt aus der Gruppe, die aus Morbus Alzheimer, Morbus Parkinson, Morbus Huntington und Multipler Sklerose besteht; Schizophrenie; chronischer diskontinuierlicher Hypoxie; Krämpfen; oder hyperaktiver Störung (ADHD, Aufmerksamdefizit/hyperaktive Störung).

35. Verwendung zumindest einer Verbindung der allgemeinen Formel Ia nach einem oder mehreren der Ansprüche 17 bis 31 für die Herstellung eines Medikaments für die Verbesserung der Kognition (Kognitionsteigerung) und/oder für die Verbesserung von kognitivem Gedächtnis (kognitive Gedächtnissteigerung).

36. Verwendung zumindest einer Verbindung der allgemeinen Formel Ia nach einem oder mehreren der Ansprüche 17 bis 31 für die Herstellung eines Medikaments für die Prophylaxe und/oder Behandlung von Drogenabhängigkeit und/oder -entzug, bevorzugt für die Prophylaxe und/oder Behandlung von Abhängigkeit und/oder Entzug in Zusammenhang mit einer oder mehreren Drogen, die aus der Gruppe ausgewählt sind, die aus Benzodiazepin, natürlichen-, semisynthetischen- oder synthetischen Opiaten, wie Kokain, Ethanol und/oder Nikotin, besteht.

## Revendications

1. Composé de phényl-pipérazine substitué par un nitro de formule générale I, 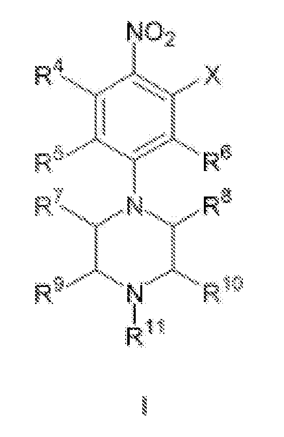 dans laquelle
X représente un groupe fonctionnel -NR¹R² ou un groupe fonctionnel -OR³ ;
R¹ représente un radical cycloaliphatique de 3 à 9 chaînons, saturé ou insaturé, qui peut être substitué par 1, 2 ou 3 substituants indépendamment choisis dans le groupe constitué des groupes oxo (=O), thioxo (=S), alkyle en C₁ à C₅, -O-alkyle en C₁ à C₅, -S-alkyle en C₁ à C₅, - C(=O)-OH, -C(=O)-alkyle en C₁ à C₅, -C(=O)-O-alkyle en C₁ à C₅, -OC(=O)-alkyle en C₁ à C₅, des atomes de F, CI, Br et I, et des groupes -CN, - CF₃, - OCF₃, -SCF₃, -OH, -SH, -NH₂, -NH(alkyle en C₁ à C₅), -N(alkyle en C₁ à C₅)₂, -NO₂, -CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle et qui peut contenir 1, 2 ou 3 hétéroatomes indépendamment choisis dans le groupe constitué des atomes d'azote, d'oxygène et de soufre en tant qu'élément(s) du noyau et qui est relié par l'intermédiaire d'un groupe alkylène en C₁ à C₆, alcénylène en C₂ à C₆ ou alkinylène en C₂ à C₆, linéaire ou ramifié, qui peut être non substitué ou substitué par 1, 2 ou 3 substituants indépendamment choisis dans le groupe constitué des atomes de F, CI, Br et I, et des groupes -CN, -CF₃, - OCF₃, -SCF₃, -OH, -SH, -NH₂, -O-alkyle en C₁ à C₅, -S-alkyle en C₁ à C₅, - NH-(alkyle en C₁ à C₅) et -N(alkyle en C₁ à C₅)₂ ;
à condition que R¹ ne représente pas un radical tétrahydrofuranyle ;
un radical hétéroaryle choisi dans le groupe constitué des groupes furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridinyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle qui peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, -C(=O)-OH, - C(=O)-alkyle en C₁ à C₅, -C(=O)-O-alkyle en C₁ à C₅, -O-C(=O)-alkyle en C₁ à C₅, des atomes de F, CI, Br et I, et des groupes -CN, -CF₃, -OCF₃, -SCF₃, - OH, -SH, -NH₂, -NH(alkyle en C₁ à C₅), -N(alkyle en C₁ à C₅)₂, -NO₂, -CHO, - CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle ;
un radical aryle ou hétéroaryle choisi dans le groupe constitué des groupes phényle, naphtyle, thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle, moyennant quoi ledit radical aryle ou hétéroaryle est relié par l'intermédiaire d'un groupe -(CH₂) et peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, - C(=O)-alkyle en C₁ à C₅, -C(=O)-O-alkyle en C₁ à C₅, -O-C(=O)-alkyle en C₁ à C₅, des atomes de F, Br et I, et des groupes -CN, -CF₃, -OCF₃, -SCF₃, -OH, -SH, -NH₂, -NH(alkyle en C₁ à C₅), -N(alkyle en C₁ à C₅)₂, -NO₂, -CHO, - CF₂H, -CFH₂, -C(=O)-NH₂, -C(=O)-NH(alkyle en C₁ à C₅), -C(=O)-N(alkyle en C₁ à C₅)2, -S(=O)₂-alkyle en C₁ à C₅, -S(=O)₂-phényle, phényle, phénoxy et benzyle ;
un radical aryle ou hétéroaryle choisi dans le groupe constitué des groupes naphtyle, furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridinyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle, moyennant quoi ledit radical aryle ou hétéroaryle est relié par l'intermédiaire d'un groupe -(CH₂)₂ et/ou peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, - C(=O)-OH, -C(=O)-alkyle en C₁ à C₅, -C(=O)-O-alkyle en C₁ à C₅, -OC(=O)-alkyle en C₁ à C₅, des atomes de F, Cl, Br et I, et des groupes -CN, - CF₃, -OCF₃, -SCF₃, -OH, -SH, -NH₂, -NH(alkyle en C₁ à C₅), -N(alkyle en C₁ à C₅)₂, -NO₂, -CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle ;
un radical aryle ou hétéroaryle choisi dans le groupe constitué des groupes phényle, naphtyle, furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridinyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle, moyennant quoi ledit radical aryle ou hétéroaryle est relié par l'intermédiaire d'un groupe -(CH₂)₃ et/ou peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, - C(=O)-OH, -C(=O)-alkyle en C₁ à C₅, -C(=O)-O-alkyle en C₁ à C₅, -OC(=O)-alkyle en C₁ à C₅, des atomes de F, Cl, Br et I, et des groupes -CN, - CF₃, -OCF₃, -SCF₃, -OH, -SH, -NH₂, -NH(alkyle en C₁ à C₅), -N(alkyle en C₁ à C₅)₂, -NO₂, -CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle ;
ou
un groupe fonctionnel -C(=O)-R¹² ;
R² représente un atome d'hydrogène ou un radical aliphatique en C₁ à C₁₀, linéaire ou ramifié, saturé ou insaturé, qui peut être non substitué ou substitué par 1, 2 ou 3 substituants indépendamment choisis dans le groupe constitué des atomes de F, Cl, Br et I, et des groupes -CN, -CF₃,-OCF₃, -SCF₃, -OH, -SH, -NH₂, -O-alkyle en C₁ à C₅, -S-alkyle en C₁ à C₅, - NH-(alkyle en C₁ à C₅) et -N(alkyle en C₁ à C₅)₂ ; ou
R¹ et R², conjointement avec l'atome d'azote de pontage, forment un noyau hétérocyclique de 3 à 6 chaînons saturé ou insaturé, mais pas aromatique, qui contient 1, 2 ou 3 hétéroatomes supplémentaires indépendamment choisis dans le groupe constitué des atomes d'azote et de soufre en tant qu'élément(s) du noyau et qui peut être substitué par 1, 2 ou 3 substituants indépendamment choisis dans le groupe constitué des groupes alkyle en C₁ à C₅, -O-alkyle en C₁ à C₅, -S-alkyle en C₁ à C₅, oxo (=O), thioxo (=S), -C(=O)-OH, -C(=O)-alkyle en C₁ à C₅, -C(=O)-O-alkyle en C₁ à C₅, -O-C(=O)-alkyle en C₁ à C₅, des atomes de F, Cl, Br et I, et des groupes -CN, -CF₃, -OCF₃, -SCF₃, -OH, -SH, -NH₂, -NH(alkyle en C₁ à C₅), - N(alkyle en C₁ à C₅)₂, -NO₂, -CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle,
R³ représente un radical aryle ou hétéroaryle de 5 à 10 chaînons, qui peut être non substitué ou qui peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, -C(=O)-OH, - C(=O)-alkyle en C₁ à C₅, -C(=O)-O-alkyle en C₁ à C₅, -O-C(=O)-alkyle en C₁ à C₅, des atomes de F, Cl, Br et I, et des groupes -CN, -CF₃, -OCF₃, -SCF₃,-OH, -SH, -NH₂, -NH(alkyle en C₁ à C₅), -N(alkyle en C₁ à C₅)₂, -NO₂, -CHO, - CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle et qui peut être relié par l'intermédiaire d'un groupe alkylène en C₁ à C₆, alcénylène en C₂ à C₆ ou alkinylène en C₂ à C₆, linéaire ou ramifié, qui peut être non substitué ou substitué par 1, 2 ou 3 substituants indépendamment choisis dans le groupe constitué des atomes de F, Cl, Br et I, et des groupes -CN, -CF₃, -OCF₃, -SCF₃, -OH, -SH, -NH₂, -O-alkyle en C₁ à C₅, -S-alkyle en C₁ à C₅, -NH-(alkyle en C₁ à C₅) et -N(alkyle en C₁ à C₅)₂ et moyennant quoi le radical hétéroaryle contient 1, 2 ou 3 hétéroatomes indépendamment choisis dans le groupe constitué des atomes d'azote, d'oxygène et de soufre en tant qu'élément(s) du noyau ;
R⁴, R⁵ et R⁶ représentent chacun un atome d'hydrogène ;
R⁷ et R⁸, identiques ou différents, représentent chacun un atome d'hydrogène ou un radical aliphatique en C₁ à C₁₀, linéaire ou ramifié, saturé ou insaturé, qui peut être non substitué ou substitué par 1, 2 ou 3 substituants indépendamment choisis dans le groupe constitué des atomes de F, Cl, Br et I, et des groupes -CN, -CF₃, -OCF₃, -SCF₃, -OH, -SH, -NH₂,-O-alkyle en C₁ à C₅, -S-alkyle en C₁ à C₅, -NH-(alkyle en C₁ à C₅) et - N(alkyle en C₁ à C₅)₂ ;
R⁹ et R¹⁰, identiques ou différents, représentent chacun un atome d'hydrogène ou un radical aliphatique en C₁ à C₁₀, linéaire ou ramifié, saturé ou insaturé, qui peut être non substitué ou substitué par 1, 2 ou 3 substituants indépendamment choisis dans le groupe constitué des atomes de F, Cl, Br et I, et des groupes -CN, -CF₃, -OCF₃, -SCF₃, -OH, -SH, -NH₂,-O-alkyle en C₁ à C₅, -S-alkyle en C₁ à C₅, -NH(alkyle en C₁ à C₅) et -N(alkyle en C₁ à C₅)₂ ;
R¹¹ représente un radical aliphatique non substitué en C₁ à C₁₀, linéaire ou ramifié, saturé ou insaturé ou un radical aryle ou hétéroaryle de 5 à 10 chaînons qui peut être non substitué ou qui peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, -C(=O)-OH, -C(=O)-alkyle en C₁ à C₅, -C(=O)-O-alkyle en C₁ à C₅, -O-C(=O)-alkyle en C₁ à C₅, des atomes de F, Cl, Br et I, et des groupes-CN, -CF₃, -OCF₃, -SCF₃, -OH, -SH, -NH₂, -NH(alkyle en C₁ à C₅), -N(alkyle en C₁ à C₅)₂, -NO₂, -CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle et moyennant quoi le radical hétéroaryle contient 1, 2 ou 3 hétéroatomes indépendamment choisis dans le groupe constitué des atomes d'azote, d'oxygène et de soufre en tant qu'élément(s) du noyau ;
à condition que R¹¹ ne représente pas un radical thiazolyle ;
et
R¹² représente un radical aryle de 5 à 10 chaînons qui peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, -C(=O)-OH, -C(=O)-alkyle en C₁ à C₅, -C(=O)-O-alkyle en C₁ à C₅, -O-C(=O)-alkyle en C₁ à C₅, des atomes de F, Br et I, et des groupes -CN, -CF₃, -OCF₃, -SCF₃, -SH, -NH₂, -NH(alkyle en C₁ à C₅), - N(alkyle en C₁ à C₅)₂, -CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, - S(=O)₂-phényle, phényle, phénoxy et benzyle,
un radical hétéroaryle choisi dans le groupe constitué des groupes furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridinyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle qui peut être non substitué ou qui peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, -C(=O)-OH, -C(=O)-alkyle en C₁ à C₅, -C(=O)-O-alkyle en C₁ à C₅, -O-C(=O)-alkyle en C₁ à C₅, des atomes de F, Cl, Br et I, et des groupes -CN, -CF₃, -OCF₃, -SCF₃, -OH, -SH, -NH₂, -NH(alkyle en C₁ à C₅), - N(alkyle en C₁ à C₅)₂, -NO₂, -CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle ;
ou
un radical aryle ou hétéroaryle choisi dans le groupe constitué des groupes phényle, naphtyle, furyle (furanyle), thiényle (thiophényle), pyrrolyle, oxazolyle, isoxazolyle, thiazolyle, isothiazolyle, pyrazolyle, oxadiazolyle, thiadiazolyle, triazolyle, pyridinyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle, qui peut être non substitué ou qui peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, -C(=O)-OH, - C(=O)-alkyle en C₁ à C₅, -C(=O)-O-alkyle en C₁ à C₅, -O-C(=O)-alkyle en C₁ à C₅, des atomes de F, Cl, Br et I, et des groupes -CN, -CF₃, -OCF₃, -SCF₃,-OH, -SH, -NH₂, -NH(alkyle en C₁ à C₅), -N(alkyle en C₁ à C₅)₂, -NO₂, -CHO, - CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle et qui est relié par l'intermédiaire d'un groupe -(CH₂)_{1,2 ou 3} ;
à condition que le composé suivant
(1) Benzyl-[5-(4-méthyl-pipérazin-1-yl)-2-nitro-phényl]-amine
soit exclu ;
optionnellement sous la forme de l'un de ses stéréoisomères, de préférence des énantiomères ou des diastéréomères, d'un racémate ou sous la forme d'un mélange d'au moins deux de ses stéréoisomères, de préférence des énantiomères et/ou des diastéréomères, dans n'importe quel rapport de mélange, ou d'un sel physiologiquement acceptable de celui-ci, ou d'un solvate correspondant de celui-ci.

2. Composé selon la revendication 1, **caractérisé en ce que**
X représente un groupe fonctionnel -NR¹R² ou un groupe fonctionnel -OR³ ;
R¹ représente un radical (hétéro)cycloaliphatique choisi dans le groupe constitué des groupes cyclopropyle, cyclobutyle, cyclopentyle, cyclopentényle, cyclohexyle, cyclohexényle, cycloheptyle, cycloheptényle, cyclooctyle, cyclooctényle, cyclononyle, imidazolidinyle, aziridinyle, azétidinyle, pyrrolidinyle, pipéridinyle, morpholinyle, thiomorpholinyle, pipérazinyle, pyrazolidinyle, tétrahydrothiophényle et azépanyle, qui peut être substitué par 1, 2 ou 3 substituants indépendamment choisis dans le groupe constitué des groupes oxo (=O), thioxo (=S), alkyle en C₁ à C₅, -O-alkyle en C₁ à C₅, -S-alkyle en C₁ à C₅, -C(=O)-OH, -C(=O)-alkyle en C₁ à C₅, -C(=O)-O-alkyle en C₁ à C₅, -O-C(=O)-alkyle en C₁ à C₅, des atomes de F, Cl, Br et I, et des groupes -CN, -CF₃, -OCF₃, -SCF₃, -OH, -SH, -NH₂,-NH(alkyle en C₁ à C₅), -N(alkyle en C₁ à C₅)₂, -NO₂, -CHO, -CF₂H, -CFH₂, - C(=O)-NH₂, -C(=O)-NH(alkyle en C₁ à C₅), -C(=O)-N(alkyle en C₁ à C₅)₂, - S(=O)₂-alkyle en C₁ à C₅, -S(=O)₂-phényle, phényle, phénoxy et benzyle et qui est relié par l'intermédiaire d'un groupe alkylène en C₁ à C₆, alcénylène en C₂ à C₆ ou alcynylène en C₂ à C₆, linéaire ou ramifié, qui peut être non substitué ou substitué par 1, 2 ou 3 substituants indépendamment choisis dans le groupe constitué des atomes de F, Cl, Br et I, et des groupes -CN,-CF₃, -OCF₃, -SCF₃, -OH, -SH, -NH₂, -O-alkyle en C₁ à C₅, -S-alkyle en C₁ à C₅, -NH(alkyle en C₁ à C₅) et -N(alkyle en C₁ à C₅)₂ ;
un radical hétéroaryle choisi dans le groupe constitué des groupes furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridinyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle qui peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, -C(=O)-OH, - C(=O)-alkyle en C₁ à C₅, -C(=O)-O-alkyle en C₁ à C₅, -O-C(=O)-alkyle en C₁ à C₅, des atomes de F, Cl, Br et I, et des groupes -CN, -CF₃, -OCF₃, -SCF₃,-OH, -SH, -NH₂, -NH(alkyle en C₁ à C₅), -N(alkyle en C₁ à C₅)₂, -NO₂, -CHO, - CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle ;
un radical aryle ou hétéroaryle choisi dans le groupe constitué des groupes phényle, naphtyle, thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle, moyennant quoi ledit radical aryle ou hétéroaryle est relié par l'intermédiaire d'un groupe -(CH₂) et peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, - C(=O)-alkyle en C₁ à C₅, -C(=O)-O-alkyle en C₁ à C₅, -O-C(=O)-alkyle en C₁ à C₅, des atomes de F, Br et I, et des groupes -CN, -CF₃, -OCF₃, -SCF₃, -OH, -SH, -NH₂, -NH(alkyle en C₁ à C₅), -N(alkyle en C₁ à C₅)₂, -NO₂, -CHO, - CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle ;
un radical aryle ou hétéroaryle choisi dans le groupe constitué des groupes naphtyle, furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridinyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle, moyennant quoi ledit radical aryle ou hétéroaryle est relié par l'intermédiaire d'un groupe -(CH₂)₂ et/ou peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, - C(=O)-OH, -C(=O)-alkyle en C₁ à C₅, -C(=O)-O-alkyle en C₁ à C₅, -OC(=O)-alkyle en C₁ à C₅, des atomes de F, Cl, Br et I, et des groupes -CN, - CF₃, -OCF₃, -SCF₃, -OH, -SH, -NH₂, -NH(alkyle en C₁ à C₅), -N(alkyle en C₁ à C₅)₂, -NO₂, -CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle;
un radical aryle ou hétéroaryle choisi dans le groupe constitué des groupes phényle, naphtyle, furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridinyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle, moyennant quoi ledit radical aryle ou hétéroaryle est relié par l'intermédiaire d'un groupe -(CH₂)₃ et/ou peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, - C(=O)-O_{H}, -C(=O)-alkyle en C₁ à C₅, -C(=O)-O-alkyle en C₁ à C₅, -OC(=O)-alkyle en C₁ à C₅, des atomes de F, Cl, Br et I, et des groupes -CN,-CF₃, -OCF₃, -SCF₃, -OH, -SH, -NH₂, -NH(alkyle en C₁ à C₅), -N(alkyle en C₁ à C₅)₂, -NO₂, -CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle;
ou
un groupe fonctionnel -C(=O)-R¹²;
R² représente un atome d'hydrogène ou un radical aliphatique en C₁ à C₁₀, linéaire ou ramifié, saturé ou insaturé, qui peut être non substitué ou substitué par 1, 2 ou 3 substituants indépendamment choisis dans le groupe constitué des atomes de F, Cl, Br et I, et des groupes -CN, -CF₃,-OCF₃, -SCF₃, -OH, -SH, -NH₂, -O-alkyle en C₁ à C₅, -S-alkyle en C₁ à C₅, - NH-(alkyle en C₁ à C₅) et -N(alkyle en C₁ à C₅)₂; ou
R¹ et R², conjointement avec l'atome d'azote de pontage, forment un noyau hétérocyclique de 3 à 6 chaînons saturé ou insaturé, mais pas aromatique, qui contient 1, 2 ou 3 hétéroatomes supplémentaires indépendamment choisis dans le groupe constitué des atomes d'azote et de soufre en tant qu'élément(s) du noyau et qui peut être substitué par 1, 2 ou 3 substituants indépendamment choisis dans le groupe constitué des groupes alkyle en C₁ à C₅, -O-alkyle en C₁ à C₅, -S-alkyle en C₁ à C₅, oxo (=O), thioxo (=S), -C(=O)-OH, -C(=O)-alkyle en C₁ à C₅, -C(=O)-O-alkyle en C₁ à C₅, -O-C(=O)-alkyle en C₁ à C₅, des atomes de F, Cl, Br et I, et des groupes -CN, -CF₃, -OCF₃, -SCF₃, -OH, -SH, -NH₂, -NH(alkyle en C₁ à C₅), - N(alkyle en C₁ à C₅)₂, -NO₂, -CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=0)₂-phényle, phényle, phénoxy et benzyle;
R³ représente un radical aryle ou hétéroaryle de 5 à 10 chaînons, qui peut être non substitué ou qui peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, -CC =O)-OH, - C(=O)-alkyle en C₁ à C₅, -C(=O)-O-alkyle en C₁ à C₅, -O-C(=O)-alkyle en C₁ à C₅, des atomes de F, Cl, Br et I, et des groupes -CN, -CF₃, -OCF₃, -SCF₃,-OH, -SH, -NH₂, -NH(alkyle en C₁ à C₅), -N(alkyle en C₁ à C₅)₂, -NO₂, -CHO, - CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle et qui peut être relié par l'intermédiaire d'un groupe alkylène en C₁ à C₆, alcénylène en C₂ à C₆ ou alkinylène en C₂ à C₆, linéaire ou ramifié, qui peut être non substitué ou substitué par 1, 2 ou 3 substituants indépendamment choisis dans le groupe constitué des atomes de F, Cl, Br et I, et des groupes -CN, -CF₃, -OCF₃, -SCF₃, -OH, -SH, -NH₂, -O-alkyle en C₁ à C₅, -S-alkyle en C₁ à C₅, -NH-(alkyle en C₁ à C₅) et -N(alkyle en C₁ à C₅)₂ et moyennant quoi le radical hétéroaryle contient 1, 2 ou 3 hétéroatomes indépendamment choisis dans le groupe constitué des atomes d'azote, d'oxygène et de soufre en tant qu'élément(s) du noyau,
R⁴, R⁵ et R⁶ représentent chacun un atome d'hydrogène ;
R⁷ et R⁸, identiques ou différents, représentent chacun un atome d'hydrogène ou un radical aliphatique en C₁ à C₁₀, linéaire ou ramifié, saturé ou insaturé, qui peut être non substitué ou substitué par 1, 2 ou 3 substituants indépendamment choisis dans le groupe constitué des atomes de F, Cl, Br et I, et des groupes -CN, -CF₃, -OCF₃, -SCF₃, -OH, -SH, -NH₂,-O-alkyle en C₁ à C₅, -S-alkyle en C₁ à C₅, -NH-(alkyle en C₁ à C₅) et-N(alkyle en C₁ à C₅)₂ ;
R⁹ et R¹⁰, identiques ou différents, représentent chacun un atome d'hydrogène ou un radical aliphatique en C₁ à C₁₀, linéaire ou ramifié, saturé ou insaturé, qui peut être non substitué ou substitué par 1, 2 ou 3 substituants indépendamment choisis dans le groupe constitué des atomes de F, Cl, Br et I, et des groupes -CN, -CF₃, -OCF₃, -SCF₃, -OH, -SH, -NH₂,-O-alkyle en C₁ à C₅, -S-alkyle en C₁ à C₅, -NH(alkyle en C₁ à C₅) et -N(alkyle en C₁ à C₅)₂ ;
R¹¹ représente un radical aliphatique non substitué en C₁ à C₁₀, linéaire ou ramifié, saturé ou insaturé ou un radical aryle ou hétéroaryle choisi dans le groupe constitué des groupes phényle, naphtyle, furyle (furanyle), thiényle (thiophényle), pyrrolyle, oxazolyle, isoxazolyle, isothiazolyle, imidazolyle, pyrazolyle, oxadiazolyle, thiadiazolyle, triazolyle, pyridazinyle, pyrimidinyle, pyridinyle, pyrazinyle, quinolinyle, isoquinolinyle, benzo[b]furanyle, benzo[b]thiophényle, benzothiadiazolyle et imidazo[2,1-b]thiazolyle, qui peut être non substitué ou qui peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅,-C(=O)-OH, -C(=O)-alkyle en C₁ à C₅, -C(=O)-O-alkyle en C₁ à C₅, -OC(=O)-alkyle en C₁ à C₅, des atomes de F, Cl, Br et I, et des groupes -CN,-CF₃, -OCF₃, -SCF₃, -OH, -SH, -NH₂, -NH(alkyle en C₁ à C₅), -N(alkyle en C₁ à C₅)₂, -NO₂, -CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(O)₂-phényle, phényle, phénoxy et benzyle;
et
R¹² représente un radical aryle de 5 à 10 chaînons qui peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, -C(=O)-OH, -C(=O)-alkyle en C₁ à C₅,-C(=O)-O-alkyle en C₁ à C₅, -O-C(=O)-alkyle en C₁ à C₅, des atomes de F, Br et I, et des groupes -CN, -CF₃, -OCF₃, -SCF₃, -SH, -NH₂, -NH(alkyle en C₁ à C₅), - N(alkyle en C₁ à C₅)₂, -CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, - S(=O)₂-phényle, phényle, phénoxy et benzyle;
un radical hétéroaryle choisi dans le groupe constitué des groupes furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridinyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle qui peut être non substitué ou qui peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, -C(=O)-OH, -C(=O)-alkyle en C₁ à C₅, -C(=O)-O-alkyle en C₁ à C₅, -O-C(=O)-alkyle en C₁ à C₅, des atomes de F, Cl, Br et I, et des groupes -CN, -CF₃, -OCF₃, -SCF₃, -OH, -SH, -NH₂, -NH(alkyle en C₁ à C₅), - N(alkyle en C₁ à C₅)₂, -NO₂, -CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=0)₂-phényle, phényle, phénoxy et benzyle;
ou
un radical aryle ou hétéroaryle choisi dans le groupe constitué des groupes phényle, naphtyle, furyle (furanyle), thiényle (thiophényle), pyrrolyle, oxazolyle, isoxazolyle, thiazolyle, isothiazolyle, pyrazolyle, oxadiazolyle, thiadiazolyle, triazolyle, pyridinyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle, qui peut être non substitué ou qui peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, -C(=O)-OH, - C(=O)-alkyle en C₁ à C₅, -C(=O)-O-alkyle en C₁ à C₅, -O-C(=O)-alkyle en C₁ à C₅, des atomes de F, Cl, Br et I, et des groupes -CN, -CF₃, -OCF₃, -SCF₃,-OH, -SH, -NH₂, -NH(alkyle en C₁ à C₅), -N(alkyle en C₁ à C₅)₂, -NO₂, -CHO, - CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle et qui est relié par l'intermédiaire d'un groupe -(CH₂)_{1,2 ou 3} ;
optionnellement sous la forme de l'un de ses stéréoisomères, de préférence des énantiomères ou des diastéréomères, d'un racémate ou sous la forme d'un mélange d'au moins deux de ses stéréoisomères, de préférence des énantiomères et/ou des diastéréomères, dans n'importe quel rapport de mélange, ou d'un sel physiologiquement acceptable de celui-ci, ou d'un solvate correspondant de celui-ci.

3. Composé selon la revendication 1 ou 2, **caractérisé en ce que** R¹ représente
un radical (hétéro)cycloaliphatique choisi dans le groupe constitué des groupes cyclopropyle, cyclobutyle, cyclopentyle, cyclopentényle, cyclohexyle, cyclohexényle, cycloheptyle, cycloheptényle, cyclooctyle, cyclooctényle, cyclononyle, imidazolidinyle, aziridinyle, azétidinyle, pyrrolidinyle, pipéridinyle, morpholinyle, thiomorpholinyle, pipérazinyle, pyrazolidinyle, tétrahydrothiophényle et azépanyle, moyennant quoi ledit radical (hétéro)cycloaliphatique peut être relié par l'intermédiaire d'un groupe -(CH₂)_{1, 2 ou 3} et/ou peut être substitué par 1, 2 ou 3 substituants indépendamment choisis dans le groupe constitué des groupes oxo (=O), thioxo (=S), méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, des atomes de F, CI, Br et I, et des groupes -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ et -S(=O)₂-CH₃,
un radical hétéroaryle choisi dans le groupe constitué des groupes furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridinyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle, moyennant quoi ledit radical hétéroaryle peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, - S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, des atomes de F, CI, Br et I, et des groupes -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ et -S(=O)₂-CH₃,
un radical aryle ou hétéroaryle choisi dans le groupe constitué des groupes phényle, naphtyle, thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle, moyennant quoi ledit radical aryle ou hétéroaryle est relié par l'intermédiaire d'un groupe -(CH₂) et/ou peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, des atomes de F, Br et I, et des groupes -CN, -CF₃, -OCF₃, -SCF₃, -OH, -SH, -NH₂, -NH-CH₃, -NH-C₂H₅, - NH-CH₂-CH₂-CH₃, -NH-CH(CH₃)₂, -NH-C(CH₃)₃, -N(CH₃)₂, -N(C₂H₅)₂, -NO₂, - CHO, -CF₂H, -CFH₂, -C(=O)-NH₂, -C(=O)-NH-CH₃, -C(=O)-NH-C₂H₅, - C(=O)-N(CH₃)₂, -C(=O)-N(C₂H₅)₂ et -S(=O)₂-CH₃,
un radical aryle ou hétéroaryle choisi dans le groupe constitué des groupes naphtyle, furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridinyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle, moyennant quoi ledit radical aryle ou hétéroaryle est relié par l'intermédiaire d'un groupe -(CH₂)₂ et/ou peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -OC(CH₃)₃, ₋S-CH₃, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, des atomes de F, CI, Br et I, et des groupes -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - NO₂, -CHO, -CF₂H, -CFH₂, -C(=O)-NH₂, -C(=O)-NH-CH₃, -C(=O)-NH-C₂H₅, - C(=O)-N(CH₃)₂, -C(=O)-N(C₂H₅)₂ et -S(=O)₂-CH₃,
un radical aryle ou hétéroaryle choisi dans le groupe constitué des groupes phényle, naphtyle, furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridinyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle, moyennant quoi ledit radical aryle ou hétéroaryle est relié par l'intermédiaire d'un groupe -(CH₂)₃ et/ou peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -OC(CH₃)₃, -S-CH₃, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, des atomes de F, CI, Br et I, et des groupes -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - NO₂, -CHO, -CF₂H, -CFH₂, -C(=O)-NH₂, -C(=O)-NH-CH₃, -C(=O)-NH-C₂H₅, - C(=O)-N(CH₃)₂, -C(=O)-N(C₂H₅)₂ et -S(=O)₂-CH₃ ;
ou
un groupe fonctionnel -C(=O)-R¹² ;
de préférence R¹ représente
un radical pyrrolyle non substitué ;
un radical aryle ou hétéroaryle choisi dans le groupe constitué des groupes phényle et pyrrolyle, moyennant quoi ledit radical phényle est relié par l'intermédiaire d'un groupe -(CH₂) et ledit radical pyrrolyle est relié par l'intermédiaire d'un groupe -(CH₂)₂ et/ou ledit radical phényle ou pyrrolyle peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, - S-CH(CH₃)₂, -S-C(CH₃)₃, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, des atomes de F, Br et I, et des groupes -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ et -S(=O)₂-CH₃ ; ou
un groupe fonctionnel -C(=O)-R¹² ;
plus préférablement R¹ représente un radical pyrrolyle non substitué ;
un radical aryle ou hétéroaryle choisi dans le groupe constitué des groupes phényle et pyrrolyle, moyennant quoi ledit radical phényle est relié par l'intermédiaire d'un groupe -(CH₂) et ledit radical pyrrolyle est relié par l'intermédiaire d'un groupe -(CH₂)₂ et/ou ledit radical phényle ou pyrrolyle peut être substitué par 1, 2 ou 3 substituants indépendamment choisis dans le groupe constitué des groupes méthyle, éthyle, n-propyle, isopropyle, n-butyle, tert-butyle, sec-butyle, isobutyle et n-pentyle, et des atomes de F, Br et I ou
un groupe fonctionnel -C(=O)-R¹².

4. Composé selon une ou plusieurs des revendications 1 à 3, **caractérisé en ce que**
R² représente un atome d'hydrogène ou un radical alkyle non substitué en C₁ à C₁₀, linéaire ou ramifié ;
de préférence R² représente un atome d'hydrogène ou un radical alkyle choisi dans le groupe constitué des groupes méthyle, éthyle, n-propyle, isopropyle, n-butyle, sec-butyle, isobutyle et tert-butyle ;
plus préférablement R² représente un atome d'hydrogène ou un radical méthyle.

5. Composé selon une ou plusieurs des revendications 1 à 4, **caractérisé en ce que**
R¹ et R², conjointement avec l'atome d'azote de pontage, forment un groupe fonctionnel choisi dans le groupe constitué des groupes 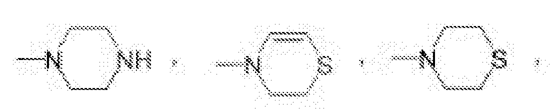 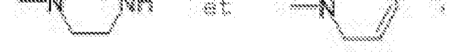 moyennant quoi chacun de ces groupes fonctionnels cycliques mentionnés ci-dessus peut être substitué au niveau de n'importe quelle position y compris les groupes -NH par 1, 2 ou 3 substituants indépendamment choisis dans le groupe constitué des groupes oxo (=O), thioxo (=S), méthyle, éthyle, n-propyle, isopropyle, n-butyle, tert-butyle, sec-butyle, isobutyle, n-pentyle, -O-CH₃, -O-C₂H₅, -O-OH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, des atomes de F, Cl, Br et I, et des groupes -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=0)-N(CH₃)₂, -C(=O)-N(C₂H₅)₂, -S(=O)₂-CH₃, -S(=O)₂-phényle, phényle, phénoxy et benzyle ;
de préférence R¹ et R², conjointement avec l'atome d'azote de pontage, forment un groupe fonctionnel pyridazin-3-one qui peut être substitué par 1, 2 ou 3 substituants indépendamment choisis dans le groupe constitué des groupes méthyle, éthyle, n-propyle, isopropyle, n-butyle, tert-butyle, -O-CH₃, -O-C₂H₅, -C(=O)-OH, -C(=O)-O-CH₃, -C(=O)-O-C₂H₅, - C(=O)-CH₃, -C(=O)-C₂H₅, des atomes de F, Cl, Br et I, et des groupes -CN, -CF₃, -OCF₃, -SCF₃, -OH, -SH, -NH₂, -NH-CH₃, -NH-C₂H₅, -NO₂, -CHO, -CF₂H, -CFH₂, -C(=O)-NH₂, phényle, phénoxy et benzyle ;
plus préférablement R¹ et R², conjointement avec l'atome d'azote de pontage, forment un groupe fonctionnel pyridazin-3-one non substitué.

6. Composé selon une ou plusieurs des revendications 1 à 5, **caractérisé en ce que**
R³ représente un radical aryle ou hétéroaryle choisi dans le groupe constitué des groupes phényle, naphtyle, furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridinyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle, moyennant quoi ledit radical aryle ou hétéroaryle peut être relié par l'intermédiaire d'un groupe -(CH₂)_{1, 2 ou 3} et/ou peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, des atomes de F, CI, Br et I, et des groupes -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ et -S(=O)₂-CH₃ ;
de préférence R³ représente un radical phényle qui peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, - S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, des atomes de F, CI, Br et I, et des groupes -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ et -S(=O)₂-CH₃ ;
plus préférablement R³ représente un radical phényle non substitué.

7. Composé selon une ou plusieurs des revendications 1 à 6, **caractérisé en ce que**
R⁷ et R⁸, identiques ou différents, représentent chacun un atome d'hydrogène ou un radical alkyle non substitué en C₁ à C₁₀, linéaire ou ramifié ;
de préférence R⁷ et R⁸, identiques ou différents, représentent chacun un atome d'hydrogène ou un radical alkyle choisi dans le groupe constitué des groupes méthyle, éthyle, n-propyle, isopropyle, n-butyle, sec-butyle, isobutyle et tert-butyle ;
plus préférablement R⁷ et R⁸ représentent chacun un atome d'hydrogène.

8. Composé selon une ou plusieurs des revendications 1 à 7, **caractérisé en ce que** R⁹ et R¹⁰, identiques ou différents, représentent chacun un atome d'hydrogène ou un radical alkyle non substitué en C₁ à C₁₀, linéaire ou ramifié ;
de préférence R⁹ et R¹⁰, identiques ou différents, représentent chacun un atome d'hydrogène ou un radical alkyle choisi dans le groupe constitué des groupes méthyle, éthyle, n-propyle, isopropyle, n-butyle, sec-butyle, isobutyle et tert-butyle ;
plus préférablement R⁹ et R¹⁰ représentent chacun un atome d'hydrogène.

9. Composé selon une ou plusieurs des revendications 1 à 8, **caractérisé en ce que**
R¹¹ représente un radical alkyle choisi dans le groupe constitué des groupes méthyle, éthyle, n-propyle, isopropyle, n-butyle, sec-butyle, isobutyle et tert-butyle ; ou un radical aryle ou hétéroaryle choisi dans le groupe constitué des groupes phényle, naphtyle, furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridazinyle, pyrimidinyle, pyridinyle, pyrazinyle, quinolinyle, isoquinolinyle, benzo[b]furanyle, benzo[b]thiophényle, benzothiadiazolyle et imidazo[2,1-b]thiazolyle, moyennant quoi
ledit groupe aryle ou hétéroaryle peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -OC(CH₃)₃, -S-CH₃, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, des atomes de F, CI, Br et I, et des groupes -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - NO₂, -CHO, -CF₂H, -CFH₂, -C(=O)-NH₂, -C(=O)-NH-CH₃, -C(=O)-NH-C₂H₅, - C(=O)-N(CH₃)₂, -C(=O)-N(C₂H₅)₂ et -S(=O)₂-CH₃ ;
de préférence R¹¹ représente un radical alkyle choisi dans le groupe constitué des groupes méthyle, éthyle, n-propyle, isopropyle et n-butyle ; ou un radical aryle ou hétéroaryle choisi dans le groupe constitué des groupes phényle et pyridinyle, moyennant quoi ledit groupe aryle ou hétéroaryle peut être substitué par 1, 2 ou 3 substituants indépendamment choisis dans le groupe constitué des groupes méthyle, éthyle, n-propyle, isopropyle, n-butyle, tert-butyle, -O-CH₃, -O-C₂H₅, -C(=O)-OH, -C(=O)-O-CH₃, -C(=O)-O-C₂H₅, des atomes de F, Cl, Br et I, et des groupes -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, - C(=O)-NH₂, -C(=O)-NH-CH₃, -C(=O)-NH-C₂H₅, -C(=O)-N(CH₃)₂, -C(=O)-N(C₂H₅)₂ et -S(=O)₂-CH₃ ;
plus préférablement R¹¹ représente un radical alkyle choisi dans le groupe constitué des groupes méthyle, éthyle, n-propyle et n-butyle ; ou un radical phényle ou pyridinyle non substitué.

10. Composé selon une ou plusieurs des revendications 1 à 9, **caractérisé en ce que**
R¹² représente un radical aryle de 5 à 10 chaînons qui peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, - S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, des atomes de F, Br et I, et des groupes -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - CHO, -CF₂H, -CFH₂, -C(=O)-NH₂, -C(=O)-NH-CH₃, -C(=O)-NH-C₂H₅, - C(=O)-N(CH₃)₂, -C(=O)-N(C₂H₅)₂ et -S(=O)₂-CH₃ ;
un radical hétéroaryle choisi dans le groupe constitué des groupes furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridinyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle, moyennant quoi ledit radical hétéroaryle peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, - S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, des atomes de F, CI, Br et I, et des groupes -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ et -S(=O)₂-CH₃ ;
ou
un radical aryle ou hétéroaryle choisi dans le groupe constitué des groupes phényle, naphtyle, furyle (furanyle), thiényle (thiophényle), pyrrolyle, oxazolyle, isoxazolyle, thiazolyle, isothiazolyle, pyrazolyle, oxadiazolyle, thiadiazolyle, triazolyle, pyridinyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle, moyennant quoi ledit radical aryle ou hétéroaryle est relié par l'intermédiaire d'un groupe -(CH₂)₁, _{2 ou 3} et/ou peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, des atomes de F, CI, Br et I, et des groupes -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ et -S(=O)₂-CH₃ ;
de préférence R¹² représente un radical phényle qui peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, - S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, des atomes de F, Br et I, et des groupes -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - CHO, -CF₂H, -CFH₂, -C(=O)-NH₂, -C(=O)-NH-CH₃, -C(=O)-NH-C₂H₅, - C(=O)-N(CH₃)₂, -C(=O)-N(C₂H₅)₂ et -S(=O)₂-CH₃ ;
plus préférablement R¹² représente un radical phényle non substitué.

11. Composé selon une ou plusieurs des revendications 1 à 10, **caractérisé en ce que**
X représente un groupe fonctionnel -NR¹R² ou un groupe fonctionnel - OR³ ;
R¹ représente un radical pyrrolyle non substitué ; un radical aryle ou hétéroaryle choisi dans le groupe constitué des groupes phényle et pyrrolyle, moyennant quoi ledit radical phényle est relié par l'intermédiaire d'un groupe -(CH₂) et ledit radical pyrrolyle est relié par l'intermédiaire d'un groupe -(CH₂)₂ et/ou ledit radical phényle ou pyrrolyle peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, des atomes de F, Br et I, et des groupes -CN, -CF₃, -OCF₃, -SCF₃, -OH, -SH, -NH₂, -NH-CH₃, -NH-C₂H₅, - NH-CH₂-CH₂-CH₃, -NH-CH(CH₃)₂, -NH-C(CH₃)₃, -N(CH₃)₂, -N(C₂H₅)₂, -NO₂, - CHO, -CF₂H, -CFH₂, -C(=O)-NH₂, -C(=O)-NH-CH₃, -C(=O)-NH-C₂H₅, - C(=O)-N(CH₃)₂, -C(=O)-N(C₂H₅)₂ et -S(=O)₂-CH₃ ou un groupe fonctionnel - C(=O)-R¹² ;
R² représente un atome d'hydrogène ou un radical alkyle choisi dans le groupe constitué des groupes méthyle, éthyle, n-propyle, isopropyle, n-butyle, sec-butyle, isobutyle et tert-butyle ;
ou
R¹ et R², conjointement avec l'atome d'azote de pontage, forment un groupe fonctionnel pyridazin-3-one qui peut être substitué par 1, 2 ou 3 substituants indépendamment choisis dans le groupe constitué des groupes méthyle, éthyle, n-propyle, isopropyle, n-butyle, tert-butyle, -O-CH₃, -OC₂H₅, -C(=O)-OH, -C(=O)-O-CH₃, -C(=O)-O-C₂H₅, -C(=O)-CH₃, -C(=O)-C₂H₅, des atomes de F, Cl, Br et I, et des groupes -CN, -CF₃, -OCF₃, -SCF₃, - OH, -SH, -NH₂, -NH-CH₃, -NH-C₂H₅, -NO₂, -CHO, -CF₂H, -CFH₂, -C(=O)-NH₂, phényle, phénoxy et benzyle ;
R³ représente un radical phényle qui peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -OC(CH₃)₃, -S-CH₃, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, des atomes de F, CI, Br et I, et des groupes -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - NO₂, -CHO, -CF₂H, -CFH₂, -C(=O)-NH₂, -C(=O)-NH-CH₃, -C(=O)-NH-C₂H₅, - C(=O)-N(CH₃)₂, -C(=O)-N(C₂H₅)₂ et -S(=O)₂-CH₃ ;
R⁴, R⁵ et R⁶ représentent chacun un atome d'hydrogène ;
R⁷ et R⁸, identiques ou différents, représentent chacun un atome d'hydrogène ou un radical alkyle choisi dans le groupe constitué des groupes méthyle, éthyle, n-propyle, isopropyle, n-butyle, sec-butyle, isobutyle et tert-butyle ;
R⁹ et R¹⁰, identiques ou différents, représentent chacun un atome d'hydrogène ou un radical alkyle choisi dans le groupe constitué des groupes méthyle, éthyle, n-propyle, isopropyle, n-butyle, sec-butyle, isobutyle et tert-butyle ;
R¹¹ représente un radical alkyle choisi dans le groupe constitué des groupes méthyle, éthyle, n-propyle, isopropyle et n-butyle ; ou un radical aryle ou hétéroaryle choisi dans le groupe constitué des groupes phényle et pyridinyle, moyennant quoi ledit groupe aryle ou hétéroaryle peut être substitué par 1, 2 ou 3 substituants indépendamment choisis dans le groupe constitué des groupes méthyle, éthyle, n-propyle, isopropyle, n-butyle, tert-butyle, -O-CH₃, -O-C₂H₅, -C(=O)-OH, -C(=O)-O-CH₃, -C(=O)-O-C₂H₅, des atomes de F, CI, Br et I, et des groupes -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, - N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=0)-N(CH₃)₂, -C(=O)-N(C₂H₅)₂ et -S(=O)₂-CH₃ ;
et
R¹² représente un radical phényle qui peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, des atomes de F, Br et I, et des groupes -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - CHO, -CF₂H, -CFH₂, -C(=O)-NH₂, -C(=O)-NH-CH₃, -C(=O)-NH-C₂H₅, - C(=O)-N(CH₃)₂, -C(=O)-N(C₂H₅)₂ et -S(=O)₂-CH₃.

12. Composé selon une ou plusieurs des revendications 1 à 11, **caractérisé en ce que**
X représente un groupe fonctionnel -NR¹R² ou un groupe fonctionnel - OR³ ;
R¹ représente un radical pyrrolyle non substitué ; un radical aryle ou hétéroaryle choisi dans le groupe constitué des groupes phényle et pyrrolyle, moyennant quoi ledit radical phényle est relié par l'intermédiaire d'un groupe -(CH₂) et ledit radical pyrrolyle est relié par l'intermédiaire d'un groupe -(CH₂)₂ et/ou ledit radical phényle ou pyrrolyle peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, des atomes de F, Br et I ou un du groupe fonctionnel -C(=O)-R¹² ;
R² représente un atome d'hydrogène ou un radical méthyle ;
ou
R¹ et R², conjointement avec l'atome d'azote de pontage, forment un groupe fonctionnel pyridazin-3-one non substitué ;
R³ représente un radical phényle non substitué ;
R⁴, R⁵ et R⁶ représentent chacun un atome d'hydrogène ;
R⁷ et R⁸ représentent chacun un atome d'hydrogène ;
R⁹ et R¹¹ représentent chacun un atome d'hydrogène ;
R¹¹ représente un radical alkyle choisi dans le groupe constitué des groupes méthyle, éthyle, n-propyle et n-butyle ; ou un radical phényle ou pyridinyle non substitué
et
R¹² représente un radical phényle non substitué.

13. Composé selon une ou plusieurs des revendications 1 à 12 choisi dans le groupe constitué des composés suivants :
[1] [5-(4-Méthyl-pipérazin-1-yl)-2-nitro-phényl]-(2-pyrrol-1-yl-éthyl)-amine,
[2] (4-Fluoro-benzyl)-[5-(4-méthyl-pipérazin-1-yl)-2-nitro-phényl]-amine,
[3] N-[5-(4-Méthyl-pipérazin-1-yl)-2-nitro-phényl]-benzamide,
[4] N-Méthyl-N-[5-(4-méthyl-pipérazin-1-yl)-2-nitro-phényl]-benzamide,
[5] [5-(4-Méthyl-pipérazin-1-yl)-2-nitro-phényl]-pyrrol-1-yl-amine,
[6] 2-[5-(4-Méthyl-pipérazin-1-yl)-2-nitro-phényl]-2H-pyridazin-3-one,
[7] 1-Méthyl-4-(4-nitro-3-phénoxy-phényl)-pipérazine,
[8] Benzyl-[5-(4-butyl-pipérazin-1-yl)-2-nitro-phényl]-amine,
[9] Benzyl-[2-nitro-5-(4-pyridin-2-yl-pipérazin-1-yl)-phényl]-amine et
[10] Benzyl-[2-nitro-5-(4-phényl-2-yl-pipérazin-1-yl)-phényl]-amine ;
optionnellement sous la forme d'un sel physiologiquement acceptable de celui-ci, ou d'un solvate correspondant de celui-ci.

14. Procédé de préparation d'un composé selon une ou plusieurs des revendications 1 à 13, **caractérisé en ce qu'**au moins un composé nitrobenzène de formule générale II, 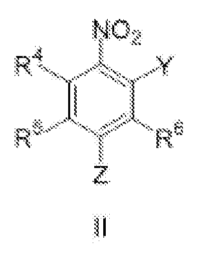 dans laquelle R⁴ à R⁶ ont une signification selon une ou plusieurs des revendications 1 à 13, Y représente un atome de chlore, et Z représente un atome de brome ou d'iode ; est mis à réagir avec au moins un composé de formule générale III, 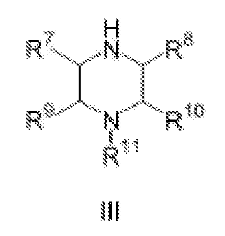 dans laquelle R⁷ à R¹¹ ont une signification selon une ou plusieurs des revendications 1 à 13, dans un milieu réactionnel adéquat, de préférence en présence d'au moins un catalyseur et/ou d'au moins un agent auxiliaire et/ou d'au moins une base pour donner un composé de formule générale IV, 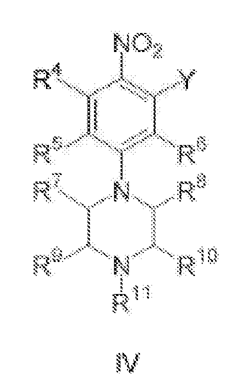 dans laquelle R⁴ à R¹¹ ont une signification selon une ou plusieurs des revendications 1 à 13 et Y représente un atome de chlore ; qui est optionnellement purifié et/ou isolé, et le composé de formule générale IV est mis à réagir avec au moins un composé de formule générale V, 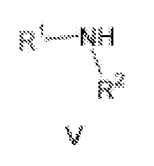 dans laquelle R¹ et R² ont une signification selon une ou plusieurs des revendications 1 à 13, dans un milieu réactionnel adéquat, de préférence en présence d'au moins un catalyseur et/ou d'au moins un agent auxiliaire et/ou d'au moins une base pour donner un composé de formule générale VI, 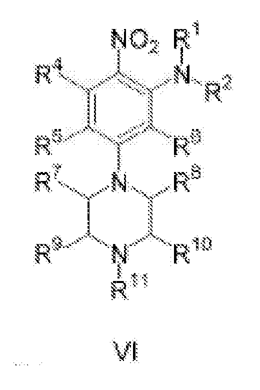 dans laquelle R¹, R² et R⁴ à R¹¹ ont une signification selon une ou plusieurs des revendications 1 à 13, qui est optionnellement purifié et/ou isolé.

15. Procédé de préparation d'un composé selon une ou plusieurs des revendications 1 à 13, **caractérisé en ce qu'**au moins un composé nitrobenzène de formule générale VII, 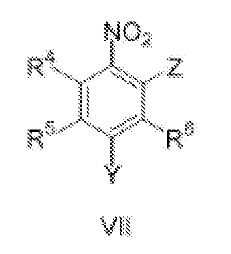 dans laquelle R⁴ à R⁶ ont une signification selon une ou plusieurs des revendications 1 à 13, Z représente un atome de brome ou d'iode, et Y représente un atome de chlore, est mis à réagir avec au moins un composé de formule générale V, 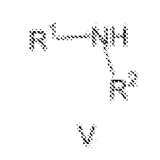 dans laquelle R¹ et R² ont une signification selon une ou plusieurs des revendications 1 à 13, dans un milieu réactionnel adéquat, de préférence en présence d'au moins un catalyseur et/ou d'au moins un agent auxiliaire et/ou d'au moins une base pour donner un composé de formule générale VIII, 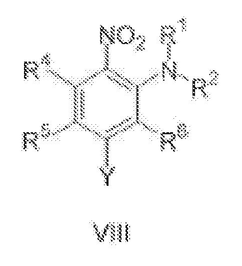 dans laquelle R¹, R² et R⁴ à R⁶ ont une signification selon une ou plusieurs des revendications 1 à 13 et Y représente un atome de chlore ; qui est optionnellement purifié et/ou isolé, et le composé de formule générale VIII est mis à réagir avec au moins un composé de formule générale III, 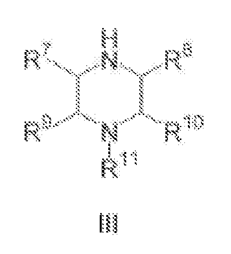 dans laquelle R⁷ à R¹¹ ont une signification selon une ou plusieurs des revendications 1 à 13, dans un milieu réactionnel adéquat, de préférence en présence d'au moins un catalyseur et/ou d'au moins un agent auxiliaire et/ou d'au moins une base pour donner un composé de formule générale VI, 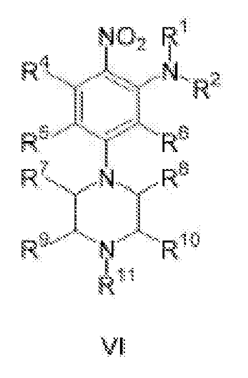 dans laquelle R¹, R² et R⁴ à R¹¹ ont une signification selon une ou plusieurs des revendications 1 à 13, qui est optionnellement purifié et/ou isolé.

16. Procédé de préparation d'un composé selon une ou plusieurs des revendications 1 à 13, **caractérisé en ce qu'**au moins un composé nitrobenzène de formule générale II, 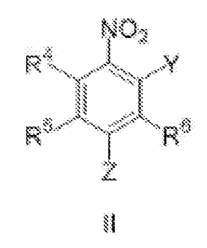 dans laquelle R⁴ à R⁶ ont une signification selon une ou plusieurs des revendications 1 à 13, Y représente un atome de chlore, et Z représente un atome de brome ou d'iode ;
est mis à réagir avec au moins un composé de formule générale III, 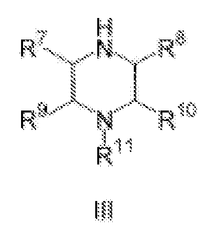 dans laquelle R⁷ à R¹¹ ont une signification selon une ou plusieurs des revendications 1 à 13, dans un milieu réactionnel adéquat, de préférence en présence d'au moins un catalyseur et/ou d'au moins un agent auxiliaire et/ou d'au moins une base pour donner un composé de formule générale IV, 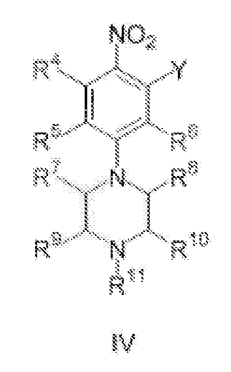 dans laquelle R⁴ à R¹¹ ont une signification selon une ou plusieurs des revendications 1 à 13 et Y représente un atome de chlore ; qui est optionnellement purifié et/ou isolé, et le composé de formule générale IV est mis à réagir avec au moins un composé de formule générale IX, 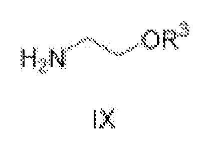 dans laquelle R³ a une signification selon une ou plusieurs des revendications 1 à 13, dans un milieu réactionnel adéquat, de préférence en présence d'au moins un catalyseur et/ou d'au moins un agent auxiliaire et/ou d'au moins une base pour donner un composé de formule générale X, 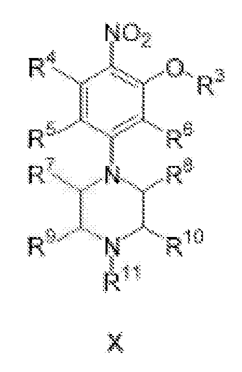 dans laquelle R³ à R¹¹ ont une signification selon une ou plusieurs des revendications 1 à 13, qui est optionnellement purifié et/ou isolé.

17. Médicament comprenant au moins un composé de phényl-pipérazine substitué par un nitro de formule générale Ia, 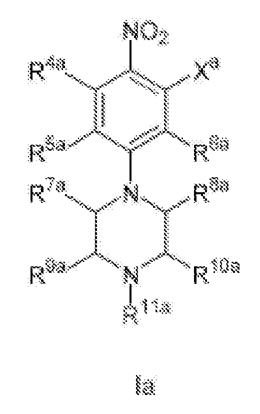 dans laquelle
X^{a} représente un groupe fonctionnel -NR^{1a}R^{2a} ou un groupe fonctionnel -OR^{3a};
R^{1a} représente un radical aliphatique en C₁ à C₁₀, linéaire ou ramifié, saturé ou insaturé, qui est substitué par 1, 2 ou 3 substituants indépendamment choisis dans le groupe constitué des atomes de F, Cl, Br et I, et des groupes -CN, -CF₃, -OCF₃, -SCF₃, -OH, -SH, -NH₂, -O-alkyle en C₁ à C₅, -S-alkyle en C₁ à C₅, -NH-(alkyle en C₁ à C₅) et -N(alkyle en C₁ à C₅)₂;
un radical choisi dans le groupe constitué des groupes adamantyle, bicyclo[2.2.1]heptyle et bicyclo[3.1.1]heptyle qui peut être substitué par 1, 2 ou 3 substituants indépendamment choisis dans le groupe constitué des groupes oxo (=O), thioxo (=S), alkyle en C₁ à C₅, -O-alkyle en C₁ à C₅, -S-alkyle en C₁ à C₅, -C(=O)-OH, -C(=O)-alkyle en C₁ à C₅, -C(=O)-O-alkyle en C₁ à C₅, -O-C(=O)-alkyle en C₁ à C₅, des atomes de F, Cl, Br et I, et des groupes -CN, -CF₃, -OCF₃, -SCF₃, -OH, -SH, -NH₂, -NH(alkyle en C₁ à C₅), - N(alkyle en C₁ à C₅)₂, -NO₂, -CHO, -CF₂H, -CFH₂, -C(=O)-NH₂, -C(=O)-NH(alkyle en C₁ à C₅), -C(=O)-N(alkyle en C₁ à C₅)₂ et -S(=O)₂-alkyle en C₁ à C₅ et/ou qui est relié par l'intermédiaire d'un groupe alkylène en C₁ à C₆ linéaire ou ramifié qui peut contenir 1, 2 ou 3 hétéroatomes indépendamment choisis dans le groupe constitué des atomes d'azote, d'oxygène et de soufre en tant qu'élément(s) de la chaîne ;
un radical cycloaliphatique de 3 à 9 chaînons, saturé ou insaturé, qui peut être substitué par 1, 2 ou 3 substituants indépendamment choisis dans le groupe constitué des groupes oxo (=O), thioxo (=S), alkyle en C₁ à C₅, - O-alkyle en C₁ à C₅, -S-alkyle en C₁ à C₅, -C(=O)-OH, -C(=O)-alkyle en C₁ à C₅, -C(=O)-O-alkyle en C₁ à C₅, -O-C(=O)-alkyle en C₁ à C₅, des atomes de F, Cl, Br et I, et des groupes -CN, -CF₃, -OCF₃, -SCF₃, -OH, -SH, -NH₂,-NH(alkyle en C₁ à C₅), -N(alkyle en C₁ à C₅)₂, -NO₂, -CHO, -CF₂H, -CFH₂, - C(=O)-NH₂, -C(=O)-NH(alkyle en C₁ à C₅), -C(=O)-N(alkyle en C₁ à C₅)₂, - S(=O)₂-alkyle en C₁ à C₅, -S(=O)₂-phényle, phényle, phénoxy et benzyle et qui est relié par l'intermédiaire d'un groupe alkylène en C₁ à C₆, alcénylène en C₂ à C₆ ou alcynylène en C₂ à C₆, linéaire ou ramifié, qui peut être non substitué ou substitué par 1, 2 ou 3 substituants indépendamment choisis dans le groupe constitué des atomes de F, Cl, Br et I, et des groupes -CN,-CF₃, -OCF₃, -SCF₃, -OH, -SH, -NH₂, -O-alkyle en C₁ à C₅, -S-alkyle en C₁ à C₅, phényle, -NH-(alkyle en C₁ à C₅) et -N(alkyle en C₁ à C₅)₂ et/ou qui peut contenir 1, 2 ou 3 hétéroatomes indépendamment choisis dans le groupe constitué des atomes d'azote, d'oxygène et de soufre en tant qu'élément(s) du noyau ;
un radical aryle ou hétéroaryle de 5 à 10 chaînons qui peut être substitué par 1, 2 ou 3 substituants indépendamment choisis dans le groupe constitué des groupes alkyle en C₁ à C₅, -O-alkyle en C₁ à C₅, -S-alkyle en C₁ à C₅, -C(=O)-OH, -C(=O)-alkyle en C₁ à C₅, -C(=O)-O-alkyle en C₁ à C₅, -O-C(=O)-alkyle en C₁ à C₅, des atomes de F, Cl, Br et I, et des groupes-CN, -CF₃, -OCF₃, -SCF₃, -OH, -SH, -NH₂, -NH(alkyle en C₁ à C₅), -N(alkyle en C₁ à C₅)₂, -NO₂, -CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle et moyennant quoi ledit radical hétéroaryle contient 1, 2 ou 3 hétéroatomes indépendamment choisis dans le groupe constitué des atomes d'azote, d'oxygène et de soufre en tant qu'élément(s) du noyau ;
un radical aryle de 5 à 10 chaînons, moyennant quoi ledit radical aryle peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, -C(=O)-O-alkyle en C₁ à C₅, -O-C(=O)-alkyle en C₁ à C₅, des atomes de F, Cl, Br et I, et des groupes -CN, -CF₃, -OCF₃,-SCF₃, -OH, -SH, -NH₂, -NH(alkyle en C₁ à C₅), -N(alkyle en C₁ à C₅)₂, -NO₂, - CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle et ledit radical aryle est relié par l'intermédiaire d'un groupe alkylène en C₁ à C₆, alcénylène en C₂ à C₆ ou alcynylène en C₂ à C₆, linéaire ou ramifié, qui peut être non substitué ou substitué par 1, 2 ou 3 substituants indépendamment choisis dans le groupe constitué des atomes de F, Cl, Br et I, et des groupes -CN, -CF₃, -OCF₃, -SCF₃, -OH, -SH, -NH₂,-O-alkyle en C₁ à C₅, -S-alkyle en C₁ à C₅, phényle, -NH-(alkyle en C₁ à C₅) et -N(alkyle en C₁ à C₅)₂ et qui peut contenir 1, 2 ou 3 hétéroatomes indépendamment choisis dans le groupe constitué des atomes d'azote, d'oxygène et de soufre en tant qu'élément(s) de la chaîne ;
un radical hétéroaryle de 5 à 10 chaînons, qui peut être substitué par 1, 2 ou 3 substituants indépendamment choisis dans le groupe constitué des groupes alkyle en C₁ à C₅, -O-alkyle en C₁ à C₅, -S-alkyle en C₁ à C₅, - C(=O)-OH, -C(=O)-alkyle en C₁ à C₅, -C(=O)-O-alkyle en C₁ à C₅, -OC(=O)-alkyle en C₁ à C₅, des atomes de F, Cl, Br et I, et des groupes -CN,-CF₃, -OCF₃, -SCF₃, -OH, -SH, -NH₂, -NH(alkyle en C₁ à C₅), -N(alkyle en C₁ à C₅)₂, -NO₂, -CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle et qui est relié par l'intermédiaire d'un groupe alkylène en C₁ à C₆, alcénylène en C₂ à C₆ ou alcynylène en C₂ à C₆, linéaire ou ramifié, qui peut être non substitué ou substitué par 1, 2 ou 3 substituants indépendamment choisis dans le groupe constitué des atomes de F, Cl, Br et I, et des groupes -CN, -CF₃, -OCF₃, -SCF₃, -OH, -SH, -NH₂,-O-alkyle en C₁ à C₅, -S-alkyle en C₁ à C₅, phényle, -NH-(alkyle en C₁ à C₅) et -N(alkyle en C₁ à C₅)₂ et qui peut contenir 1, 2 ou 3 hétéroatomes indépendamment choisis dans le groupe constitué des atomes d'azote, d'oxygène et de soufre en tant qu'élément(s) de la chaîne et moyennant quoi le radical hétéroaryle contient 1, 2 ou 3 hétéroatomes indépendamment choisis dans le groupe constitué des atomes d'azote, d'oxygène et de soufre en tant qu'élément(s) du noyau ;
ou un groupe fonctionnel -C(=O)-R^{12a} ;
R^{2a} représente un atome d'hydrogène ou un radical aliphatique en C₁ à C₁₀, linéaire ou ramifié, saturé ou insaturé, qui peut être non substitué ou substitué par 1, 2 ou 3 substituants indépendamment choisis dans le groupe constitué des atomes de F, Cl, Br et I, et des groupes -CN, -CF₃,-OCF₃, -SCF₃, -OH, -SH, -NH₂, -O-alkyle en C₁ à C₅, -S-alkyle en C₁ à C₅, - NH-(alkyle en C₁ à C₅) et -N(alkyle en C₁ à C₅)₂,
ou
R^{1a} et R^{2a}, conjointement avec l'azote de pontage, forment un noyau hétérocyclique de 3 à 6 chaînons, saturé, insaturé ou aromatique, qui peut être substitué par 1, 2 ou 3 substituants indépendamment choisis dans le groupe constitué des groupes alkyle en C₁ à C₅, -O-alkyle en C₁ à C₅, -S-alkyle en C₁ à C₅, oxo (=O), thioxo (=S), -C(=O)-OH, -C(=O)-alkyle en C₁ à C₅, -C(=O)-O-alkyle en C₁ à C₅, -O-C(=O)-alkyle en C₁ à C₅, des atomes de F, Cl, Br et I, et des groupes -CN, -CF₃, -OCF₃, -SCF₃, -OH, -SH, -NH₂,-NH(alkyle en C₁ à C₅), -N(alkyle en C₁ à C₅)₂, -NO₂, -CHO, -CF₂H, -CFH₂, - C(=O)-NH₂, -C(=O)-NH(alkyle en C₁ à C₅), -C(=O)-N(alkyle en C₁ à C₅)₂, - S(=O)₂-alkyle en C₁ à C₅, -S(=O)₂-phényle, phényle, phénoxy et benzyle et qui peut contenir 1, 2 ou 3 hétéroatomes supplémentaires indépendamment choisis dans le groupe constitué des atomes d'azote, d'oxygène et de soufre en tant qu'élément(s) du noyau et/ou qui peut être condensé avec un système de noyaux mono- ou bicycliques qui peut être substitué par 1, 2 ou 3 substituants indépendamment choisis dans le groupe constitué des groupes alkyle en C₁ à C₅, -O-alkyle en C₁ à C₅, -S-alkyle en C₁ à C₅, oxo (=O), thioxo (=S), -C(=O)-OH, -C(=O)-alkyle en C₁ à C₅, -C(=O)-O-alkyle en C₁ à C₅, -O-C(=O)-alkyle en C₁ à C₅, des atomes de F, Cl, Br et I, et des groupes -CN, -CF₃, -OCF₃, -SCF₃, -OH, -SH, -NH₂, -NH(alkyle en C₁ à C₅), - N(alkyle en C₁ à C₅)₂, -NO₂, -CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle, moyennant quoi ledit ou lesdits substituants cycliques phényle, phénoxy et benzyle peuvent être non substitués ou substitués par 1, 2 ou 3 substituants indépendamment choisis dans le groupe constitué des groupes méthyle, éthyle, n-propyle, isopropyle, méthoxy, éthoxy, des atomes de F, Cl et Br, et des groupes-CN, -CF₃, -OCF₃, -SCF₃, -OH, -SH, -NH₂ et NO₂ ;
moyennant quoi les noyaux du système de noyaux comportent 5, 6 ou 7 chaînons et peuvent contenir 1, 2 ou 3 hétéroatomes indépendamment choisis dans le groupe constitué des atomes d'azote, d'oxygène et de soufre ;
R^{3a} représente un radical aryle ou hétéroaryle de 5 à 10 chaînons qui peut être substitué par 1, 2 ou 3 substituants indépendamment choisis dans le groupe constitué des groupes alkyle en C₁ à C₅, -O-alkyle en C₁ à C₅, -S-alkyle en C₁ à C₅, -C(=O)-OH, -C(=O)-alkyle en C₁ à C₅, -C(=O)-O-alkyle en C₁ à C₅, -O-C(=O)-alkyle en C₁ à C₅, des atomes de F, Cl, Br et I, et des groupes -CN, -CF₃, -OCF₃, -SCF₃, -OH, -SH, -NH₂, -NH(alkyle en C₁ à C₅), - N(alkyle en C₁ à C₅)₂, -NO₂, -CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle et qui peut être relié par l'intermédiaire d'un groupe alkylène en C₁ à C₆, alcénylène en C₂ à C₆ ou alkinylène en C₂ à C₆, linéaire ou ramifié, et qui peut être non substitué ou substitué par 1, 2 ou 3 substituants indépendamment choisis dans le groupe constitué des atomes de F, Cl, Br et I, et des groupes -CN, -CF₃, -OCF₃,-SCF₃, -OH, -SH, -NH₂, -O-alkyle en C₁ à C₅, -S-alkyle en C₁ à C₅, -NH-(alkyle en C₁ à C₅) et -N(alkyle en C₁ à C₅)₂ et moyennant quoi le radical hétéroaryle contient 1, 2 ou 3 hétéroatomes indépendamment choisis dans le groupe constitué des atomes d'azote, d'oxygène et de soufre en tant qu'élément(s) du noyau ;
R^{4a}, R^{5a} et R^{6a}, identiques ou différents, représentent chacun un atome d'hydrogène ou un atome d'halogène ;
ou
R^{4a} et R^{5a},conjointement avec les atomes de carbone de pontage, forment un noyau hétérocyclique non substitué de 5 ou 6 chaînons qui contient 1, 2 ou 3 hétéroatomes indépendamment choisis dans le groupe constitué des atomes d'azote, d'oxygène et de soufre en tant qu'élément(s) du noyau et qui, conjointement avec le noyau phényle avec lequel il est fusionné, forme un système de noyaux aromatiques bicycliques à 9 ou 10 chaînons ;
R^{7a} et R^{8a}, identiques ou différents, représentent chacun un atome d'hydrogène ou un radical aliphatique en C₁ à C₁₀, linéaire ou ramifié, saturé ou insaturé, qui peut être non substitué ou substitué par 1, 2 ou 3 substituants indépendamment choisis dans le groupe constitué des atomes de F, Cl, Br et I, et des groupes -CN, -CF₃, -OCF₃, -SCF₃, -OH, -SH, -NH₂,-O-alkyle en C₁ à C₅, -S-alkyle en C₁ à C₅, -NH-(alkyle en C₁ à C₅) et - N(alkyle en C₁ à C₅)₂ ;
R^{9a} et R^{10a}, identiques ou différents, représentent chacun un atome d'hydrogène ou un radical aliphatique en C₁ à C₁₀, linéaire ou ramifié, saturé ou insaturé, qui peut être non substitué ou substitué par 1, 2 ou 3 substituants indépendamment choisis dans le groupe constitué des atomes de F, Cl, Br et I, et des groupes -CN, -CF₃, -OCF₃, -SCF₃, -OH, -SH, -NH₂,-O-alkyle en C₁ à C₅, -S-alkyle en C₁ à C₅, -NH-(alkyle en C₁ à C₅) et - N(alkyle en C₁ à C₅)₂ ;
R^{11a} représente un atome d'hydrogène ; un radical aliphatique en C₁ à C₁₀, linéaire ou ramifié, saturé ou insaturé, qui peut être non substitué ou substitué par 1, 2 ou 3 substituants indépendamment choisis dans le groupe constitué des atomes de F, Cl, Br et I, et des groupes -CN, -CF₃, -OCF₃,-SCF₃, -OH, -SH, -NH₂, -O-alkyle en C₁ à C₅, -S-alkyle en C₁ à C₅, -NH-(alkyle en C₁ à C₅) et -N(alkyle en C₁ à C₅)₂ et qui peut contenir 1, 2 ou 3 hétéroatomes indépendamment choisis dans le groupe constitué des atomes d'azote, d'oxygène et de soufre en tant qu'élément(s) de la chaîne ;
un radical aryle ou hétéroaryle de 5 à 10 chaînons qui peut être substitué par 1, 2 ou 3 substituants indépendamment choisis dans le groupe constitué des groupes alkyle en C₁ à C₅, -O-alkyle en C₁ à C₅, -S-alkyle en C₁ à C₅, -C(=O)-OH, -C(=O)-alkyle en C₁ à C₅, -C(=O)-O-alkyle en C₁ à C₅, -O-C(=O)-alkyle en C₁ à C₅, des atomes de F, Cl, Br et I, et des groupes-CN, -CF₃, -OCF₃, -SCF₃, -OH, -SH, -NH₂, -NH(alkyle en C₁ à C₅), -N(alkyle en C₁ à C₅)₂, -NO₂, -CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle et qui peut être relié par l'intermédiaire d'un groupe alkylène en C₁ à C₆, alcénylène en C₂ à C₆ ou alcynylène en C₂ à C₆, linéaire ou ramifié, qui peut être non substitué ou substitué par 1, 2 ou 3 substituants indépendamment choisis dans le groupe constitué des atomes de F, Cl, Br et I, et des groupes -CN, -CF₃, -OCF₃,-SCF₃, -OH, -SH, -NH₂, -O-alkyle en C₁ à C₅, -S-alkyle en C₁ à C₅, -NH-(alkyle en C₁ à C₅) et -N(alkyle en C₁ à C₅)₂ et moyennant quoi le radical hétéroaryle contient 1, 2 ou 3 hétéroatomes indépendamment choisis dans le groupe constitué des atomes d'azote, d'oxygène et de soufre en tant qu'élément(s) du noyau ; un groupe fonctionnel -C(=O)-R^{13a} ou un groupe fonctionnel -S(=O)₂-R^{14a} ;
R^{12a} représente un radical aliphatique en C₁ à C₁₀, linéaire ou ramifié, saturé ou insaturé, qui peut être non substitué ou substitué par 1, 2 ou 3 substituants indépendamment choisis dans le groupe constitué des atomes de F, Cl, Br et I, et des groupes -CN, -CF₃, -OCF₃, -SCF₃, -OH, -SH, -NH₂,-O-alkyle en C₁ à C₅, -S-alkyle en C₁ à C₅, -NH-(alkyle en C₁ à C₅) et - N(alkyle en C₁ à C₅)₂ ;
ou un radical aryle ou hétéroaryle de 5 à 10 chaînons, qui peut être substitué par 1, 2 ou 3 substituants indépendamment choisis dans le groupe constitué des groupes alkyle en C₁ à C₅, -O-alkyle en C₁ à C₅, -S-alkyle en C₁ à C₅, -C(=O)-OH, -C(=O)-alkyle en C₁ à C₅, -C(=O)-O-alkyle en C₁ à C₅, -O-C(=O)-alkyle en C₁ à C₅, des atomes de F, Cl, Br et I, et des groupes-CN, -CF₃, -OCF₃, -SCF₃, -OH, -SH, -NH₂, -NH(alkyle en C₁ à C₅), -N(alkyle en C₁ à C₅)₂, -NO₂, -CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle et qui peut être relié par l'intermédiaire d'un groupe alkylène en C₁ à C₆, alcénylène en Cl à C₆ ou alcynylène en C₂ à C₆, linéaire ou ramifié, qui peut être non substitué ou substitué par 1, 2 ou 3 substituants indépendamment choisis dans le groupe constitué des atomes de F, Cl, Br et I, et des groupes -CN, -CF₃, -OCF₃,-SCF₃, -OH, -SH, -NH₂, -O-alkyle en C₁ à C₅, -S-alkyle en C₁ à C₅, -NH-(alkyle en C₁ à C₅) et -N(alkyle en C₁ à C₅)₂ et moyennant quoi le radical hétéroaryle contient 1, 2 ou 3 hétéroatomes indépendamment choisis dans le groupe constitué des atomes d'azote, d'oxygène et de soufre en tant qu'élément(s) du noyau ;
et
R^{13a} et R^{14a}, identiques ou différents, représentent chacun un radical aliphatique en C₁ à C₁₀, linéaire ou ramifié, saturé ou insaturé, qui peut être non substitué ou substitué par 1, 2 ou 3 substituants indépendamment choisis dans le groupe constitué des atomes de F, Cl, Br et I, et des groupes -CN, -CF₃, -OCF₃, -SCF₃, -OH, -SH, -NH₂, -O-alkyle en C₁ à C₅, -S-alkyle en C₁ à C₅, -NH-(alkyle en C₁ à C₅) et -N(alkyle en C₁ à C₅)₂ ;
ou un radical aryle ou hétéroaryle de 5 à 10 chaînons, qui peut être substitué par 1, 2 ou 3 substituants indépendamment choisis dans le groupe constitué des groupes alkyle en C₁ à C₅, -O-alkyle en C₁ à C₅, -S-alkyle en C₁ à C₅, -C(=O)-OH, -C(=O)-alkyle en C₁ à C₅, -C(=O)-O-alkyle en C₁ à C₅, -O-C(=O)-alkyle en C₁ à C₅, des atomes de F, Cl, Br et I, et des groupes-CN, -CF₃, -OCF₃, -SCF₃, -OH, -SH, -NH₂, -NH(alkyle en C₁ à C₅), -N(alkyle en C₁ à C₅)₂, -NO₂, -CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle et/ou qui peut être relié par l'intermédiaire d'un groupe alkylène en C₁ à C₆, alcénylène en C₂ à C₆ ou alcynylène en C₂ à C₆, linéaire ou ramifié, qui peut être non substitué ou substitué par 1, 2 ou 3 substituants indépendamment choisis dans le groupe constitué des atomes de F, Cl, Br et I, et des groupes -CN, -CF₃, -OCF₃,-SCF₃, -OH, -SH, -NH₂, -O-alkyle en C₁ à C₅, -S-alkyle en C₁ à C₅, -NH-(alkyle en C₁ à C₅) et -N(alkyle en C₁ à C₅)₂ et moyennant quoi le radical hétéroaryle contient 1, 2 ou 3 hétéroatomes indépendamment choisis dans le groupe constitué des atomes d'azote, d'oxygène et de soufre en tant qu'élément(s) du noyau.
optionnellement sous la forme de l'un de ses stéréoisomères, de préférence des énantiomères ou des diastéréomères, d'un racémate ou sous la forme d'un mélange d'au moins deux de ses stéréoisomères, de préférence des énantiomères et/ou des diastéréomères, dans n'importe quel rapport de mélange, ou d'un sel physiologiquement acceptable de celui-ci, ou d'un solvate correspondant de celui-ci ;
et optionnellement au moins un agent auxiliaire physiologiquement compatible.

18. Médicament selon la revendication 17, **caractérisé en ce que**
R^{1a} représente un radical alkyle choisi dans le groupe constitué des groupes méthyle, éthyle, n-propyle, isopropyle, n-butyle, sec-butyle, isobutyle et tert-butyle qui est substitué par 1, 2 ou 3 substituants indépendamment choisis dans le groupe constitué des atomes de F, Cl, Br et I, et des groupes -CN, -CF₃, -OCF₃, -SCF₃, -OH, -SH et -NH₂ ;
un radical choisi dans le groupe constitué des groupes adamantyle, bicyclo[2.2.1]heptyle et bicyclo[3.1.1]heptyle qui peut être substitué par 1, 2 ou 3 substituants indépendamment choisis dans le groupe constitué des groupes alkyle en C₁ à C₅, -O-alkyle en C₁ à C₅, des atomes de F, Cl, Br et I, et des groupes -CN, -CF₃ et -OCF₃ et/ou qui est relié par l'intermédiaire d'un groupe alkylène en C₁ à C₆, linéaire ou ramifié, qui peut contenir 1, 2 ou 3 atomes d'oxygène en tant qu'éléments de la chaîne ;
un radical aryle ou hétéroaryle choisi dans le groupe constitué des groupes phényle, naphtyle, furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridinyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle, qui peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, -C(=O)-OH, -C(=O)-O-alkyle en C₁ à C₅, -OC(=O)-alkyle en C₁ à C₅, des atomes de F, Cl, Br et I, et des groupes -CN,-CF₃, -OCF₃, -SCF₃, -OH, -SH, -NH₂, -NH(alkyle en C₁ à C₅), -N(alkyle en C₁ à C₅)₂, -NO₂, -CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle ;
un radical aryle choisi dans le groupe constitué des groupes phényle et naphtyle qui peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, -C(=O)-O-alkyle en C₁ à C₅, -O-C(=O)-alkyle en C₁ à C₅, des atomes de F, Cl, Br et I, et des groupes-CN, -CF₃, -OCF₃, -SCF₃, -OH, -SH, -NH₂, -NH(alkyle en C₁ à C₅), -N(alkyle en C₁ à C₅)₂, -NO₂, -CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle et ledit radical aryle est relié par l'intermédiaire d'un groupe alkylène en C₁ à C₆, linéaire ou ramifié, qui peut contenir 1, 2 ou 3 atomes d'oxygène en tant qu'éléments de la chaîne et qui peut être substitué par un radical phényle ;
un radical hétéroaryle choisi dans le groupe constitué des groupes furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridinyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle, moyennant quoi ledit radical hétéroaryle peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, -C(=O)-OH, -C(=O)-O-alkyle en C₁ à C₅, -O-C(=O)-alkyle en C₁ à C₅, des atomes de F, Cl, Br et I, et des groupes -CN, -CF₃, -OCF₃,-SCF₃, -OH, -SH, -NH₂, -NH(alkyle en C₁ à C₅), -N(alkyle en C₁ à C₅)₂, -NO₂, - CHO, -CF₂H, -CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle et ledit radical hétéroaryle est relié par l'intermédiaire d'un groupe alkylène en C₁ à C₆, linéaire ou ramifié, qui peut contenir 1, 2 ou 3 atomes d'oxygène en tant qu'éléments de la chaîne,
ou un groupe fonctionnel -C(=O)-R^{12a} ;
de préférence R^{1a} représente un radical alkyle choisi dans le groupe constitué des groupes méthyle, éthyle, n-propyle et isopropyle, moyennant quoi ledit radical alkyle est substitué par 1, 2 ou 3 substituants indépendamment choisis dans le groupe constitué du groupe -OH, des atomes de F, Cl et Br, et du groupe -CN ;
un radical choisi dans le groupe constitué des groupes adamantyle, bicyclo[2.2.1]heptyle et bicyclo[3.1.1]heptyle qui peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -O-C(CH₃)₃, des atomes de F, Cl, Br et I, et des groupes -CN,-CF₃ et -OCF₃ et/ou qui est relié par l'intermédiaire d'un groupe alkylène choisi dans le groupe constitué des groupes -CH₂-, -CH(CH₃)-, -CH₂-CH₂-, - CH₂-CH₂-CH₂- et -CH₂-CH₂-O- ;
un radical aryle ou hétéroaryle choisi dans le groupe constitué des groupes phényle, naphtyle, furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle et pyridinyle, moyennant quoi ledit radical aryle ou hétéroaryle peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -OC(CH3)₃, -S-CH₃, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, des atomes de F, Cl, Br et I, et des groupes -CN, -CF₃, -OCF₃, -SCF₃, -SH, -NH₂, -NH-CH₃, -NH-C₂H₅, - NH-CH₂-CH₂-CH₃, -NH-CH(CH₃)₂, -NH-C(CH₃)₃, -N(CH₃)₂, -N(C₂H₅)₂, -CHO, - CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ et -S(=O)₂-CH₃ ;
un radical aryle choisi dans le groupe constitué des groupes phényle et naphtyle, moyennant quoi ledit groupe aryle peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(OH₃)₃, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, des atomes de F, Cl, Br et I, et des groupes -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, - CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ et -S(=O)₂-CH₃ et/ou est relié par l'intermédiaire d'un groupe alkylène choisi dans le groupe constitué des groupes -CH₂-, - CH(CH₃)-, -CH(phényle)-, -CH₂-CH₂-, -CH₂-CH₂-CH₂- et -CH₂-CH₂-O- ;
un radical hétéroaryle choisi dans le groupe constitué des groupes furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle et pyridinyle, moyennant quoi ledit radical hétéroaryle peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, - S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, des atomes de F, Cl, Br et I, et des groupes -CN, -CF₃, -OCF₃, -SCF₃, -SH, -NH₂, -NH-CH₃, - NH-C₂H₅, -NH-CH₂-CH₂-CH₃, -NH-CH(CH₃)₂, -NH-C(CH₃)₃, -N(CH₃)₂, - N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ et -S(=O)₂-CH₃ et/ou est relié par l'intermédiaire d'un groupe alkylène choisi dans le groupe constitué des groupes -cher, -CH(CH₃)-, -CH₂-CH₂-, -CH₂-CH₂-CH₂- et -CH₂-CH₂-O- ;
ou un groupe fonctionnel -C(=O)-R^{12a} ;
plus préférablement R^{1a} représente un radical alkyle choisi dans le groupe constitué des groupes -CH₂-CH₂-OH et -CH₂-CH₂-CH₂-OH ;
un radical adamantyle non substitué ;
un radical phényle ou pyrrolyle non substitué ;
un radical naphtyle non substitué qui est relié par l'intermédiaire d'un groupe alkylène choisi dans le groupe constitué des groupes -CH₂-, - CH(CH₃)-, -CH₂-CH₂-, -CH₂-CH₂-CH₂- et -CH₂-CH₂-O- ;
un radical phényle qui peut être substitué par 1, 2 ou 3 substituants indépendamment choisis dans le groupe constitué des groupes méthyle, éthyle, tert-butyle et méthoxy, et des atomes de F et Cl et ledit radical phényle est relié par l'intermédiaire d'un groupe alkylène choisi dans le groupe constitué des groupes -CH₂-, -CH(CH₃)-, -CH(phényle)-, -CH₂-CH₂-, -CH₂-CH₂-CH₂- et -CH₂-CH₂-O- ;
un radical hétéroaryle choisi dans le groupe constitué des groupes pyridinyle, furanyle et pyrrolyle, moyennant quoi ledit radical pyridinyle, furanyle ou pyrrolyle peut être substitué par 1, 2 ou 3 substituants indépendamment choisis dans le groupe constitué des groupes méthyle, éthyle, tert-butyle et méthoxy, et des atomes de F et Cl et ledit radical pyridinyle, furanyle ou pyrrolyle est relié par l'intermédiaire d'un groupe alkylène choisi dans le groupe constitué des groupes -CFH₂-, -CH(CH₃)-, - CH₂-CH₂-, -CH₂-CH₂-CH₂- et -CH₂-CH₂-O- ;
ou un groupe fonctionnel -C(=O)-R^{12a}.

19. Médicament selon la revendication 17 ou 18, **caractérisé en ce que**
R^{2a} représente un atome d'hydrogène ou un radical alkyle non substitué en C₁ à C₁₀, linéaire ou ramifié ;
de préférence R^{2a} représente un atome d'hydrogène ou un radical alkyle choisi dans le groupe constitué des groupes méthyle, éthyle, n-propyle, isopropyle, n-butyle, sec-butyle, isobutyle et tert-butyle ;
plus préférablement R^{2a} représente un atome d'hydrogène ou un radical méthyle.

20. Médicament selon une ou plusieurs des revendications 17 à 19, **caractérisé en ce que**
R^{1a} et R^{2a}, conjointement avec l'atome d'azote de pontage, forment un groupe fonctionnel choisi dans le groupe constitué des groupes 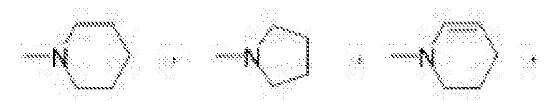 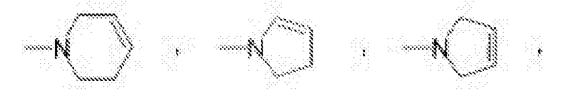 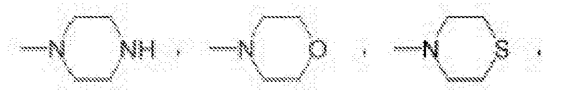 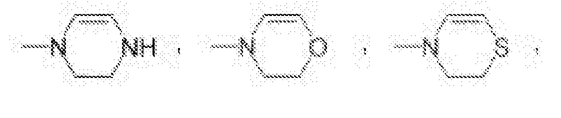 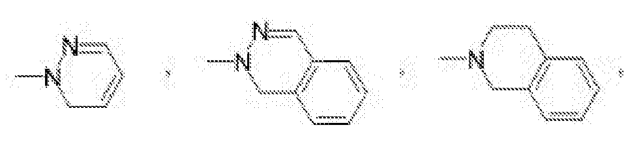 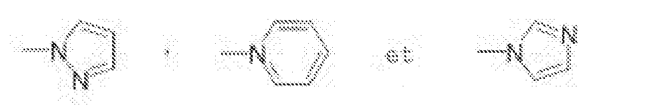 moyennant quoi chacun de ces groupes fonctionnels cycliques mentionnés ci-dessus peut être substitué au niveau de n'importe quelle position y compris les groupes -NH- par 1, 2 ou 3 substituants indépendamment choisis dans le groupe constitué des groupes alkyle en C₁ à C₅, -O-alkyle en C₁ à C₅, -S-alkyle en C₁ à C₅, -C(=O)-OH, oxo (=O), thioxo (=S), -C(=O)-O-alkyle en C₁ à C₅, -O-C(=O)-alkyle en C₁ à C₅, des atomes de F, Cl, Br et I, et des groupes -CN, -CF₃, -OCF₃, -SCF₃, -OH, -SH, -NH₂, -NH(alkyle en C₁ à C₅), -N(alkyle en C₁ à C₅)₂, -NO₂, -CHO, -CF₂H, - CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-Phényle, phényle, phénoxy et benzyle ; moyennant quoi ledit ou lesdits substituants cycliques phényle, phénoxy et benzyle peuvent être non substitués ou substitués par 1, 2 ou 3 substituants indépendamment choisis dans le groupe constitué des groupes méthyle, éthyle, n-propyle, isopropyle, méthoxy, éthoxy, des atomes de F, Cl, Br, et des groupes -CN, -CF₃, -OCF₃, -SCF₃, -OH, -SH, -NH₂ et NO₂ ;
de préférence R^{1a} et R^{2a}, conjointement avec l'atome d'azote de pontage, forment un groupe fonctionnel choisi dans le groupe constitué des groupes 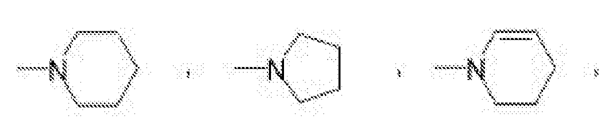 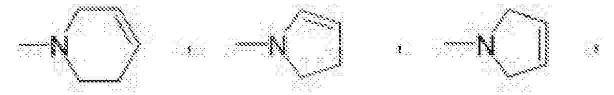 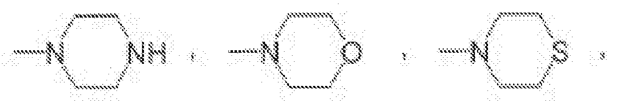 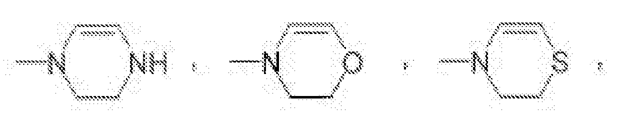 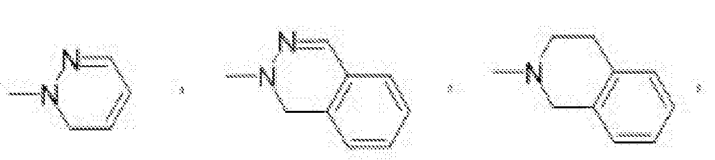 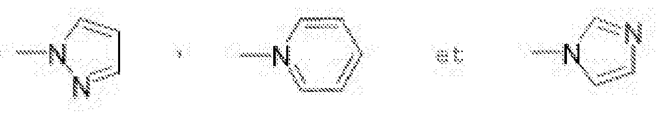 moyennant quoi chacun de ces groupes fonctionnels cycliques mentionnés ci-dessus peut être substitué par 1, 2 ou 3 substituants indépendamment choisis dans le groupe constitué des groupes oxo (=O), thioxo (=S), méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, des atomes de F, Cl, Br et I, et des groupes -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂CH₃, -S(=O)₂-phényle, phényle, phénoxy et benzyle ; moyennant quoi ledit ou lesdits substituants cycliques phényle, phénoxy et benzyle peuvent être non substitués ou substitués par 1, 2 ou 3 substituants indépendamment choisis dans le groupe constitué des groupes méthyle, éthyle, n-propyle, isopropyle, méthoxy, éthoxy, des atomes de F, Cl et Br, et des groupes -CN, -CF₃,-OCF₃, -SCF₃, -OH, -SH, -NH₂ et NO₂ ;
plus préférablement R^{1a} et R^{2a}, conjointement avec l'atome d'azote de pontage, forment un groupe fonctionnel choisi dans le groupe constitué des groupes

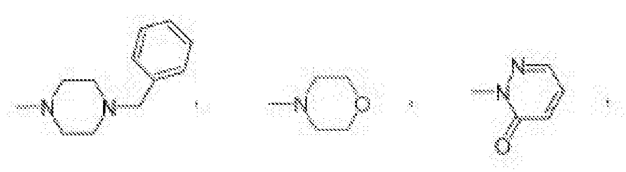

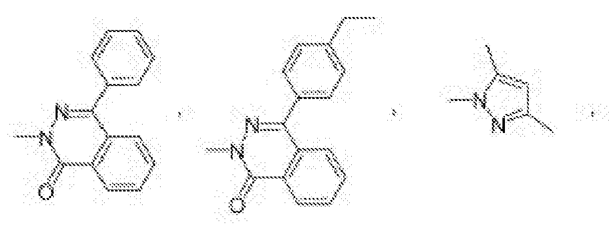

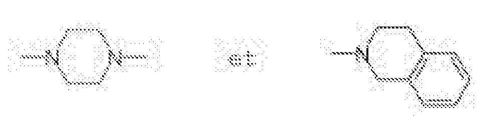

21. Médicament selon une ou plusieurs des revendications 17 à 20, **caractérisé en ce que**
R^{3a} représente un radical aryle ou hétéroaryle choisi dans le groupe constitué des groupes phényle, naphtyle, furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle et pyridinyle, moyennant quoi ledit radical aryle ou hétéroaryle peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, des atomes de F, Cl, Br et I, et des groupes -CN, -CF₃, -OCF₃, -SCF₃, -SH, -NH₂, -NH-CH₃, - NH-C₂H₅, -NH-CH₂-CH₂-CH₃, -NH-CH(CH₃)₂, -NH-C(CH₃)₃, -N(CH₃)₂, - N(C₂H₅)₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ et -S(=O)₂-CH₃ ;
de préférence R^{3a} représente un radical phényle qui peut être substitué par 1, 2 ou 3 substituants indépendamment choisis dans le groupe constitué des groupes méthyle, éthyle, n-propyle, isopropyle, n-butyle, tert-butyle, -O-CH₃, -O-C₂H₅, -O-CH₂-CH₂-CH₃, -C(=O)-OH, -C(=O)-O-CH₃, - C(=O)-O-C₂H₅, -C(=O)-CH₃, -C(=O)-C₂H₅, des atomes de F, Cl, Br et I, et des groupes -CN, -CF₃, -OCF₃, -SCF₃, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -CHO, - CF₂H, -CFH₂ et -S(=O)₂-CH³;
plus préférablement R^{3a} représente un radical phényle non substitué.

22. Médicament selon une ou plusieurs des revendications 17 à 21, **caractérisé en ce que**
R^{4a}, R^{5a} et R^{6a}, identiques ou différents, représentent chacun un atome d'hydrogène ou un atome d'halogène choisi dans le groupe constitué des atomes de F, Cl et Br ;
de préférence R^{4a}, R^{5a} et R^{6a}, identiques ou différents, représentent chacun un atome d'hydrogène ou un atome de fluor.

23. Médicament selon une ou plusieurs des revendications 17 à 22, **caractérisé en ce que**
R^{4a} et R^{5a}, conjointement avec les atomes de carbone de pontage, forment un groupe fonctionnel choisi dans le groupe constitué des groupes 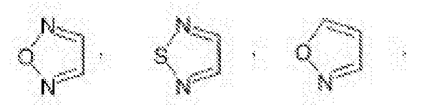 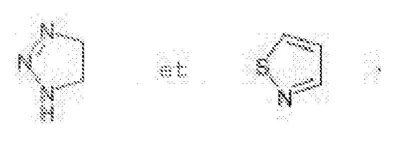 qui, conjointement avec le noyau phényle avec lequel il est fusionné, forme un système de noyaux aromatiques bicycliques substitués choisi dans le groupe constitué des groupes 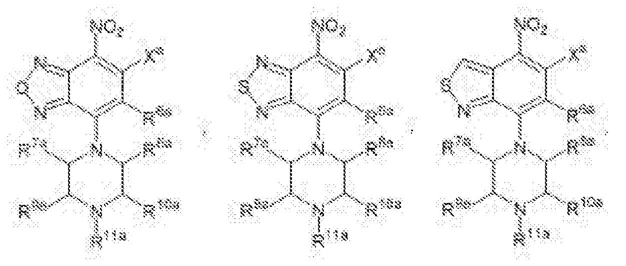 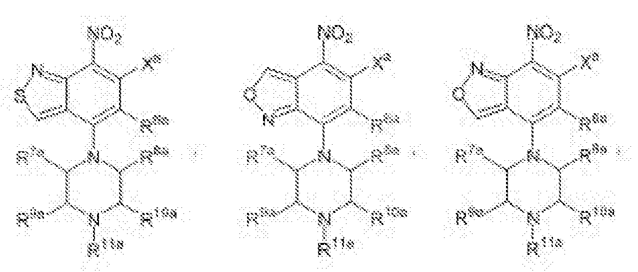 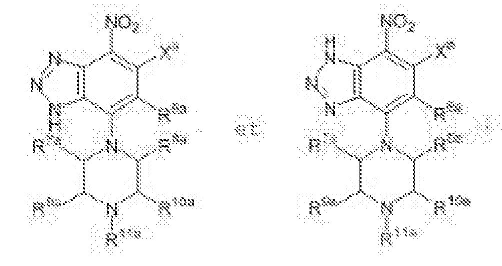 de préférence R^{4a} et R^{5a}, conjointement avec les atomes de carbone de pontage, forment le groupe fonctionnel suivant, 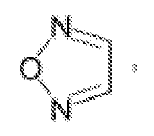 qui, conjointement avec le noyau phényle avec lequel il est fusionné, forme le système de noyaux aromatiques bicycliques substitués suivant

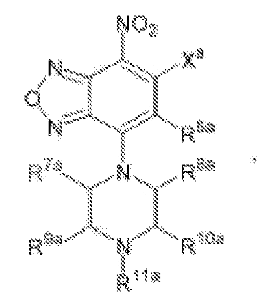

24. Médicament selon une ou plusieurs des revendications 17 à 23, **caractérisé en ce que**
R^{7a} et R^{8a}, identiques ou différents, représentent chacun un atome d'hydrogène ou un radical alkyle non substitué en C₁ à C₁₀, linéaire ou ramifié ;
de préférence R^{7a} et R^{8a}, identiques ou différents, représentent chacun un atome d'hydrogène ou un radical alkyle choisi dans le groupe constitué des groupes méthyle, éthyle, n-propyle, isopropyle, n-butyle, sec-butyle, isobutyle et tert-butyle ;
plus préférablement R^{7a} et R^{8a} représentent chacun un atome d'hydrogène.

25. Médicament selon une ou plusieurs des revendications 17 à 24, **caractérisé en ce que**
R^{9a} et R^{10a}, identiques ou différents, représentent chacun un atome d'hydrogène ou un radical alkyle non substitué en C₁ à C₁₀, linéaire ou ramifié ;
de préférence R^{9a} et R^{10a}, identiques ou différents, représentent chacun un atome d'hydrogène
ou un radical alkyle choisi dans le groupe constitué des groupes méthyle, éthyle, n-propyle, isopropyle, n-butyle, sec-butyle, isobutyle et tert-butyle ;
plus préférablement R^{9a} et R^{10a}, identiques ou différents, représentent chacun un atome d'hydrogène ou un radical méthyle.

26. Médicament selon une ou plusieurs des revendications 17 à 25, **caractérisé en ce que**
R^{11a} représente un atome d'hydrogène ;
un radical alkyle choisi dans le groupe constitué des groupes méthyle, éthyle, n-propyle, isopropyle, n-butyle, sec-butyle, isobutyle et tert-butyle, moyennant quoi ledit radical alkyle peut être substitué par 1, 2 ou 3 substituants indépendamment choisis dans le groupe constitué du groupe - OH, des atomes de F, Cl et Br et du groupe -CN ;
un radical aryle ou hétéroaryle choisi dans le groupe constitué des groupes phényle, naphtyle, furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridinyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle, moyennant quoi ledit radical aryle ou hétéroaryle peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, - C(=O)-OH, -C(=O)-O-alkyle en C₁ à C₅, -O-C(=O)-alkyle en C₁ à C₅, des atomes de F, Cl, Br et I, et des groupes -CN, -CF₃, -OCF₃, -SCF₃, -OH, -SH, -NH₂, -NH(alkyle en C₁ à C₅), -N(alkyle en C₁ à C₅)₂, -NO₂, -CHO, -CF₂H, - CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-Phényle, phényle, phénoxy et benzyle et/ou peut être relié par l'intermédiaire d'un groupe -(CH₂)_{1,2 ou 3} ;
un groupe fonctionnel -C(=O)-R^{13a} ou un groupe fonctionnel -S(=O)₂-R^{14a} ;
de préférence R^{11a} représente un atome d'hydrogène ;
un radical alkyle choisi dans le groupe constitué des groupes méthyle, éthyle, n-propyle, isopropyle, n-butyle, sec-butyle, isobutyle et tert-butyle, moyennant quoi ledit radical alkyle peut être substitué par 1, 2 ou 3 substituants indépendamment choisis dans le groupe constitué du groupe - OH, des atomes de F, Cl et Br, et du groupe -CN ;
un radical aryle ou hétéroaryle choisi dans le groupe constitué des groupes phényle, naphtyle, furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridazinyle, pyrimidinyle, pyridinyle, pyrazinyle, quinolinyle, isoquinolinyle, benzo[b]furanyle, benzo[b]thiophényle, benzothiadiazolyle et imidazo[2,1-b]thiazolyle, moyennant quoi ledit groupe aryle ou hétéroaryle peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, des atomes de F, Cl, Br et I, et des groupes -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ et -S(=O)₂-CH₃ et/ou peut être relié par l'intermédiaire d'un groupe -(CH₂)_{1,2} ou 3 ;
un groupe fonctionnel -C(=O)-R^{13a} ou un groupe fonctionnel -S(=O)₂-R^{14a} ;
plus préférablement R^{11a} représente un atome d'hydrogène ;
un radical alkyle choisi dans le groupe constitué des groupes méthyle, n-butyle et -CH₂-CH₂-OH ;
un radical phényle ou pyridinyle non substitué, moyennant quoi ledit radical phényle ou pyridinyle peut être relié par l'intermédiaire d'un groupe -(CH₂) ;
un groupe fonctionnel -C(=O)-R^{12a} ou un groupe fonctionnel -S(=O)₂-R^{13a}.

27. Médicament selon une ou plusieurs des revendications 17 à 26, **caractérisé en ce que**
R^{12a} représente un radical alkyle choisi dans le groupe constitué des groupes méthyle, éthyle, n-propyle, isopropyle, n-butyle, sec-butyle, isobutyle et tert-butyle ;
ou un radical aryle ou hétéroaryle choisi dans le groupe constitué des groupes phényle, naphtyle, furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridinyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle, moyennant quoi ledit groupe aryle ou hétéroaryle peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, - C(=O)-OH, -C(=O)-O-alkyle en C₁ à C₅, -O-C(=O)-alkyle en C₁ à C₅, des atomes de F, Cl, Br et I, et des groupes -CN, -CF₃, -OCF₃, -SCF₃, -OH, -SH, -NH₂, -NH(alkyle en C₁ à C₅), -N(alkyle en C₁ à C₅)₂, -NO₂, -CHO, -CF₂H, - CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-Phényle, phényle, phénoxy et benzyle et/ou peut être relié par l'intermédiaire d'un groupe -(CH₂)_{1, 2 ou 3};
de préférence R^{12a} représente un radical alkyle choisi dans le groupe constitué des groupes méthyle, éthyle, n-propyle et isopropyle,
ou un radical aryle ou hétéroaryle choisi dans le groupe constitué des groupes phényle, naphtyle, furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle et pyridinyle, moyennant quoi ledit radical aryle ou hétéroaryle peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -OC(CH₃)₃, -S-CH₃, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, des atomes de F, Cl, Br et I, et des groupes -CN, -CF₃, -OCF₃, -SCF₃, -SH, -NH₂, -NH-CH₃, -NH-C₂H₅, - NH-CH₂-CH₂-CH₃, -NH-CH(CH₃)₂, -NH-C(CH₃)₃, -N(CH₃)₂, -N(C₂H₅)₂, -CHO, - CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ et -S(=O)₂-CH₃ et/ou peut être relié par l'intermédiaire d'un groupe -(CH₂) ;
plus préférablement R^{12a} représente un radical phényle ou thiophényle, moyennant quoi ledit radical phényle ou thiophényle peut être substitué par 1, 2 ou 3 substituants choisis dans le groupe constitué du groupe méthyle et de l'atome de chlore.

28. Médicament selon une ou plusieurs des revendications 17 à 27, **caractérisé en ce que**
R^{13a} représente un radical alkyle choisi dans le groupe constitué des groupes méthyle, éthyle, n-propyle, isopropyle, n-butyle, sec-butyle, isobutyle et tert-butyle, moyennant quoi ledit radical alkyle peut être substitué par 1, 2 ou 3 substituants indépendamment choisis dans le groupe constitué du groupe -OH, des atomes de F, Cl et Br et du groupe -CN ;
ou un radical aryle ou hétéroaryle choisi dans le groupe constitué des groupes phényle, naphtyle, furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridazinyle, pyrimidinyle, pyridinyle, pyrazinyle, quinolinyle, isoquinolinyle, benzo[b]furanyle, benzo[b]thiophényle, benzothiadiazolyle et imidazo[2,1-b]thiazolyle, moyennant quoi ledit groupe aryle ou hétéroaryle peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -O-C(CH₃)₃, -S-CH₃, -S-C₂H₅, -S-CH₅-CH₅-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)-C(CH₃)₃, des atomes de F, Cl, Br et I, et des groupes -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -NO₅, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ et -S(=O)₂-CH₃ et/ou peut être relié par l'intermédiaire d'un groupe -(CH₂)_{1, 2 ou 3} ;
de préférence R^{13a} représente un radical alkyle choisi dans le groupe constitué des groupes méthyle, éthyle, n-propyle, isopropyle, n-butyle, sec-butyle, isobutyle et tert-butyle ;
ou un radical aryle ou hétéroaryle choisi dans le groupe constitué des groupes phényle et thiényle (thiophényle), moyennant quoi ledit groupe aryle ou hétéroaryle peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, - C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, des atomes de F, Cl, Br et I, et des groupes -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, - CHO, -CF₂H, -CFH₂, -C(=O)-NH₂, -C(=O)-NH-CH₃, -C(=O)-NH-C₂H₅, - C(=O)-N(CH₃)₂, -C(=O)-N(C₂H₅)₂ et -S(=O)₂-CH₃ et/ou peut être relié par l'intermédiaire d'un groupe -(CH₂)_{1, 2 ou 3} ;
plus préférablement R^{13a} représente un radical méthyle ou un radical phényle ou thiophényle, moyennant quoi ledit radical phényle ou thiophényle peut être substitué par 1, 2 ou 3 substituants choisis dans le groupe constitué du groupe méthyle et de l'atome de chlore.

29. Médicament selon une ou plusieurs des revendications 17 à 28, **caractérisé en ce que**
R^{14a} représente un radical alkyle choisi dans le groupe constitué des groupes méthyle, éthyle, n-propyle, isopropyle, n-butyle, sec-butyle, isobutyle et tert-butyle ;
ou un radical aryle ou hétéroaryle choisi dans le groupe constitué des groupes phényle, naphtyle, furyle (furanyle), thiényle (thiophényle), pyrrolyle, oxazolyle, isoxazolyle, thiazolyle, isothiazolyle, imidazolyle, pyrazolyle, oxadiazolyle, thiadiazolyle, triazolyle, pyridinyle, pyridazinyle, pyrimidinyle, pyrazinyle, quinolinyle, isoquinolinyle, benzo[b]furanyle, benzo[b]thiophényle, benzothiadiazolyle et imidazo[2,1-b]thiazolyle, moyennant quoi ledit groupe aryle ou hétéroaryle peut être substitué par 1, 2, 3, 4 ou 5 substituants indépendamment choisis dans le groupe constitué des groupes alkyle en C₁ à C₅, -O-alkyle en C₁ à C₅, -S-alkyle en C₁ à C₅, - C(=O)-OH, -C(=O)-O-alkyle en C₁ à C₅, -O-C(=O)-alkyle en C₁ à C₅, des atomes de F, Cl, Br et I, et des groupes -CN, -CF₃, -OCF₃, -SCF₃, -OH, -SH, -NH₂, -NH(alkyle en C₁ à C₅), -N(alkyle en C₁ à C₅)₂, -NO₂, -CHO, -CF₂H, - CFH₂, -C(=O)-NH₂, -C(=O)-NH(alkyle en C₁ à C₅), -C(=O)-N(alkyle en C₁ à C₅)₂, -S(=O)₂-alkyle en C₁ à C₅, -S(=O)₂-phényle, phényle, phénoxy et benzyle et/ou peut être relié par l'intermédiaire d'un groupe -(CH₂)_{1, 2 ou 3} ;
de préférence R^{14a} représente un radical alkyle choisi dans le groupe constitué des groupes méthyle, éthyle, n-propyle et isopropyle,
ou un radical phényle, qui peut être substitué par 1, 2 ou 3 substituants indépendamment choisis dans le groupe constitué des groupes méthyle, éthyle, n-propyle, isopropyle, n-butyle, tert-butyle, sec-butyle, isobutyle, n-pentyle, -O-CH₃, -O-C₂H₅, -O-CH₂-CH₂-CH₃, -O-CH(CH₃)₂, -OC(CH₃)₃, -S-CH₃, -S-C₂H₅, -S-CH₂-CH₂-CH₃, -S-CH(CH₃)₂, -S-C(CH₃)₃, - C(=O)-OH, -C(=O)-O-CH₃, -C(=O)-O-C₂H₅, -C(=O)-O-CH₂-CH₂-CH₃, - C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, des atomes de F, Cl, Br et I, et des groupes -CN, -CF₃, -OCF₃, -SCF₃, -SH, -NH₂, -NH-CH₃, -NH-C₂H₅, - NH-CH₂-CH₂-CH₃, -NH-CH(CH₃)₂, -NH-C(CH₃)₃, -N(CH₃)₂, -N(C₂H₅)₂, -CHO, - CF₂H, -CFH₂, -C(=O)-NH₂, -C(=O)-NH-CH₃, -C(=O)-NH-C₂H₅, -C(=O)-N(CH₃)₂, -C(=O)-N(C₂H₂)₂ et -S(=O)₂-CH₃ ;
plus préférablement R^{14a} représente un radical méthyle ou un radical phényle qui peut être substitué par 1, 2 ou 3 radicaux méthyles.

30. Médicament selon une ou plusieurs des revendications 17 à 29, **caractérisé en ce que**
X^{a} représente un groupe fonctionnel -NR^{1a}R^{2a} ou un groupe fonctionnel -OR^{3a} ;
R^{1a} représente un radical alkyle choisi dans le groupe constitué des groupes -CH₂-CH₂-OH et -CH₂-CH₂-CH₂-OH ;
un radical adamantyle non substitué ;
un radical phényle ou pyrrolyle non substitué ;
un radical naphtyle non substitué qui est relié par l'intermédiaire d'un groupe alkylène choisi dans le groupe constitué des groupes -CH₂-, - CH(CH₃)-, -CH₂-CH₂-, -CH₂-CH₂-CH₂- et -CH₂-CH₂-O- ;
un radical phényle qui peut être substitué par 1, 2 ou 3 substituants indépendamment choisis dans le groupe constitué des groupes méthyle, éthyle, tert-butyle et méthoxy, et des atomes de F et Cl et ledit radical phényle est relié par l'intermédiaire d'un groupe alkylène choisi dans le groupe constitué des groupes -CH₂-, -CH(CH₃)-, -CH(phényle)-, -CH₂-CH₂-, -CH₂-CH₂-CH₂- et -CH₂-CH₂-O- ;
un radical hétéroaryle choisi dans le groupe constitué des groupes pyridinyle, furanyle et pyrrolyle, moyennant quoi ledit radical pyridinyle, furanyle ou pyrrolyle peut être substitué par 1, 2 ou 3 substituants indépendamment choisis dans le groupe constitué des groupes méthyle, éthyle, tert-butyle et méthoxy, et des atomes de F et Cl et ledit radical pyridinyle, furanyle ou pyrrolyle est relié par l'intermédiaire d'un groupe alkylène choisi dans le groupe constitué des groupes -CH₂-, -CH(CH₃)-, - CH₂-CH₂-, -CH₂-CH₂-CH₂- et -CH₂-CH₂-O- ;
ou un groupe fonctionnel -C(=O)-R^{12a} ;
R^{2a} représente un atome d'hydrogène ou un radical méthyle ;
ou
R^{1a} et R^{2a}, conjointement avec l'atome d'azote de pontage, forment un groupe fonctionnel choisi dans le groupe constitué des groupes 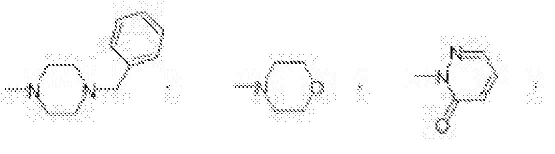 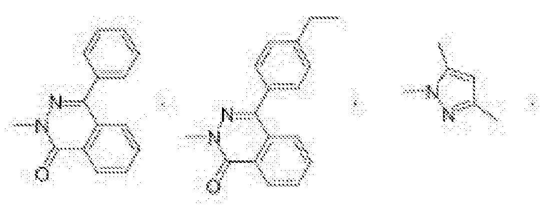 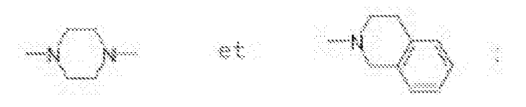 R^{3a} représente un radical phényle non substitué ;
R^{4a}, R^{5a} et R^{6a}, identiques ou différents, représentent chacun un atome d'hydrogène ou un atome de fluor ;
ou
R^{4a} et R^{5a}, conjointement avec les atomes de carbone de pontage, forment le groupe fonctionnel suivant, 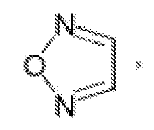 qui, conjointement avec le noyau phényle avec lequel il est fusionné, forme le système de noyaux aromatiques bicycliques substitués suivant 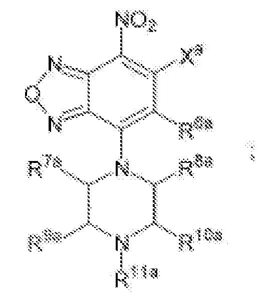 R^{7a} et R^{8a} représentent chacun un atome d'hydrogène ;
R^{9a} et R¹⁰, identiques ou différents, représentent chacun un atome d'hydrogène ou un radical méthyle ;
R^{11a} représente un atome d'hydrogène ;
un radical alkyle choisi dans le groupe constitué des groupes méthyle, n-butyle et -CH₂-CH₂-OH ;
un radical phényle ou pyridinyle non substitué, moyennant quoi ledit radical phényle ou pyridinyle peut être relié par l'intermédiaire d'un groupe -(CH₂) ;
un groupe fonctionnel -C(=O)-R^{12a} ou un groupe fonctionnel -S(=O)₂-R^{13a} ;
R^{12a} représente un radical phényle ou thiophényle, moyennant quoi ledit radical phényle ou thiophényle peut être substitué par 1, 2 ou 3 substituants choisis dans le groupe constitué du groupe méthyle et de l'atome de chlore ;
R^{13a} représente un radical méthyle ou un radical phényle ou thiophényle, moyennant quoi ledit radical phényle ou thiophényle peut être substitué par 1, 2 ou 3 substituants choisis dans le groupe constitué du groupe méthyle et de l'atome de chlore
et
R^{14a} représente un radical méthyle ou un radical phényle qui peut être substitué par 1, 2 ou 3 radicaux méthyles ;
optionnellement sous la forme de l'un de ses stéréoisomères, de préférence des énantiomères ou des diastéréomères, d'un racémate ou sous la forme d'un mélange d'au moins deux de ses stéréoisomères, de préférence des énantiomères et/ou des diastéréomères, dans n'importe quel rapport de mélange, ou d'un sel physiologiquement acceptable de celui-ci, ou d'un solvate correspondant de celui-ci.

31. Médicament selon une ou plusieurs des revendications 17 à 30 comprenant au moins un composé choisi dans le groupe constitué des composés suivants
[1] [5-(4-Méthyl-pipérazin-1-yl)-2-nitro-phényl]-(2-pyrrol-1-yl-éthyl)-amine,
[2] (4-Fluoro-benzyl)-[5-(4-méthyl-pipérazin-1-yl)-2-nitro-phényl]-amine,
[3] N-[5-(4-Méthyl-pipérazin-1-yl)-2-nitro-phényl]-benzamide,
[4] N-Méthyl-N-[5-(4-méthyl-pipérazin-1-yl)-2-nitro-phényl]-benzamide,
[5] [5-(4-Méthyl-pipérazin-1-yl)-2-nitro-phényl]-pyrrol-1-yl-amine,
[6] 2-[5-(4-Méthyl-pipérazin-1-yl)-2-nitro-phényl]-2H-pyridazin-3-one,
[7] 1-Méthyl-4-(4-nitro-3-phénoxy-phényl)-pipérazine,
[8] Benzyl-[5-(4-butyl-pipérazin-1-yl)-2-nitro-phényl]-amine,
[9] Benzyl-[2-nitro-5-(4-pyridin-2-yl-pipérazin-1-yl)-phényl]-amine et
[10] Benzyl-[2-nitro-5-(4-phényl-2-yl-pipérazin-1-yl)-phényl]-amine ;
[11] Furan-2-ylméthyl-[5-(4-méthyl-pipérazin-1-yl)-2-nitro-phényl]amine,
[12] 2-[4-(4-Nitro-3-phénéthylamino-phényl)-pipérazin-1-yl]-éthanol,
[13] (2-Nitro-5-pipérazin-1-yl-phényl)-(2-o-tolyloxy-éthyl)-amine
[14] 2-[4-(3-Benzylamino-4-nitro-phényl)-pipérazin-1-yl]-éthanol,
[15] 4-[5-(4-Méthyl-pipérazin-1-yl)-2-nitro-phényl]-morpholine,
[16] 2-[5-(4-Méthyl-pipérazin-1-yl)-2-nitro-phényl]-4-phényl-2H-phtalazin-1-one,
[17] [2-(4-Chloro-phénoxy)-éthyl]-[5-(3,5-diméthyl-pipérazin-1-yl)-2-nitro-phényl]-amine,
[18] 2-[4-[3-(Benzhydryl-amino)-4-nitro-phényl]-pipérazin-1-yl]-éthanol,
[19] 4-[4-Fluoro-5-(4-méthyl-pipérazin-1-yl)-2-nitro-phényl]-morpholine,
[20] 2-[2-Nitro-5-[4-(toluène-4-sulfonyl)-pipérazin-1-yl]-phényl]-1, 2, 3, 4-tétrahydro-isoquinoline,
[21] 1-[3-(3,5-Diméthyl-pyrazol-1-yl)-4-nitro-phényl]-4-méthyl-pipérazine,
[22] Benzyl-(2-nitro-5-pipérazin-1-yl-phényl)-amine,
[23] [5-(4-Méthyl-pipérazin-1-yl)-2-nitro-phényl]-phénéthyl-amine,
[24] [5-(4-Méthyl-pipérazin-1-yl)-2-nitro-phényl]-pyridin-3-ylméthyl-amine,
[25] (3-Chloro-phényl)-[4-[3-[(furanyl-2-ylméthyl)-amino]-4-nitro-phényl]-pipérazin-1-yl]-méthanone,
[26] (2-Nitro-5-pipérazin-1-yl-phényl)-pyridin-3-ylméthyl-amine,
[27] 1-Benzyl-4-(2-nitro-5-pipérazin-1-yl-phényl)-pipérazine,
[28] Furan-2-ylméthyl-[5-(4-méthanesulfonyl-pipérazin-1-yl)-2-nitro-phényl]-amine,
[29] Benzhydryl-[5-(4-méthyl-pipérazin-1-yl)-2-nitro-phényl]-amine,
[30] (2-Nitro-5-pipérazin-1-yl-phényl)-(2-phénoxy-éthyl)-amine,
[31] 2-[5-(4-Méthyl-pipérazin-1-yl)-2-nitro-phényl]-4-phényl-2H-phtalazin-1-one,
[32] 1-[3-(3,5-Diméthyl-pyrazol-1-yl)-4-nitro-phényl]-pipérazine,
[33] (2-Nitro-5-pipérazin-1-yl-phényl)-phénéthyl-amine,
[34] [5-(4-Benzènesulfonyl-pipérazin-1-yl)-2-nitro-phényl]-furan-2-ylméthyl-amine,
[35] [2-(3,4-Diméthoxy-phényl)-éthyl]-(2-nitro-5-pipérazin-1-yl-phényl)-amine,
[36] [4-[3-[(Furan-2-ylméthyl)-amino]-4-nitro-phényl]-pipérazin-1-yl]-m-tolyl-méthanone,
[37] [4-[3-[(Furan-2-ylméthyl)-amino]-4-nitro-phényl]-pipérazin-1-yl]-phényl-méthanone,
[38] [4-[3-(3,5-Diméthyl-pyrazol-1-yl)-4-nitro-phényl]-pipérazin-1-yl]-thiophén-2-yl-méthanone,
[39] 4-(4-Ethyl-phényl)-2-[5-[4-méthyl-pipérazin-1-yl)-2-nitro-phényl]-2H-phtalalazin-1-one,
[40] 3-[7-(4-Méthyl-pipérazin-1-yl)-4-nitro-benzo[1,2,5]oxadiazol-5-ylamino]propan-1-ol,
[41] 3-[4-Nitro-7-(4-phényl-pipérazin-1-yl)-benzo[1,2,5]oxadiazol-5-ylamino]-éthan-1-ol,
[42] 3-[4-Nitro-7-(4-pyridin-pipérazin-1-yl)-benzo[1,2,5]oxadiazol-5-ylamino]-propan-1-ol,
[43] [2-(3,4-Diméthoxy-phényl)-éthyl]-[5-(4-méthyl-pipérazin-1-yl)-2-nitro-phényl]-amine,
[44] [2-(3,4-Diméthyl-phényl)-éthyl]-[5-(4-méthyl-pipérazin-1-yl)-2-nitro-phényl]-amine,
[45] [2-(4-tert-Butyl-phénoxy)-éthyl]-[5-(4-méthyl-pipérazin-1-yl)-2-nitro-phényl]-amine,
[46] [2-(4-Méthoxy-phénoxy)-éthyl]-[5-(4-méthyl-pipérazin-1-yl)-2-nitro-phényl]-amine,
[47] [5-(4-Méthyl-pipérazin-1-yl)-2-nitro-phényl]-(2-m-tolyloxy-éthyl)-amine,
[48] [2-(4-Chloro-phénoxy)-éthyl]-[5-(4-méthyl-pipérazin-1-yl)-2-nitro-phényl]-amine,
[49] [2-Nitro-5-pipérazin-1-yl-phényl)-(1-phényl-éthyl)-amine,
[50] [2-(3-Méthoxy-phénoxy)-éthyl]-[5-(4-méthyl-pipérazin-1-yl)-2-nitro-phényl]-amine,
[51] [2-(2-Méthoxy-phénoxy)-éthyl]-[5-(4-méthyl-pipérazin-1-yl)-2-nitro-phényl]-amine,
[52] (4-Chloro-benzyl)-(2-nitro-5-pipérazin-1-yl-phényl)-amine,
[53] Benzyl-[5-(4-benzyl-pipérazin-1-yl)-2-nitro-phényl]-amine,
[54] Benzyl-[5-(4-méthyl-pipérazin-1-yl)-2-nitro-phényl]-amine,
[55] [5-(4-Méthyl-pipérazin-1-yl)-2-nitro-phényl]-(2-o-tolyloxy-éthyl)-amine,
[56] (4-Chloro-benzyl)-[5-(4-méthyl-pipérazin-1-yl)-2-nitro-phényl]-amine,
[57] Furan-2-ylméthyl-(2-nitro-5-pipérazin-1-yl-phényl)-amine,
[58] [2-(4-Chloro-phénoxy)-éthyl]-(2-nitro-5-pipérazin-1-yl-phényl)-amine,
[59] [5-(4-Méthyl-pipérazin-1-yl)-2-nitro-phényl]-(1-phényl-éthyl)-amine
[60] 1-[4-(3-Benzylamino-4-nitro-phényl)-pipérazin-1-yl]-éthanone,
[61] 2-[4-[3-(4-Méthylpipérazin-1-yl)-4-nitrophényl]pipérazin-1-yl]éthanol,
[62] 2-[4-[3-[2-(Naphtalén-2-yloxy)éthylamino]-4-nitrophényl]pipérazin-1-yl]éthanol,
[63] 2-[4-(3-{[1-(1-Adamantyl)éthyl)amino}-4-nitrophényl)pipérazin-1-yl]éthanol et
[64] 2-[4-[3-(3,4-Diméthoxyphénéthylamino)-4-nitrophényl]pipérazin-1-yl]éthanol ;
optionnellement sous la forme de l'un de ses stéréoisomères, de préférence des énantiomères ou des diastéréomères, d'un racémate ou sous la forme d'un mélange d'au moins deux de ses stéréoisomères, de préférence des énantiomères et/ou des diastéréomères, dans n'importe quel rapport de mélange, ou d'un sel physiologiquement acceptable de celui-ci, ou d'un solvate correspondant de celui-ci.

32. Médicament selon une ou plusieurs des revendications 17 à 31 pour la prophylaxie et/ou le traitement d'un trouble ou d'une maladie liés à l'absorption de nourriture, de préférence pour la régulation de l'appétit, pour le maintien, l'augmentation ou la réduction de la masse corporelle, pour la prophylaxie et/ou le traitement de l'obésité, de la boulimie, de l'anorexie, de la cachexie, du diabète de type 2, de préférence du diabète non insulinodépendant de type 2 causé par l'obésité ; pour la prophylaxie et/ou le traitement de l'accident vasculaire cérébral ; de la migraine ; du traumatisme crânien ; de l'épilepsie ; du syndrome du côlon irritable ; du syndrome de l'intestin irritable ; des troubles du système nerveux central ; de l'anxiété ; des attaques de panique ; de la dépression ; des troubles bipolaires ; du trouble obsessionnel-compulsif ; des troubles cognitifs ; du dysfonctionnement cognitif associé aux maladies psychiatriques ; des troubles de la mémoire ; de la démence sénile ; des troubles de l'humeur ; des troubles du sommeil ; de la psychose ; des troubles neurodégénératifs, de préférence choisis dans le groupe constitué de la maladie d'Alzheimer, de la maladie de Parkinson, de la chorée de Huntington et de la sclérose en plaques ; de la schizophrénie ; de l'hypoxie intermittente chronique, des convulsions ; ou du trouble de l'hyperactivité (TDAH, trouble déficitaire de l'attention avec hyperactivité) ; pour l'amélioration de la cognition (augmentation cognitive) ou de la mémoire cognitive (augmentation de la mémoire cognitive) ; pour la prophylaxie et/ou le traitement de la dépendance à la drogue et/ou le sevrage ; pour la prophylaxie et/ou le traitement de la dépendance à l'alcool et/ou le sevrage, pour la prophylaxie et/ou le traitement de la dépendance à la nicotine et/ou le sevrage.

33. Utilisation d'au moins un composé de formule générale Ia selon une ou plusieurs des revendications 17 à 31 pour la fabrication d'un médicament pour la prophylaxie et/ou le traitement d'un trouble ou d'une maladie liés à l'absorption de nourriture, de préférence pour la régulation de l'appétit ; pour le maintien, l'augmentation ou la réduction de la masse corporelle ; ou pour la prophylaxie et/ou le traitement de l'obésité, de la boulimie, de l'anorexie, de la cachexie ou du diabète de type 2 (diabète non insulinodépendant), plus préférablement pour la prophylaxie et/ou le traitement de l'obésité.

34. Utilisation d'au moins un composé de formule générale la selon une ou plusieurs des revendications 17 à 31 pour la fabrication d'un médicament pour la prophylaxie et/ou le traitement de l'accident vasculaire cérébral ; de la migraine ; du traumatisme crânien ; de l'épilepsie ; du syndrome du côlon irritable ; du syndrome de l'intestin irritable ; des troubles du système nerveux central ; de l'anxiété ; des attaques de panique ; de la dépression ; des troubles bipolaires ; du trouble obsessionnel-compulsif ; des troubles cognitifs ; du dysfonctionnement cognitif associé aux maladies psychiatriques ; des troubles de la mémoire ; de la démence sénile ; des troubles de l'humeur ; des troubles du sommeil ; de la psychose ; des troubles neurodégénératifs, de préférence choisis dans le groupe constitué de la maladie d'Alzheimer, de la maladie de Parkinson, de la chorée de Huntington et de la sclérose en plaques ; de la schizophrénie ; de l'hypoxie intermittente chronique ; des convulsions ; ou du trouble de l'hyperactivité (TDAH, trouble déficitaire de l'attention avec hyperactivité).

35. Utilisation d'au moins un composé de formule générale Ia selon une ou plusieurs des revendications 17 à 31 pour la fabrication d'un médicament pour l'amélioration de la cognition (augmentation cognitive) et/ou pour l'amélioration de la mémoire cognitive (augmentation de la mémoire cognitive).

36. Utilisation d'au moins un composé de formule générale Ia selon une ou plusieurs des revendications 17 à 31 pour la fabrication d'un médicament pour la prophylaxie et/ou le traitement de la dépendance à la drogue et/ou le sevrage, de préférence pour la prophylaxie et/ou le traitement de la dépendance et/ou le sevrage liés à une ou plusieurs des drogues choisies dans le groupe constitué des benzodiazépines, des opioïdes naturels, semi-synthétiques ou synthétiques tels que la cocaïne, l'éthanol et/ou la nicotine.
